# EUROPEAN PATENT APPLICATION

(11) **EP 4 830 624 A1**
(43) Date of publication of application: **28.10.2026**
(21) Application number: 25826302.9
(22) Date of filing: 27.06.2025
(51) Int. Cl.: A61B 5/145, A61B 5/1473

(54) **PHYSIOLOGICAL SIGN DETECTION APPARATUS**

(30) Priority: 29.06.2024 CN 202410869608; 30.09.2024 CN 202411403418
(71) Applicant: Huawei Technologies Co., Ltd., Shenzhen, Guangdong 518129 (CN)
(72) Inventor: LIN, Nan, Shenzhen, Guangdong 518129 (CN); CHEN, Zhiyuan, Shenzhen, Guangdong 518129 (CN); ZOU, Lin, Shenzhen, Guangdong 518129 (CN); XIE, Songlin, Shenzhen, Guangdong 518129 (CN); ZHAO, Menglong, Shenzhen, Guangdong 518129 (CN); ZHANG, Lei, Shenzhen, Guangdong 518129 (CN); YANG, Wenjian, Shenzhen, Guangdong 518129 (CN)
(74) Representative: Gill Jennings & Every LLP
(86) International application number: PCT/CN2025/104621
(87) International publication number: WO 2026/002231

(57) **Abstract**

This application provides a physiological sign monitoring apparatus. The physiological sign monitoring apparatus includes a lower housing, a base, a support, a first elastic member, a sensor assembly, an upper housing, and a stopper. The lower housing includes a top wall and a side wall. The base is mounted on an inner side of the lower housing and is slidably connected to the side wall of the lower housing. The base includes an abutting end. The support is mounted on an inner side of the base and is slidably connected to the base. The first elastic member is connected between the top wall of the lower housing and the support. The sensor assembly is fastened to the support and is located on a side, away from the top wall of the lower housing, of the support. The sensor assembly includes a sensor and a puncture needle. The upper housing is detachably fastened to an end, away from the top wall of the housing, of the side wall of the lower housing. The upper housing covers an opening. When the physiological sign monitoring apparatus is in an unused state, the stopper is configured to prevent the base from moving relative to the lower housing in an axial direction of the physiological sign monitoring apparatus. The physiological sign monitoring apparatus has a function of preventing accidental triggering upon falling, providing better user experience.

## Description

This application claims priorities to Chinese Patent Application No. 202410869608.1, filed with the China National Intellectual Property Administration on June 29, 2024 and entitled "PHYSIOLOGICAL SIGN MONITORING APPARATUS", and to Chinese Patent Application No. 202411403418.7, filed with the China National Intellectual Property Administration on September 30, 2024 and entitled "PHYSIOLOGICAL SIGN MONITORING APPARATUS", both of which are incorporated herein by reference in their entireties.

### TECHNICAL FIELD

This application relates to the field of medical device technologies, and in particular, to a physiological sign monitoring apparatus.

### BACKGROUND

Diabetes is a chronic disease that may cause many complications. In a conventional diabetes monitoring manner, a blood glucose meter is used to prick a finger to take blood and perform single-point measurement on a glucose concentration in venous blood. Some current minimally invasive blood glucose monitoring products may implement continuous monitoring for more than 7 days by implanting a biosensor under skin to be in contact with tissue fluid. The minimally invasive blood glucose monitoring product applies a small sensor apparatus (usually referred to as a patch) to human skin through an implantation apparatus. A sensor of the patch is guided into subcutaneous tissue by a puncture needle, to be in contact with body fluid. The patch transmits analyte data obtained by the sensor to a terminal, for example, a mobile phone or a display instrument. Most existing physiological sign monitoring apparatuses are of a button type, and a patch is ejected by pressing a button of the physiological sign monitoring apparatus. However, a button-type physiological sign monitoring apparatus is prone to accidental triggering. When the physiological sign monitoring apparatus is not aligned with skin, a patch is ejected when a button is accidentally touched. Consequently, the physiological sign monitoring apparatus malfunctions, and a sensor cannot be implanted into subcutaneous tissue, leading to a failure of blood glucose monitoring and poor user experience.

### SUMMARY

This application provides a physiological sign monitoring apparatus.

According to a first aspect, an embodiment of this application provides a physiological sign monitoring apparatus. The physiological sign monitoring apparatus includes a lower housing, a base, a support, a first elastic member, a sensor assembly, an upper housing, and a stopper. The lower housing includes a top wall and a side wall. The side wall of the lower housing is connected around a periphery of the top wall of the lower housing. An opening is formed on a side, away from the top wall of the lower housing, of the side wall of the lower housing. The base is mounted on an inner side of the lower housing and is slidably connected to the side wall of the lower housing. The base includes an abutting end, and the abutting end protrudes from the lower housing through the opening. The support is mounted on an inner side of the base and is slidably connected to the base. The first elastic member is connected between the top wall of the lower housing and the support. The sensor assembly is fastened to the support and is located on a side, away from the top wall of the lower housing, of the support. The sensor assembly includes a sensor and a puncture needle. The upper housing is detachably fastened to an end, away from the top wall of the housing, of the side wall of the lower housing. The upper housing covers the opening. When the physiological sign monitoring apparatus is in an unused state, the stopper is configured to prevent the base from moving relative to the lower housing in an axial direction of the physiological sign monitoring apparatus. When the upper housing is detached from the lower housing, the abutting end abuts against an organism, and the lower housing moves relative to the base in a direction toward the organism, the first elastic member pushes the support toward the organism, the sensor assembly abuts against the organism, and the sensor is implanted into the organism under guidance of the puncture needle.

It can be understood that the lower housing, the base, and the support may constitute a trigger mechanism of the physiological sign monitoring apparatus. A trigger device of the trigger mechanism needs to axially move by a specific stroke to complete triggering, to push the support. When the physiological sign monitoring apparatus is in the unused state, the stopper may be disposed to limit a trend of relative movement between the base and the lower housing in the axial direction, to prevent the base from moving relative to the lower housing in the axial direction of the physiological sign monitoring apparatus, in other words, limit an axial movement stroke of the base in a trigger direction. An axial movement stroke of the device of the trigger mechanism is limited, so that a limited axial stroke is less than a trigger stroke, to prevent accidental triggering. When the physiological sign monitoring apparatus falls with the top facing down, the stopper limits large relative movement between the base and the lower housing in the axial direction, so that the physiological sign monitoring apparatus is not disengaged or triggered upon falling or vibration.

In some possible implementations, the stopper includes a first stop structure and a second stop structure. The first stop structure is fastened to an outer side of the base. The second stop structure is fastened to the upper housing. In the axial direction of the physiological sign monitoring apparatus, the first stop structure and the second stop structure are arranged opposite to each other, and the second stop structure is located on a side, close to the top wall of the lower housing, of the first stop structure.

It can be understood that the second stop structure is located on the side, close to the bottom wall of the lower housing, of the first stop structure. When the physiological sign monitoring apparatus falls or vibrates, the base moves relative to the lower housing in the axial direction of the physiological sign monitoring apparatus. Before the support is triggered, the first stop structure abuts against the second stop structure, so that relative movement between the base and the lower housing stops.

In some possible implementations, both the first stop structure and the second stop structure are threaded, and a thread pitch of the first stop structure is the same as a thread pitch of the second stop structure.

It can be understood that a threaded structure is simple with low preparation difficulty. The first stop structure and the second stop structure are designed to be of a threaded structure, to help reduce production costs and simplify a mechanism.

In some possible implementations, engaging threads are provided on an outer side of the side wall of the lower housing, and the upper housing is detachably fastened to the lower housing through the engaging threads. The thread pitch of the first stop structure is the same as a thread pitch of the engaging threads.

In this way, when the upper housing is detachably fastened to the lower housing through the threads, the first stop structure and the second stop structure can be in good contact and fit.

In some possible implementations, the lower housing further includes a first limiting block. The first limiting block is fastened to an inner side of the side wall of the lower housing and is exposed from the opening. The base further includes a second limiting block, the second limiting block is fastened to an outer side of the abutting end. In the axial direction of the physiological sign monitoring apparatus, the first limiting block and the second limiting block are arranged opposite to each other. The stopper is detachably connected to the base, and abuts between the first limiting block and the second limiting block. When the physiological sign monitoring apparatus switches from the unused state to an in-use state, the upper housing is detached from the lower housing, the stopper is detached from the base, and the abutting end abuts against the organism.

It can be understood that, in the axial direction of the physiological sign monitoring apparatus, the first limiting block and the second limiting block are arranged opposite to each other, and when the physiological sign monitoring apparatus is subject to collision, the stopper may abut between the first limiting block and the second limiting block, and the stopper may limit a trend of relative movement between the base and the lower housing in the axial direction, to prevent the base from moving relative to the lower housing in the axial direction of the physiological sign monitoring apparatus, in other words, limit an axial movement stroke of the base in a trigger direction. The stopper may alternatively be disposed as a separate mechanical part that is detachably connected.

In some possible implementations, the stopper is of a semi-ring structure, and a central angle of the semi-ring structure is greater than 180°.

It can be understood that the stopper is of the semi-ring structure, so that when using the physiological sign monitoring apparatus, a user may remove the stopper for unlocking after removing a housing cap assembly, and the trigger mechanism can properly operate. In addition, the central angle of the semi-ring structure is greater than 180°, so that the stopper can snap into the base through a structure of the stopper, without a need to additionally dispose a mechanical part for fastening.

In some possible implementations, a surface, away from the base, of the stopper is provided with an anti-skid pattern. This provides convenience for the user to take the stopper.

In some possible implementations, the abutting end is provided with a limiting groove, and an opening of the limiting groove is located on an outer side surface of the abutting end.

The upper housing includes a top wall and a side wall. The side wall of the upper housing is connected around a periphery of the top wall of the upper housing. The stopper is fastened to an inner side of the side wall of the upper housing. When the upper housing is mounted to the lower housing, the stopper is partially located in the limiting groove.

It can be understood that, when the upper housing is mounted to the lower housing, the stopper is partially located in the limiting groove. In a direction parallel to the axial direction (a Z axis) of the physiological sign monitoring apparatus, the stopper is arranged opposite to the base. When the physiological sign monitoring apparatus is subject to collision, the stopper may limit a trend of relative movement between the base and the upper housing in the axial direction. In addition, because the lower housing is fastened to the upper housing, the stopper may limit a trend of relative movement between the base and the lower housing in the axial direction, to prevent the base from moving relative to the lower housing in the axial direction of the physiological sign monitoring apparatus, in other words, limit an axial movement stroke of the base in a trigger direction.

**In** some possible implementations, the stopper is threaded, the limiting groove is a threaded groove, and a thread pitch of the limiting groove is the same as a thread pitch of the stopper.

It can be understood that the stopper may well fit with the limiting groove. The stopper may alternatively be designed to be long in an axial direction of the upper housing. A contact area between the stopper and the base may be large, to well prevent the base from moving relative to the lower housing in the axial direction of the physiological sign monitoring apparatus.

**In** some possible implementations, there are a plurality of stoppers, the plurality of stoppers are spaced apart, and the plurality of stoppers are evenly arranged around the axial direction of the physiological sign monitoring apparatus.

It can be understood that, in comparison with a solution in which there is one stopper, a large quantity of stoppers is disposed, to help increase a contact area between the stopper and the base, and well prevent the base from moving relative to the lower housing in the axial direction of the physiological sign monitoring apparatus.

**In** some possible implementations, the base further includes a plurality of seventh resilient arms, the plurality of seventh resilient arms are spaced apart around an axial direction of the base, a tail end of the seventh resilient arm is provided with a hook, and the hook faces the inner side of the base.

When the support moves in a direction toward the organism and the sensor assembly abuts against the organism, the hook of the seventh resilient arm snaps into the support.

It can be understood that the plurality of seventh resilient arms jointly perform position limiting for the support in a horizontal direction (the horizontal direction is perpendicular to the axial direction of the physiological sign monitoring apparatus), to prevent the support from shaking upon interaction with skin of the organism, keep a relative position between the support and the skin fixed, reduce shaking of the support relative to the skin, and reduce wound damage.

In some possible implementations, the support further includes a fastening boss. The fastening boss is protrudingly disposed on an outer side surface of the support. When the physiological sign monitoring apparatus is in the unused state, the hook of the seventh resilient arm and the fastening boss are spaced apart. When the support moves in the direction toward the organism and the sensor assembly abuts against the organism, the hook of the seventh resilient arm snaps into the fastening boss.

It can be understood that the seventh resilient arm and the fastening boss constitute a locking mechanism for preventing a push mechanism from retracting. This can effectively reduce a risk of sliding of the support, reduce an amount of oscillation that occurs after the sensor is implanted, and reduce wound damage caused by the implantation.

In some possible implementations, when the hook of the seventh resilient arm faces away from the abutting end and the sensor assembly abuts against the organism, the hook of the seventh resilient arm snaps into a side, close to the abutting end, of the fastening boss. Alternatively, when the hook of the seventh resilient arm faces the abutting end and the sensor assembly abuts against the organism, the hook of the seventh resilient arm snaps into a side, away from the abutting end, of the fastening boss.

It can be understood that an orientation of the hook of the seventh resilient arm may be changed, and when the orientation of the hook of the seventh resilient arm is changed, a joint surface between the hook of the seventh resilient arm and the fastening boss also correspondingly changes.

In some possible implementations, the base further includes a plurality of elastic latches. The elastic latches are spaced apart around the axial direction of the base. An end, away from the top wall of the lower housing, of the elastic latch is spaced away from the abutting end. The elastic latch protrudes from the inner side of the base.

When the support moves in the direction toward the organism and the sensor assembly abuts against the organism, the elastic latch abuts against a side, close to the top wall of the lower housing, of the support.

It can be understood that the plurality of elastic latches may be configured to perform position limiting for the support in an axial direction, to prevent the support from sliding in the axial direction. The plurality of elastic latches abut against the support around the axial direction of the support, and perform position limiting for the support in the horizontal direction, to prevent the support from shaking upon interaction with skin, keep a relative position between the support and the skin fixed, reduce shaking of the support relative to the skin, and reduce wound damage.

In some possible implementations, the support further includes a flange, and the flange is protrudingly disposed on the outer side surface of the support. When the support moves in the direction toward the organism and the sensor assembly abuts against the organism, the elastic latch abuts against a side, close to the top wall of the lower housing, of the flange.

It can be understood that, after the support is unlocked, in a process in which the support moves down under the action of the first elastic member, the flange of the support may spread open the elastic latch. Then, when the support pushes the sensor assembly into position, the elastic latch rebounds and abuts against the side, close to the top wall of the lower housing, of the flange of the support, to implement position limiting for the support in the axial direction.

In some possible implementations, the sensor includes a switch magnet, and the switch magnet is configured to control energization/de-energization of the sensor.

The physiological sign monitoring apparatus further includes a first magnet. The first magnet is fastened to the support and is located between the support and the sensor assembly. In the axial direction of the physiological sign monitoring apparatus, the first magnet and the switch magnet are arranged opposite to each other. When the physiological sign monitoring apparatus is in the unused state, the first magnet is configured to de-energize the sensor.

It can be understood that the switch magnet and the first magnet are disposed, so that when the physiological sign monitoring apparatus is in the unused state, the first magnet may act on the switch magnet to de-energize the sensor. Through physical energization/de-energization, power consumption of the physiological sign monitoring apparatus during storage is reduced, and product battery life is improved. When the physiological sign monitoring apparatus is in the in-use state, during a period of time after the upper housing is open and before the sensor assembly abuts against the organism, the sensor may be energized or de-energized. This may be specifically set according to a requirement.

In some possible implementations, the physiological sign monitoring apparatus further includes a second magnet. The second magnet is fastened to the upper housing. In the axial direction of the physiological sign monitoring apparatus, the second magnet and the switch magnet are arranged opposite to each other.

When the physiological sign monitoring apparatus is in the unused state, the second magnet is configured to de-energize the sensor.

It can be understood that, in comparison with the solution in which only the first magnet is disposed, both the first magnet and the second magnet are disposed to act on the switch magnet, to de-energize the sensor. The first magnet and the second magnet may apply a larger acting force to the switch magnet. This helps ensure de-energization of the sensor when the physiological sign monitoring apparatus is in the unused state.

In some possible implementations, the sensor assembly may further include a first electrode plate and a second electrode plate. The first electrode plate and the second electrode plate are electrically connected to a positive electrode and a negative electrode respectively. In the axial direction of the physiological sign monitoring apparatus, the switch magnet is located between the first electrode plate and the second electrode plate. The switch magnet is fastened to the first electrode plate. The first electrode plate is deformable. When the physiological sign monitoring apparatus is in the unused state, the switch magnet and the second electrode plate are spaced apart, and the sensor is de-energized. When the physiological sign monitoring apparatus is in the in-use state, the switch magnet is in contact with and electrically connected to the second electrode plate, and the sensor is energized.

It can be understood that, in comparison with a case in which the switch magnet is located on a same side of the first electrode plate and the second electrode plate and the switch magnet needs to be in contact with both the first electrode plate and the second electrode plate to energize the sensor, in this embodiment, the switch magnet needs to be in contact with only an unconnected electrode plate to energize the sensor. This reduces a risk of failing to properly energize the sensor.

In some possible implementations, the first electrode plate includes an ultra-thin steel sheet. It can be understood that the ultra-thin steel sheet is conductive and has high strength.

In some possible implementations, the sensor assembly further includes a needle holder. The puncture needle is fastened to the needle holder. A puncture tip of the puncture protrudes from the needle holder. The physiological sign monitoring apparatus further includes a needle retraction holder. The needle retraction holder snaps into the support, the lower housing, or the base. The needle holder snaps into the needle retraction holder. When the support moves in a direction toward the organism, the support pushes the needle holder toward the organism through the needle retraction holder, and the puncture tip of the puncture needle penetrates into the organism.

It can be understood that the needle holder snaps into the needle retraction holder, so that the needle holder can move synchronously with the needle retraction holder. When the support moves in an implantation direction, the support may push the sensor assembly to also move in the implantation direction, so that a bonding member of the sensor assembly can be attached to the organism. In addition, the support pushes the needle holder toward the organism through the needle retraction holder, so that the puncture needle can puncture into the organism. When the needle retraction holder moves in a direction opposite to implantation direction, the needle retraction holder drives, through the needle holder, the puncture needle to move in the direction opposite to the implantation direction, so that the puncture needle is detached from the organism.

In some possible implementations, the physiological sign monitoring apparatus further includes a second elastic member, and the second elastic member is located between the support and the needle retraction holder, or the second elastic member is located on a side, close to the top wall of the lower housing, of the needle retraction holder. After the sensor punctures into and is implanted into the organism, the second elastic member is configured to enable the needle retraction holder to move away from the organism, the needle retraction holder and the puncture needle move toward a side away from the organism, and the puncture needle is detached from the organism.

In some possible implementations, the second elastic member abuts between the support and the needle retraction holder, and the second elastic member is in a compressed state.

It can be understood that the second elastic member may be of a structure like a compression spring. The second elastic member pushes, from a side, close to the support, of the needle retraction holder, the needle retraction holder to move away from the support, in other words, away from the organism.

In some possible implementations, the second elastic member is located on the side, close to the top wall of the lower housing, of the needle retraction holder, one end of the second elastic member is connected to the needle retraction holder, the other end of the second elastic member is connected to the lower housing, and the second elastic member is in a stretched state.

It can be understood that the second elastic member may be of a structure like a tension spring or a mainspring. The second elastic member pulls, from a side of the needle retraction holder that is away from the support, the needle retraction holder to move away from the support, in other words, away from the organism.

In some possible implementations, the second elastic member is located between the support and the needle retraction holder, and the second elastic member is a spring. Alternatively, the second elastic member is located on the side, close to the top wall of the lower housing, of the needle retraction holder, and the second elastic member is a needle retraction mainspring or a tension spring.

In some possible implementations, the support includes a side plate and a bottom plate. The side plate of the support is in a tubular shape and is fastened to a side, close to the top wall of the lower housing, of the bottom plate of the support. The needle retraction holder is located on an inner side of the side plate of the support. The sensor assembly is located on a side, away from the top wall of the lower housing, of the bottom plate of the support. The base includes a pre-retraction pressure rib and a retraction pressure rib. Both the pre-retraction pressure rib and the retraction pressure rib are protrudingly disposed on an inner side surface of the base. A snap-fit hole is provided on the side plate of the support. The snap-fit hole passes through the side plate of the support. A wall surface of the snap-fit hole includes a first stepped surface and a second stepped surface. A distance between the first stepped surface and the base is less than a distance between the second stepped surface and the base. A distance between the first stepped surface and the bottom plate of the support is less than a distance between the second stepped surface and the bottom plate of the support. The needle retraction holder includes an elastic fastener. When the physiological sign monitoring apparatus is in the unused state, one end of the elastic fastener abuts against the first stepped surface, and a middle part of the elastic fastener protrudes from an outer side of the side plate of the support through the snap-fit hole. In the axial direction of the physiological sign monitoring apparatus, the middle part of the elastic fastener is arranged opposite to the pre-retraction pressure rib and is arranged opposite to the retraction pressure rib. In a process in which the first elastic member pushes the support toward the organism until the sensor assembly abuts against the organism, when the support moves to a first position, the middle part of the elastic fastener abuts against the pre-retraction pressure rib, and one end of the elastic fastener abuts against the second stepped surface; when the support moves to a second position, the middle part of the elastic fastener abuts against the retraction pressure rib, and the elastic fastener is fully located on an inner side of the side plate of the support; when the support moves to a third position, the sensor assembly abuts against the organism; and after the sensor punctures into and is implanted into the organism, the second elastic member is configured to enable the needle retraction holder to move away from the organism, the needle retraction holder and the puncture needle move toward a side away from the organism, and the puncture needle is detached from the organism.

It can be understood that this embodiment shows a design of staged needle retraction. The puncture needle may be pulled for a specific distance through pre-extraction, so that threaded plastic on the needle holder pre-retracts during implantation, to prevent a plastic part from getting in contact with skin and pressing against a wound.

In some possible implementations, the sensor assembly further includes a sealed cartridge. The sealed cartridge is detachably fastened to the needle holder. The sealed cartridge has a hollow cavity. A part of the sensor and the puncture tip of the puncture needle are located in the hollow cavity of the sealed cartridge.

The sealed cartridge includes a plurality of first elastic arms. The first elastic arms are protrudingly disposed on an outer side surface of the sealed cartridge. The upper housing further includes an unlock ratchet wheel. The unlock ratchet wheel fits with the plurality of first elastic arms. In a process in which the upper housing is tightened relative to the lower housing, the first elastic arm is slidably connected to the unlock ratchet wheel. In a process in which the upper housing is detached from the lower housing, the first elastic arm snaps into the unlock ratchet wheel, and pushes the sealed cartridge to detach from the needle holder.

It can be understood that the sealed cartridge may be configured to protect the puncture needle against microbial contamination. The unlock ratchet wheel fits with the first elastic arm. When the upper housing is detached from the lower housing, the sealed cartridge may be pushed to detach from the needle holder, so that the puncture needle and the sensor are exposed. This helps simplify operation steps of the physiological sign monitoring apparatus, and improve user experience.

In some possible implementations, the sealed cartridge further includes a plurality of second elastic arms, the second elastic arms are protrudingly disposed on the outer side surface of the sealed cartridge, and the ratchet wheel fits with the plurality of second elastic arms. The plurality of first elastic arms form a first elastic arm group. The plurality of second elastic arms form a second elastic arm group. The first elastic arm group and the second elastic arm group are disposed in a staggered manner in the axial direction of the physiological sign monitoring apparatus.

It can be understood that different groups of elastic arms have different rotation angles, and a rotation angle a exists between two groups of elastic arms. To be specific, any one of the two groups of elastic arms rotates relative to the other group of elastic arms by the angle a, and the two groups of elastic arms coincide in the axial direction. For example, when the sealed cartridge is mounted to the unlock ratchet wheel, end faces of the first group of elastic arms may be engaged with short edges of inclined ratchet wheel teeth, and end faces of the second group of elastic arms are spaced away from the short edges of the inclined ratchet wheel teeth. In this case, the sealed cartridge needs to rotate relative to the unlock ratchet wheel by an angle a1, so that the end faces of the second group of elastic arms can be engaged with the short edges of the inclined ratchet wheel teeth. a may be equal to C×(360°/n)+a1, where C is a non-zero integer. a1 may be greater than 0° and less than (360°/n). For example, a1=(360°/2n). When n=12, a may be (C×30°+15°), and a1 may be (360°/24)=15°. In this way, regardless of rotating forward or reversely, the second group of elastic arms need to rotate relative to the unlock ratchet wheel by only 15° to be engaged with the short edges of the inclined ratchet wheel teeth.

In some possible implementations, the sensor assembly further includes a needle holder. The puncture needle is fastened to the needle holder. A puncture tip of the puncture protrudes from the needle holder. The sensor assembly further includes a sealed cartridge. The sealed cartridge is detachably fastened to the needle holder. The sealed cartridge has a hollow cavity. A part of the sensor and the puncture tip of the puncture needle are located in the hollow cavity of the sealed cartridge. The upper housing includes a bottom wall, a side wall, a clamping arm, and a snap-fit boss. The side wall of the upper housing is connected around a periphery of the bottom wall of the upper housing. An end, away from the bottom wall of the upper housing, of the side wall of the upper housing is detachably fastened to the lower housing. The snap-fit boss is fastened to a side, facing the top wall of the lower housing, of the bottom wall of the upper housing. The snap-fit boss is provided with a snap-fit slot. An opening of the snap-fit slot faces the top wall of the lower housing. When the physiological sign monitoring apparatus is in the unused state, an end, away from the needle holder, of the sealed cartridge abuts in the snap-fit slot.

A mounting hole is provided on the bottom wall of the upper housing. The mounting hole passes through the bottom wall. One end of the clamping arm is detachably connected to the bottom wall of the upper housing. The other end of the clamping arm extends, through the mounting hole, into a side, facing the top wall of the lower housing, of the bottom wall of the upper housing, and is detachably connected to the sealed cartridge. In a process in which the upper housing is detached from the lower housing, the clamping arm clamps the sealed cartridge, and pushes the sealed cartridge to detach from the needle holder.

It can be understood that, in this embodiment, the clamping arm is an independent detachable mechanical part, and the sealed cartridge is first assembled, to avoid displacement of the sealed cartridge during assembly of the clamping arm and therefore avoid difficulty in subsequent removal of the sealed cartridge.

In some possible implementations, the sealed cartridge has a limiting groove, and an opening of the limiting groove is located on a side wall of the sealed cartridge. An end, away from a top plate of the upper housing, of the clamping arm is provided with a hook. The hook of the clamping arm faces the sealing member. The hook of the clamping arm is at least partially located in the limiting groove. A groove wall of the limiting groove includes a first limiting surface and a second limiting surface. The first limiting surface and the second limiting surface are spaced apart in a circumferential direction of the sealed cartridge. The hook of the clamping arm is located between the first limiting surface and the second limiting surface.

It can be understood that, in this embodiment, in the circumferential direction of the sealed cartridge, the limiting groove on a side surface of the sealed cartridge has two limiting surfaces (the first limiting surface and the second limiting surface). In a process in which the upper housing is detached from the lower housing and the upper housing drives the sealed cartridge to rotate, a contact surface between the clamping arm and the sealed cartridge is large, and there are limiting surfaces on both sides of the hook of the clamping arm, so that the sealed cartridge is not likely to detach from the upper housing. This reduces a risk that the sealed cartridge falls off during removal of the sealed cartridge, causing damage to the puncture needle and the bonding member.

In some possible implementations, a groove surface of the limiting groove further includes a third limiting surface. The third limiting surface is connected to the first limiting surface and the second limiting surface. In a direction parallel to an axial direction of the sealed cartridge, the third limiting surface is arranged opposite to the hook of the clamping arm.

It can be understood that, when the physiological sign monitoring apparatus switches from the unused state to the in-use state, in a process in which the upper housing is detached from the lower housing, the clamping arm may clamp the sealed cartridge through the third limiting surface, and push the sealed cartridge to detach from the needle holder.

In some possible implementations, there are a plurality of limiting grooves, the plurality of limiting grooves are spaced apart in the circumferential direction of the sealed cartridge, there are a plurality of clamping arms, and the plurality of clamping arms and the plurality of limiting grooves are arranged in a one-to-one correspondence.

In this way, a plurality of clamping arms may be disposed to clamp the sealed cartridge from around the sealed cartridge, to reduce a risk that the sealed cartridge falls off during removal of the sealed cartridge.

In some possible implementations, an outer diameter of the snap-fit boss is greater than an outer diameter of the sealed cartridge. In this way, the clamping arm is not likely to collide with the sealed cartridge during mounting, to reduce a risk of damage to the sealed cartridge, and avoid a case that the sealed cartridge cannot be removed together with the upper housing when the upper housing is detached from the lower housing.

According to a second aspect, an embodiment of this application provides a physiological sign monitoring apparatus. The physiological sign monitoring apparatus includes a housing, a support, a first elastic member, a sensor assembly, a button module, and a front sealing cover. The housing includes a middle plate, an upper side wall, and a lower side wall. The middle plate includes a first surface and a second surface that face away from each other. The upper side wall is fastened to the first surface. The lower side wall is fastened to the second surface. Both the upper side wall and the lower side wall are connected around a periphery of the middle plate. The upper side wall and the middle plate enclose first space. The lower side wall and the middle plate enclose second space. The middle plate has a first snap-fit hole. The first snap-fit hole passes through the middle plate and is communication with the first space and the second space. The support includes a body portion, two first snap-fit arms, and at least one second snap-fit arm. The body portion is located on an inner side of the lower side wall and is slidably connected to the lower side wall. Both the first snap-fit arm and the second snap-fit arm are fastened to an end, close to the middle plate, of the body portion. The two first snap-fit arms and the second snap-fit arm are connected around a periphery of the body portion at spacings. An end, away from the body portion, of the first snap-fit arm has a first hook. An end, away from the body portion, of the second snap-fit arm has a second hook. Both the first snap-fit arm and the second snap-fit arm are partially located in the first snap-fit hole. The first hook is located in the first space and snaps into the middle plate. The second hook is located in the first space and snaps into the middle plate. The first elastic member abuts between the middle plate and the support. The sensor assembly is fastened to the body portion and located on a side, away from the middle plate of the housing, of the body portion. The sensor assembly is located in the second space. The sensor assembly includes a sensor and a puncture needle. The button module includes two buttons, two first push blocks, and at least one second push block. Both the first push block and the second push block are located in the first space. The upper side wall has two mounting holes. The mounting hole passes through the upper side wall and is in communication with the first space and the outside. The button is mounted in the mounting hole. One end of the button is exposed to the outside, and the other end of the button abuts against the first push block. Two ends of the second push block respectively abut against the two first push blocks. The first snap-fit arm and the first push block are arranged opposite to each other in a first direction. The second snap-fit arm and the second push block are arranged opposite to each other in a second direction. The first direction and the second direction form an included angle. The front sealing cover is detachably fastened to an end, away from the middle plate, of the lower side wall. When the front sealing cover is detached from the lower side wall, the end, away from the middle plate, of the lower side wall abuts against an organism, both of the two buttons are subject to pressure toward a central axis of the physiological sign monitoring apparatus and push the two first push blocks to the central axis of the physiological sign monitoring apparatus, the two first push blocks push the second push block to the central axis of the physiological sign monitoring apparatus, the first hook and the second hook enter the second space from the first space, the first elastic member pushes the support toward the organism, the sensor assembly abuts against the organism, and the sensor is implanted into the organism under guidance of the puncture needle.

It can be understood that the button module in this embodiment includes the two buttons. When the physiological sign monitoring apparatus falls or is accidentally triggered, a button on one side is pressed against, and only one first push block is pushed to move, without simultaneously driving the two second push blocks to move. Therefore, at most one snap-fit arm is detached, and the other three snap-fit arms can still remain in snap-fit with the housing, to prevent falling or accidental triggering. In this way, in the physiological sign monitoring apparatus in this embodiment, pushing is not accidentally triggered when only one button is pressed, or when the button touches the ground upon falling, or during vibration.

In some possible implementations, the two ends of the second push block are respectively located on sides, close to the central axis of the physiological sign monitoring apparatus, of the two first push blocks. The first snap-fit arm is partially located on a side, close to the central axis of the physiological sign monitoring apparatus, of the first push block. The second snap-fit arm is partially located on a side, close to the central axis of the physiological sign monitoring apparatus, of the second push block.

In some possible implementations, the housing further includes a boss. The boss is fastened to a middle part of the middle plate and is located in the first space. Both the two first push blocks and the at least one second push block are located between the boss and the upper side wall. The button module further includes an elastic member, and the elastic member is located between the boss and the second push block.

It can be understood that the elastic member may be configured to enable the first push block or the second push block to reset after a limitation on the support is released. When a user simultaneously presses the two buttons, the first push block presses against the second push block toward the middle, and the elastic member is pressed against. After the user releases pressure on a first button and a second button, a restoring force of the elastic member may push the second push block to return to an original initial position. In addition, the second push block abuts against the first push block. In a process in which the second push block resets under a force of the elastic member, the first push block may also be pushed back to an initial position. The first push block moves outward to further push the two buttons back to positions at which the buttons are located before being pressed.

In some possible implementations, there are two elastic members, the second push block includes a first abutting end and a second abutting end, the first abutting end abuts between one first push block and one elastic member, and the second abutting end abuts between the other first push block and the other elastic member.

It can be understood that a quantity of elastic members may be twice a quantity of second push blocks, and two abutting ends (the first abutting end and the second abutting end) of the second push block that abut against the first push block each correspond to one elastic member. In this way, the second push block is subject to uniform forces.

In some possible implementations, there are two second push blocks, and the two second push blocks are spaced apart in the second direction and are respectively located on two sides of the central axis of the physiological sign monitoring apparatus. In this way, the button module is subject to symmetrical forces.

In some possible implementations, the sensor assembly further includes a needle holder. The puncture needle is fastened to the needle holder. A puncture tip of the puncture protrudes from the needle holder. The physiological sign monitoring apparatus further includes a needle retraction holder. The needle retraction holder snaps into the housing. The needle holder snaps into the needle retraction holder. When the support moves in a direction toward the organism, the support pushes the needle holder toward the organism through the needle retraction holder, and the puncture tip of the puncture needle penetrates into the organism.

It can be understood that the needle holder snaps into the needle retraction holder, so that the needle holder can move synchronously with the needle retraction holder. When the support moves in an implantation direction, the support may push the sensor assembly to also move in the implantation direction, so that a bonding member of the sensor assembly can be attached to the organism. In addition, the support pushes the needle holder toward the organism through the needle retraction holder, so that the puncture needle can puncture into the organism. When the needle retraction holder moves in a direction opposite to implantation direction, the needle retraction holder drives, through the needle holder, the puncture needle to move in the direction opposite to the implantation direction, so that the puncture needle is detached from the organism.

In some possible implementations, the physiological sign monitoring apparatus further includes a second elastic member, and the second elastic member is located between the support and the needle retraction holder, or the second elastic member is located on a side, close to the middle plate of the housing, of the needle retraction holder. After the sensor punctures into and is implanted into the organism, the second elastic member is configured to enable the needle retraction holder to move away from the organism, the needle retraction holder and the puncture needle move toward a side away from the organism, and the puncture needle is detached from the organism.

It can be understood that the second elastic member may be of a structure like a compression spring. The second elastic member pushes, from a side, close to the support, of the needle retraction holder, the needle retraction holder to move away from the support, in other words, away from the organism. The second elastic member may alternatively be of a structure like a tension spring or a mainspring. The second elastic member pulls, from a side of the needle retraction holder that is away from the support, the needle retraction holder to move away from the support, in other words, away from the organism.

In some possible implementations, the physiological sign monitoring apparatus further includes a helical fastener. The helical fastener includes a limiting rib and a rotation portion. The rotation portion is fastened to the support. The limiting rib is fastened to the rotation portion. The rotation portion is in a tubular shape. An inner side surface of the rotation portion is provided with inclined teeth. An outer side surface of the needle retraction holder is provided with inclined teeth. The inclined teeth of the rotation portion are engaged with the inclined teeth of the needle retraction holder. The needle retraction holder is rotatably connected to an inner side of the rotation portion. The housing further includes a limiting rib. When the physiological sign monitoring apparatus is in an unused state, the limiting rib of the helical fastener abuts against the limiting rib of the housing in a circumferential direction of the rotation portion. When the support moves in the direction toward the organism, the limiting rib of the helical fastener is detached from the limiting rib of the housing, the second elastic member is configured to enable the needle retraction holder to move away from the organism, the needle retraction holder and the puncture needle move toward a side away from the organism, and the puncture needle is detached from the organism.

It can be understood that the support is subject to an acting force of the first elastic member, and the support moves in the direction toward the organism, to drive the sensor assembly to move down. When the bonding member of the sensor assembly is approximately flush with an end face (a trigger position of a needle retraction action) of an abutting end of the housing, a constraint between a first limiting rib and a second limiting rib is released, and the first limiting rib is detached from the second limiting rib. The helical fastener may be driven, by an acting force applied by the needle retraction holder through the inclined teeth, to rotate. A constraint on the needle retraction holder is released, and the needle retraction holder is unlocked. Under the action of an abutting force of the second elastic member, the needle retraction holder may move up together with a guide needle, to complete needle retraction.

In some possible implementations, the physiological sign monitoring apparatus further includes a plurality of lever fasteners. The plurality of lever fasteners are arranged around the central axis of the physiological sign monitoring apparatus. The lever fastener includes a head portion, a middle portion, and a tail portion. The middle portion is connected between the head portion and the tail portion. The head portion abuts against a side, close to the middle plate the housing, of the needle retraction holder. The middle portion is rotatably connected to the support. The tail portion snaps into the housing. When the support moves in the direction toward the organism, the tail portion is detached from the housing, the needle retraction holder and the puncture needle move toward a side away from the organism, the head portion is pushed away from the central axis of the physiological sign monitoring apparatus, and the puncture needle is detached from the organism.

It can be understood that the support is subject to an acting force of the first elastic member, and the support moves in the direction toward the organism, to drive the sensor assembly to move down, so that the lever fastener also moves down correspondingly. When the lever fastener moves to an end, close to first accommodation space, of a limiting rod, the sensor assembly is approximately flush with the end face of the abutting end of the housing. The second elastic member applies an upward pulling force to the needle retraction holder. The needle retraction holder may apply an upward pushing force to the head portion of the lever fastener. The limiting rod releases a limitation on the lever fastener. The second elastic member may drive the needle retraction holder to enable the tail portion of the lever fastener to rotate downward, and the head portion of the lever fastener is detached from the needle retraction holder. Under the action of the second elastic member, the needle retraction holder may move up together with the guide needle, to complete needle retraction.

In some possible implementations, the physiological sign monitoring apparatus further includes a rear sealing cover that is detachably fastened to an end, away from the middle plate, of the upper side wall, and the rear sealing cover covers the end, away from the middle plate, of the upper side wall and closes the first space.

It can be understood that the rear sealing cover, the front sealing cover, and the housing may constitute an enclosure of the physiological sign monitoring apparatus, to protect internal devices of the physiological sign monitoring apparatus.

According to a third aspect, an embodiment of this application provides a physiological sign monitoring apparatus. The physiological sign monitoring apparatus includes a housing, a support, a first elastic member, a sensor assembly, a rear sealing cover, a button module, and a front sealing cover. The housing includes a middle plate, an upper side wall, a lower side wall, and a boss. The middle plate includes a first surface and a second surface that face away from each other. The upper side wall is fastened to the first surface. The lower side wall is fastened to the second surface. Both the upper side wall and the lower side wall are connected around a periphery of the middle plate. The upper side wall and the middle plate enclose first space. The lower side wall and the middle plate enclose second space. The boss is fastened to the middle plate and is located in the first space. The boss and the upper side wall are spaced apart and enclose rotation space. The middle plate has a snap-fit hole. The snap-fit hole passes through the middle plate and is in communication with the rotation space and the second space. The support includes a body portion and a snap-fit arm. The body portion is on an inner side of the lower side wall and is slidably connected to the lower side wall. The snap-fit arm is fastened to an end, close to the middle plate, of the body portion. An end, away from the body portion, of the snap-fit arm has a hook. The snap-fit arm enters the rotation space through the snap-fit hole. The first elastic member abuts between the middle plate and the body portion. The sensor assembly is fastened to the body portion and located on a side, away from the middle plate of the housing, of the body portion. The sensor assembly is located in the second space. The sensor assembly includes a sensor and a puncture needle. The rear sealing cover is detachably fastened to an end, away from the middle plate, of the upper side wall. The rear sealing cover covers the end, away from the middle plate, of the upper side wall. The rear sealing cover is provided with a mounting hole. The mounting hole passes through the rear sealing cover and is in communication with the outside and the first space. The button module includes a button, a first reset spring, and a slide block. The button is fastened in the mounting hole. One end of the button is exposed to the first space, and the other end of the button is exposed to the outside through the mounting hole. The first reset spring abuts between the button and the middle plate. The slide block is located in the rotation space. The slide block includes a ring portion and a snap-fit portion. The ring portion is sleeved on the boss. The snap-fit portion is connected to a side, away from the boss, of the ring portion. The hook of the snap-fit arm snaps into the snap-fit portion of the slide block. The front sealing cover is detachably fastened to an end, away from the middle plate, of the lower side wall. The rear sealing cover covers an end, away from the middle plate, of the lower side wall and closes the second space. When the front sealing cover is detached from the lower side wall, the end, away from the middle plate, of the lower side wall abuts against an organism, the button is subject to pressure toward the first space and pushes the slide block to rotate around a central axis of the physiological sign monitoring apparatus, the hook of the snap-fit arm is detached from the slide block, the first elastic member pushes the support toward the organism, the sensor assembly abuts against the organism, and the sensor is implanted into the organism under guidance of the puncture needle.

It can be understood that, in comparison with a conventional button solution, in this embodiment, a force applied to the button in an axial direction is converted into a rotation force in a horizontal direction, to avoid a case that the support is detached due to collision caused by the force in the axial direction to the physiological sign monitoring apparatus during falling.

In some possible implementations, the button module further includes a push block. The push block is located between the slide block and the button. The push block includes a first surface and a second surface that face away from each other. The button abuts against the first surface. The first reset spring abuts between the second surface and the middle plate. The second surface of the push block is provided with a first inclined surface. A surface on a side, facing the push block, of the slide block is provided with a second inclined surface. The first inclined surface fits with the second inclined surface. When the button is subject to pressure toward the first space and the button moves relative to the housing in a direction toward the organism, the button enables the push block to move relative to the housing in the direction toward the organism, and the push block pushes, through fitting between the first inclined surface and the second inclined surface of the slide block, the slide block to rotate around the central axis of the physiological sign monitoring apparatus.

It can be understood that, when a user presses the button, the button may abut against the push block, and the push block abuts against an abutting ramp of the slide block. Through fitting between the first inclined surface of the push block and the second inclined surface of the slide block, the slide block is pushed to axially rotate relative to the housing. During rotation of the slide block, the support may remain stationary relative to the housing. The snap-fit portion of the slide block rotates relative to the snap-fit arm of the support until the snap-fit portion and the snap-fit arm are staggered. A constraint on the snap-fit arm is released, and the snap-fit arm exits the first space from a first snap-fit hole, to release a limitation on the support. Under the action of the first elastic member, the support moves down.

In some possible implementations, the push block further includes a limiting portion. The limiting portion is protrudingly disposed on the second surface. The boss is provided with a limiting groove. An opening of the limiting groove is located on a surface on a side, facing the button, of the boss. The limiting portion is configured to fit with the limiting groove to prevent the push block from moving relative to the housing around the central axis of the physiological sign monitoring apparatus.

It can be understood that the limiting portion may be configured to snap into the boss of the housing, to limit rotation of a bump, so that the push block can move in the axial direction. A second ramp is configured to fit with an inclined surface of the ramp of the slide block to push the slide block to rotate.

In some possible implementations, there are a plurality of snap-fit arms, the plurality of snap-fit arms are connected around a periphery of the body portion at spacings, there are a plurality of snap-fit portions, the plurality of snap-fit portions are arranged around the ring portion, and the plurality of snap-fit arms and the plurality of snap-fit portions are arranged in a one-to-one correspondence.

It can be understood that, in comparison with a case in which there is one snap-fit arm, the plurality of snap-fit arms need to be simultaneously unbound from the snap-fit portion to deploy the support. Therefore, the plurality of snap-fit arms help reduce a risk of accidental triggering of the support.

In some possible implementations, the button module further includes a second reset spring, and the housing further includes a limiting block. The limiting block and the snap-fit portion are spaced apart around the central axis of the physiological sign monitoring apparatus. The second reset spring abuts between the limiting block and the snap-fit portion.

In a process in which the button is subject to pressure toward the first space and pushes the slide block to rotate around the central axis of the physiological sign monitoring apparatus, the snap-fit portion moves close to the limiting block, to compress the second reset spring.

It can be understood that the second reset spring is disposed, to avoid a case that the slide block rotates in the housing due to impact of an external force when the apparatus is not in use, causing accidental triggering of the support.

In some possible implementations, an end face of an end, away from the middle plate, of the button is located in the mounting hole and is recessed toward the first space.

It can be understood that the rear sealing cover protrudes above the end face of the button. During falling, after the apparatus is in contact with the ground, the rear sealing cover is first deformed, and an amount of deformation of the button is much less than a stroke needed for triggering (a distance by which the button needs to move in the axial direction to release a limitation on the support in a normal state), to prevent accidental triggering of the button of the physiological sign monitoring apparatus in this embodiment during falling.

In some possible implementations, the sensor assembly further includes a needle holder. The puncture needle is fastened to the needle holder. A puncture tip of the puncture protrudes from the needle holder. The physiological sign monitoring apparatus further includes a needle retraction holder. The needle retraction holder snaps into the housing. The needle holder snaps into the needle retraction holder. When the support moves in a direction toward the organism, the support pushes the needle holder toward the organism through the needle retraction holder, and the puncture tip of the puncture needle penetrates into the organism.

It can be understood that the needle holder snaps into the needle retraction holder, so that the needle holder can move synchronously with the needle retraction holder. When the support moves in an implantation direction, the support may push the sensor assembly to also move in the implantation direction, so that a bonding member of the sensor assembly can be attached to the organism. In addition, the support pushes the needle holder toward the organism through the needle retraction holder, so that the puncture needle can puncture into the organism. When the needle retraction holder moves in a direction opposite to implantation direction, the needle retraction holder drives, through the needle holder, the puncture needle to move in the direction opposite to the implantation direction, so that the puncture needle is detached from the organism.

In some possible implementations, the physiological sign monitoring apparatus further includes a second elastic member, and the second elastic member is located between the support and the needle retraction holder, or the second elastic member is located on a side, close to the middle plate of the housing, of the needle retraction holder. After the sensor punctures into and is implanted into the organism, the second elastic member is configured to enable the needle retraction holder to move away from the organism, the needle retraction holder and the puncture needle move toward a side away from the organism, and the puncture needle is detached from the organism.

It can be understood that the second elastic member may be of a structure like a compression spring. The second elastic member pushes, from a side, close to the support, of the needle retraction holder, the needle retraction holder to move away from the support, in other words, away from the organism. The second elastic member may alternatively be of a structure like a tension spring or a mainspring. The second elastic member pulls, from a side of the needle retraction holder that is away from the support, the needle retraction holder to move away from the support, in other words, away from the organism.

In some possible implementations, the physiological sign monitoring apparatus further includes a helical fastener. The helical fastener includes a limiting rib and a rotation portion. The rotation portion is fastened to the support. The limiting rib is fastened to the rotation portion. The rotation portion is in a tubular shape. An inner surface of the rotation portion is provided with inclined teeth. An outer side surface of the needle retraction holder is provided with inclined teeth. The inclined teeth of the rotation portion are engaged with the inclined teeth of the needle retraction holder. The needle retraction holder is rotatably connected to an inner side of the rotation portion. The housing further includes a limiting rib. When the physiological sign monitoring apparatus is in an unused state, the limiting rib of the helical fastener abuts against the limiting rib of the housing in a circumferential direction of the rotation portion. When the support moves in the direction toward the organism, the limiting rib of the helical fastener is detached from the limiting rib of the housing, the second elastic member is configured to enable the needle retraction holder to move away from the organism, the needle retraction holder and the puncture needle move toward a side away from the organism, and the puncture needle is detached from the organism.

It can be understood that the support is subject to an acting force of the first elastic member, and the support moves in the direction toward the organism, to drive the sensor assembly to move down. When the bonding member of the sensor assembly is approximately flush with an end face (a trigger position of a needle retraction action) of an abutting end of the housing, a constraint between a first limiting rib and a second limiting rib is released, and the first limiting rib is detached from the second limiting rib. The helical fastener may be driven, by an acting force applied by the needle retraction holder through the inclined teeth, to rotate. A constraint on the needle retraction holder is released, and the needle retraction holder is unlocked. Under the action of an abutting force of the second elastic member, the needle retraction holder may move up together with a guide needle, to complete needle retraction.

In some possible implementations, the physiological sign monitoring apparatus further includes a plurality of lever fasteners. The plurality of lever fasteners are arranged around the central axis of the physiological sign monitoring apparatus. The lever fastener includes a head portion, a middle portion, and a tail portion. The middle portion is connected between the head portion and the tail portion. The head portion abuts against a side, close to the middle plate the housing, of the needle retraction holder. The middle portion is rotatably connected to the support. The tail portion snaps into the housing. When the support moves in the direction toward the organism, the tail portion is detached from the housing, the needle retraction holder and the puncture needle move toward a side away from the organism, the head portion is pushed away from the central axis of the physiological sign monitoring apparatus, and the puncture needle is detached from the organism.

It can be understood that the support is subject to an acting force of the first elastic member, and the support moves in the direction toward the organism, to drive the sensor assembly to move down, so that the lever fastener also moves down correspondingly. When the lever fastener moves to an end, close to first accommodation space, of a limiting rod, the sensor assembly is approximately flush with the end face of the abutting end of the housing. The second elastic member applies an upward pulling force to the needle retraction holder. The needle retraction holder may apply an upward pushing force to the head portion of the lever fastener. The limiting rod releases a limitation on the lever fastener. The second elastic member may drive the needle retraction holder to enable the tail portion of the lever fastener to rotate downward, and the head portion of the lever fastener is detached from the needle retraction holder. Under the action of the second elastic member, the needle retraction holder may move up together with the guide needle, to complete needle retraction.

According to a fourth aspect, an embodiment of this application provides a physiological sign monitoring apparatus. The physiological sign monitoring apparatus includes a lower housing, a base, a support, a first elastic member, a sensor assembly, an upper housing, and a concealing mechanism. The lower housing includes a top wall and a side wall. The side wall of the lower housing is connected around a periphery of the top wall of the lower housing. An opening is formed on a side, away from the top wall of the lower housing, of the side wall of the lower housing. The base is mounted on an inner side of the lower housing and is slidably connected to the side wall of the lower housing. The support is mounted on an inner side of the base and is slidably connected to the base. The first elastic member is connected between the top wall of the housing and the support. The sensor assembly is fastened to the support and is located on a side, away from the top wall of the lower housing, of the support. The sensor assembly includes a sensor and a puncture needle. The upper housing is detachably fastened to an end, away from the top wall of the housing, of the side wall of the lower housing. The upper housing covers the opening. The concealing mechanism is configured to conceal the puncture needle. The concealing mechanism is fastened to a side, away from the top wall of the lower housing, of the base, and a part of the concealing mechanism protrudes from the lower housing through the opening. When the upper housing is detached from the lower housing, the concealing mechanism abuts against an organism, and the lower housing moves relative to the base in a direction toward the organism, the concealing structure switches from a closed state to an open state, the puncture needle is exposed, the first elastic member pushes the support toward the organism, the sensor assembly abuts against the organism, and the sensor is implanted into the organism under guidance of the puncture needle.

It can be understood that, after the upper housing is detached from the lower housing, the concealing mechanism switches from the open state to the closed state, and the concealing mechanism conceals the puncture needle. This helps eliminate a fear of a user for the puncture needle when the user uses the physiological sign monitoring apparatus.

In some possible implementations, when the physiological sign monitoring apparatus is in an unused state, the upper housing is mounted to the lower housing, and the concealing mechanism is in the open state.

After the upper housing is detached from the lower housing, the concealing mechanism switches from the open state to the closed state, and the concealing mechanism conceals the puncture needle. This helps eliminate a fear of the user for the puncture needle when the user uses the physiological sign monitoring apparatus.

In some possible implementations, the concealing mechanism includes a plurality of hinges. When the concealing mechanism is in the closed state, the plurality of hinges are located on a side, away from the top wall of the lower housing, of the puncture needle, to conceal the puncture needle.

In some possible implementations, when the concealing mechanism is in the open state, an included angle between a plane on which the hinge is located and an axial direction of the physiological sign monitoring apparatus is less than or equal to 10°.

It can be understood that the hinge does not necessarily need to be completely parallel to the axial direction of the physiological sign monitoring apparatus, and the hinge may alternatively be disposed to a larger angle, so that the hinge can be open wider, to reduce interference to deployment of the support.

In some possible implementations, the concealing mechanism further includes a fastening pillar, a hinge pin, and a torsion spring. A quantity of fastening pillars, a quantity of hinge pins, a quantity of torsion springs, and a quantity of hinges are equal. The fastening pillar is fastened to an end, away from the top wall of the lower housing, of the base. A plurality of fastening pillars are connected around a periphery of the base. The hinge pin is connected to an end, away from the base, of the fastening pillar. The torsion spring is sleeved on the hinge pin. The hinge is rotatably connected to the fastening pillar. The torsion spring abuts against a side, close to the base, of the hinge.

It can be understood that, when the concealing mechanism switches from the open state to the closed state, the torsion spring may be configured to provide a force for the hinge to rotate; and when the concealing mechanism is in the closed state, the torsion spring may be configured to support the hinge to conceal the puncture needle.

In some possible implementations, the hinge includes a rotation portion, a limiting portion, and a concealing portion. The rotation portion is connected between the limiting portion and the concealing portion. The concealing portion is in a plate shape. The rotation portion is rotatably connected to the fastening pillar. The plurality of fastening pillars enclose third space. The concealing portion is located in the third space. The limiting portion is located outside the third space. The lower housing has an abutting portion. The abutting portion is protrudingly disposed on an inner side surface of the side wall of the lower housing. In a direction parallel to the axial direction of the physiological sign monitoring apparatus, the limiting portion is arranged opposite to the abutting portion of the housing, and the limiting portion is located on a side, away from the top wall of the lower housing, of the abutting portion. In a process in which the lower housing moves relative to the base in the direction toward the organism, the abutting portion pushes the limiting portion, an end, away from the concealing portion, of the limiting portion moves toward the organism, the concealing portion moves toward a side away from the organism, the concealing structure switches from the closed state to the open state, and the puncture needle is exposed.

In some possible implementations, there are three hinges, and the three hinges are sequentially arranged around a central axis of the physiological sign monitoring apparatus.

In some possible implementations, the support is provided with a sixth guide structure, and the sixth guide structure is fastened to an end, away from the top wall of the lower housing, of the support and is protrudingly disposed on an outer side of the support. The physiological sign monitoring apparatus further includes a guide pillar. The guide pillar is fastened to the end, away from the top wall of the lower housing, of the base. The guide pillar and the fastening pillar are connected around the periphery of the base. A seventh guide structure is disposed on a side, facing the third space, of the guide pillar. In a process in which the first elastic member pushes the support toward the organism, the sixth guide structure is slidably connected to the second guide structure, to implement directional movement of the support.

It can be understood that in the process in which the first elastic member pushes the support toward the organism, the sixth guide structure is slidably connected to the seventh guide structure, to implement directional movement of the support. In this way, there is no movement clearance between the support and the base. Therefore, when the support moves down, an amount of shaking of the support in a horizontal direction is minimized, to minimize an amount of horizontal shaking of a hard needle during implantation, and minimize subcutaneous damage.

In some possible implementations, the concealing mechanism further includes a first limiting block. The first limiting block is fastened to the fastening pillar and is located on a side, away from the base, of the concealing portion of the hinge. In a direction parallel to an axial direction of the physiological sign monitoring apparatus, the first limiting block is arranged opposite to the concealing portion of the hinge. When the concealing mechanism is in the closed state, the first limiting block abuts against the concealing portion of the hinge.

It can be understood that the first limiting block is disposed, so that when the concealing mechanism is in the closed state, the first limiting block abuts against the concealing portion of the hinge, to prevent the hinge from being subject to an abutting force of the torsion spring, and avoid exposing the puncture needle due to an excessively large opening angle.

In some possible implementations, the concealing mechanism further includes a limiting pillar, a bearing plate, a second limiting block, a first push block reset spring, and a first push block. The bearing plate is fastened to the end, away from the top wall of the lower housing, of the base. Both the limiting pillar and the fastening pillar are fastened to a side, away from the base, of the bearing plate. The fastening pillar and the limiting pillar are spaced apart. There are a plurality of limiting pillars. The plurality of limiting pillars are spaced apart around a central axis of the physiological sign monitoring apparatus. The second limiting block is fastened to the limiting pillar and is spaced away from the bearing plate. The first push block is movably connected to the limiting pillar. The first push block is partially located between the second limiting block and the bearing plate. The first push block reset spring abuts between the first push block and the bearing plate. The first push block includes an abutting end. The abutting end protrudes from the lower housing through the opening. When the physiological sign monitoring apparatus is in the unused state, the upper housing abuts against the first push block, and the first push block abuts against the hinge, to enable the concealing mechanism to be in the open state. In a process in which the upper housing is detached from the lower housing, the first push block reset spring pushes the first push block to move toward a side away from the top wall of the lower housing, the first push block and the hinge are spaced apart, the hinge is pushed by the torsion spring to rotate toward the side away from the top wall of the lower housing, and the concealing mechanism switches from the open state to the closed state to conceal the puncture needle. When the abutting end abuts against the organism and the lower housing moves relative to the base in a direction toward the organism, the first push block reset spring is compressed, the first push block abuts against the hinge, the concealing mechanism switches from the closed state to the open state, and the puncture needle is exposed.

It can be understood that, when using the physiological sign monitoring apparatus, the user removes the upper housing, the first push block reset spring may apply, to the first push block, a force for moving away from the base, the first push block moves in a direction away from the base, two torsion springs respectively apply, to two hinges, forces for moving away from the base, and the hinges are closed to conceal the puncture needle of the sensor assembly. In this case, sight of the user may be blocked by the hinges, and the user cannot see the puncture needle from the outside. This helps eliminate a fear of the user for the puncture needle when the user uses the physiological sign monitoring apparatus.

During assembly of the physiological sign monitoring apparatus, after the sensor assembly is mounted to the support, the concealing structure is mounted to the base, and then the upper housing is mounted. The upper housing may apply, to the push block of the concealing mechanism, a force for moving toward the base. The first push block moves toward the base, and the first push block 5085 abuts against the hinge, so that one end of the hinge moves toward the bearing plate of the base, the torsion spring and the reset spring are compressed, and the upper housing is fastened to the lower housing through threads. In this case, the hinge of the concealing mechanism remains in an open state.

In some possible implementations, the concealing mechanism further includes a bearing plate, a drive wheel, a second push block, a second push block reset spring, and a drive wheel reset spring. The hinge includes a blade, a first guide pillar, and a second guide pillar. The blade includes a first surface and a second surface that face away from each other. The first guide pillar is fastened to the first surface, and the second guide pillar is fastened to the second surface. The bearing plate is fastened to the end, away from the top wall of the lower housing, of the base. The bearing plate is provided with a first guide hole. The drive wheel is movably connected to the base. The drive wheel is provided with a second guide hole. The first guide pillar is slidably connected to the first guide hole. The second guide pillar is slidably connected to the second guide hole. The push block reset spring abuts between the push block and the drive wheel. The push block includes an abutting end. The abutting end protrudes from the lower housing through the opening. One end of the drive wheel reset spring is fastened to the base, and the other end of the drive wheel reset spring is fastened to the drive wheel. When the physiological sign monitoring apparatus is in the unused state, the drive wheel reset spring is in a stretched state. In a process in which the upper housing is detached from the lower housing, the push block reset spring pushes the push block to move toward a side away from the bottom wall of the lower housing, the drive wheel reset spring resets to pull the drive wheel to rotate reversely, and the plurality of hinges rotate, to enable the concealing mechanism to be in the closed state. When the abutting end abuts against the organism and the lower housing moves relative to the base in a direction toward the organism, the push block moves relative to the base in a direction toward the bottom wall of the lower housing to push the directional wheel to rotate, the drive wheel reset spring is stretched, and the plurality of hinges rotate, to enable the concealing mechanism to be in the open state and the puncture needle to be exposed.

It can be understood that, when using the apparatus, the user removes the upper housing, and the plurality of hinges rotate, to enable the concealing mechanism to be in the closed state. When the user presses the apparatus against skin, the second push block first pushes the drive wheel to rotate by a specific angle to enable the concealing mechanism to open, without affecting pushing of the sensor assembly. During use, the user needs to first press the apparatus by a specific stroke. Then the concealing mechanism is open, and the user continues to press the apparatus, so that the support is pushed out.

In some possible implementations, the concealing mechanism includes an alloy limiting cap and a plurality of alloy sheets. The alloy is in a curved-surface shape, and the alloy is fastened to an inner side of the base. The alloy limiting cap is fastened to the lower housing. When the physiological sign monitoring apparatus is in an unused state, an end, away from the base, of the alloy sheet is located between the base and the alloy limiting cap. After the upper housing is detached from the lower housing, the alloy limiting cap is detached from the base, and ends, away from the base, of the alloy converge to conceal the puncture needle. The base includes an abutting end. The abutting end protrudes from the lower housing through the opening. When the abutting end abuts against an organism and the lower housing moves relative to the base in a direction toward the organism, the first elastic member pushes the support toward the organism, and the support pushes the alloy sheets open, to enable the concealing mechanism to be in the open state and the puncture needle to be exposed.

It can be understood that, in comparison with the foregoing embodiments of the concealing mechanism, in this embodiment, during implantation of the sensor assembly, the hinge of the concealing mechanism opens while moving, without a need to first press a door plate to enable the hinge to open and then trigger implantation. Therefore, a deployment stroke of pressing is small, and a length of the physiological sign monitoring apparatus in the axial direction can be set to be small. In addition, there are a large quantity of hinges, and when the concealing mechanism is in the closed state, the hinges can better conceal the puncture needle, to ensure that the user cannot see the puncture needle, and eliminate a fear of the user for the needle.

In some possible implementations, the concealing mechanism includes an alloy sheet, the alloy is in a curved-surface shape, and the alloy is at least partially fastened to an inner side of the base. When the physiological sign monitoring apparatus is in an unused state, an end, away from the base, of the alloy sheet is located between the base and the upper housing. After the upper housing is detached from the lower housing, ends, away from the base, of the alloy converge to conceal the puncture needle. The base includes an abutting end. The abutting end protrudes from the lower housing through the opening. When the abutting end abuts against an organism and the lower housing moves relative to the base in a direction toward the organism, the first elastic member pushes the support toward the organism, and the support pushes the alloy sheets open, to enable the concealing mechanism to be in the open state and the puncture needle to be exposed.

It can be understood that, in comparison with the foregoing embodiments of the concealing mechanism, in this embodiment, the concealing mechanism has two hinges, occupying small space. In addition, during implantation of the sensor assembly, the hinges open while moving, without a need to first press a door plate to enable the hinges to open and then trigger implantation. Therefore, a deployment stroke of pressing is small, and a length of the physiological sign monitoring apparatus in the axial direction can be set to be small.

According to a fifth aspect, an embodiment of this application provides a physiological sign monitoring apparatus. The physiological sign monitoring apparatus includes a lower housing, a base, a support, a first elastic member, a sensor assembly, and an upper housing.

The lower housing includes a top wall and a side wall. The side wall of the lower housing is connected around a periphery of the top wall of the lower housing. An opening is formed on a side, away from the top wall of the lower housing, of the side wall of the lower housing.

The base is mounted on an inner side of the lower housing and is slidably connected to the side wall of the lower housing. The base includes a top plate, a side wall, and a rebound arm. The side wall of the base is connected around a periphery of the top plate. The rebound arm is fastened to a side, away from the side wall of the base, of the top plate. The rebound arm abuts against the top wall of the lower housing. The base includes an abutting end. The abutting end protrudes from the lower housing through the opening. The support is mounted on an inner side of the base and is slidably connected to the base. The first elastic member abuts between the base and the support. The sensor assembly is fastened to the support and is located on a side, away from the top wall of the lower housing, of the support. The sensor assembly includes a sensor and a puncture needle. The upper housing is detachably fastened to an end, away from the top wall of the housing, of the side wall of the lower housing. The upper housing covers the abutting end and closes the opening. When the upper housing is detached from the lower housing, the abutting end abuts against an organism, and the lower housing moves relative to the base in a direction toward the organism, the first elastic member pushes the support toward the organism, the sensor assembly abuts against the organism, and the sensor is implanted into the organism under guidance of the puncture needle.

It can be understood that, when the physiological sign monitoring apparatus is started, the first elastic member is not further compressed, a pressing force of the lower housing is decoupled from a pushing force of the first elastic member, and the pressing force may be adjusted by using a size of an elastic arm of the base, to reduce the pressing force. For example, in the foregoing embodiments, a pressing force = a fastener spreading force + a compression force of the first elastic member; and in this embodiment, the pressing force = a fastener spreading force + a force of pressing the rebound arm.

In some possible implementations, the sensor assembly further includes a needle holder. The puncture needle is fastened to the needle holder. A puncture tip of the puncture protrudes from the needle holder. The physiological sign monitoring apparatus further includes a needle retraction holder. The needle retraction holder snaps into the support, the lower housing, or the base. The needle holder snaps into the needle retraction holder. The physiological sign monitoring apparatus further includes a second elastic member, and the second elastic member is located between the support and the needle retraction holder, or the second elastic member is located on a side, close to the top wall of the lower housing, of the needle retraction holder. When the support moves in a direction toward the organism, the support pushes the needle holder toward the organism through the needle retraction holder, and the puncture tip of the puncture needle penetrates into the organism. After the sensor punctures into and is implanted into the organism, the second elastic member is configured to enable the needle retraction holder to move away from the organism, the needle retraction holder and the puncture needle move toward a side away from the organism, and the puncture needle is detached from the organism. The first elastic member is sleeved on an outer side of the second elastic member.

It can be understood that, in comparison with the solution in which the first elastic member and the second elastic member are arranged in the axial direction of the physiological sign monitoring apparatus in the foregoing embodiments, in this embodiment, the first elastic member and the second elastic member are approximately located on one horizontal plane, and an overall position is near the abutting end of the physiological sign monitoring apparatus. During use of the physiological sign monitoring apparatus, the first elastic member and the second elastic member are more stable with slight shaking.

### BRIEF DESCRIPTION OF DRAWINGS

To describe technical solutions in embodiments of this application, the following describes accompanying drawings used in embodiments of this application.
FIG. 1 is a diagram of a physiological sign monitoring apparatus in some application scenarios according to this application;
FIG. 2 is a diagram of a structure of a physiological sign monitoring apparatus shown in FIG. 1 in some embodiments;
FIG. 3 is a schematic exploded view of a partial structure of a physiological sign monitoring apparatus shown in FIG. 2 according to an implementation;
FIG. 4 is a diagram of a structure of a lower housing shown in FIG. 3 from another perspective;
FIG. 5 is a diagram of a structure of a base of a physiological sign monitoring apparatus shown in FIG. 3;
FIG. 6 is a schematic cross-sectional view of a structure of a base shown in FIG. 5 along A-A according to an implementation;
FIG. 7 is a schematic cross-sectional view of a partial structure of a physiological sign monitoring apparatus shown in FIG. 2 that is cut along B-B;
FIG. 8 is a schematic cross-sectional view of a partial structure of a physiological sign monitoring apparatus shown in FIG. 2 that is cut along C-C;
FIG. 9 is a schematic cross-sectional view of a partial structure of a physiological sign monitoring apparatus shown in FIG. 2 that is cut along D-D;
FIG. 10 is a diagram of a structure of a support shown in FIG. 3 according to an implementation;
FIG. 11 is a diagram of a structure of a support shown in FIG. 3 from another perspective;
FIG. 12 is a diagram of a structure of a spring seat shown in FIG. 3 according to an implementation;
FIG. 13 is a diagram of assembly of a spring seat and a support shown in FIG. 3 according to an implementation;
FIG. 14 is a partial cross-sectional view of an embodiment shown in FIG. 13 along E-E according to an implementation;
FIG. 15 is a diagram of a structure of a structure shown in FIG. 2 along a section line F-F according to an implementation;
FIG. 16 is a diagram of a structure of a needle retraction holder shown in FIG. 3 according to an implementation;
FIG. 17 is a diagram of a structure of a needle retraction holder shown in FIG. 16 from another perspective according to an implementation;
FIG. 18 is a diagram of assembly of a needle retraction holder and a support shown in FIG. 3 according to an implementation;
FIG. 19 is a partial cross-sectional view of a physiological sign monitoring apparatus shown in FIG. 2 along a section line B-B according to an implementation;
FIG. 20 is a schematic cross-sectional view of a partial structure of a physiological sign monitoring apparatus shown in FIG. 2 that is cut along B-B;
FIG. 21 is a schematic cross-sectional view of a partial structure of a physiological sign monitoring apparatus shown in FIG. 2 that is cut along C-C;
FIG. 22 is a schematic cross-sectional view of a partial structure of a physiological sign monitoring apparatus shown in FIG. 2 that is cut along B-B;
FIG. 23 is a diagram of a structure of a sensor assembly shown in FIG. 3 according to an implementation;
FIG. 24 is a schematic exploded view of a sensor assembly shown in FIG. 23 according to an implementation;
FIG. 25a is a diagram of a structure of a sensor shown in FIG. 24 according to an implementation;
FIG. 25b is a diagram of assembly of a sensor, a needle holder, and a puncture needle shown in FIG. 24 according to an implementation;
FIG. 26 is a partial cross-sectional view of a sensor assembly shown in FIG. 23 that is cut along G-G according to an implementation;
FIG. 27 is a diagram of assembly of an electrical connection assembly and a battery assembly shown in FIG. 24 according to an implementation;
FIG. 28 is a diagram of assembly of a sensor, a needle holder, a puncture needle, and an electrical connection assembly shown in FIG. 24 according to an implementation;
FIG. 29a is a diagram of a structure of a gasket shown in FIG. 24 according to an implementation;
FIG. 29b is a diagram of a structure of an upper cover shown in FIG. 24 according to an implementation;
FIG. 29c is a diagram of a structure of a lower cover shown in FIG. 24 according to an implementation;
FIG. 29d is a partial cross-sectional view of a sensor assembly shown in FIG. 23 along G-G according to an implementation;
FIG. 30 is a partial cross-sectional view of a physiological sign monitoring apparatus shown in FIG. 2 along F-F according to an implementation;
FIG. 31a is a diagram of a structure of a housing cap assembly shown in FIG. 3 according to an implementation;
FIG. 31b is a schematic exploded view of a housing cap assembly shown in FIG. 30 according to an implementation;
FIG. 32 is a diagram of a structure of an upper housing 81 shown in FIG. 31b according to an implementation;
FIG. 33 is a cross-sectional view of a housing cap assembly shown in FIG. 31a along H-H according to an implementation;
FIG. 34 is a cross-sectional view of a housing cap assembly shown in FIG. 31a along I-I according to an implementation;
FIG. 35 is a partial cross-sectional view of a physiological sign monitoring apparatus shown in FIG. 2 along B-B according to an implementation;
FIG. 36a is a diagram of an upper housing according to an implementation;
FIG. 36b is a diagram of a lower housing according to an implementation;
FIG. 36c is a diagram of a lower housing shown in FIG. 36b from another perspective according to an implementation;
FIG. 37 is a diagram of assembly of an upper housing and a sealed cartridge shown in FIG. 35 according to an implementation;
FIG. 38 is a partial schematic cross-sectional view of a physiological sign monitoring apparatus shown in FIG. 2 along J-J according to an implementation;
FIG. 39 is a partial cross-sectional view of a physiological sign monitoring apparatus shown in FIG. 2 along B-B during use according to an implementation;
FIG. 40 is a partial cross-sectional view of a physiological sign monitoring apparatus shown in FIG. 2 along B-B during use according to an implementation;
FIG. 41 is a partial cross-sectional view of a physiological sign monitoring apparatus shown in FIG. 2 along B-B during use according to an implementation;
FIG. 42 is a partial cross-sectional view of a physiological sign monitoring apparatus shown in FIG. 2 along K-K before use according to an implementation;
FIG. 43 is a partial cross-sectional view of a physiological sign monitoring apparatus shown in FIG. 2 along K-K during use according to an implementation;
FIG. 44a is a partial cross-sectional view of a physiological sign monitoring apparatus shown in FIG. 2 along K-K during use according to an implementation;
FIG. 44b is a partial cross-sectional view of a physiological sign monitoring apparatus shown in FIG. 2 along K-K according to another implementation;
FIG. 45 is a diagram of a physiological sign monitoring apparatus shown in FIG. 2 according to another implementation;
FIG. 46 is a schematic exploded view of a physiological sign monitoring apparatus shown in FIG. 45 according to an implementation;
FIG. 47 is a diagram of a structure of a lower housing shown in FIG. 46 according to an implementation;
FIG. 48 is a diagram of a structure of a base shown in FIG. 46 according to an implementation;
FIG. 49 is a diagram of a structure of a stopper shown in FIG. 46 according to an implementation;
FIG. 50 is a diagram of assembly of a base and a lower housing shown in FIG. 46 according to an implementation;
FIG. 51 is a diagram of assembly of a snap ring, a base, and a lower housing shown in FIG. 46 according to an implementation;
FIG. 52 is a schematic cross-sectional view of a physiological sign monitoring apparatus shown in FIG. 45 along A2-A2 according to an implementation;
FIG. 53 is a diagram of a physiological sign monitoring apparatus shown in FIG. 2 according to another implementation;
FIG. 54 is a schematic exploded view of a physiological sign monitoring apparatus shown in FIG. 53 according to an implementation;
FIG. 55 is a diagram of a structure of an upper housing shown in FIG. 54 according to an implementation;
FIG. 56 is a diagram of a structure of a base shown in FIG. 54 according to an implementation;
FIG. 57 is a schematic cross-sectional view of a physiological sign monitoring apparatus shown in FIG. 53 along A3-A3 according to an implementation;
FIG. 58 is a diagram of a structure of a support shown in FIG. 3 according to another implementation;
FIG. 59 is a diagram of a structure of a base shown in FIG. 3 according to another implementation;
FIG. 60 is a partial cross-sectional view of a physiological sign monitoring apparatus shown in FIG. 2 along F-F according to another implementation;
FIG. 61 is a partial cross-sectional view of a physiological sign monitoring apparatus shown in FIG. 2 along F-F during use according to an implementation;
FIG. 62 is a partial cross-sectional view of a physiological sign monitoring apparatus shown in FIG. 2 along F-F during use according to an implementation;
FIG. 63 is a schematic exploded view of a housing cap assembly shown in FIG. 3 according to another implementation;
FIG. 64a is a diagram of assembly of an unlock ratchet wheel, an upper housing, and a sealed cartridge shown in FIG. 63 according to an implementation;
FIG. 64b is a diagram of a structure of an unlock ratchet wheel shown in FIG. 63 according to an implementation;
FIG. 65a is a diagram of a structure of a sealed cartridge shown in FIG. 64a according to another implementation;
FIG. 65b is a partial cross-sectional view of a structure shown in FIG. 64a along A4-A4 according to an implementation;
FIG. 66a is a diagram of a structure of a sealed cartridge shown in FIG. 64a according to still another implementation;
FIG. 66b is a partial cross-sectional view of a structure shown in FIG. 64a along A4-A4 according to another implementation;
FIG. 67a is a diagram of a structure of a physiological sign monitoring apparatus shown in FIG. 2 according to still another implementation;
FIG. 67b is a diagram of a partial structure of a housing cap assembly shown in FIG. 67a according to an implementation;
FIG. 67c is a diagram of a structure of a housing cap assembly shown in FIG. 67b according to an implementation;
FIG. 67d is a partial cross-sectional view of a housing cap assembly shown in FIG. 67a along B4-B4 according to an implementation;
FIG. 68a is a diagram of a partial structure of an upper housing shown in FIG. 67b according to an implementation;
FIG. 68b is a diagram of a partial structure of an upper housing shown in FIG. 68a from another perspective according to an implementation;
FIG. 68c is a diagram of a structure of a clamping arm assembly shown in FIG. 67c according to an implementation;
FIG. 68d is a diagram of assembly of a clamping arm assembly and an upper housing shown in FIG. 67b according to an implementation;
FIG. 68e is a diagram of a structure of a sealed cartridge provided in embodiments according to still another implementation;
FIG. 69a is a partial cross-sectional view of a physiological sign monitoring apparatus shown in FIG. 67a along C4-C4 according to an implementation;
FIG. 69b is a partial cross-sectional view of a physiological sign monitoring apparatus shown in FIG. 67a along D4-D4 during assembly according to an implementation;
FIG. 69c is a partial cross-sectional view of a physiological sign monitoring apparatus shown in FIG. 67a along D4-D4 during assembly according to an implementation;
FIG. 69d is a partial cross-sectional view of a physiological sign monitoring apparatus shown in FIG. 67a along D4-D4 during assembly according to an implementation;
FIG. 69e is a partial cross-sectional view of a physiological sign monitoring apparatus shown in FIG. 67a along D4-D4 during assembly according to an implementation;
FIG. 70a is a diagram of a structure of a lower housing provided in this application according to another implementation;
FIG. 70b is a diagram of a structure of a lower housing shown in FIG. 70a from another perspective;
FIG. 70c is a diagram of a structure of a lower housing shown in FIG. 70a from still another perspective;
FIG. 71a is a diagram of a structure of an upper housing provided in this application according to another implementation;
FIG. 71b is a diagram of a structure of an upper housing shown in FIG. 71a from another perspective;
FIG. 71c is a diagram of a structure of an upper housing shown in FIG. 71a from still another perspective;
FIG. 72 is a schematic exploded view of a physiological sign monitoring apparatus shown in FIG. 2 according to yet another implementation;
FIG. 73 is a diagram of a structure of a needle retraction holder shown in FIG. 72 according to an implementation;
FIG. 74 is a diagram of a structure of a needle retraction holder shown in FIG. 73 from another perspective;
FIG. 75 is a diagram of a structure of a support shown in FIG. 72 according to an implementation;
FIG. 76 is a diagram of assembly of a support and a needle retraction holder shown in FIG. 72 according to an implementation;
FIG. 77 is a schematic cross-sectional view of assembly of a support and a base shown in FIG. 72 according to an implementation;
FIG. 78 is a diagram of a structure of a needle holder and a puncture needle of a sensor assembly shown in FIG. 72 according to an implementation;
FIG. 79 to FIG. 82 are partial cross-sectional views of a physiological sign monitoring apparatus in a process of deploying a sensor assembly according to an embodiment;
FIG. 83 is a diagram of a physiological sign monitoring apparatus shown in FIG. 2 according to yet another implementation;
FIG. 84 is a schematic exploded view of a physiological sign monitoring apparatus shown in FIG. 83 according to an implementation;
FIG. 85 is a diagram of a structure of a lower housing shown in FIG. 84 according to an implementation;
FIG. 86 is a partial cross-sectional view of a structure shown in FIG. 83 along A5-A5 according to an implementation;
FIG. 87 is a diagram of a structure of a needle retraction holder shown in FIG. 84 according to an implementation;
FIG. 88 is a diagram of assembly of a needle retraction holder and a support in FIG. 84 according to an implementation;
FIG. 89 is a partial cross-sectional view of a structure shown in FIG. 83 along A5-A5 according to an implementation;
FIG. 90 is a partial cross-sectional view of a physiological sign monitoring apparatus shown in FIG. 83 along B5-B5 during use according to an implementation;
FIG. 91 is a partial cross-sectional view of a physiological sign monitoring apparatus shown in FIG. 83 along B5-B5 during use according to an implementation;
FIG. 92 is a diagram of assembly of a battery assembly shown in FIG. 24 according to an implementation;
FIG. 93 is a partial cross-sectional view of a physiological sign monitoring apparatus shown in FIG. 2 along B-B according to another implementation;
FIG. 94 is a diagram of a structure of a first electrode plate shown in FIG. 91 from another perspective according to an implementation;
FIG. 95 is a diagram of a structure of a physiological sign monitoring apparatus shown in FIG. 2 according to yet another implementation;
FIG. 96 is a schematic exploded view of a partial structure of a physiological sign monitoring apparatus shown in FIG. 95 according to an implementation;
FIG. 97 is a diagram of a structure of a housing shown in FIG. 96 according to an implementation;
FIG. 98 is a diagram of a structure of a housing shown in FIG. 97 from another perspective;
FIG. 99 is a diagram of a structure of a support shown in FIG. 96 according to an implementation;
FIG. 100 is a diagram of a structure of a support shown in FIG. 99 from another perspective;
FIG. 101 is a diagram of a structure of a helical fastener shown in FIG. 96 according to an implementation;
FIG. 102 is a diagram of a structure of a needle retraction holder shown in FIG. 96 according to an implementation;
FIG. 103 is a diagram of a structure of a needle retraction holder shown in FIG. 102 from another perspective;
FIG. 104 is a diagram of assembly of a needle retraction holder and a helical fastener shown in FIG. 96 according to an implementation;
FIG. 105 is a partial cross-sectional view of a physiological sign monitoring apparatus shown in FIG. 95 along A6-A6 according to an implementation;
FIG. 106 is a partial cross-sectional view of a physiological sign monitoring apparatus shown in FIG. 95 along B6-B6 according to an implementation;
FIG. 107 to FIG. 109 are partial cross-sectional views of a physiological sign monitoring apparatus shown in FIG. 95 along B6-B6 during use according to an implementation;
FIG. 110 is a diagram of a structure of a trigger button module shown in FIG. 96 according to an implementation;
FIG. 111 is a diagram of assembly of a trigger button module, a housing, and a support shown in FIG. 96 according to an implementation;
FIG. 112 is a partial cross-sectional view of a physiological sign monitoring apparatus shown in FIG. 95 along C6-C6 according to an implementation;
FIG. 113 is a diagram of a structure of a sealing pad shown in FIG. 112 according to an implementation;
FIG. 114 is a diagram of a structure of a sealing ring shown in FIG. 112 according to an implementation;
FIG. 115 is a diagram of a structure of a first "O"-shaped sealing ring and a button shown in FIG. 112 according to an implementation;
FIG. 116 is a diagram of an assembly structure of a first "O"-shaped sealing ring and a button shown in FIG. 115 according to an implementation;
FIG. 117 is a diagram of a structure of a physiological sign monitoring apparatus shown in FIG. 2 according to yet another implementation;
FIG. 118 is a schematic exploded view of a partial structure of a physiological sign monitoring apparatus shown in FIG. 117 according to an implementation;
FIG. 119 is a diagram of a structure of a rear sealing cover shown in FIG. 118 according to an implementation;
FIG. 120 is a diagram of a structure of a housing shown in FIG. 118 according to an implementation;
FIG. 121 is a diagram of a structure of a support shown in FIG. 118 according to an implementation;
FIG. 122 is a diagram of a structure of a support shown in FIG. 121 from another perspective according to an implementation;
FIG. 123 is a diagram of a structure of a lever fastener shown in FIG. 118 according to an implementation;
FIG. 124 is a partial cross-sectional view of a physiological sign monitoring apparatus shown in FIG. 117 along A7-A7 according to an implementation;
FIG. 125 is a partial cross-sectional view of a physiological sign monitoring apparatus shown in FIG. 117 along B7-B7 according to an implementation;
FIG. 126 is a partial cross-sectional view of a physiological sign monitoring apparatus shown in FIG. 117 along B7-B7 according to an implementation;
FIG. 127 is a partial cross-sectional view of a physiological sign monitoring apparatus shown in FIG. 117 along B7-B7 according to an implementation;
FIG. 128 is a partial cross-sectional view of a physiological sign monitoring apparatus shown in FIG. 117 along B7-B7 according to an implementation;
FIG. 129 to FIG. 132 are partial cross-sectional views of a physiological sign monitoring apparatus shown in FIG. 117 along B7-B7 during use according to an implementation;
FIG. 133 is a schematic exploded cross-sectional view of a trigger button module shown in FIG. 118 according to an implementation;
FIG. 134 is a diagram of a slide block shown in FIG. 133 according to an implementation;
FIG. 135 is a diagram of assembly of a slide block and a housing according to an implementation;
FIG. 136 is a diagram of assembly of a second reset spring, a slide block, and a housing according to an implementation;
FIG. 137 is a diagram of assembly of a support, a second reset spring, a slide block, and a housing according to an implementation;
FIG. 138 is a partial cross-sectional view of a physiological sign monitoring apparatus shown in FIG. 117 along C7-C7 according to an implementation;
FIG. 139 is a diagram of a push block shown in FIG. 133 according to an implementation;
FIG. 140 is a diagram of a structure of a sealing pad shown in FIG. 128 according to an implementation;
FIG. 141 is a diagram of a structure of a sealing ring shown in FIG. 128 according to an implementation;
FIG. 142 is a diagram of a structure of an "O"-shaped sealing ring and a button shown in FIG. 128 according to an implementation;
FIG. 143 is a diagram of an assembly structure of an "O"-shaped sealing ring and a button shown in FIG. 141 according to an implementation;
FIG. 144 is a diagram of a structure of a physiological sign monitoring apparatus shown in FIG. 2 according to yet another implementation;
FIG. 145 is a schematic exploded view of a partial structure of a physiological sign monitoring apparatus shown in FIG. 144 according to an implementation;
FIG. 146 is a diagram of a structure of a housing shown in FIG. 145 according to an implementation;
FIG. 147 is a schematic cross-sectional view of a housing shown in FIG. 146 along A8 to A8 according to an implementation;
FIG. 148 is a diagram of a structure of a support shown in FIG. 145 according to an implementation;
FIG. 149 is a diagram of a structure of a support shown in FIG. 148 from another perspective according to an implementation;
FIG. 150 is a diagram of a structure of a needle retraction holder shown in FIG. 148 according to an implementation;
FIG. 151 is a diagram of a structure of a needle retraction holder shown in FIG. 150 from another perspective according to an implementation;
FIG. 152 is a partial schematic cross-sectional view of a physiological sign monitoring apparatus shown in FIG. 144 along B8-B8 according to an implementation;
FIG. 153 is a partial schematic cross-sectional view of a physiological sign monitoring apparatus shown in FIG. 144 along B8-B8 during use according to an implementation;
FIG. 154 is a partial schematic cross-sectional view of a physiological sign monitoring apparatus shown in FIG. 144 along C8-C8 during use according to an implementation;
FIG. 155 is a partial schematic cross-sectional view of a physiological sign monitoring apparatus shown in FIG. 144 along B8-B8 during use according to an implementation;
FIG. 156 is a partial schematic cross-sectional view of a physiological sign monitoring apparatus shown in FIG. 144 along B8-B8 during use according to an implementation;
FIG. 157 is a diagram of a structure of a physiological sign monitoring apparatus shown in FIG. 2 according to yet another implementation;
FIG. 158 is a partial cross-sectional view of a physiological sign monitoring apparatus shown in FIG. 157 along A9-A9 according to an implementation;
FIG. 159 is a partial schematic exploded view of a physiological sign monitoring apparatus shown in FIG. 157 according to an implementation;
FIG. 160 is a diagram of a structure of a physiological sign monitoring apparatus shown in FIG. 157 with a housing cap assembly removed according to an implementation;
FIG. 161 is a schematic exploded view of a partial structure shown in FIG. 159 according to an implementation;
FIG. 162 is a diagram of a structure of a base and a fastening pillar shown in FIG. 161 according to an implementation;
FIG. 163 is a diagram of assembly of a base, a fastening pillar, a hinge pin, and a torsion spring shown in FIG. 161 according to an implementation;
FIG. 164 is a diagram of assembly of a base, a fastening pillar, a hinge, a hinge pin, and a torsion spring shown in FIG. 161 according to an implementation;
FIG. 165 is a partial cross-sectional view of a structure shown in FIG. 164 along B9-B9 according to an implementation;
FIG. 166 is a diagram of a structure of a support shown in FIG. 159 according to an implementation;
FIG. 167 to FIG. 170a are partial schematic cross-sectional views of a physiological sign monitoring apparatus shown in FIG. 157 along C9-C9 during use according to an implementation;
FIG. 170b is a diagram of a structure of a support shown in FIG. 159 according to another implementation;
FIG. 170c is a partial schematic cross-sectional view of a physiological sign monitoring apparatus shown in FIG. 157 along C9-C9 according to another implementation;
FIG. 171 is a diagram of a structure of a physiological sign monitoring apparatus shown in FIG. 2 according to yet another implementation;
FIG. 172 is a partial cross-sectional view of a physiological sign monitoring apparatus shown in FIG. 171 along A10-A10 according to an implementation;
FIG. 173 is a partial schematic exploded view of a physiological sign monitoring apparatus shown in FIG. 171 according to an implementation;
FIG. 174 is a diagram of a structure of a physiological sign monitoring apparatus shown in FIG. 171 with a housing cap assembly removed according to an implementation;
FIG. 175 is a schematic exploded view of a concealing mechanism and a base shown in FIG. 173 according to an implementation;
FIG. 176 is a partial diagram of assembly of a base and a concealing mechanism shown in FIG. 173;
FIG. 177 is a diagram of a partial structure of a physiological sign monitoring apparatus shown in FIG. 174 according to an implementation;
FIG. 178 is a partial schematic cross-sectional view of a physiological sign monitoring apparatus shown in FIG. 174 along B10-B10 according to an implementation;
FIG. 179 is a diagram of a structure of a physiological sign monitoring apparatus shown in FIG. 2 according to yet another implementation;
FIG. 180 is a partial cross-sectional view of a physiological sign monitoring apparatus shown in FIG. 179 along A11-A 11 according to an implementation;
FIG. 181 is a partial schematic exploded view of a physiological sign monitoring apparatus shown in FIG. 179 according to an implementation;
FIG. 182 is a diagram of a structure of a physiological sign monitoring apparatus shown in FIG. 179 with a housing cap assembly removed according to an implementation;
FIG. 183 is a diagram of a lower housing shown in FIG. 180 according to an implementation;
FIG. 184 is a diagram of a partial structure of a concealing mechanism shown in FIG. 181 according to an implementation;
FIG. 185 is a schematic exploded view of a concealing mechanism shown in FIG. 184 according to an implementation;
FIG. 186 is a diagram of a base shown in FIG. 180 according to an implementation;
FIG. 187 is a diagram of a structure of a hinge shown in FIG. 185 according to an implementation;
FIG. 188 is a diagram of a partial structure of a physiological sign monitoring apparatus shown in FIG. 179;
FIG. 189 is a partial cross-sectional view of a structure shown in FIG. 188 along B11-B11 according to an implementation;
FIG. 190 is a diagram of a partial structure of a physiological sign monitoring apparatus shown in FIG. 179;
FIG. 191 is a partial cross-sectional view of a structure shown in FIG. 190 along C11-C11 according to an implementation;
FIG. 192 is a diagram of a partial structure of a physiological sign monitoring apparatus shown in FIG. 179;
FIG. 193 is a partial cross-sectional view of a structure shown in FIG. 192 along D11-D11 according to an implementation;
FIG. 194 is a partial cross-sectional view of a mechanism shown in FIG. 182 along E11-E11 according to an implementation;
FIG. 195 is a diagram of a structure of a physiological sign monitoring apparatus shown in FIG. 2 according to yet another implementation;
FIG. 196 is a partial cross-sectional view of a physiological sign monitoring apparatus shown in FIG. 195 along A12-A12 according to an implementation;
FIG. 197 is a partial schematic exploded view of a physiological sign monitoring apparatus shown in FIG. 195 according to an implementation;
FIG. 198 is a diagram of a structure of a physiological sign monitoring apparatus shown in FIG. 195 with a housing cap assembly removed according to an implementation;
FIG. 199 is a diagram of a partial structure of a physiological sign monitoring apparatus shown in FIG. 196;
FIG. 200 is a diagram of a structure of an alloy sheet shown in FIG. 199 according to an implementation;
FIG. 201 is a diagram of a structure of an alloy limiting cap shown in FIG. 199 according to an implementation;
FIG. 202 is a partial cross-sectional view of a physiological sign monitoring apparatus shown in FIG. 195 along A12-A12 according to an implementation;
FIG. 203 is a diagram of a structure of an upper housing shown in FIG. 197 according to an implementation;
FIG. 204 is a diagram of a structure of an upper housing shown in FIG. 203 from another perspective;
FIG. 205 is a diagram of assembly of a partial structure shown in FIG. 197 according to an implementation;
FIG. 206 is a partial cross-sectional view of a physiological sign monitoring apparatus shown in FIG. 195 along A12-A12 in an in-use state according to an implementation;
FIG. 207 is a diagram of a structure of a physiological sign monitoring apparatus shown in FIG. 2 according to yet another implementation;
FIG. 208 is a partial cross-sectional view of a physiological sign monitoring apparatus shown in FIG. 207 along A13-A13 according to an implementation;
FIG. 209 is a partial schematic exploded view of a physiological sign monitoring apparatus shown in FIG. 207 according to an implementation;
FIG. 210 is a diagram of a structure of a physiological sign monitoring apparatus shown in FIG. 207 with a housing cap assembly removed according to an implementation;
FIG. 211 is a diagram of a structure of a base shown in FIG. 209 according to an implementation;
FIG. 212 is a partial schematic cross-sectional view of a physiological sign monitoring apparatus shown in FIG. 210 along B13-B13 according to an implementation;
FIG. 213 is a diagram of a partial structure of a physiological sign monitoring apparatus shown in FIG. 207 according to an implementation;
FIG. 214 is a partial cross-sectional view of a physiological sign monitoring apparatus shown in FIG. 210 along B13-B13 according to an implementation;
FIG. 215 is a partial cross-sectional view of a physiological sign monitoring apparatus shown in FIG. 207 along A13-A13 in an in-use state according to an implementation;
FIG. 216 is a partial cross-sectional view of a physiological sign monitoring apparatus shown in FIG. 207 along A13-A13 in an in-use state according to an implementation;
FIG. 217 is a diagram of a structure of a physiological sign monitoring apparatus shown in FIG. 2 according to yet another implementation;
FIG. 218 is a partial cross-sectional view of a physiological sign monitoring apparatus shown in FIG. 217 along A14-A14 according to an implementation;
FIG. 219 is a partial schematic exploded view of a physiological sign monitoring apparatus shown in FIG. 217 according to an implementation;
FIG. 220 is a diagram of a structure of a lower housing shown in FIG. 219 according to an implementation;
FIG. 221 is a diagram of a structure of a base shown in FIG. 219 according to an implementation;
FIG. 222 is a diagram of a structure of a base shown in FIG. 221 from another perspective;
FIG. 223 is a diagram of a structure of a base shown in FIG. 221 from still another perspective;
FIG. 224 is a diagram of a structure of a support shown in FIG. 219 according to an implementation;
FIG. 225 is a diagram of a structure of a support shown in FIG. 224 from another perspective;
FIG. 226 is a diagram of a structure of a needle retraction holder shown in FIG. 219 according to an implementation;
FIG. 227 is a diagram of a structure of a needle retraction holder shown in FIG. 226 from another perspective;
FIG. 228 is a diagram of assembly of a needle retraction holder, a support, and a second elastic member shown in FIG. 219 according to an implementation;
FIG. 229 is a partial cross-sectional view of a physiological sign monitoring apparatus shown in FIG. 217 along C14-C14 according to an implementation;
FIG. 230 is a partial cross-sectional view of a physiological sign monitoring apparatus shown in FIG. 217 along B14-B14 according to an implementation;
FIG. 231 is a diagram of assembly of a base, a first elastic member, a needle retraction holder, a support, and a second elastic member shown in FIG. 219 according to an implementation;
FIG. 232 is a partial cross-sectional view of a physiological sign monitoring apparatus shown in FIG. 231 along C14-C14 according to an implementation;
FIG. 233 is a partial cross-sectional view of a physiological sign monitoring apparatus shown in FIG. 231 along D14-D14 according to an implementation;
FIG. 234 is a diagram of assembly of a base, a first elastic member, a needle retraction holder, a support, a second elastic member, and a sensor assembly shown in FIG. 219 according to an implementation;
FIG. 235 is a partial cross-sectional view of a physiological sign monitoring apparatus shown in FIG. 217 along C14-C14 according to an implementation;
FIG. 236 is a partial cross-sectional view of a physiological sign monitoring apparatus shown in FIG. 217 along E14-E14 according to an implementation;
FIG. 237 is a partial cross-sectional view of a physiological sign monitoring apparatus shown in FIG. 217 along B14-B14 according to an implementation;
FIG. 238 is a partial cross-sectional view of a physiological sign monitoring apparatus shown in FIG. 217 along C14-C14 according to an implementation;
FIG. 239 is a partial cross-sectional view of a physiological sign monitoring apparatus shown in FIG. 217 along A14-A14 in an in-use state according to an implementation;
FIG. 240 is a partial cross-sectional view of a physiological sign monitoring apparatus shown in FIG. 217 along C14-C14 in an in-use state according to an implementation;
FIG. 241 is a partial cross-sectional view of a physiological sign monitoring apparatus shown in FIG. 217 along B14-B14 in an in-use state according to an implementation;
FIG. 242 is a partial cross-sectional view of a physiological sign monitoring apparatus shown in FIG. 217 along C14-C14 in an in-use state according to an implementation; and
FIG. 243 is a partial cross-sectional view of a physiological sign monitoring apparatus shown in FIG. 217 along A14-A14 in an in-use state according to an implementation.

### DESCRIPTION OF EMBODIMENTS

The following describes embodiments of this application with reference to the accompanying drawings in embodiments of this application. Embodiments described with reference to the accompanying drawings in this specification are examples, and are intended to explain this application, but shall not be construed as a limitation on this application.

In the descriptions of embodiments of this application, it should be noted that the terms "mount" and "connection" should be understood in a broad sense, unless otherwise clearly specified and limited. For example, the "connection" may be a detachable connection or a non-detachable connection, or may be a direct connection or a connection through an intermediate medium. It should be understood that, in this application, an "electrical connection" may be understood as physical contact and electrical conduction between components; or may be understood as a form in which different components in a line structure are connected through a physical line that can transmit an electrical signal, for example, a printed circuit board (printed circuit board, PCB) copper foil or a conducting wire. Both "connected" and "connection" may indicate a mechanical connection relationship or a physical connection relationship. For example, that A is connected to B or a connection between A and B may indicate that there is a fastening component (for example, a screw, a bolt, or a rivet) between A and B, or A and B are in contact with each other and it is difficult to separate A from B.

In addition, "fastening" in this specification should also be understood in a broad sense. For example, "fastening" may be direct fastening or indirect fastening through an intermediate medium. The term "fastening" indicates that two parts are connected to each other and a relative positional relationship remains unchanged after the two parts are connected to each other. Orientation terms mentioned in embodiments of this application, for example, "upper" and "lower", merely indicate directions with reference to the accompanying drawings. Therefore, the orientation terms are intended for better and more clearly describing and understanding embodiments of this application, but not to indicate or imply that a specified apparatus or element needs to have a specific orientation or be constructed and operated in a specific orientation, and therefore cannot be construed as a limitation on embodiments of this application. "A plurality of" means two or more.

In the descriptions of embodiments of this application, "/" indicates or, unless otherwise specified. For example, A/B may indicate A or B. In this specification, "and/or" describes only an association relationship between associated objects, and indicates that three relationships may exist. For example, A and/or B may indicate the following three cases: Only A exists, both A and B exist, and only B exists. In addition, in the descriptions of embodiments of this application, "a plurality of" means two or more than two.

In embodiments of this application, the terms "first", "second", "third", and "fourth" are merely intended for description, and shall not be understood as an indication or implication of relative importance or an implicit indication of a quantity of indicated technical features. Therefore, a feature limited by "first", "second", "third", or "fourth" may explicitly or implicitly include one or more features.

In addition, limitations on relative positional relationships, for example, being parallel, perpendicular, or aligned, are mentioned in embodiments of this application. All of these limitations are defined based on a current technique level, but are not absolutely strict limitations. A small deviation is allowed, and being approximately parallel, approximately perpendicular, approximately aligned, or the like is allowed. For example, that A is parallel to B means that A is parallel or approximately parallel to B, and an included angle between A and B may range from 0 degrees to 10 degrees. For example, that A is perpendicular to B means that A is perpendicular or approximately perpendicular to B, and an included angle between A and B may range from 80 degrees to 100 degrees.

In embodiments of this application, arrangement around an axial direction of a mechanical part and arrangement around a circumferential direction of the mechanical part indicate one direction.

It can be understood that an axis of a mechanical part is also referred to as a central axis of the mechanical part, and may also be referred to as a central axis of the mechanical part.

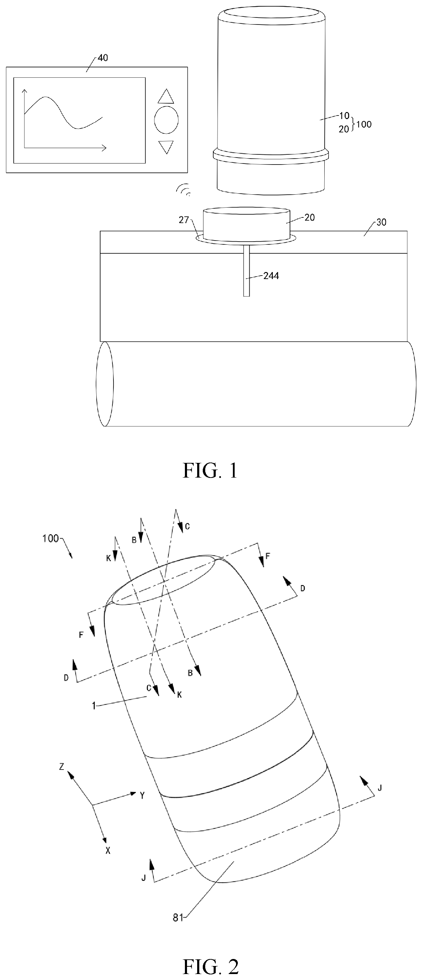

FIG. 1 is a diagram of a physiological sign monitoring apparatus 100 in some application scenarios according to this application. FIG. 2 is a diagram of a structure of the physiological sign monitoring apparatus 100 shown in FIG. 1 in some embodiments.

As shown in FIG. 1 and FIG. 2, the physiological sign monitoring apparatus 100 may include an implantation apparatus 10 and a sensor assembly 20. The sensor assembly 20 is detachably mounted on an inner side of the implantation apparatus 10. The implantation apparatus 10 is configured to push the sensor assembly 20 to a target monitoring position on an organism 30 (for example, skin of a belly or a fat-rich part of an arm of the organism 30). The sensor assembly 20 may include a sensor 24244 and a bonding member 2827. After the implantation apparatus 10 pushes the sensor assembly 20 out, the sensor assembly 20 is fastened to the organism 30 through the bonding member 2827 at the bottom of the sensor assembly 20. A part of the sensor 24244 of the sensor assembly 20 extends from the sensor assembly 20 and is implanted into subcutaneous tissue of skin of the organism 30, to collect and transmit blood information, so that the sensor 24244 can monitor and quantify one or more physiological target substances such as blood glucose, lactic acid, uric acid, dissolved oxygen, hydrogen peroxide, and ions. The sensor assembly 20 may transmit information to a terminal device 40 in a short-range transmission mode, for example, through Bluetooth or a near field communication (Near Field Communication, NFC) technology. The terminal device 40 may be an electronic device with a display, for example, a display instrument, a mobile phone, or an electronic watch. In another implementation, the terminal device may alternatively have no display. After the sensor 24244 is implanted into the organism 30, setting may be performed by using an application (Application, APP) on the terminal device 40, to start monitoring on the physiological target substance, for example, blood glucose. The sensor assembly 20 transmits collected physiological target substance data to the terminal device 40. The terminal device 40 is configured to observe a concentration or other information of the physiological target substance, to monitor and manage a physical and chemical indicator like blood glucose. The sensor assembly 20 is a consumable. For example, one sensor assembly 20 may be continuously used for 14 days or more. In another implementation, the sensor assembly 20 may alternatively be permanent.

The physiological sign monitoring apparatus 100 is an integral apparatus. The sensor assembly 20 is pre-mounted on the inner side of the implantation apparatus 10 before packaging. A user does not need to assemble the implantation apparatus 10 and the sensor assembly 20 during use. The user only needs to open a package and attach the physiological sign monitoring apparatus 100 to the target monitoring position on the organism 30, and the implantation apparatus 10 of the physiological sign monitoring apparatus 100 may push the sensor assembly 20 to the target monitoring position. The integral physiological sign monitoring apparatus 100 helps simplify steps of user operations, to avoid a risk of biological contamination during assembly by the user while facilitating the user operations. In addition, the physiological sign monitoring apparatus 100 needs only one package, and the sensor assembly 20 and the implantation apparatus 10 do not need to be separately packaged. This helps reduce product costs and reduce packages and waste.

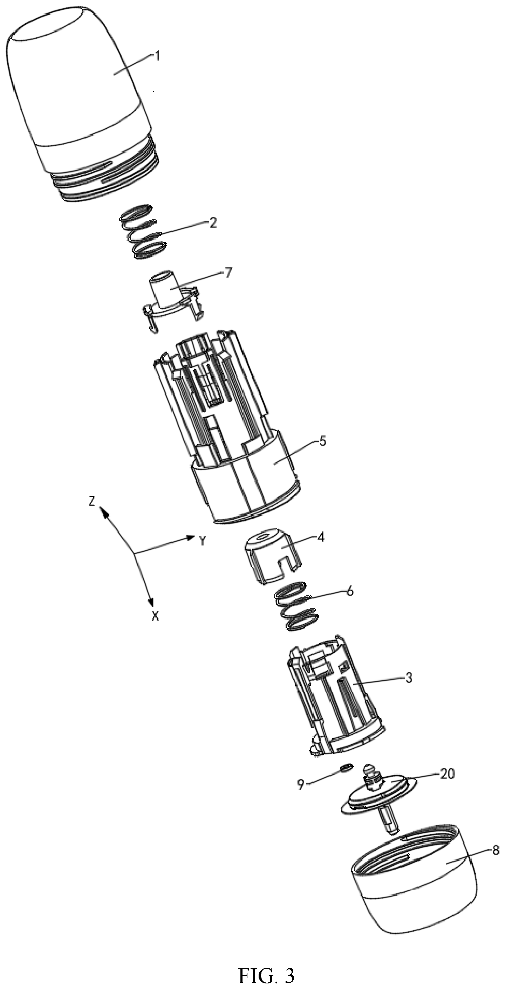

FIG. 3 is a schematic exploded view of a partial structure of the physiological sign monitoring apparatus 100 shown in FIG. 2 according to an implementation.

As shown in FIG. 2 and FIG. 3, the implantation apparatus 10 of the physiological sign monitoring apparatus 100 may include a lower housing 1, a first elastic member 2, a spring seat 7, a base 5, a needle retraction holder 4, a second elastic member 6, a support 3, a first magnet 9, and a housing cap assembly 8. In some other embodiments, the physiological sign monitoring apparatus 100 may include more or fewer components, or some components may be combined or split.

For ease of understanding, an axial direction of the physiological sign monitoring apparatus 100 is a Z-axis direction, a length direction of the physiological sign monitoring apparatus 100 is an X direction, and a width direction of the physiological sign monitoring apparatus 100 is a Y direction.

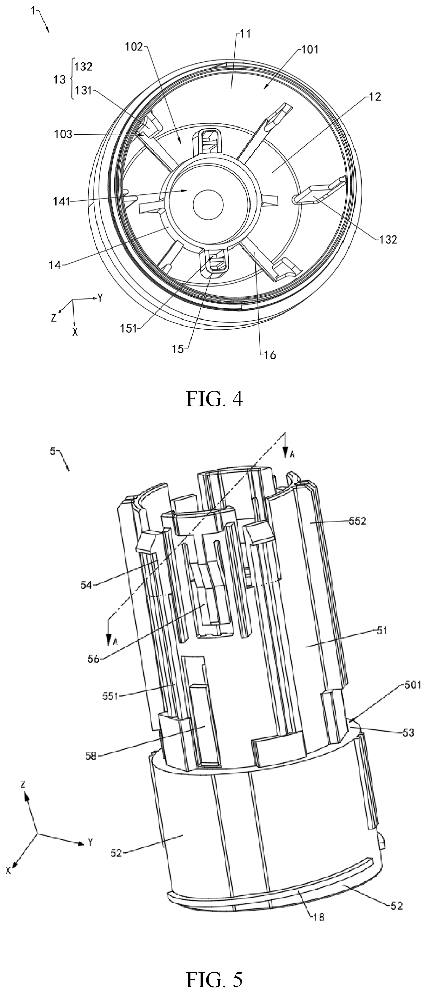

FIG. 4 is a diagram of a structure of the lower housing 1 shown in FIG. 3 from another perspective.

As shown in FIG. 4, the lower housing 1 may include a side wall 11 and a top wall 12, and the side wall 11 of the lower housing 1 is connected around a periphery of the top wall 12 of the lower housing 1 . An opening 101 is formed on a side, away from the top wall 12 of the lower housing 1, of the side wall 11 of the lower housing 1. The lower housing 1 has an internal cavity 102, and the internal cavity 102 is in communication with the opening 101. The lower housing 1 may be approximately in a cylindrical shape. The lower housing 1 has an axial direction. The side wall 11 of the lower housing 1 is arranged around the axial direction of the lower housing 1. The top wall 12 of the lower housing 1 intersects the axial direction of the lower housing 1.

In some implementations, the lower housing 1 may further include a first guide structure 13, and the first guide structure 13 may be fastened to an inner side surface of the side wall 11 of the lower housing 1. The housing 1 has an inner side and an outer side. The inner side surface of the side wall 11 of the lower housing 1 is a surface on an inner side of the side wall 11 of the lower housing 1. An inner side surface in the following descriptions is understood in a similar manner, and details are not described in this application again. The first guide structure 13 may extend in the axial direction of the lower housing 1. There may be one or more first guide structures 13. When there are a plurality of first guide structures 13, the plurality of first guide structures 13 are spaced apart around a circumferential direction of the side wall 11 of the lower housing 1.

In some implementations, the first guide structure 13 may include a first slide block 131 and a second slide block 132, and the first slide block 131 and the second slide block 132 are spaced apart. Both the first slide block 131 and the second slide block 132 extend in the axial direction of the lower housing 1. Limiting space 103 is reserved between the first slide block 131 and the top wall 12 of the lower housing 1. There may be one or more first slide blocks 131 and one or more second slide blocks 132. When there are a plurality of first slide blocks 131 and a plurality of second slide blocks 132, the plurality of first slide blocks 131 and the plurality of second slide blocks 132 are spaced apart around the circumferential direction of the side wall 11 of the lower housing 1, and the plurality of first slide blocks 131 and the plurality of second slide blocks 132 may be arranged alternately. For example, there are two first slide blocks 131 and two second slide blocks 132, the two first slide blocks 131 are arranged opposite to each other, and the two second slide blocks 132 are arranged opposite to each other.

In some implementations, the lower housing 1 may further include a first limiting boss 14 and a toggle 15. Both the first limiting boss 14 and the toggle 15 are fastened to a side, facing the opening 101 of the lower housing 1, of the top wall 12 of the lower housing 1.

In some implementations, the first limiting boss 14 may be in a circular shape or a ring shape. The first limiting boss 14 and the top wall 12 may enclose first space 141.

In some implementations, the toggle 15 may be located on an outer side of the first limiting boss 14. There may be one or more toggles 15. When there are a plurality of toggles 15, the plurality of toggles 15 are spaced apart around a circumferential direction of the first limiting boss 14. For example, there are two toggles 15, and the two toggles 15 are arranged opposite to each other. The toggle 15 is provided with an open slot 151. The open slot 151 of the toggle 15 helps reduce consumables for manufacturing the lower housing 1 and reduce a weight of the lower housing 1.

In some implementations, the lower housing 1 may further include a reinforcing rib 16. The reinforcing rib 16 may be fastened to the side, facing the opening 101 of the lower housing 1, of the top wall 12 of the lower housing 1. The reinforcing rib 16 may be located on the outer side of the first limiting boss 14. There may be one or more reinforcing ribs 16. When there are a plurality of toggles 15, the plurality of toggles 15 are spaced apart around the circumferential direction of the first limiting boss 14. The reinforcing rib 16 and the toggle 15 are spaced apart. For example, there are six reinforcing ribs 16.

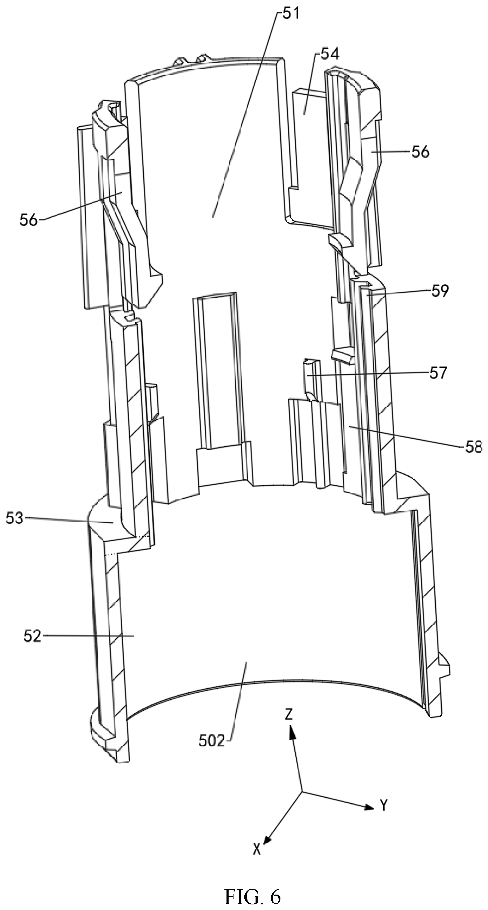

FIG. 5 is a diagram of a structure of the base 5 of the physiological sign monitoring apparatus 100 shown in FIG. 3. FIG. 6 is a schematic cross-sectional view of a structure of the base 5 shown in FIG. 5 along A-A according to an implementation.

As shown in FIG. 5 and FIG. 6, the base 5 may include an upper side wall 51, a lower side wall 52, and a connection wall 53. The connection wall 53 of the base 5 is connected to the upper side wall 51 of the base 5 and the lower side wall 52 of the base 5. On an outer side of the base 5, a stepped surface 501 is formed between the upper side wall 51 of the base 5 and the lower side wall 52 of the base 5. The stepped surface 501 is an upper surface of the connection wall 53 of the base 5. A direction from the upper side wall 51 of the base 5 to the lower side wall 52 of the base 5 is a top-down direction. For example, the upper side wall 51 of the base 5 is located above the lower side wall 52 of the base 5. The base 5 may be approximately in a cylindrical shape. The base 5 has an abutting end 502. The abutting end 502 is an end, away from the upper side wall 51, of the base 5.

In some implementations, the base 5 further includes a first resilient arm 54. An end, away from the lower side wall 52, of the first resilient arm 54 is provided with a hook. The hook of the first resilient arm 54 protrudes from an outer side surface of the base 5. The other end of the first resilient arm 54 is fastened to the upper side wall 51 of the base 5. The other end of the first resilient arm 54 is an end, away from the hook, of the first resilient arm 54. The first resilient arm 54 is spaced away from the side wall 11 of the base 5 in a circumferential direction. The hook of the first resilient arm 54 is provided with an inclined end face. The inclined end face of the first resilient arm 54 is inclined relative to an axial direction of the base 5.

In some implementations, the base 5 may further include a second guide structure 55, and the second guide structure 55 is fastened to the outer side surface of the base 5. The second guide structure 55 may extend in the axial direction of the base 5. For example, the second guide structure 55 may include a first guide arm 551, and the first guide arm 551 is fastened to an outer side surface of the upper side wall 51 of the base 5. The first guide arm 551 has a slide groove, and an opening 101 of the slide groove of the first guide arm 551 faces away from the side wall 11 of the base 5. The slide groove of the first guide arm 551 extends in the axial direction of the base 5, and the slide groove of the first guide arm 551 may be a strip-shaped groove.

In some implementations, the second guide structure 55 may further include a second guide arm 552. The second guide arm 552 is fastened to the outer side surface of the upper side wall 51 of the base 5. The second guide arm 552 has a slide groove. An opening of the slide groove of the second guide arm 552 may face away from the upper side wall 51 of the base 5. The slide groove of the second guide arm 552 extends in the axial direction of the base 5, and the slide groove of the second guide arm 552 may be a strip-shaped groove. There may be one or more first guide arms 551 and/or one or more second guide arms 552, and the second guide arms 552 and the first guide arms 551 may be arranged alternately in a circumferential direction of the base 5.

In some implementations, the base 5 may further include a second resilient arm 56. The second resilient arm 56 is located on a side, away from the abutting end 502, of the base 5. A tail end of the second resilient arm 56 is provided with a hook. The tail end of the second resilient arm 56 is an end, close to the abutting end 502, of the second resilient arm 56. The other end of the second resilient arm 56 is fastened to the upper side wall 51 of the base 5. The second resilient arm 56 is spaced away from the upper side wall 51 of the base 5 in the circumferential direction. The hook of the second resilient arm 56 is located on an inner side of the base 5 and faces away from the abutting end 502. There may be one or more second resilient arms 56, and a plurality of second resilient arms 56 may be spaced apart around the axial direction of the base 5. For example, there are two second resilient arms 56, and the two second resilient arms 56 are arranged opposite to each other.

In some implementations, the base 5 may further include a third guide structure 57, and the third guide structure 57 is fastened to an inner side surface of the base 5. The third guide structure 57 extends in the axial direction of the base 5. For example, there may be one or more third guide structures 57. When there are a plurality of third guide structures 57, the plurality of third guide structures 57 are spaced apart around the circumferential direction of the side wall 11 of the lower housing 1. For example, the third guide structure 57 is a slide block. The third guide structure 57 is protrudingly disposed on an inner side surface of the upper side wall 51 of the base 5. For example, there may be three third guide structures 57. The third guide structure 57 is located at an end, close to the lower side wall 52 of the base 5, of the upper side wall 51 of the base 5.

In some implementations, the base 5 may further include a seventh resilient arm 58. For example, the seventh resilient arm 58 is fastened to an end, close to the abutting end 502, of the upper side wall 51 of the base 5. A tail end of the seventh resilient arm 58 is provided with a hook. The tail end of the seventh resilient arm 58 is an end, away from the abutting end 502, of the seventh resilient arm 58. The hook of the seventh resilient arm 58 is located on the inner side of the base 5 and faces the abutting end 502. There may be one or more seventh resilient arms 58, and a plurality of seventh resilient arms 58 may be spaced apart around the axial direction of the base 5. For example, there may be two seventh resilient arms 58, and the two seventh resilient arms 58 are arranged opposite to each other.

In some implementations, the seventh resilient arm 58 may alternatively be fastened to an end, away from the abutting end 502, of the upper side wall 51 of the base 5. A tail end of the seventh resilient arm 58 is provided with a hook. The tail end of the seventh resilient arm 58 is an end, close to the abutting end 502, of the seventh resilient arm 58. The hook of the seventh resilient arm 58 is located on the inner side of the base 5 and faces away from the abutting end 502.

In some implementations, the base 5 may further include a fifth guide structure 59. The fifth guide structure 59 may be fastened to the inner side surface of the upper side wall 51 of the base 5. The fifth guide structure 59 has a slide groove. The slide groove extends in the axial direction of the base 5. The slide groove may be a strip-shaped groove. There may be one or more fifth guide structures 59. When there are a plurality of fifth guide structures 59, the plurality of fifth guide structures 59 may be spaced apart around the axial direction of the base 5. For example, there may be two fifth guide structures 59.

In some implementations, the base 5 may be an integrally formed mechanical part. The base 5 is made of engineering plastic such as polycarbonate or an acrylonitrile-butadiene-styrene mixture. In this embodiment, the polycarbonate and acrylonitrile-butadiene-styrene plastic are characterized by heat resistance, impact resistance, good mechanical performance, high hardness, good processing performance, and the like. The base 5 is made of the engineering plastic such as the polycarbonate or the acrylonitrile-butadiene-styrene mixture, to help reduce manufacturing costs of the base 5, and ensure mechanical strength of the base 5.

In some other embodiments, the base 5 may alternatively be made of another material, for example, engineering plastic such as polyvinyl chloride resin or polystyrene resin, or metal such as stainless steel or an aluminum alloy. This is not strictly limited in this application.

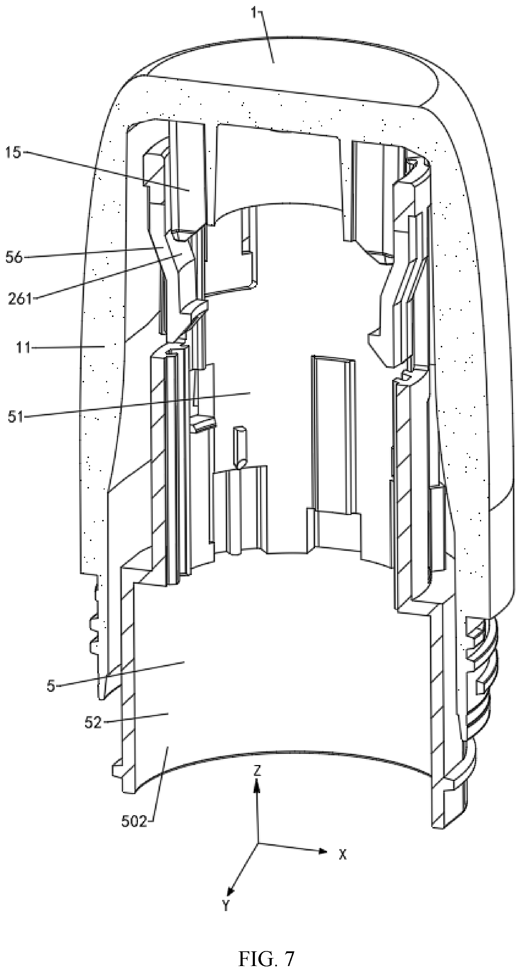

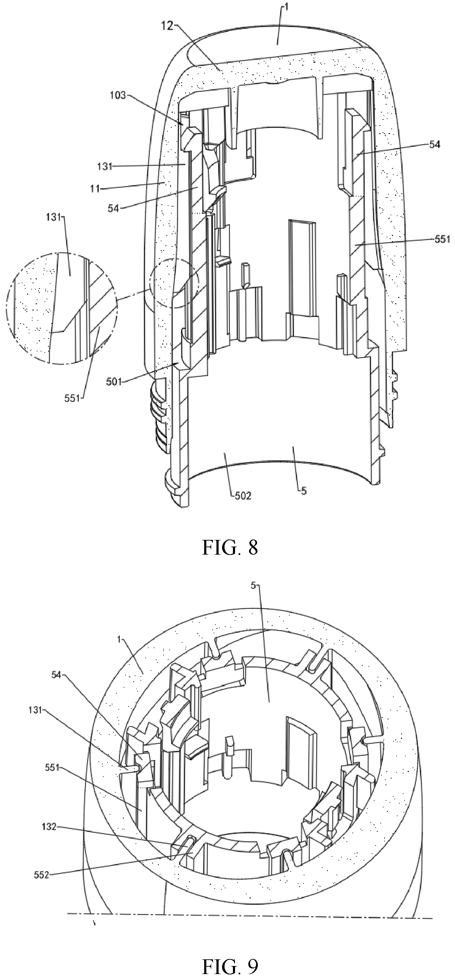

FIG. 7 is a schematic cross-sectional view of a partial structure of the physiological sign monitoring apparatus 100 shown in FIG. 2 that is cut along B-B. FIG. 8 is a schematic cross-sectional view of a partial structure of the physiological sign monitoring apparatus 100 shown in FIG. 2 that is cut along C-C. FIG. 9 is a schematic cross-sectional view of a partial structure of the physiological sign monitoring apparatus 100 shown in FIG. 2 that is cut along D-D.

As shown in FIG. 7 to FIG. 9, the base 5 may be mounted on the inner side of the lower housing 1 and slidably connected to the side wall 11 of the lower housing 1. The axial direction of the base 5 may coincide with the axial direction of the lower housing 1. An end, away from the upper side wall 51, of the lower side wall 52 of the base 5 is the abutting end 502. The abutting end 502 protrudes from the lower housing 1 through the opening 101 of the lower housing 1.

In some implementations, the hook of the first resilient arm 54 may snap into the side wall 11 of the lower housing 1, to limit movement of the lower housing 1 relative to the base 5 in a direction away from the abutting end 502. For example, the hook of the first resilient arm 54 may snap into the first slide block 131, and the hook of the first resilient arm 54 is mounted in the limiting space 103 between the first slide block 131 and the top wall 12 of the lower housing 1. The first slide block 131 abuts against the hook of the first resilient arm 54, to limit movement of the lower housing 1 relative to the base 5 in a direction away from the lower side wall 52. It can be understood that, in this embodiment, the hook of the first resilient arm 54 performs position limiting for the first slide block 131 in the axial direction, to prevent the lower housing 1 from moving relative to the base 5 in the direction away from the lower side wall 52, and prevent the lower housing 1 from detaching from the base 5.

In some implementations, the first resilient arm 54 is spaced away from the upper side wall 51 of the base 5 in the circumferential direction, and the hook of the first resilient arm 54 has an inclined end face. During assembly of the base 5 and the lower housing 1, the first resilient arm 54 may perform elastic avoidance for the first slide block 131 in a radial direction, and the inclined end face of the first resilient arm 54 performs avoidance for the first slide block 131 in the axial direction, so that the base 5 can be smoothly mounted into the inner side of the lower housing 1. This reduces snap-fit resistance, and can avoid damage to the base 5 and the lower housing 1 during assembly.

In some other embodiments, the side wall 11 of the lower housing 1 may alternatively have snap-fit space or a snap-fit block, and the hook of the first resilient arm 54 may snap into the snap-fit space or the snap-fit block of the lower housing 1, to implement snap-fit between the first resilient arm 54 and the lower housing 1. This is not strictly limited in this application.

In some other embodiments, a position of the hook of the first resilient arm 54 and a position of the first slide block 131 may alternatively be interchanged.

In some other embodiments, the first guide structure 13 is slidably connected to the second guide structure 55. The slide groove of the first guide arm 551 and a slide groove of the first resilient arm 54 may be used for guiding. For example, the first slide block 131 (the first guide structure 13) is mounted in the slide groove of the first guide arm 551 and the slide groove of the first resilient arm 54. To be specific, the first slide block 131 can slide between the stepped surface 501 and the hook of the first resilient arm 54. A quantity of first guide arms 551 matches a quantity of first slide blocks 131. The second slide block 132 is mounted in the slide groove of the second guide arm 552. The second slide block 132 can slide in the slide groove of the second guide arm 552. A quantity of second guide arms 552 matches a quantity of second slide blocks 132.

It can be understood that the first guide structure 13 is slidably connected to and fits with the second guide structure 55, to guide the lower housing 1 and the base 5 to slide relative to each other in the axial direction of the lower housing 1, and enable the lower housing 1 and the base 5 to perform position limiting for each other in a circumferential direction of the lower housing 1, so that the base 5 does not rotate relative to the lower housing 1 in the circumferential direction. When there are a plurality of first guide structures 13 and a plurality of second guide structures 55, forces applied to the lower housing 1 and the base 5 can be balanced, to prevent the base 5 from tilting relative to the lower housing 1. In addition, the first slide block 131 can slide between the stepped surface 501 and the hook of the first resilient arm 54, so that the lower housing 1 can move relative to the base 5 in the axial direction, and movement of the lower housing 1 relative to the base 5 in the axial direction is limited, to limit a distance by which the lower housing 1 can move relative to the base 5 in an implantation direction. The implantation direction is parallel to the Z-axis direction.

In some other embodiments, the first guide arm 551 may alternatively be disposed on an inner side surface of the lower housing 1, and the first slide block 131 of the base 5 may fit with the first guide arm 551 of the lower housing 1 to implement mutual position limiting between the lower housing 1 and the base 5 in the circumferential direction. This is not strictly limited in this application. In some other embodiments, the second slide block 132 may alternatively be fastened to the outer side surface of the base 5, the second guide arm 552 may alternatively be fastened to the inner side surface of the side wall 11 of the lower housing 1, and the second slide block 132 may fit with the second guide arm 552 to implement mutual position limiting between the lower housing 1 and the base 5 in the circumferential direction. This is not strictly limited in this application. In some other embodiments, fitting between the first guide structure 13 and the second guide structure 55 may alternatively not include fitting between the first slide block 131 and the first guide arm 551, or may not include fitting between the second slide block 132 and the second guide arm 552. This is not strictly limited in this application.

In some implementations, the toggle 15 may be located on a side, facing the base 5, of the top wall 12 of the lower housing 1. When the lower housing 1 moves relative to the base 5 in a direction toward the abutting end 502, the toggle 15 pushes the second resilient arm 56, and the second resilient arm 56 moves toward the outer side of the base 5.

For example, a wall surface of a guide slot of the second resilient arm 56 may further include a guide surface 261. The guide surface 261 is axially inclined relative to the base 5. One end of the toggle 15 is disposed close to the guide surface 261 of the guide slot. When the toggle 15 moves in a direction toward the abutting end 502, the toggle 15 can move along the guide surface 261.

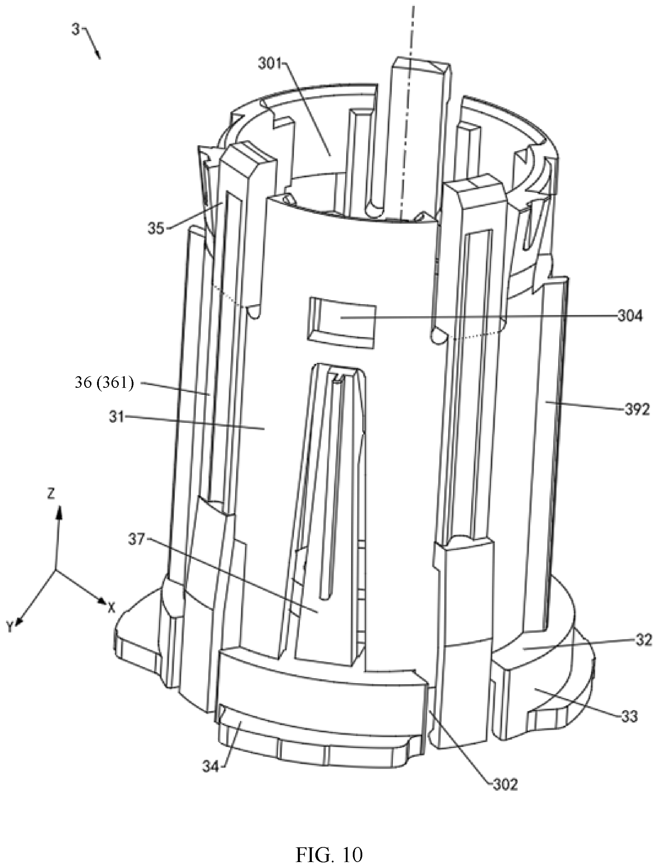

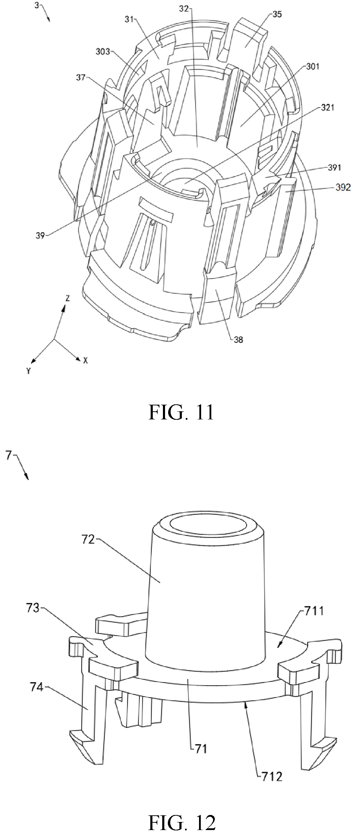

FIG. 10 is a diagram of a structure of the support 3 shown in FIG. 3 according to an implementation. FIG. 11 is a diagram of a structure of the support 3 shown in FIG. 3 from another perspective.

As shown in FIG. 10 and FIG. 11, the support 3 may include a side plate 31, a bottom plate 32, and a lower side plate 33. The bottom plate 32 of the support 3 is connected to the side plate 31 of the support 3 and the lower side plate 33 of the support 3. The side plate 31 of the support 3 is fastened to a periphery of the bottom plate 32 of the support 3. The side plate 31 of the support 3 surrounds the bottom plate 32 of the support 3 to form first accommodation space 301. The lower side plate 33 of the support 3 is fastened to the periphery of the bottom plate 32 of the support 3. The lower side plate 33 of the support 3 surrounds the bottom plate 32 of the support 3 to form second accommodation space 302. The support 3 is approximately a cylindrical assembly.

In some implementations, the support 3 may further include a flange 34. The flange 34 may be protrudingly disposed on an outer side surface of the support 3. For example, the flange 34 may be fastened to an outer side surface of the lower side plate 33 of the support 3. There may be one or more flanges 34, and a plurality of flanges 34 are spaced apart in a circumferential direction of the support 3. The flange 34 may be configured to increase a contact area between the support 3 and the user, and reduce pressure. A bottom surface 341 of the flange 34 is approximately flush with a bottom surface of the support 3. In view of a process error, the bottom surface 341 of the flange 34 may protrude downward. The bottom surface 341 of the flange 34 is a surface on a side, away from the side plate 31, of the flange 34.

In some implementations, the support 3 may further include a third resilient arm 35, one end of the third resilient arm 35 is fastened to the side plate 31 of the support 3, and the third resilient arm 35 is located at an end, away from the bottom plate 32 of the support 3, of the side plate 31 of the support 3. The third resilient arm 35 is spaced away from the side plate 31 of the support 3 in the circumferential direction. An end, away from the bottom plate 32 of the support 3, of the third resilient arm 35 is provided with an inclined end face. The inclined end face of the third resilient arm 35 is inclined relative to an axial direction of the support 3.

In some implementations, the support 3 may further include a fourth guide structure 36. The fourth guide structure 36 may be fastened to the outer side surface of the support 3. For example, the fourth guide structure 36 may include a third guide arm 361, and the third guide arm 361 is fastened to an outer side surface of the side plate 31 of the support 3. The third guide arm 361 has a slide groove, and an opening of the slide groove of the third guide arm 361 may face an outer side of the support 3. The slide groove of the first guide arm 551 extends in the axial direction of the support 3. The slide groove of the third guide arm 361 may be a strip-shaped groove.

In some implementations, the third guide arm 361 may extend from the bottom plate 32 of the support 3 to the third resilient arm 35 (distinguished by a dashed line in the figure). The third resilient arm 35 also has a slide groove. The slide groove of the third guide arm 361 is in communication with the slide groove of the third resilient arm 35. The third resilient arm 35 is also a part of the fourth guide structure 36. There may be one or more third resilient arms 35 and one or more third guide arms 361. When there are a plurality of third resilient arms 35 and a plurality of third guide arms 361, the plurality of third resilient arms 35 and the plurality of third guide arms 361 are arranged around the circumferential direction of the support 3.

In some implementations, the support 3 is provided with first snap-fit space 303, and the first snap-fit space 303 is located at the end, away from the bottom plate 32 of the support 3, of the side plate 31 of the support 3. For example, the first snap-fit space 303 may be a snap-fit slot. An opening 101 of the snap-fit slot faces the outer side of the support 3. In addition, the first snap-fit space 303 may alternatively be a snap-fit hole that passes through the side plate 31 of the support 3. The first snap-fit space 303 and the third resilient arm 35 are spaced apart.

In some implementations, the support 3 may be further provided with second snap-fit space 304. The second snap-fit space 304 is located at the end, away from the bottom plate 32 of the support 3, of the side plate 31 of the support 3. For example, the second snap-fit space 304 may be a snap-fit hole that passes through the side plate 31 of the support 3. The second snap-fit space 304 may be spaced away from the first snap-fit space 303 and the third resilient arm 35.

In some implementations, the support 3 may further include a fourth resilient arm 37. The fourth resilient arm 37 is spaced away from the side plate 31 of the support 3 in the circumferential direction, and one end of the fourth resilient arm 37 is fastened to the bottom plate 32 of the support 3 or the side plate 31 of the support 3. The other end of the fourth resilient arm 37 is provided with a hook. The other end of the fourth resilient arm 37 is an end, away from the bottom plate 32 of the support 3, of the fourth resilient arm 37. The hook of the fourth resilient arm 37 faces an inner side of the support 3. There may be one or more fourth resilient arms 37. When there are a plurality of fourth resilient arms 37, the plurality of fourth resilient arms 37 are arranged around the circumferential direction of the support 3.

In some implementations, the bottom plate 32 of the support 3 has a mounting hole 321. The support 3 may further include a fifth resilient arm 38. The fifth resilient arm 38 is spaced away from the lower side plate 33 of the support 3 in the circumferential direction. One end of the fifth resilient arm 38 is fastened to the bottom plate 32 of the support 3 or the lower side plate 33 of the support 3. The other end of the fifth resilient arm 38 is provided with a hook. The other end of the fifth resilient arm 38 is an end, away from the bottom plate 32 of the support 3, of the fifth resilient arm 38. The hook faces the inner side of support 3. There may be one or more fifth resilient arms 38. When there are a plurality of fifth resilient arms 38, the plurality of fifth resilient arms 38 are spaced apart around the circumferential direction of the support 3. For example, there may be three fifth resilient arms 38.

In some implementations, the support 3 may further include a fourth limiting boss 39. The fourth limiting boss 39 may be arranged around a periphery of the mounting hole 321, and is fastened to a side, facing the first accommodation space 301, of the bottom plate 32 of the support 3.

In some implementations, the support 3 may further include a fastening boss 391. The fastening boss 391 may be fastened to the outer side surface of the side plate 31 of the support 3, and the fastening boss 391 is located at the end, away from the bottom plate 32 of the support 3, of the side plate 31 of the support 3. For example, a top surface 3911 of the fastening boss 391 may be approximately flush with a top surface 311 of the side plate 31. The top surface 3911 of the fastening boss 391 is an end face, away from the bottom plate 32 of the support 3, of the fastening boss 391. The top surface 311 of the side plate 31 is an end face, away from the bottom plate 32 of the support 3, of the side plate 31. For example, the fastening boss 391 further has a side surface 3912. The side surface 3912 of the fastening boss 391 is a surface on a side, away from the side plate 31 of the support 3, of the fastening boss 391. The side surface 3912 of the fastening boss 391 is an inclined surface. The side surface 3912 of the fastening boss 391 may be inclined relative to the axial direction of the support 3, and face a side of the bottom plate 32 of the support 3.

In some implementations, a guide slot 312 may be further provided on an inner side of the side plate 31 of the support 3. The guide slot 312 may extend in the axial direction of the support 3. The guide slot 312 and the fourth resilient arm 37 are spaced apart. For example, there may be one or more guide slots 312. When there are a plurality of guide slots 312, the plurality of guide slots 312 are spaced apart around the axial direction of the support 3. For example, there may be three guide slots 312.

In some implementations, the support 3 may further include a slide bar 392. The slide bar 392 may be fastened to the outer side surface of the side plate 31 of the support 3. The slide bar 392 may extend in the axial direction of the support 3. For example, there may be one or more slide bars 392. When there are a plurality of slide bars 392, the plurality of slide bars 392 are spaced apart around the axial direction of the support 3. For example, there may be two slide bars 392. The two slide bars 392 are symmetrically distributed.

In some implementations, the support 3 may be an integrally formed mechanical part. The support 3 may be made of engineering plastic such as polycarbonate or an acrylonitrile-butadiene-styrene mixture. In this embodiment, the polycarbonate and acrylonitrile-butadiene-styrene plastic are characterized by heat resistance, impact resistance, good mechanical performance, high hardness, good processing performance, and the like. The support 3 is made of the engineering plastic such as the polycarbonate or the acrylonitrile-butadiene-styrene mixture, to help reduce manufacturing costs of the support 3, and ensure mechanical strength of the support 3.

In some other embodiments, the support 3 may alternatively be made of another material, for example, engineering plastic such as polyvinyl chloride resin or polystyrene resin, or metal such as stainless steel or an aluminum alloy. This is not strictly limited in this application.

FIG. 12 is a diagram of a structure of the spring seat 7 shown in FIG. 3 according to an implementation.

As shown in FIG. 12, the spring seat 7 may include an abutting portion 71, a third limiting boss 72, and a plurality of clamping blocks 73. The abutting portion 71 is approximately in a disc shape. The abutting portion 71 may include a first surface 711 and a second surface 712 that face away from each other. The third limiting boss 72 may be fastened to the first surface 711 of the abutting portion 71.

In some implementations, the plurality of clamping blocks 73 may be fastened to the first surface 711 of the abutting portion 71 and spaced away from the third limiting boss 72. The plurality of clamping blocks 73 are spaced apart. The plurality of clamping blocks 73 are spaced apart around the third limiting boss 72.

In some implementations, the abutting portion 71 may further include a circumferential side surface 713. The circumferential side surface 713 is connected between the first surface 711 and the second surface 712. The spring seat 7 may further include an eighth resilient arm 74. One end of the eighth resilient arm 74 is fastened to the circumferential side surface 713 of the abutting portion 71. The other end of the eighth resilient arm 74 is provided with a hook. The hook faces an outer side of the spring seat 7.

In some implementations, the spring seat 7 may be made of engineering plastic such as polycarbonate or an acrylonitrile-butadiene-styrene mixture.

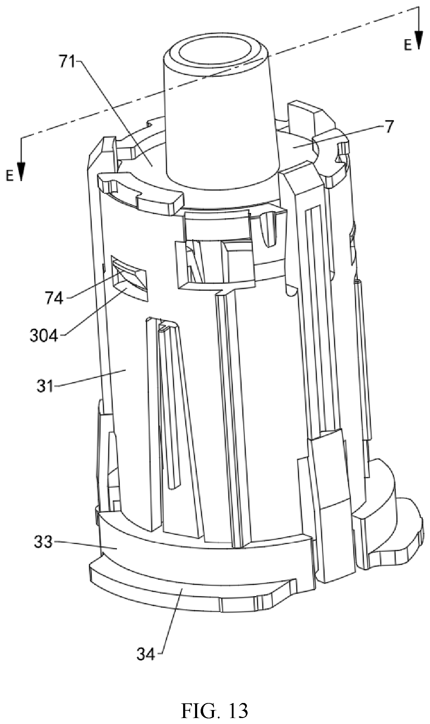

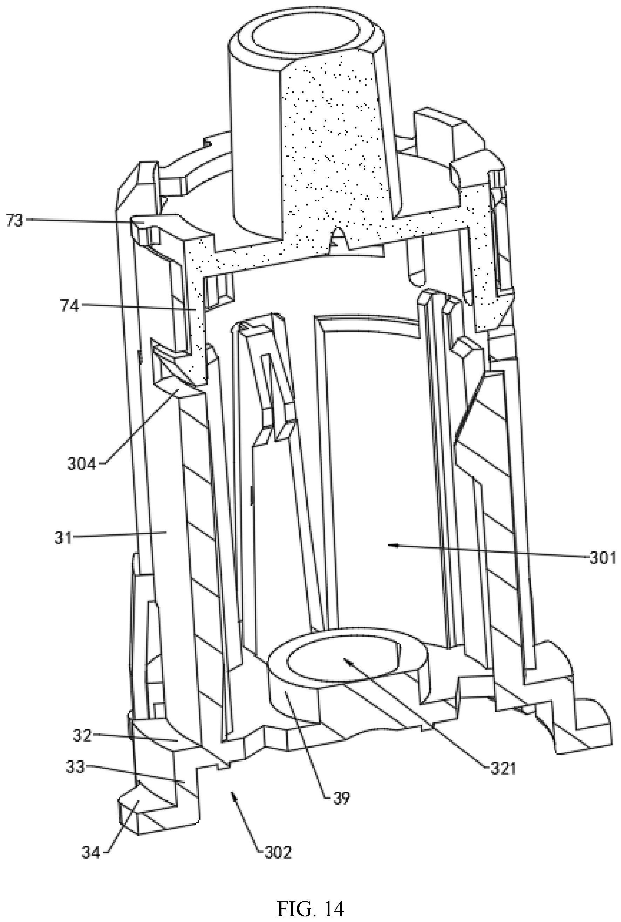

FIG. 13 is a diagram of assembly of the spring seat 7 and the support 3 shown in FIG. 3 according to an implementation. FIG. 14 is a partial cross-sectional view of the embodiment shown in FIG. 13 along E-E according to an implementation.

As shown in FIG. 13 and FIG. 14, the spring seat 7 may be mounted on the inner side of the support 3. For example, the hook of the eighth resilient arm 74 of the spring seat 7 snaps into the second snap-fit space 304 of the support 3. The clamping block 73 of the spring seat 7 is at least partially located on a side, away from the lower side plate 33 of the support 3, of the side plate 31 of the support 3. In this way, axial movement and circumferential rotation of the spring seat 7 relative to the support 3 can be limited.

It can be understood that the clamping block 73 of the spring seat 7 is at least partially located on the side, away from the lower side plate 33 of the support 3, of the side plate 31 of the support 3, to help increase an abutting area between the spring seat 7 and the support 3, and improve connection reliability.

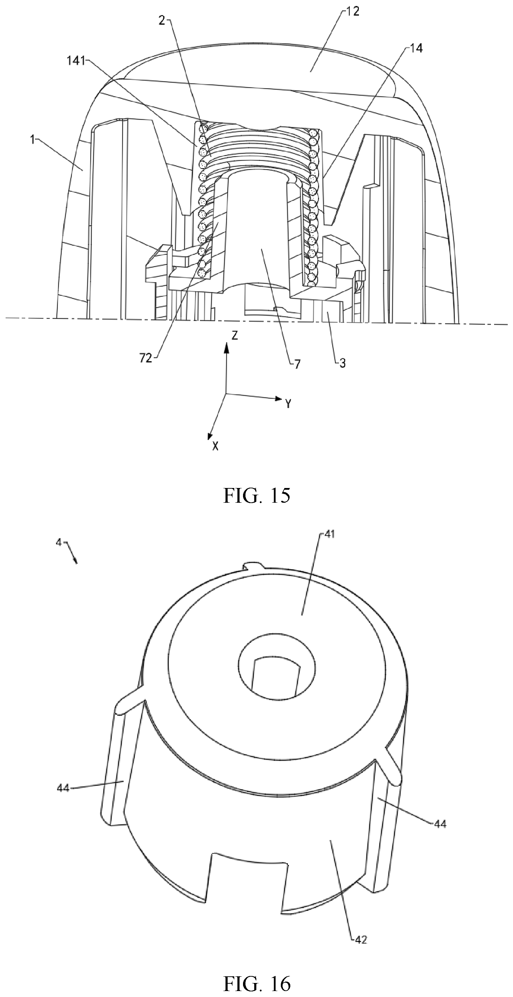

FIG. 15 is a diagram of a structure of the structure shown in FIG. 2 along a section line F-F according to an implementation.

As shown in FIG. 15, the first elastic member 2 may be connected between the top wall 12 of the lower housing 1 and the support 3. For example, the first elastic member 2 may abut between the spring seat 7 and the top wall 12 of the lower housing 1. The first elastic member 2 may be but is not limited to a spring, elastic rubber, or the like. In another implementation, the spring seat 7 may alternatively not be disposed. The first elastic member 2 may directly abut between the support 3 and the top wall 12 of the lower housing 1.

For example, one end of the first elastic member 2 may be located in the first space 141 of the first limiting boss 14 of the top wall 12 of the lower housing 1, and the other end of the first elastic member 2 may be sleeved around a periphery of the third limiting boss 72 of the spring seat 7. Both the first limiting boss 14 of the lower housing 1 and the third limiting boss 72 of the spring seat 7 perform position limiting and positioning for the first elastic member 2, to prevent the first elastic member 2 from tilting during compression and rebound, so that a position of the first elastic member 2 is stable, to improve reliability of the physiological sign monitoring apparatus 100. The first elastic member 2 may be in a compressed state, to form pre-pressure on the spring seat 7, so that a position of the spring seat 7 relative to the base 5 is stable.

In another implementation, sizes of the first limiting boss 14 and the third limiting boss 72 may be adjusted, so that one end of the first elastic member 2 may alternatively be sleeved around a periphery of the first limiting boss 14 on the top wall 12 of the lower housing 1, and the other end of the first elastic member 2 is located in the third limiting boss 72 of the first elastic member 2.

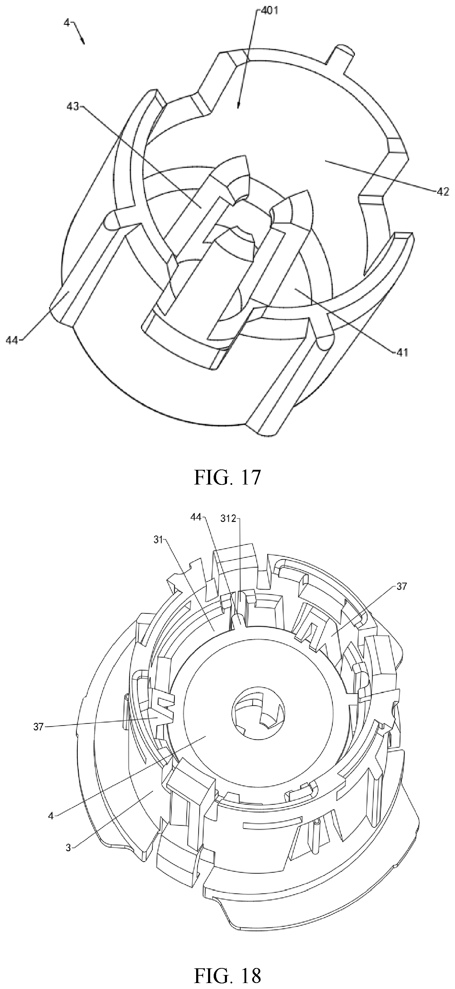

FIG. 16 is a diagram of a structure of the needle retraction holder 4 shown in FIG. 3 according to an implementation. FIG. 17 is a diagram of a structure of the needle retraction holder 4 shown in FIG. 16 from another perspective according to an implementation.

As shown in FIG. 16 and FIG. 17, the needle retraction holder 4 may include a top plate 41 and a side plate 42. The side plate 42 of the needle retraction holder 4 is arranged around the top plate 41 of the needle retraction holder 4. The needle retraction holder 4 is approximately in a cylindrical shape, and the side plate 42 of the needle retraction holder 4 surrounds third accommodation space 401.

In some implementations, the needle retraction holder 4 may further include a first cantilever 43. One end of the first cantilever 43 is fastened to a side, facing the third accommodation space 401, of the top plate 41 of the needle retraction holder 4. The other end of the first cantilever 43 is provided with a hook. The other end of the first cantilever 43 is an end, away from the top plate 41 of the needle retraction holder 4, of the first cantilever 43. The hook of the first cantilever 43 faces the top plate 41 of the needle retraction holder 4. For example, there may be a plurality of first cantilevers 43, and the plurality of first cantilevers 43 are spaced apart around an axis of the needle retraction holder 4. For example, there may be three first cantilevers 43.

In some implementations, the needle retraction holder 4 may further include a guide bar 44. The guide bar 44 may be fastened to the outer side surface of the side plate 31. The guide bar 44 may extend in an axial direction of the needle retraction holder 4. For example, there may be one or more guide bars 44. When there are a plurality of guide bars 44, the plurality of guide bars 44 are spaced apart around the axis of the needle retraction holder 4. For example, there may be three guide bars 44.

In some implementations, the needle retraction holder 4 may be an integrally formed mechanical part. The needle retraction holder 4 is made of engineering plastic such as polycarbonate or an acrylonitrile-butadiene-styrene mixture. In this embodiment, the polycarbonate and acrylonitrile-butadiene-styrene plastic are characterized by heat resistance, impact resistance, good mechanical performance, high hardness, good processing performance, and the like. The needle retraction holder 4 is made of the engineering plastic such as the polycarbonate or the acrylonitrile-butadiene-styrene mixture, to help reduce manufacturing costs of the needle retraction holder 4, and ensure mechanical strength of the needle retraction holder 4.

In some other embodiments, the needle retraction holder 4 may alternatively be made of another material, for example, engineering plastic such as polyvinyl chloride resin or polystyrene resin, or metal such as stainless steel or an aluminum alloy. This is not strictly limited in this application.

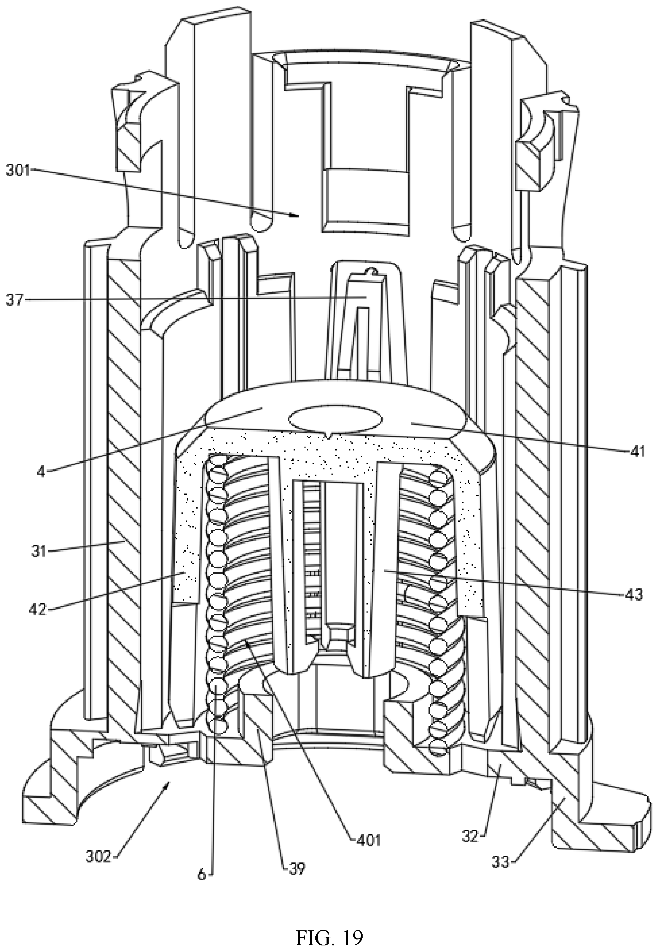

FIG. 18 is a diagram of assembly of the needle retraction holder 4 and the support 3 shown in FIG. 3 according to an implementation. FIG. 19 is a partial cross-sectional view of the physiological sign monitoring apparatus 100 shown in FIG. 2 along a section line B-B according to an implementation.

As shown in FIG. 18 and FIG. 19, the needle retraction holder 4 may be mounted on the inner side of the support 3. For example, the needle retraction holder 4 may be mounted between the bottom plate 32 of the support 3 and the hook of the fourth resilient arm 37. There may be three fourth resilient arms 37, so that the needle retraction holder 4 in snap-fit is subject to balanced forces. In this embodiment, the needle retraction holder 4 is located between the bottom plate 32 of the support 3 and the hook of the fourth resilient arm 37, so that a stable positional relationship is maintained between the needle retraction holder 4 and the support 3, and the fourth resilient arm 37 may push the needle retraction holder 4 to move together with the support 3.

In some implementations, the support 3 may further include a fourth limiting boss 39. The fourth limiting boss 39 is arranged around a periphery of the mounting hole 321, and is fastened to a side, facing the first accommodation space 301, of the bottom plate 32 of the support 3. The side plate 42 of the needle retraction holder 4 may be partially located between the fourth limiting boss 39 and the side plate 31 of the support 3.

In some implementations, the guide bar 44 of the needle retraction holder 4 may be located in the guide slot 312 on the inner side of the side plate 31 of the support 3. The guide slot 312 of the side plate 31 of the support 3 may fit with the guide bar 44 of the needle retraction holder 4, to perform position limiting for the needle retraction holder 4, and avoid rotation of the needle retraction holder 4. In another implementation, a guide slot may alternatively be provided on the needle retraction holder 4, a guide bar is disposed on an inner side surface of the side plate 31 of the support 3, and the guide slot and the guide bar fit with each other to perform position limiting for the needle retraction holder 4.

In some implementations, the guide slot of the side plate 31 of the support 3 and the fourth resilient arm 37 of the support 3 are sequentially spaced apart in the circumferential direction of the support 3. Any guide slot is located between two fourth resilient arms 37. In this way, the needle retraction holder 4 is subject to uniform forces.

In some implementations, the second elastic member 6 may be located between the support 3 and the needle retraction holder 4. Both the axial direction of the needle retraction holder 4 and an axial direction of the second elastic member 6 may coincide with the axial direction of the support 3. The second elastic member 6 is in a compressed state.

For example, the needle retraction holder 4 is mounted in the first accommodation space 301 of the support 3, and the side plate 42 of the needle retraction holder 4 is located on an outer side of the fourth limiting boss 39. The second elastic member 6 is mounted in the third accommodation space 401 of the needle retraction holder 4. The second elastic member 6 may be but is not limited to a spring, elastic rubber, or the like. The second elastic member 6 is located between the bottom plate 32 of the support 3 and the top plate 41 of the needle retraction holder 4, and is sleeved around a periphery of the first cantilever 43 and the fourth limiting boss 39. The second elastic member 6 is configured to push the needle retraction holder 4 away from the bottom plate 32 of the support 3. The first cantilever 43 and the fourth limiting boss 39 perform position limiting and positioning for the second elastic member 6, to prevent the second elastic member 6 from tilting during compression and rebound, so that a position of the second elastic member 6 is stable, to improve reliability of the physiological sign monitoring apparatus 100.

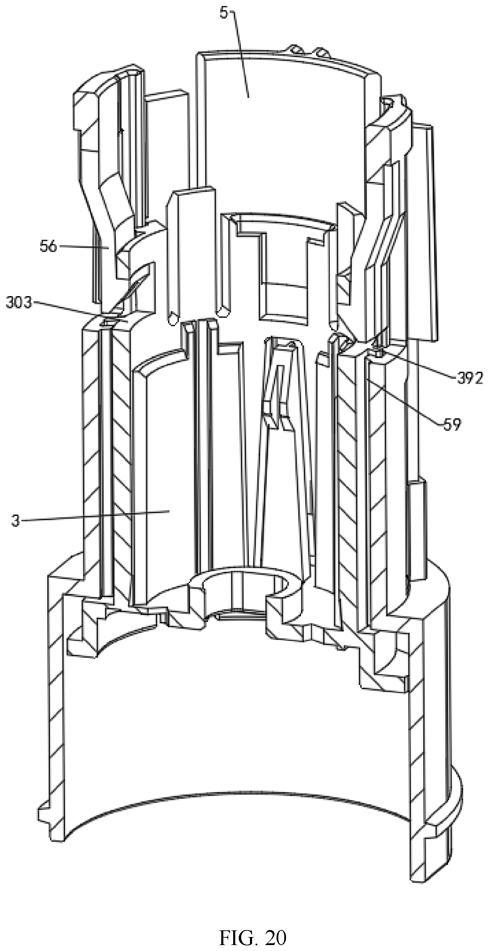

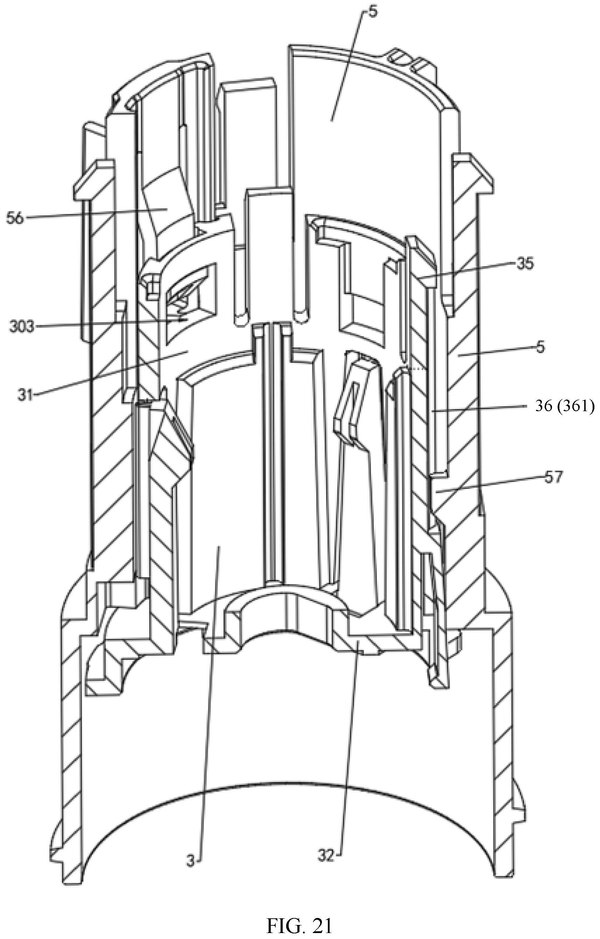

FIG. 20 is a schematic cross-sectional view of a partial structure of the physiological sign monitoring apparatus 100 shown in FIG. 2 that is cut along B-B. FIG. 21 is a schematic cross-sectional view of a partial structure of the physiological sign monitoring apparatus 100 shown in FIG. 2 that is cut along C-C. FIG. 20 and FIG. 21 are diagrams of assembly of the support 3 and the base 5 according to an implementation.

As shown in FIG. 20 and FIG. 21, the support 3 is mounted on the inner side of the base 5, and is slidably connected to the base 5. The axial direction of the support 3 may coincide with the axial direction of the base 5. For example, the slide bar 392 of the support 3 may be slidably connected to a slide groove of the fifth guide structure 59 of the base 5.

In some implementations, the third guide structure 57 of the base 5 is slidably connected to the fourth guide structure 36 of the support 3. For example, a slide block of the third guide structure 57 of the base 5 may be mounted in the slide groove of the third resilient arm 35 of the support 3 and the slide groove of the third guide arm 361, and the third slide block can slide in the slide groove of the third resilient arm 35 and the slide groove of the third guide arm 361. There may be three third resilient arms 35 and three third guide arms 361, to ensure that a connection between the support 3 and the base 5 is subject to balanced forces, and keep the support 3 and the base 5 stable in a radial direction.

It can be understood that, in this embodiment, the slide bar 392 of the support 3 is slidably connected to the slide groove of the fifth guide structure 59 of the base 5. The third guide structure 57 of the base 5 is slidably connected to and fits with the fourth guide structure 36 of the support 3, so that the base 5 and the support 3 perform position limiting for each other in the circumferential direction, to prevent the support 3 from rotating relative to the base 5 in the circumferential direction. The slide bar 392 of the support 3 is slidably connected to the slide groove of the fifth guide structure 59 of the base 5. The slide block of the third guide structure 57 can slide in the slide groove of the third resilient arm 35 and the third guide arm 361, so that the support 3 can move relative to the base 5 in the axial direction.

For example, the third resilient arm 35 may further limit movement of the support 3 in the axial direction. The third resilient arm 35 is spaced away from the side plate 31 of the support 3 in the circumferential direction, and the other end, away from the bottom plate 32 of the support 3, of the third resilient arm 35 is provided with an inclined end face. During assembly of the support 3 and the base 5, the third resilient arm 35 may perform elastic avoidance for the third slide block in the radial direction, and the inclined end face of the third resilient arm 35 performs avoidance for the third slide block in the axial direction, so that the support 3 can be smoothly mounted into the inner side of the base 5. This reduces snap-fit resistance, and can avoid damage to the support 3 and the base 5 during assembly.

In some implementations, the hook of the second resilient arm 56 is at least partially located in the first snap-fit space 303 of the support 3, so that the second resilient arm 56 snaps into the support 3, and the base 5 performs position limiting for the support 3 in the axial direction.

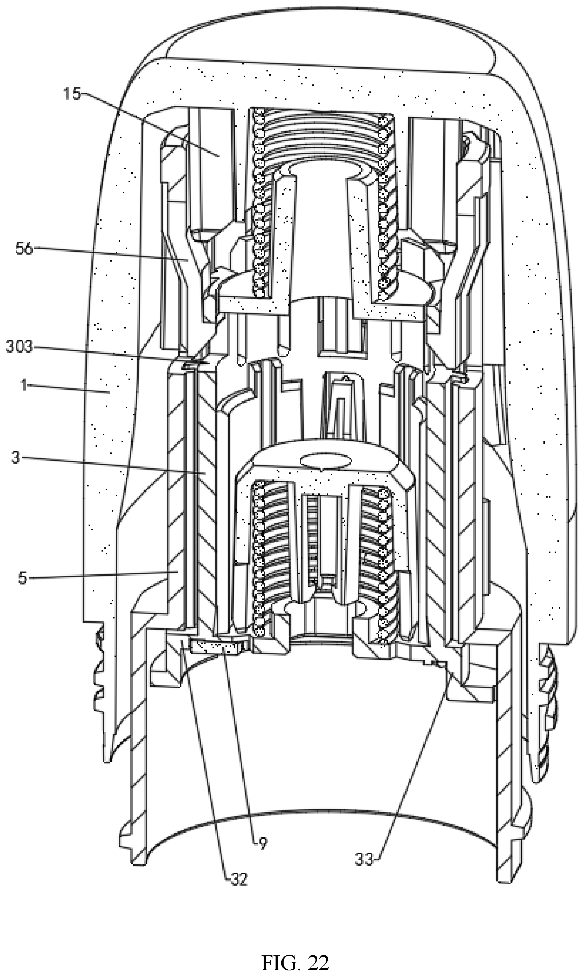

FIG. 22 is a schematic cross-sectional view of a partial structure of the physiological sign monitoring apparatus 100 shown in FIG. 2 that is cut along B-B.

As shown in FIG. 22, the hook of the second resilient arm 56 is at least partially located in the first snap-fit space 303 of the support 3, so that the second resilient arm 56 snaps into the support 3, and the base 5 performs position limiting for the support 3.

In some implementations, the first magnet 9 may be fastened to the support 3. For example, the first magnet 9 may be fastened to a side, facing the lower side plate 33 of the support 3, of the bottom plate 32 of the support 3. The first magnet 9 may be configured to form a magnetic actuator switch.

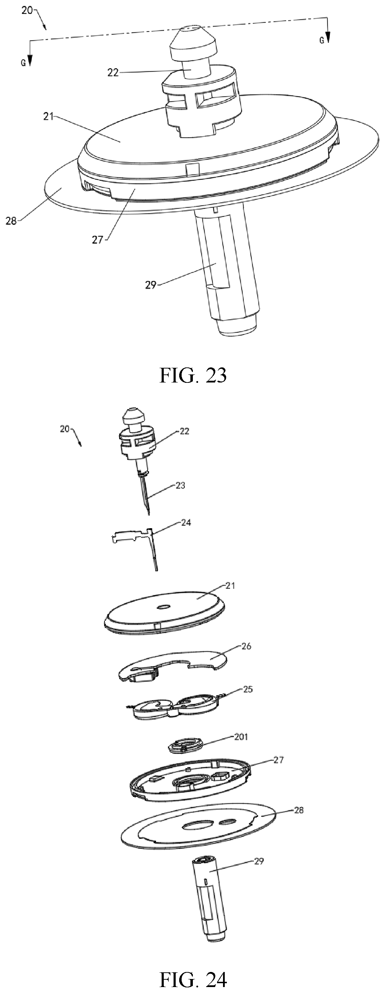

FIG. 23 is a diagram of a structure of the sensor assembly 20 shown in FIG. 3 according to an implementation. FIG. 24 is a schematic exploded view of the sensor assembly 20 shown in FIG. 23 according to an implementation.

As shown in FIG. 23 and FIG. 24, the sensor assembly 20 may include an upper cover 21, a needle holder 22, a puncture needle 23, a sensor 24, a battery assembly 25, an electrical connection assembly 26, a lower cover 27, a bonding member 28, a gasket 201, and a sealed cartridge 29.

In some implementations, both the upper cover 21 and the lower cover 27 may be made of engineering plastic such as polycarbonate or acrylonitrile-butadiene-styrene.

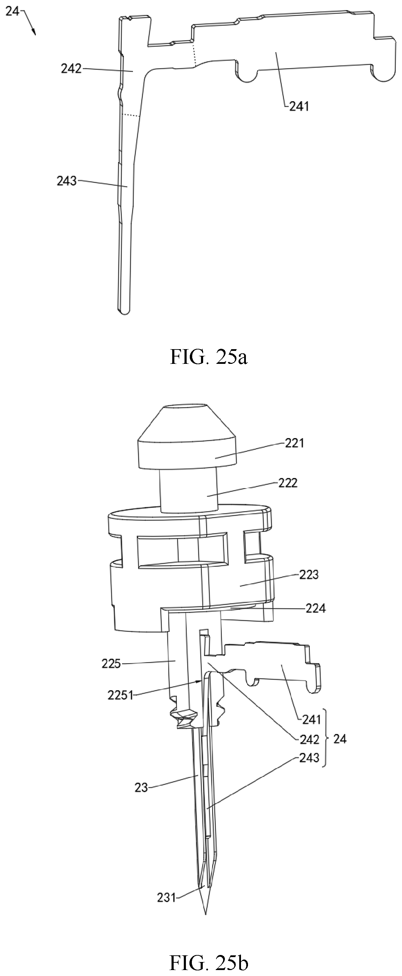

FIG. 25a is a diagram of a structure of the sensor 24 shown in FIG. 24 according to an implementation.

As shown in FIG. 25a, the sensor 24 may include a connection portion 241, a fastening portion 242, and an implantation portion 243 (distinguished by dashed lines in FIG. 25a), and the fastening portion 242 is connected to the implantation portion 243 and the connection portion 241. The connection portion 241 of the sensor 24 is configured to electrically connect to the electrical connection assembly 26 (as shown in FIG. 24), and the implantation portion 243 of the sensor 24 is configured to puncture into the organism 30.

In some implementations, the sensor 24 may include a substrate, an electrode layer, and a sensing layer (not shown in the figure). For example, the substrate is made of polyethylene terephthalate (Polyethylene terephthalate, PET). The electrode layer is fastened to a surface of the substrate. The electrode layer may be an electrode system (for example, two electrodes or a plurality of electrodes) formed on the surface of the substrate through an ink-jet printing process, a silk-screen printing process, or the like. The electrode layer may include a first part located in the implantation portion 243, a second part located in the fastening portion 242, and a third part located in the connection portion 241. The second part of the electrode layer is connected to the first part of the electrode layer and the third part of the electrode layer. The sensing layer is fastened to a partial region in the first part of the electrode layer. In other words, the sensing layer is located in the implantation portion 243. The sensing layer may be formed by applying a sensing reagent to a surface of the first part of the electrode layer through ink-jet printing or drop-casting. The sensing reagent may be a mixed solution including a bio-protease (for example, glucose oxidase), an electron transfer medium, and a cross-linking agent. After printing of the sensor 24 is completed, a shape may be formed through femtosecond or picosecond laser cutting. It can be understood that the sensing reagent of the sensing layer may be selected based on a physiological indicator that needs to be monitored.

FIG. 25b is a diagram of assembly of the sensor 24, the needle holder 22, and the puncture needle 23 shown in FIG. 24 according to an implementation.

As shown in FIG. 25b, the needle holder 22 may include a limiting fastener 221, a snap-fit portion 222, a limiting portion 223, a sealing portion 224, and a guide portion 225. The limiting fastener 221, the snap-fit portion 222, the limiting portion 223, the sealing portion 224, and the guide portion 225 are sequentially connected. The limiting portion 223 may be approximately in a cylindrical shape. A diameter of the snap-fit portion 222 is less than a diameter of a bottom surface of the limiting fastener 221 and a diameter of the limiting portion 223. The guide portion 225 has a recessed groove 2251, and the recessed groove 2251 extends in an axial direction of the needle holder 22.

In some implementations, the puncture needle 23 may be fastened to the needle holder 22 and extend in the axial direction of the needle holder 22. A puncture tip 231 of the puncture needle 23 may protrude from the guide portion 225 of the needle holder 22. The puncture needle 23 is fastened to the recessed groove 2251 of the guide portion 225, and a shape of the recessed groove 2251 matches a shape of the puncture needle 23. The puncture needle 23 and the needle holder 22 may be integrally formed. Integrating two components into an integral structure through an integral molding process means that, in a process of forming one of the two components, the component is connected to the other component, and the two components do not need to be connected through secondary processing such as bonding, welding, fastener connection, or screw connection.

For example, the needle holder 22 and the puncture needle 23 may be integrally formed through an in-mold injection molding process. The needle holder 22 may be made of engineering plastic such as polycarbonate or acrylonitrile-butadiene-styrene. The puncture needle 23 may be made of a metal material such as 316 stainless steel, 304 stainless steel, or medical-grade stainless steel (for example, SUS316L).

For example, a cross section of the puncture needle 23 may be approximately of a U-shaped structure, and the puncture needle 23 is configured to puncture into tissue of the organism 30 and push the tissue open. The puncture tip of the puncture needle 23 may be configured to cover the implantation portion 243 of the sensor 24. When the puncture needle 23 punctures into the organism 30, implantation space may be formed for the implantation portion 243 of the sensor 24 to puncture into the organism 30. In addition, the puncture tip of the puncture needle 23 may alternatively partially cover the implantation portion 243 of the sensor 24, and the implantation portion 243 of the sensor 24 can also puncture into the organism 30 together with the puncture needle 23. This is not strictly limited in this application.

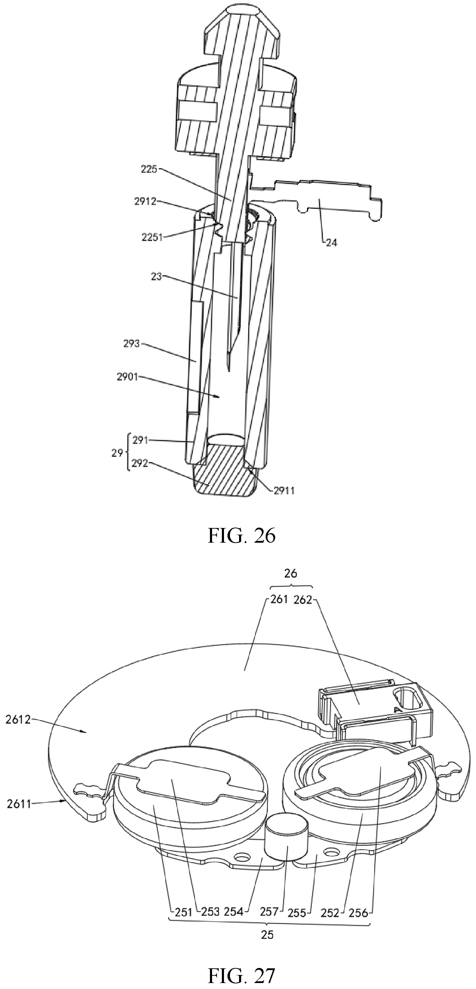

FIG. 26 is a partial cross-sectional view of the sensor assembly 20 shown in FIG. 23 that is cut along G-G according to an implementation. FIG. 26 is a diagram of assembly of the sensor 24, the needle holder 22, the puncture needle 23, and the sealed cartridge 29 according to an implementation.

As shown in FIG. 26, the sealed cartridge 29 may be configured to protect the puncture needle 23 against microbial contamination. The sealed cartridge 29 has a hollow cavity. For example, the sealed cartridge 29 may include a body 291 and a plug 292. The body 291 has placement space 2901 (a hollow cavity). The placement space 2901 may extend in an axial direction of the body 291, and pass through two ends of the body 291, to form a first opening 2911 at one end of the body 291 and form a second opening 2912 at the other end. The plug 292 may be detachably fastened to one end of the body 291, to seal the first opening 2911. The other end of the body 291 is close to the needle holder 22, and is fastened to the guide portion 225 of the needle holder 22.

In some implementations, a part of the sensor 244 and the puncture tip 231 of the puncture needle 23 may be located in the hollow cavity of the sealed cartridge 29. For example, the puncture needle 23 and the sensor 24 located in the puncture needle 23 may enter the placement space 2901 through the second opening 2912. Threads may be provided in the placement space 2901 at the other end of the body 291. Threads matching the body 291 may be provided on a circumferential side surface 2251 of the guide portion 225 of the needle holder 22. In this way, the other end of the body 291 may be fastened to the guide portion 225 of the needle holder 22 through the threads. It can be understood that the plug 292, the threads in the placement space 2901, and the threads on the circumferential side surface 2251 of the guide portion 225 are provided to seal the placement space 2901 and protect the placement space 2901 against dust and contamination.

In another implementation, the guide portion 225 of the needle holder 22 and the body 291 may alternatively be locked through an oblique slide groove.

In some implementations, the sealed cartridge 29 may further include a ratchet wheel 293. The ratchet wheel 293 may be protrudingly disposed on an outer side surface of the sealed cartridge 29. For example, there may be one or more ratchet wheels 293. When there are a plurality of ratchet wheels 293, the plurality of ratchet wheels 293 are arranged around a circumferential direction of the sealed cartridge.

In another implementation, the body 291 and the plug 292 may be an integral mechanical part and non-detachable.

FIG. 27 is a diagram of assembly of the electrical connection assembly 26 and the battery assembly 25 shown in FIG. 24 according to an implementation.

As shown in FIG. 27, the electrical connection assembly 26 may include a circuit board (Printed Circuit Board, PCB) 261 and an electrical connector 262. The electrical connector 262 is electrically connected to the circuit board 261. For example, the circuit board 261 includes a first surface 2611 and a second surface 2612 that face away from each other. The electrical connector 262 may be fastened to the second surface 2612 of the circuit board 261 and electrically connected to the circuit board 261. The electrical connector 262 may be configured to transmit an electrical signal between the circuit board 261 and the sensor 24.

It can be understood that electronic components (not shown in the figure) such as a chip, a capacitor, a resistor, a near field communication (Near Field Communication, NFC) component, or a Bluetooth component may be disposed on the circuit board 261.

For example, the battery assembly 25 may include a first battery 251, a second battery 252, a first positive electrode plate 253, a first negative electrode plate 254, a second positive electrode plate 255, a second negative electrode plate 256, and a switch magnet 257. One end of the first positive electrode plate 253 is fastened to the circuit board 261 and is electrically connected to the circuit board 261. The other end of the first positive electrode plate 253 is fastened to the first battery 251 and is electrically connected to a positive electrode of the first battery 251. One end of the first negative electrode plate 254 is fastened to the first battery 251 and is electrically connected to a negative electrode of the first battery 251. One end of the second positive electrode plate 255 is fastened to the second battery 252 and is electrically connected to a positive electrode of the second battery 252. One end of the second negative electrode plate 256 is fastened to the circuit board 261 and is electrically connected to the circuit board 261.

For example, the switch magnet 257 may be movably connected between the other end of the first negative electrode plate 254 and the other end of the second positive electrode plate 255, to control electrical conduction between the first negative electrode plate 254 and the second positive electrode plate 255. When the switch magnet 257 is connected to both the first negative electrode plate 254 and the second positive electrode plate 255, the first negative electrode plate 254 and the second positive electrode plate 255 may be electrically conducted through the switch magnet 257, a current may pass through the switch magnet 257, and the battery assembly 25 may properly transmit a current to the circuit board 261. When the switch magnet 257 disconnects the first negative electrode plate 254 from the second positive electrode plate 255, the first negative electrode plate 254 and the second positive electrode are electrically disconnected, and the battery assembly 25 cannot transmit a current to the circuit board 261. With reference to the accompanying drawings, the following specifically describes how the switch magnet 257 is connected to and disconnected from the first negative electrode plate 254 and the second positive electrode plate 255.

It can be understood that whether the switch magnet 257 is electrically connected to a positive electrode plate or a negative electrode plate is not limited in this application. In another implementation, a position of the first positive electrode plate 253 and a position of the first negative electrode plate 254 may be interchanged, and a position of the second positive electrode plate 255 and a position of the second negative electrode plate 256 may be exchanged.

For example, the first positive electrode plate 253, the first negative electrode plate 254, the second positive electrode plate 255, and the second negative electrode plate 256 may be conductive steel sheets. There are attractive forces between the switch magnet 257 and the first negative electrode plate 254 and between the switch magnet 257 and the second positive electrode plate 255.

For example, the switch magnet 257 may include one or two magnets.

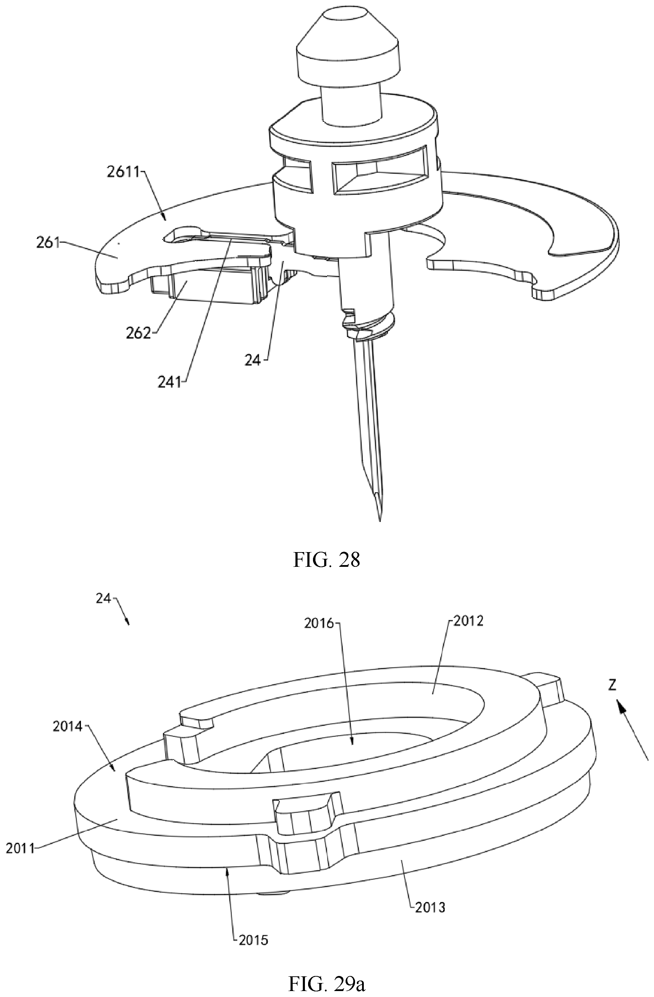

FIG. 28 is a diagram of assembly of the sensor 24, the needle holder 22, the puncture needle 23, and the electrical connection assembly 26 shown in FIG. 24 according to an implementation.

As shown in FIG. 27 and FIG. 28, the connection portion 241 of the sensor 24 is connected to the electrical connector 262, and is electrically connected to the electrical connector 262. When the switch magnet 257 is connected to both the first negative electrode plate 254 and the second positive electrode plate 255, the first battery 251 and the second battery 252 may be electrically connected to the circuit board 261, and the circuit board 261 is electrically connected to the connection portion 241 of the sensor 24 through the electrical connector 262. In this way, the first battery 251 and the second battery 252 can supply power to the sensor 24. In other words, the switch magnet 257 may be configured to control energization/de-energization of the sensor 24.

FIG. 29a is a diagram of a structure of the gasket 201 shown in FIG. 24 according to an implementation.

As shown in FIG. 29a, the gasket 201 may include a plate portion 2011, a first flange 2012, and a second flange 2013. For example, the plate portion 2011 is approximately in a disc shape. The plate portion 2011 has a top surface 2014 and a bottom surface 2015 that face away from each other. The plate portion 2011 has a first through hole 2016, and an axial direction of the first through hole 2016 may be the same as an axial direction of the plate portion 2011.

For example, the first flange 2012 is fastened to the top surface 2014 of the plate portion 2011. The first flange 2012 is arranged around the first through hole 2016. The first flange 2012 is approximately in a ring shape, and a diameter of the ring shape may be less than a diameter of the plate portion 2011.

For example, the second flange 2013 may be fastened to the bottom surface 2015 of the plate portion 2011. The second flange 2013 may be arranged around the first through hole 2016. Alternatively, the second flange 2013 may be in a ring shape, and a diameter of the ring shape may be less than the diameter of the plate portion 2011.

For example, a diameter of the first flange 2012 may be less than a diameter of the second flange 2013. In this way, during assembly of the gasket 201, the top surface and the bottom surface of the plate portion 2011 may be distinguished based on diameters of the two structures, to determine a mounting direction. In another implementation, a diameter of the first flange 2012 may alternatively be greater than a diameter of the second flange 2013, or a diameter of the first flange 2012 may be equal to a diameter of the second flange 2013.

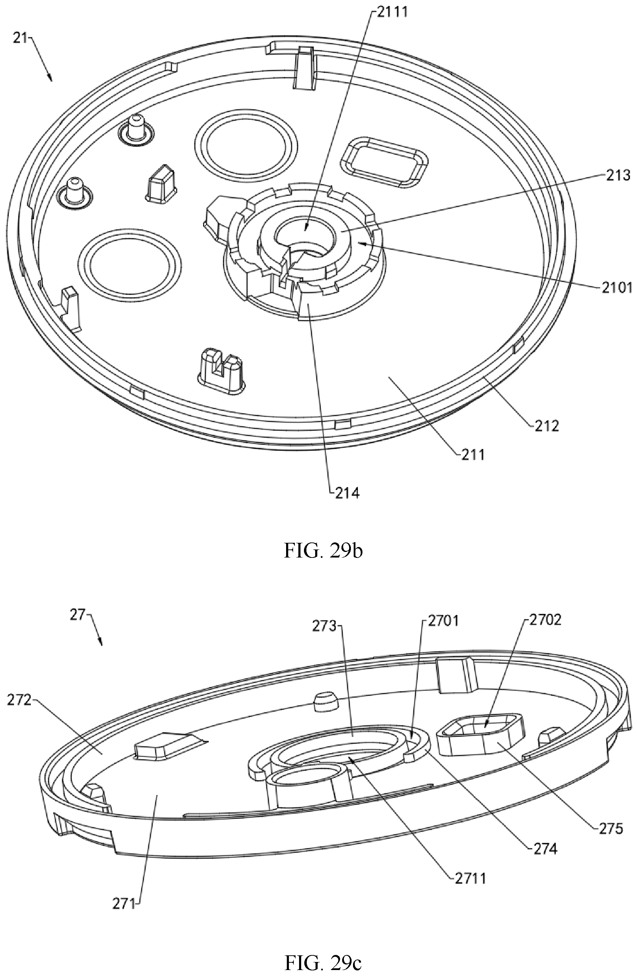

FIG. 29b is a diagram of a structure of the upper cover 21 shown in FIG. 24 according to an implementation.

As shown in FIG. 29b, the upper cover 21 may include an upper cover plate 211 and a first side plate 212. The first side plate 212 of the upper cover 21 is connected to a periphery of the upper cover plate 211 of the upper cover 21. For example, the upper cover plate 211 may be provided with a second through hole 2111, and the second through hole 2111 passes through the upper cover plate 211. The upper cover 21 may further include a fifth limiting boss 213 and a sixth limiting boss 214. Both the fifth limiting boss 213 and the sixth limiting boss 214 may be fastened to a side of the upper cover plate 211 of the upper cover 21. The fifth limiting boss 213, the sixth limiting boss 214, and the first side plate 212 of the upper cover 21 are fastened to a same side of the upper cover plate 211 of the upper cover 21. For example, the fifth limiting boss 213 may be in a circular shape or a ring shape. The sixth limiting boss 214 is located on an outer side of the fifth limiting boss 213, and surrounds one side of the fifth limiting boss 213. The upper cover plate 211, the fifth limiting boss 213, and the sixth limiting boss 214 of the upper cover 21 may form a first limiting groove 2101.

FIG. 29c is a diagram of a structure of the lower cover 27 shown in FIG. 24 according to an implementation.

As shown in FIG. 29c, the lower cover 27 may include a lower cover plate 271 and a second side plate 272. The second side plate 272 of the lower cover 27 is connected to a periphery of the lower cover plate 271 of the lower cover 27. For example, the lower cover plate 271 of the lower cover 27 may be provided with a third through hole 2711, and the third through hole 2711 passes through the lower cover plate 271 of the lower cover 27. The lower cover 27 may further include a seventh limiting boss 273 and an eighth limiting boss 274. Both the seventh limiting boss 273 and the eighth limiting boss 274 may be fastened to a side, facing the second side plate 272 of the lower cover 27, of the bottom plate 32. For example, the seventh limiting boss 273 may be in a circular shape or a ring shape. The eighth limiting boss 274 is located on an outer side of the seventh limiting boss 273, and surrounds one side of the seventh limiting boss 273. The seventh limiting boss 273 and the eighth limiting boss 274 may form a second limiting groove 2701.

For example, the lower cover 27 may further include a limiting plate 275. The limiting plate 275 is fastened to the lower cover plate 271 of the lower cover 27. The limiting plate 275 and the second side plate 272 of the lower cover 27 are fastened to a same side of the upper cover plate 211 of the upper cover 21. The limiting plate 275 and the lower cover plate 271 of the lower cover 27 may enclose second space 2702. The second space 2702 may be used to accommodate the switch magnet 257 (as shown in FIG. 27).

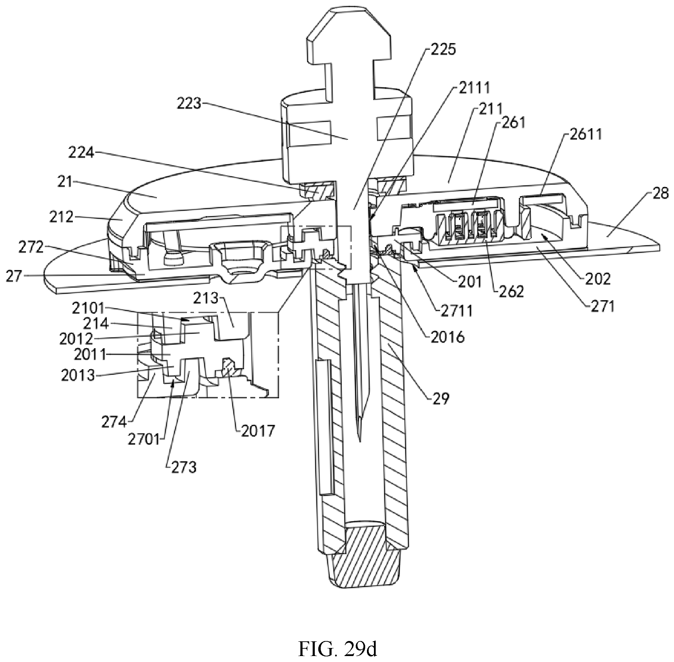

FIG. 29d is a partial cross-sectional view of the sensor assembly 20 shown in FIG. 23 along G-G according to an implementation.

As shown in FIG. 29d, the first side plate 212 of the upper cover 21 may be fastened to the second side plate 272 of the lower cover 27, to enclose mounting space 202. The electrical connection assembly 26 and the battery assembly 25 may be disposed in the mounting space 202. It can be understood that the upper cover 21 and the lower cover 27 may be configured to protect a device in the mounting space 202.

For example, the circuit board 261 of the electrical connection assembly 26 is fastened to the upper cover plate 211 of the upper cover 21. The first surface 2611 of the circuit board 261 may be fastened to the upper cover plate 211 of the upper cover 21. For example, the circuit board 261 may be fastened to the upper cover plate 211 of the upper cover 21 by using adhesive.

In some implementations, the bonding member 28 may be fastened to a side, away from the upper cover 21, of the lower cover 27. For example, the bonding member 28 may be fastened to the lower cover plate 271 of the lower cover 27. The bonding member 28 may be attached to the organism 30 when the sensor assembly 20 abuts against the organism 30, to bond the sensor assembly 20 to a skin surface of the organism 30. For example, the bonding member 28 may be approximately in a sheet-like shape. For example, the bonding member 28 may be medical adhesive, and the bonding member 28 can maintain stable adhesion for 14 days or more.

In some implementations, the guide portion 225 of the needle holder 22 may penetrate the lower cover 27 of the sensor assembly 20. For example, a part of the guide portion 225 of the needle holder 22 may enter the mounting space 202 through the second through hole 2111, and then extend out of the mounting space 202 through the third through hole 2711 of the lower cover 27. An end, provided with threads, of the guide portion 225 extends out of the lower cover 27 and is connected to the sealed cartridge 29.

In some implementations, the gasket 201 is fastened between the upper cover 21 and the lower cover 27. The first through hole of the gasket 201 is in communication with the second through hole 2111 of the upper cover 21 and the third through hole 2711 of the lower cover 27. The guide portion 225 of the needle holder 22 may extend out of the lower cover 27 through the first through hole, the second through hole 2111, and the third through hole 2711. For example, the first flange 2012 of the gasket 201 is located in the first limiting groove 5212101 of the upper cover 21, and the second flange 2013 is located in the second limiting groove 5212701 of the lower cover 27. It can be understood that the gasket 201 is intended to form a sterile package through fitting with another mechanical part, to seal a part of the sensor 24 that is implanted under skin.

In some implementations, the sealing portion 224 of the needle holder 22 may abut between the limiting portion 223 of the needle holder 22 and the upper cover plate 211 of the upper cover 21. The sealing portion 224 may be used for sealing between the needle holder 22 and the upper cover 21. For example, the sealing portion 224 may be a silicone ring. The silicone ring may be fastened to the limiting portion 223 of the needle holder 22 through bonding by using adhesive.

In some implementations, the gasket 201 may further include a sealing ring 2017. The sealing ring 2017 may be fastened to a side, facing the second flange 2013, of the plate portion 2011 of the gasket 201, and is arranged around the first through hole. The sealing ring 2017 may be located on an inner side of the second flange 2013. The sealing ring 2017 may be exposed from the lower cover 27 through the third through hole 2711 of the lower cover 27, and abut between the plate portion 2011 of the gasket 201 and the sealed cartridge 29. The sealing ring 2017 may be configured to form a sealed dust-proof structure with the sealed cartridge 29, to implement sterilization. In another implementation, the sealing ring 2017 may alternatively be fastened to the sealed cartridge 29.

For example, the sensor 24, the gasket 201, and the upper cover 21 may be sealed through glue dispensing. A sealing interface at a neck (at a position of the fastening portion 242) of the sensor 24 is formed by the upper cover 21, the gasket 201, and glue.

For example, the gasket 201, the upper cover 21, and the lower cover 27 may be fastened and sealed through glue dispensing.

For example, the first side plate 212 of the upper cover 21 and the second side plate 272 of the lower cover 27 may also be fastened and sealed through glue dispensing.

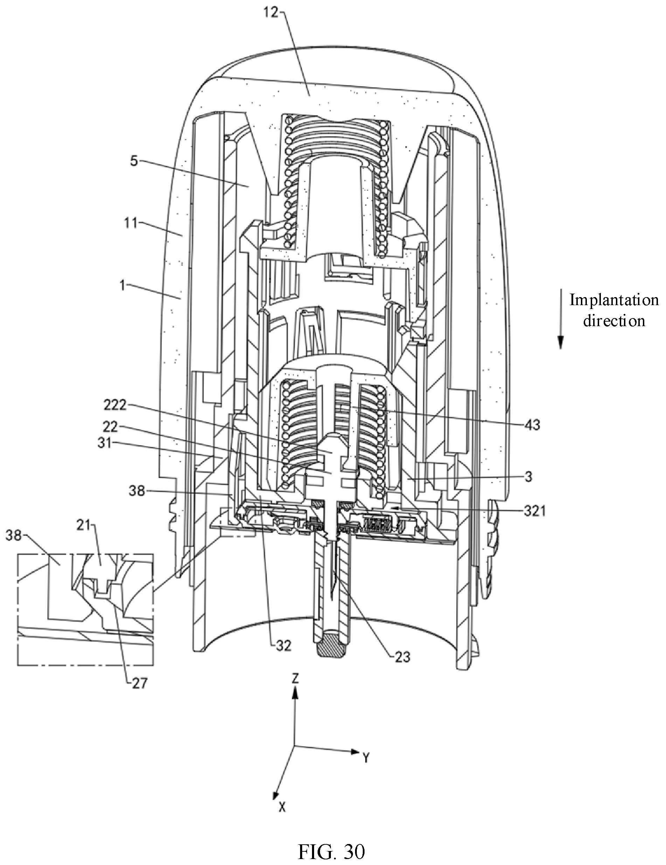

FIG. 30 is a partial cross-sectional view of the physiological sign monitoring apparatus 100 shown in FIG. 2 along F-F according to an implementation.

As shown in FIG. 30, the sensor assembly 20 may be fastened to the support 3 and located on a side, away from the top wall 12 of the lower housing 1, of the support 3. An axial direction of the sensor assembly 20 may coincide with the axial direction of the support 3. For example, the sensor assembly 20 may be mounted in the second accommodation space 302 of the support 3. The upper cover 21 of the sensor assembly 20 is located between the lower cover 27 and the bottom plate 32 of the support 3.

In some implementations, the hook of the fifth resilient arm 38 of the support 3 may snap into the lower cover 27 of the sensor assembly 20. In the axial direction, the hook of the fifth resilient arm 38 of the support 3 is at least partially located on the side, away from the upper cover 21, of the lower cover 27 of the sensor assembly 20. It can be understood that the hook of the fifth resilient arm 38 of the support 3 may be configured to perform position limiting for the sensor assembly 20 in the axial direction, to reduce a risk that the sensor assembly 20 is detached in the axial direction before being deployed. For example, when an upper housing 81 is removed, a second magnet 83 of the housing cap assembly 8 has an attractive force for the switch magnet 257 of the sensor assembly 20. The fifth resilient arm 38 is disposed to reduce a risk that the sensor assembly 20 is attracted by the attractive force of the second magnet 83 and detached.

It can be understood that, before the sensor assembly 20 is deployed, the side plate 31 of the support 3 abuts against the fifth resilient arm 38, so that the fifth resilient arm 38 is in a clamped state relative to the sensor assembly 20.

In some implementations, the needle holder 22 snaps into the needle retraction holder 4. The needle holder 22 may extend to the needle retraction holder 4 through the mounting hole 321 of the support 3. The hook of the first cantilever 43 snaps into the snap-fit portion 222 of the needle holder 22, so that the needle holder 22 can move synchronously with the needle retraction holder 4. When there are a plurality of first cantilevers 43, the needle holder 22 in snap-fit can be subject to uniform forces. When the support 3 moves in the implantation direction, the support 3 may push the sensor assembly 20 to also move in the implantation direction, so that the bonding member 28 of the sensor assembly 20 can be attached to the organism. In addition, the support 3 pushes the needle holder 22 toward the organism through the needle retraction holder 4, so that the puncture needle 23 can puncture into the organism. When the needle retraction holder 4 moves in a direction opposite to the implantation direction, the first cantilever 43 drives, through the needle holder 22, the puncture needle 23 to move in the direction opposite to the implantation direction, so that the puncture needle 23 is detached from the organism.

In some embodiments, a center of the mounting hole 321 of the support 3 is on or close to an axis of the physiological sign monitoring apparatus 100, and the plurality of first cantilevers 43 are arranged around the axis of the physiological sign monitoring apparatus 100. In this way, when the needle holder 22 of the sensor assembly 20 passes through the mounting hole 321 and snaps into the first cantilever 43, an extension direction of the puncture needle 23 is on or close to the axis of the physiological sign monitoring apparatus 100. Therefore, when the support 3 pushes the sensor assembly 20 to move in the implantation direction, the puncture needle 23 is subject to a more concentrated force. This helps alleviate pain of the user during puncturing.

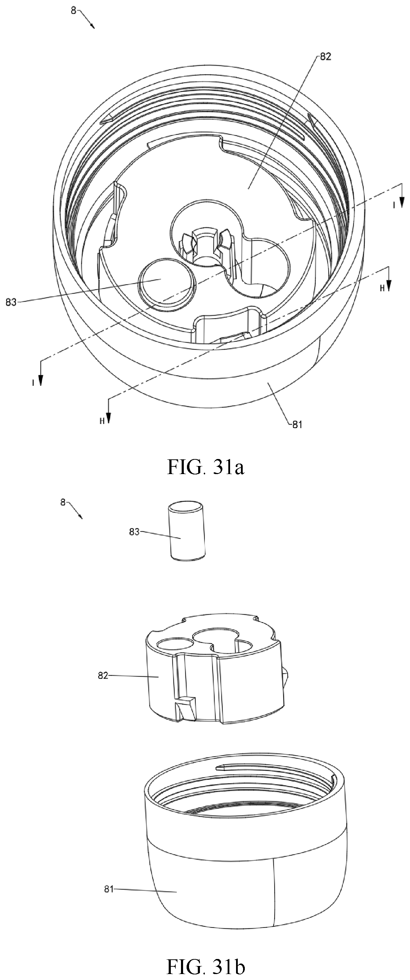

FIG. 31a is a diagram of a structure of the housing cap assembly 8 shown in FIG. 3 according to an implementation. FIG. 31b is a schematic exploded view of the housing cap assembly 8 shown in FIG. 30 according to an implementation.

As shown in FIG. 31a and FIG. 31b, the housing cap assembly 8 may include the upper housing 81, a drying member 82, and the second magnet 83. For example, the drying member 82 may be made of a material with an adsorption function. The drying member 82 is configured to absorb moisture, so that humidity in the physiological sign monitoring apparatus 100 remains within a proper range.

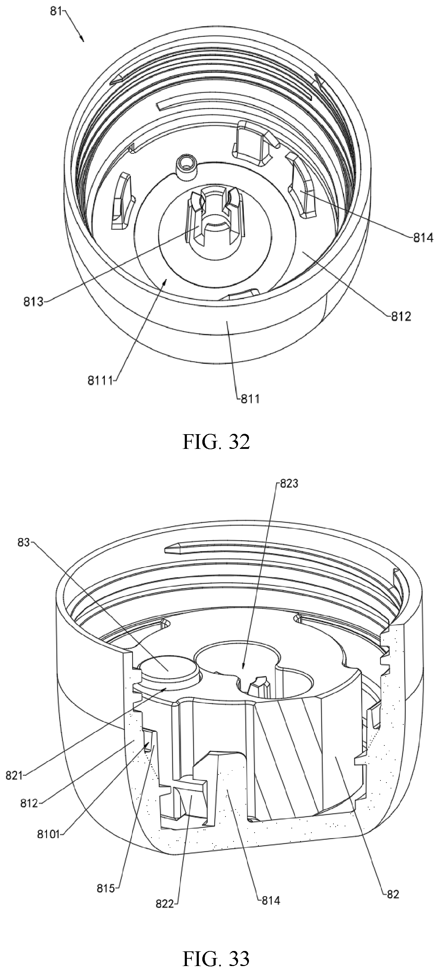

FIG. 32 is a diagram of a structure of the upper housing 81 shown in FIG. 31b according to an implementation.

As shown in FIG. 32, the upper housing 81 may include a bottom wall 811, a side wall 812, a pawl 813, and a limiting member 814. The side wall 812 of the upper housing 81 is connected around a periphery of the bottom wall 811 of the upper housing 81. The pawl 813 and the side wall 812 of the upper housing 81 are fastened to a same side of the bottom wall 811 of the upper housing 81. The pawl 813 is located on an inner side of the side wall 812 of the upper housing 81. The upper housing 81 may be approximately in a cylindrical shape.

For example, one end of the pawl 813 may be fastened to the bottom wall 811 of the upper housing 81, and the other end, away from the bottom wall 811 of the upper housing 81, of the pawl 813 is provided with a hook. The hook of the pawl 813 may be formed by a smooth protrusion from one side to another side. There may be one or more pawls 813, and a plurality of pawls 813 are spaced apart around an axis of the upper housing 81. For example, there are two pawls 813, and extension directions of the two pawls 813 are the same.

For example, the limiting member 814 may be located on an outer side of the pawl 813 and spaced away from the pawl 813. One end of the limiting member 814 is fastened to the bottom wall 811 of the upper housing 81. There may be one or more limiting members 814, and a plurality of limiting members 814 are spaced apart around the axis of the upper housing 81. The other end, away from the upper housing 81, of the limiting member 814 is provided with a hook. The hook may face the bottom wall 811. For example, there are four limiting members 814.

In some implementations, the upper housing 81 may be an integrally formed mechanical part.

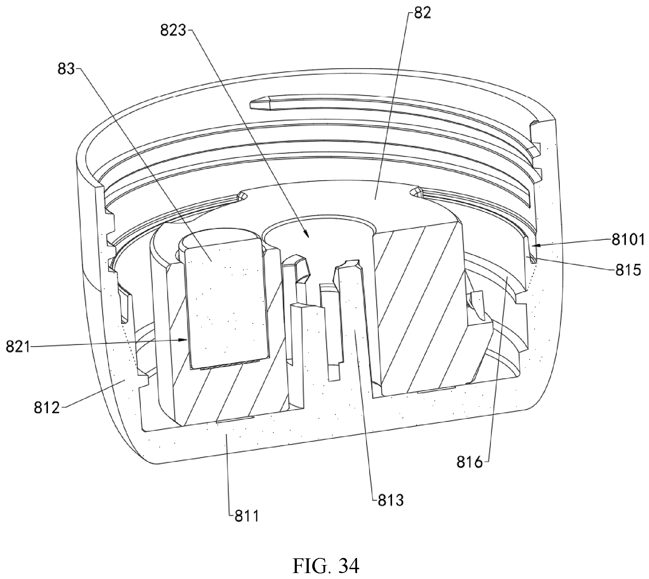

FIG. 33 is a cross-sectional view of the housing cap assembly 8 shown in FIG. 31a along H-H according to an implementation. FIG. 34 is a cross-sectional view of the housing cap assembly 8 shown in FIG. 31a along I-I according to an implementation.

As shown in FIG. 33 and FIG. 34, the drying member 82 may be provided with a first mounting slot 821. An opening of the first mounting slot 821 may face a side away from the bottom wall 811 of the upper housing 81. The second magnet 83 may be mounted in the first mounting slot 821.

In some implementations, the drying member 82 may snap into the upper housing 81. For example, a side surface of the drying member 82 may be provided with a boss 822. The hook of the limiting member 814 may snap into the boss 822 of the drying member 82. In an axial direction of the upper housing 81, the boss 822 may be partially located between the hook of the limiting member 814 and the bottom wall 811 of the upper housing 81. This can prevent the drying member 82 from sliding in the axial direction of the upper housing 81.

In some implementations, the drying member 82 may alternatively be fastened to the upper housing 81 by using adhesive. As shown in FIG. 32, the bottom wall 811 of the upper housing 81 may be provided with an adhesive groove 8111, and an opening 101 of the adhesive groove 8111 faces the side wall 812 of the upper housing 81. The adhesive groove 8111 may be configured to accommodate adhesive. When the drying member 82 is fastened to the upper housing 81 by using adhesive, there may alternatively be an adhesive layer between the drying member 82 and the bottom wall 811 of the upper housing 81.

It can be understood that the drying member 82 may be connected to the upper housing 81 in both or either of the following manners: snap-fit through the limiting member 814, and using adhesive.

In some implementations, the drying member 82 may be provided with a second mounting slot 823, and the pawl 813 of the upper housing 81 may be located in the second mounting slot 823.

In some implementations, engaging threads may be provided on an inner wall surface of the side wall 812 of the upper housing 81. The engaging threads are located on a side, away from the bottom wall 811 of the upper housing 81, of the side wall 812 of the upper housing 81. The engaging threads may be used for fastening the lower housing 1. The following provides detailed descriptions with reference to the accompanying drawings.

In some implementations, the upper housing 81 may further include a sealing strip 815. The sealing strip 815 may be fastened to the inner wall surface of the side wall 812 of the upper housing 81 (in FIG. 33, the sealing strip 815 and the side wall 812 are distinguished by a dashed line), and enclose a sealing groove 8101 together with the side wall 812 of the upper housing 81. An opening 101 of the sealing groove 8101 faces away from the bottom wall 811 of the upper housing 81. For example, the sealing strip 815 and the side wall 812 of the upper housing 81 may be an integrally formed mechanical part.

In some implementations, the upper housing 81 may further include a second stop structure 816. The second stop structure 816 may be fastened to the inner wall surface of the side wall 812 of the upper housing 81. For example, the second stop structure 816 may be threads. A thread pitch of the threads of the second stop structure 816 is equal to a thread pitch of the engaging threads.

For example, in a direction from the bottom wall 811 of the upper housing 81 to the side wall 812 of the upper housing 81, the second stop structure 816, the sealing strip 815, and the engaging threads may be sequentially arranged.

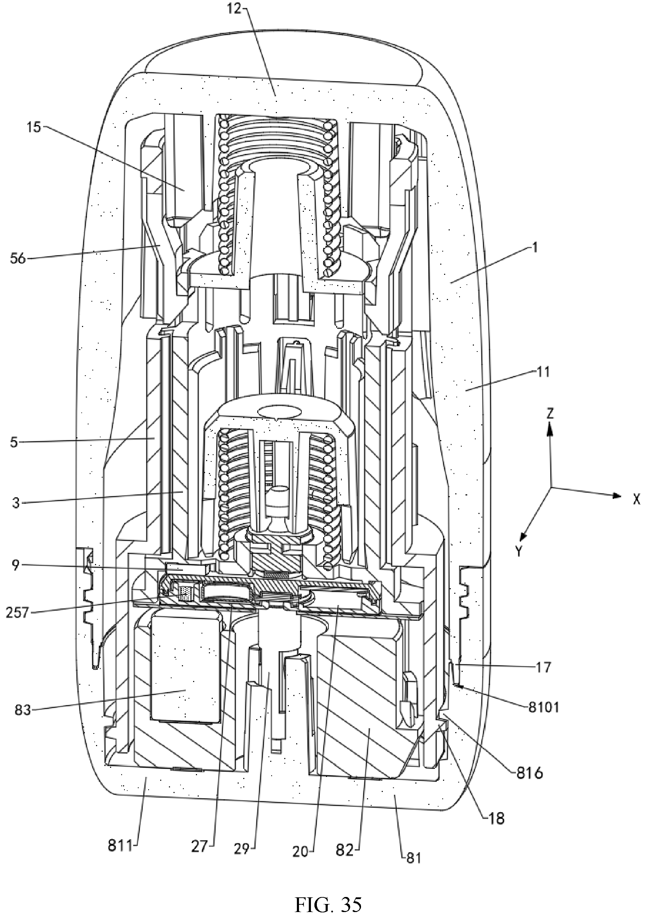

FIG. 35 is a partial cross-sectional view of the physiological sign monitoring apparatus 100 shown in FIG. 2 along B-B according to an implementation.

As shown in FIG. 27 and FIG. 35, the upper housing 81 is detachably connected to the lower housing 1. The upper housing 81 may cover the opening 101 of the lower housing 1. The upper housing 81 and the lower housing 1 may constitute a housing of the physiological sign monitoring apparatus 100. It can be understood that FIG. 35 shows an example in which the upper housing 81 and the lower housing 1 are detachable. In another embodiment, the upper housing 81 and the lower housing 1 may alternatively be integrated and non-detachable.

As shown in FIG. 34 and FIG. 35, the upper housing 81 and the lower housing 1 may be fastened through the engaging threads. For example, an outer surface at an end, away from the bottom wall 811 of the lower housing 1, of the side wall 11 of the lower housing 1 may be provided with threads. The threads of the lower housing 1 may match the press-fit threads of the upper housing 81. The upper housing 81 is fastened to the lower housing 1 through fitting between the threads.

As shown in FIG. 35, in the axial direction of the physiological sign monitoring apparatus 100, the first magnet 9, the switch magnet 257, and the second magnet 83 are arranged opposite to each other. For example, the first magnet 9 may be located between the switch magnet 257 and the support 3. The first magnet 9 is configured to provide an attractive force or a repulsive force for the switch magnet 257, so that the switch magnet 257 can be disconnected from the first negative electrode plate 254 and the first positive electrode plate 253. The second magnet 83 is configured to provide an attractive force or a repulsive force for the switch magnet 257, so that the switch magnet 257 can be disconnected from the first negative electrode plate 254 and the first positive electrode plate 253 (refer to FIG. 27). It can be understood that whether the first magnet 9 and the second magnet 83 have an attractive force or a repulsive force for the switch magnet 257 is determined based on a relative positional relationship between the switch magnet 257 and the first negative electrode plate 254 and the second positive electrode plate 255.

For example, the first negative electrode plate 254 and the first positive electrode plate 253 are located on a side, close to the first magnet 9, of the switch magnet. In this case, the first magnet 9 may be configured to provide a repulsive force for the switch magnet 257, so that the switch magnet 257 can be disconnected from the first negative electrode plate 254 and disconnected from the first positive electrode plate 253. The second magnet 83 may be configured to provide an attractive force for the switch magnet 257, so that the switch magnet 257 can be disconnected from the first negative electrode plate 254 and disconnected from the first positive electrode plate 253.

It can be understood that, with the first magnet 9, the switch magnet 257, and the second magnet 83 disposed, when the housing cap assembly 8 is fastened to the lower housing 1, the first magnet 9, the switch magnet 257, and the second magnet 83 are arranged opposite to each other, and the first magnet 9 and the second magnet 83 act. In this way, during storage of the physiological sign monitoring apparatus 100 (the physiological sign monitoring apparatus 100 is in an unused state), the switch magnet 257 can be disconnected from the first negative electrode plate 254 and disconnected from the first positive electrode plate 253, so that the sensor 24 can be de-energized. Through physical energization/de-energization, power consumption of the physiological sign monitoring apparatus 100 during storage is reduced, and product battery life is improved. When the physiological sign monitoring apparatus 100 is in use, the housing cap assembly 8 is removed. After the sensor assembly 20 is deployed, the support 3 also leaves tissue of the user. In this case, the first magnet 9 and the second magnet 83 do not act on the switch magnet 257, and there are attractive forces between the switch magnet 257 and the first negative electrode plate 254 and between the switch magnet 257 and the second positive electrode plate 255. In this way, the switch magnet 257 can be in contact with both the first negative electrode plate 254 and the second positive electrode plate 255 under the action of the attractive forces, and electrically connected to the first negative electrode plate 254 and the second positive electrode plate 255, to implement electrical conduction between the first battery 251 and the second battery 252, and energize the sensor 24. The battery assembly 25 may supply power to the sensor 24 and other electronic components on the circuit board 261, and the sensor assembly 20 may properly operate.

**In** another implementation, when an acting force of the first magnet 9 or the second magnet 83 for the switch magnet 257 meets a disconnection requirement, only either of the first magnet 9 and the second magnet 83 may alternatively be disposed. It can be understood that, when only the first magnet 9 or the second magnet 83 is disposed, a structure of the support 3 or the drying member 82 may change correspondingly. In comparison with a solution in which only either of the first magnet 9 and the second magnet 83 is disposed, both the first magnet 9 and the second magnet 83 are disposed to act on the switch magnet 257, to de-energize the sensor 24. The first magnet 9 and the second magnet 83 may apply a larger acting force to the switch magnet 257. This helps ensure de-energization of the sensor 24 when the physiological sign monitoring apparatus 100 is in the unused state.

**In** some implementations, the lower housing 1 may further include a sealing rib 17. The sealing rib 17 may be fastened to a side, away from the bottom wall 811 of the lower housing 1, of the side wall 11 of the lower housing 1. When the lower housing 1 is fastened to the upper housing 81, the sealing rib 17 is located in the sealing groove 8101 of the upper housing 81, and abuts against a wall surface of the sealing groove 8101. In other words, the sealing rib 17 and the sealing groove 8101 are in an interference fit to implement a sealing interface. It can be understood that the sealing rib 17 and the sealing groove 8101 are in an interference fit, so that a gap between the upper housing 81 and the lower housing 1 can be reduced, to reduce external water vapor that enters the physiological sign monitoring apparatus 100, and avoid impact on a function of the sensor assembly 20.

In some implementations, the upper housing 81 and the lower housing 1 may be made of a material with low water vapor permeability, for example, polypropylene (polypropylene, PP) or high-density polyethylene (High Density Polyethylene, HDPE).

It can be understood that a storage condition of the sensor 24 includes a specific requirement on humidity, and long-term storage needs to be performed in a dry environment. In this application, in the solution in which the sealing rib 17 and the sealing groove 8101 are in an interference fit to implement the sealing interface, the upper housing 81 and the lower housing 1 may be made of the material with low water vapor permeability, and the drying member 82 is disposed in the upper housing 81, the physiological sign monitoring apparatus 100 may have a dry storage environment. This facilitates long-term storage of the sensor assembly 20. The sensor assembly 20 in the physiological sign monitoring apparatus 100 in this application is not prone to a failure, and service life of the physiological sign monitoring apparatus 100 is long.

In another implementation, the upper housing 81 and the lower housing 1 may alternatively be sealed by using a sealing O-ring.

In some implementations, when the physiological sign monitoring apparatus 100 falls with the top (to be specific, an end at which the top wall 12 of the lower housing 1 is located) down, the lower housing 1 moves close to the upper housing 81, and the lower housing 1 and the base 5 are likely to move relative to each other. Consequently, the hook of the second resilient arm 56 is spread open by the toggle 15 of the lower housing 1, the hook is in a disengaged state inside, and the first elastic member 2 applies an abutting elastic force to the support 3. In this case, after the user removes the housing cap assembly 8, the support 3 is directly deployed together with the sensor assembly 20, and the product cannot be properly used.

In some implementations, the first stop structure 18 is in contact with the second stop structure 816. The second stop structure 816 and the first stop structure 18 may be arranged opposite to each other in the axial direction of the upper housing 81. The second stop structure 816 of the upper housing 81 is located on a side, away from the bottom wall 811 of the upper housing 81, of the first stop structure 18 of the base 5. In other words, the second stop structure 816 may be located on a side, close to the top wall 12 of the lower housing 1, of the first stop structure 18. It can be understood that the first stop structure 18 and the second stop structure 816 may serve as a stopper 91, configured to: when the physiological sign monitoring apparatus 100 is in the unused state, prevent the base 5 from moving relative to the lower housing 1 in the axial direction of the physiological sign monitoring apparatus 100. When the physiological sign monitoring apparatus 100 falls with the top facing down, the first stop structure 18 and the second stop structure 816 may serve as a latch for preventing triggering upon falling (the stopper 91), to limit large relative movement between the base 5 and the lower housing 1 in the axial direction, so that the physiological sign monitoring apparatus 100 is not disengaged or triggered upon falling or vibration.

In some implementations, that the first stop structure 18 is in contact with the second stop structure 816 may be understood as that the first stop structure 18 abuts against the second stop structure 816 when the physiological sign monitoring apparatus 100 falls with the top facing down. When no collision occurs, a small gap may exist between the first stop structure and the second stop structure 816 in the axial direction of the upper housing 81, to be used for assembly. For example, in the axial direction of the upper housing 81, the gap between the first stop structure and the second stop structure 816 may be less than a disengagement stroke. The disengagement stroke is a disengagement stroke by which the lower housing 1 moves relative to the base 5 in the axial direction of the upper housing 81 to deploy the support 3. For example, when the disengagement stroke is 5 mm, the gap between the first stop structure and the second stop structure 816 in the axial direction of the upper housing 81 may be less than 4 mm.

In some implementations, the first stop structure 18 and the second stop structure 816 may be threads that fit with each other. It can be understood that a threaded structure is simple with low preparation difficulty. The first stop structure 18 and the second stop structure 816 are designed to be of a threaded structure, to help reduce production costs and simplify a mechanism.

In some implementations, a thread pitch of threads of the first stop structure 18 is the same as a thread pitch of threads of the second stop structure 816. Both the thread pitch of the threads of the first stop structure 18 and the thread pitch of the threads of the second stop structure 816 may be the same as the thread pitch of the engaging threads. In this way, when the upper housing 81 is tightened with the lower housing 1, the first stop structure 18 and the second stop structure 816 can be in good contact and fit. For example, the threads of the first stop structure 18 may be a continuous turn of threads, or may be several sections of threads that are spaced apart. When the first stop structure 18 has several sections of threads that are spaced apart, thread pitches of the several sections of threads are the same.

In some implementations, the sealed cartridge 29 may abut against the bottom wall 811 of the upper housing 81. It can be understood that, when the physiological sign monitoring apparatus 100 falls with the bottom(to be specific, an end at which the bottom wall 811 of the upper housing 81 is located) facing down, the upper housing 81 has a trend of moving toward the lower housing 1, and the sealed cartridge 29 may abut against the bottom wall 811 of the upper housing 81, to limit a moving distance of the upper housing 81 in the axial direction, prevent the upper housing 81 from abutting against the base 5, and avoid disengagement of the hook of the second resilient arm 56.

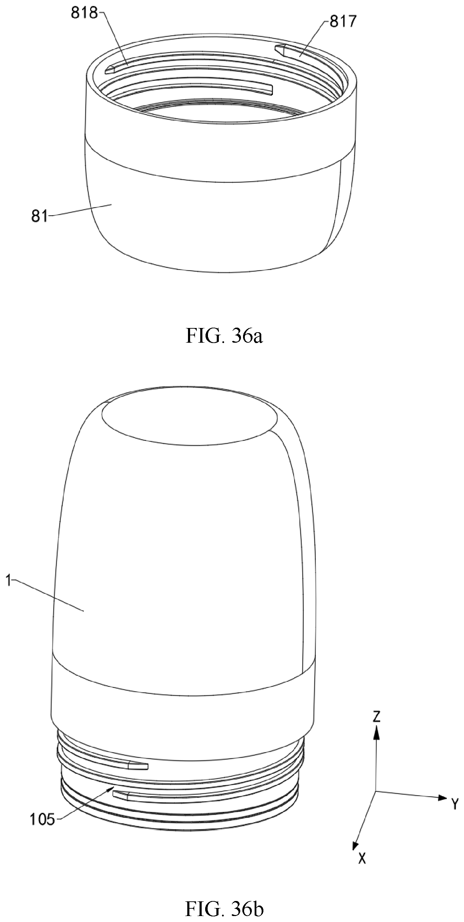

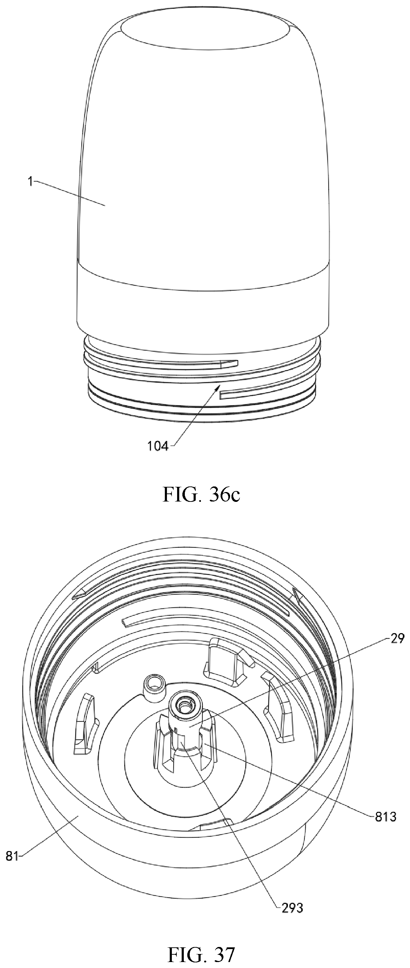

FIG. 36a is a diagram of the upper housing 81 according to an implementation. FIG. 36b is a diagram of the lower housing 1 according to an implementation. FIG. 36c is a diagram of the lower housing 1 shown in FIG. 36b from another perspective according to an implementation.

As shown in FIG. 35, FIG. 36a, FIG. 36b, and FIG. 36c, the engaging threads of the upper housing 81 may be multi-start threads. This can reduce a quantity of turns of screwing to be performed by the user. A starting position of any thread of the multi-start threads may be a starting position of screwing.

It can be understood that, in some embodiments of this application, in the axial direction of the physiological sign monitoring apparatus 100, the switch magnet 257 and the second magnet 83 need to be arranged opposite to each other, to ensure that a magnetic attractive force takes effect, and the product can be properly powered on or off. In this case, positions of the upper housing 81 and the lower housing 1 need to be fixed after they are tightened, and a fool-proofing design needs to be applied to the positions of the upper housing 81 and the lower housing 1 after they are tightened. The engaging threads of the upper housing 81 and the lower housing 1 need to have a fool-proofing function. To be specific, each time after tightening, the upper housing 81 is at a fixed position, to ensure that the second magnet 83 on a bottle cap is aligned with the switch magnet 257. In another implementation, when a Hall switch is used as a power-on/off solution, if the Hall magnet is fastened to the upper housing 81, a position of the upper housing 81 also needs to be fixed.

In some implementations, two multi-start threads with different starting heights may be used for a fool-proofing design. For example, two-start threads are used as an example. Two threads on the lower housing 1 are designed to have different starting heights, and form a coarse-pitch thread engagement opening 104 and a fine-pitch thread engagement opening 105. The engaging threads of the upper housing 81 may include a coarse-pitch thread 817 and a fine-pitch thread 818. The coarse-pitch thread 817 cannot be engaged into the fine-pitch thread engagement opening 105, so that a fool-proofing function is achieved. This ensures that a position at which the upper housing 81 is located after being tightened is fixed in the case of multi-start threads, to ensure that the second magnet 83 of the upper housing 81 is at a fixed position and is arranged opposite to the switch magnet 257.

It can be understood that, compared with single-start threads, the multi-start thread structure has higher strength, and a starting height of the multi-start threads may be designed to achieve a fool-proofing design.

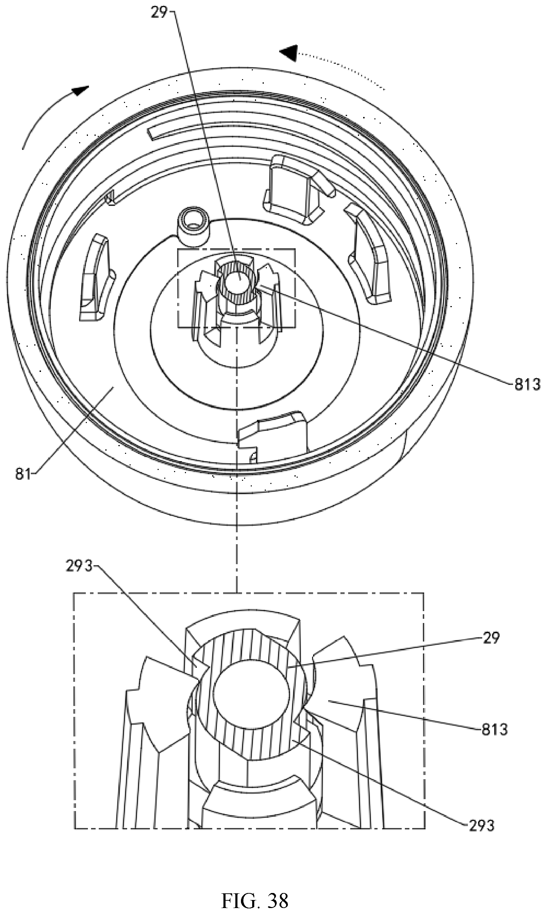

FIG. 37 is a diagram of assembly of the upper housing 81 and the sealed cartridge 29 shown in FIG. 35 according to an implementation. FIG. 38 is a partial schematic cross-sectional view of the physiological sign monitoring apparatus 100 shown in FIG. 2 along J-J according to an implementation.

As shown in FIG. 26, FIG. 37, and FIG. 38, the pawl 813 of the upper housing 81 fits with the ratchet wheel 293 of the sealed cartridge 29. A rotation direction of the engaging threads of the upper housing 81 is the same as a rotation direction of inner threads of the sealed cartridge 29 (threads used for the sealed cartridge 29 to connect the needle holder 22, as shown in FIG. 26). In a process in which the upper housing 81 is tightened (moves in a direction indicated by a dashed line with an arrow in FIG. 37) relative to the lower housing 1, the pawl 813 is slidably connected to the ratchet wheel 293. To be specific, in the process in which the upper housing 81 is tightened relative to the lower housing 1, the pawl 813 does not push the ratchet wheel 2933 to move. This prevents the sealed cartridge 29 from loosening relative to the needle holder 22 during assembly of the upper housing 81 and the lower housing 1. In a process in which the upper housing 81 is detached from (moves in a direction indicated by a solid line with an arrow in FIG. 37) the lower housing 1, the pawl 813 snaps into the ratchet wheel 293 and pushes the sealed cartridge 29 to rotate. Because the sealed cartridge 29 is threaded with the needle holder 22 (as shown in FIG. 26), the sealed cartridge 29 is screwed off relative to the needle holder 22, so that the sealed cartridge 29 is detached from the needle holder 22. A quantity of turns of inner threads of the upper housing 81 may be greater than a quantity of turns of the inner threads of the sealed cartridge 29, so that the sealed cartridge 29 can be detached from the needle holder 22 before the upper housing 81 is detached from the lower housing 1.

In this embodiment, the upper housing 81 is threaded with the lower housing 1, the sealed cartridge 29 is threaded with the needle holder 22, and the pawl 813 of the upper housing 81 fits with the ratchet wheel 293 of the sealed cartridge 29. When the upper housing 81 is detached from the lower housing 1, the sealed cartridge 29 may be pushed to detach from the needle holder 22, so that the puncture needle 23 and the sensor 24 are exposed. This helps simplify operation steps of the physiological sign monitoring apparatus 100, and improve user experience.

With reference to the accompanying drawings, the following describes fitting between components of the physiological sign monitoring apparatus 100 in movement during use.

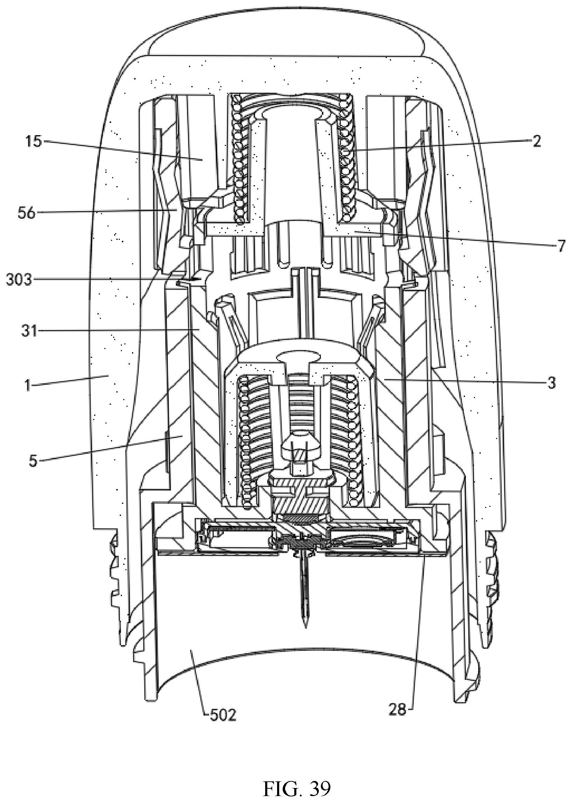

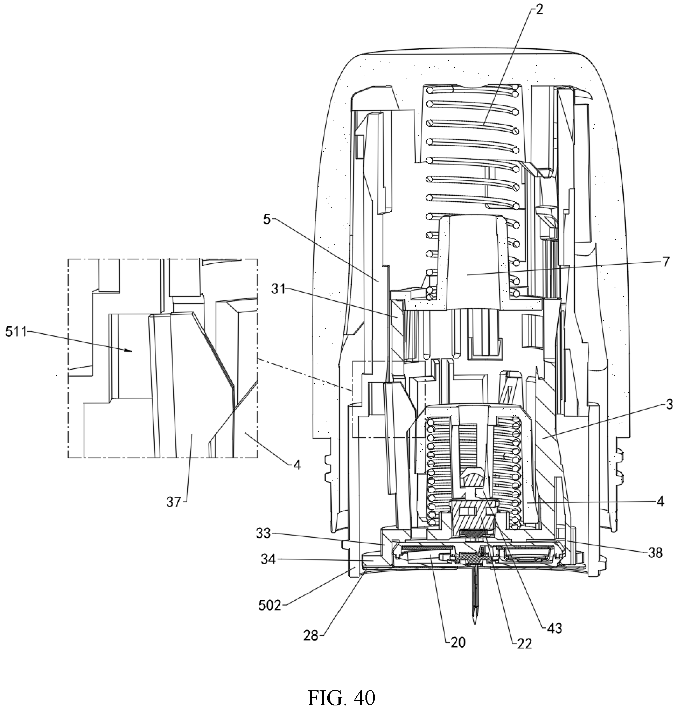

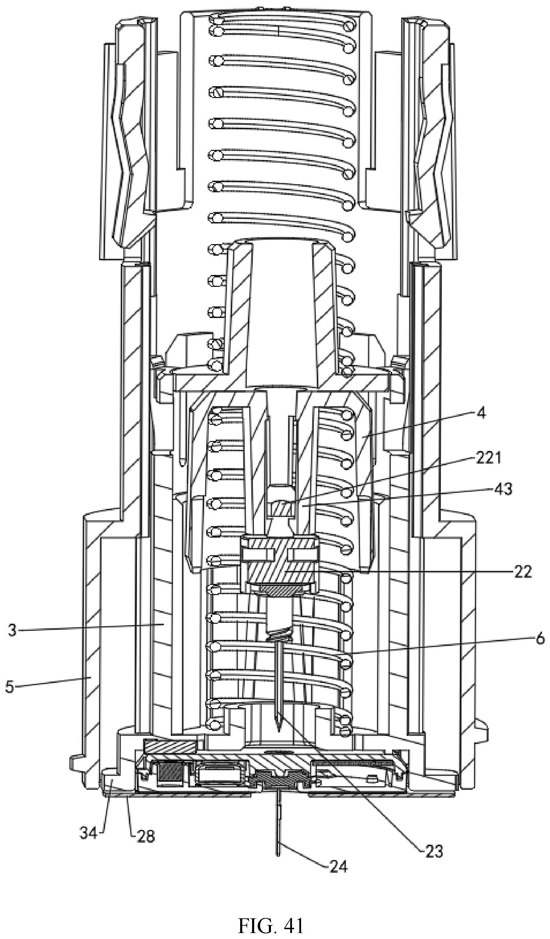

FIG. 39 is a partial cross-sectional view of the physiological sign monitoring apparatus 100 shown in FIG. 2 along B-B during use according to an implementation. FIG. 40 is a partial cross-sectional view of the physiological sign monitoring apparatus 100 shown in FIG. 2 along B-B during use according to an implementation. FIG. 41 is a partial cross-sectional view of the physiological sign monitoring apparatus 100 shown in FIG. 2 along B-B during use according to an implementation.

As shown in FIG. 35, FIG. 39, FIG. 40, and FIG. 41, FIG. 35 is a diagram of the physiological sign monitoring apparatus 100 before it is used, in other words, a diagram of the physiological sign monitoring apparatus 100 in the unused state (a storage state). FIG. 39 to FIG. 41 are diagrams of the physiological sign monitoring apparatus 100 is in an in-use state at different moments. FIG. 39 is a diagram of the support 3 at a trigger position. FIG. 40 is a diagram of a trigger position of the needle retraction holder 4. FIG. 41 is a diagram of a needle retraction action after deployment is completed.

As shown in FIG. 35 and FIG. 39, when the physiological sign monitoring apparatus 100 needs to be used, the housing cap assembly 8 is removed, the sealed cartridge 29 is removed together with the housing cap assembly 8, the abutting end 502 of the base 5 abuts against tissue of the user, and the base 5 remains stationary relative to the tissue of the user. After downward pressure is applied to the lower housing 1, the lower housing 1 moves down, and the toggle 15 of the lower housing 1 pushes away the second resilient arm 56 of the base 5. After the hook of the second resilient arm 56 is spread outward by the toggle 15 of the lower housing 1, the hook exits the first snap-fit space 303 of the support 3, and the support 3 is released.

In this embodiment, through fitting between the toggle 15 and the second resilient arm 56, position limiting performed by the base 5 for the support 3 is released, so that the support 3 is detached from the base 5 under an elastic force of the first elastic member 2. When the lower housing 1 moves in a downward direction, the toggle 15 gradually pushes a second cantilever to move toward an outer side of the side plate 31 of the support 3, and compresses the first elastic member 2. When the hook of the second resilient arm 56 is detached from the first snap-fit space 303, the support 3 moves in the downward direction under an elastic force of the first elastic member 2. A position limiting mechanism for the support 3 of the physiological sign monitoring apparatus 100 and a release mechanism thereof are of a simple and compact structure, so that transmission between the lower housing 1 and the base 5 is fast.

As shown in FIG. 39 and FIG. 40, the first elastic member 2 abuts against the lower housing 1 and the spring seat 7, and the spring seat 7 is fastened to the support 3. After the support 3 is released, the first elastic member 2 is also released. The first elastic member 2 may apply a downward pushing force to the spring seat 7 to push the spring seat 7 and the support 3 to move down. The sensor assembly 20 is fastened to the support 3. Therefore, the support 3 drives the sensor assembly 20 to move close to the tissue of the user, the puncture needle 23 of the sensor assembly 20 punctures into the tissue of the user, and the bonding member 28 of the sensor assembly 20 is tightly attached to skin of the user.

For example, the flange 34 of the support 3 is located on a side, away from the abutting end 502 of the base 5, of the bonding member 28 of the sensor assembly 20. The flange 34 of the support 3 may be approximately flush with an end face, close to the abutting end 502, of the lower side plate 33 of the support 3. During deployment of the sensor assembly 20, the flange 34 may tightly press the bonding member 28 against the skin of the user. It can be understood that, in comparison with a technical solution in which no flange 34 is disposed, when the flange 34 is disposed, a connection area between the support 3 and the skin of the user can be increased, to alleviate pain of the user during use of the physiological sign monitoring apparatus 100, and improve user experience.

It can be understood that, in view of a tolerance of a preparation process, the flange 34 of the support 3 slightly protrudes from the end face, close to the abutting end 502, of the lower side plate 33 of the support 3.

As shown in FIG. 40 and FIG. 41, in a process in which the support 3 moves down, the inner side surface of the upper side wall 51 of the base 5 may be provided with a trigger slot 511, and an opening of the trigger slot 511 faces an inner side of the upper side wall 51 of the base 5. When the fourth resilient arm 37 of the support 3 moves to the trigger slot 511, the fourth resilient arm 37 is released and springs open, and the needle retraction holder 4 is freed from a constraint and is released. The second elastic member 6 may apply an upward abutting force to the needle retraction holder 4.

The first cantilever 43 of the needle retraction holder 4 snaps into the limiting fastener 221 of the needle holder 22 of the sensor assembly 20. When the needle retraction holder 4 is subject to an upward force, the first cantilever 43 of the needle retraction holder 4 may provide an upward pulling force for the needle holder 22. The needle retraction holder 4 and the needle holder 22 move up under a force of the second elastic member 6, so that the first cantilever 43 retracts from the tissue of the user together with the puncture needle 23, and the sensor 24 is left in the tissue of the user. In this way, a needle retraction action is completed. The upper cover 21, the battery assembly 25, the electrical connection assembly 26, the lower cover 27, the bonding member 28, and the gasket 201 of the sensor assembly 20 may constitute a propulsion assembly (as shown in FIG. 24). After the needle retraction action is completed, the propulsion assembly is left in the tissue of the user, and may be configured to transmit, to a terminal device, physiological target substance data of the user that is collected by the sensor 24.

After the support 3 moves to the abutting end 502 of the base 5, the fifth resilient arm 38 of the support 3 and the base 5 are spaced apart. In this case, the base 5 does not press against the fifth resilient arm 38, in other words, the fifth resilient arm 38 is released. After the needle retraction action is completed, the user removes the lower housing 1 and other devices, and the fifth resilient arm 38 is detached from the lower housing 1 of the sensor assembly 20 along with the removal action of the user, to release the sensor assembly 20.

For example, the first elastic member 2, the spring seat 7, and the support 3 may constitute a push mechanism of the physiological sign monitoring apparatus 100. The lower housing 1, the base 5, and the support 3 may constitute a trigger mechanism of the physiological sign monitoring apparatus 100. The needle retraction holder 4, the second elastic member 6, the support 3, the needle holder 22, and the puncture needle 23 may constitute a needle retraction mechanism of the physiological sign monitoring apparatus 100. The trigger mechanism may serve as a switch for using the physiological sign monitoring apparatus 100, to control whether to deploy the sensor 24. The push mechanism may be configured to push the sensor assembly 20, and the puncture needle 23 carries the sensor 24 into tissue of a human body. The needle retraction mechanism may be configured to implement a function of retracting the puncture needle 23 after the puncture needle 23 is pushed to puncture into skin.

It can be understood that, after the sensor assembly 20 is pushed into position, the push mechanism is subject to acting forces from two springs (the first elastic member 2 and the second elastic member 6). Because the springs have a characteristic of oscillating back and forth in the axial direction, the push mechanism also oscillates together with the springs. The oscillation causes severer damage to a skin wound after the implantation. A locking mechanism for preventing the push mechanism from retracting is needed to eliminate the oscillation caused by the springs, improve implantation experience, and reduce wound damage after the implantation.

In some implementations, the base 5 may also be a part of the needle retraction mechanism of the physiological sign monitoring apparatus 100.

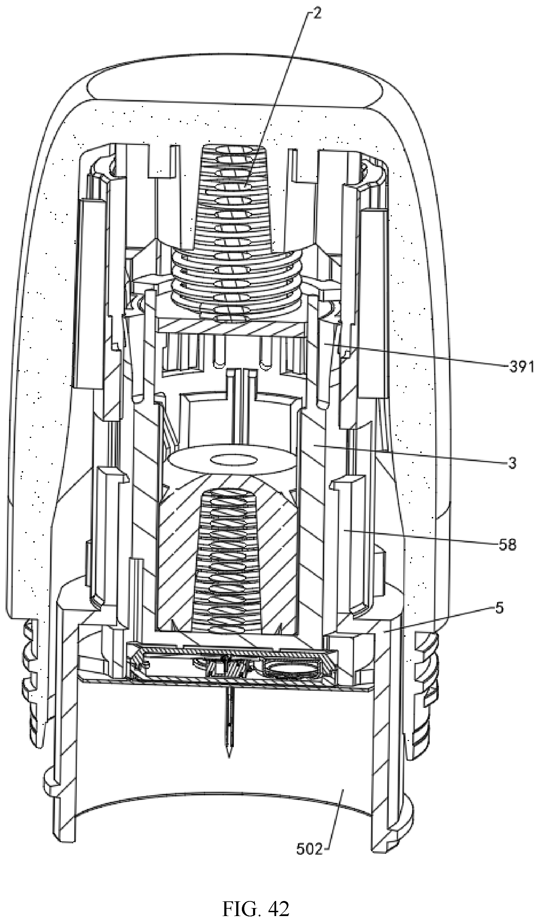

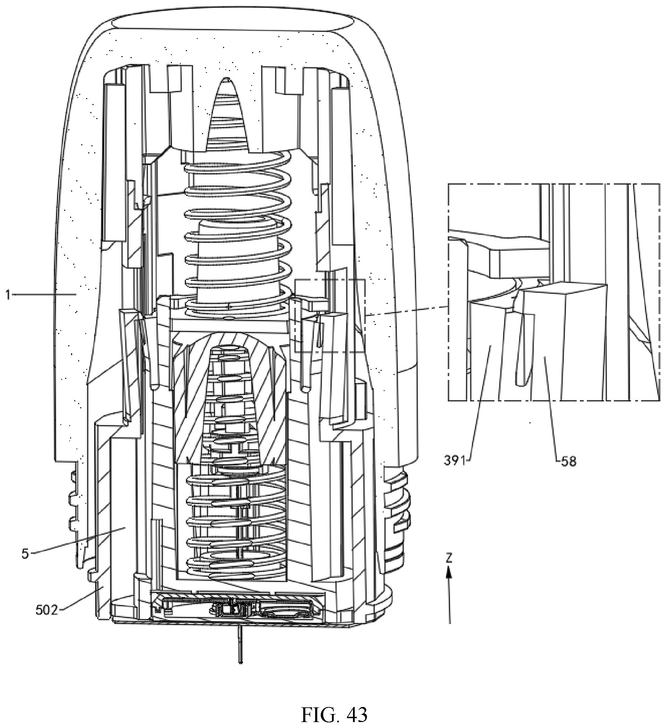

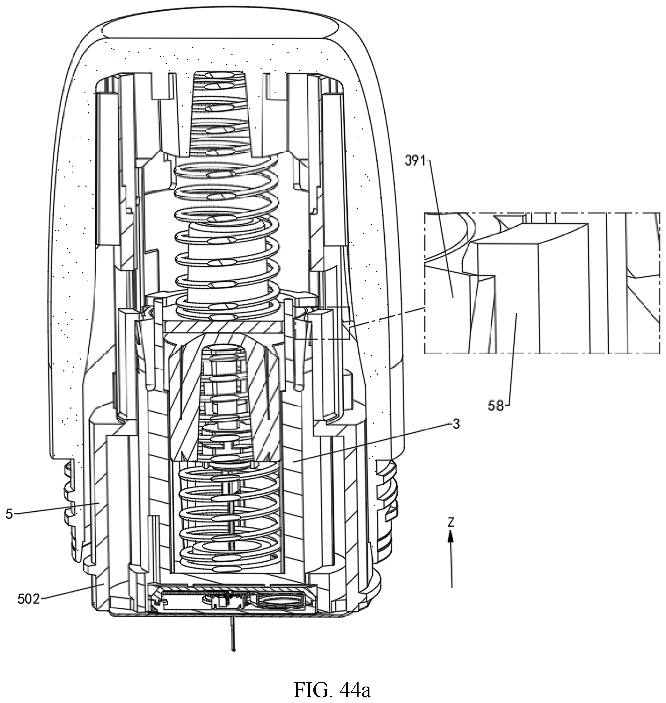

FIG. 42 is a partial cross-sectional view of the physiological sign monitoring apparatus 100 shown in FIG. 2 along K-K before use according to an implementation. FIG. 43 is a partial cross-sectional view of the physiological sign monitoring apparatus 100 shown in FIG. 2 along K-K during use according to an implementation. FIG. 44a is a partial cross-sectional view of the physiological sign monitoring apparatus 100 shown in FIG. 2 along K-K during use according to an implementation.

As shown in FIG. 42, before the physiological sign monitoring apparatus 100 is used, the fastening boss 39 of the support 3 may be located on a side, close to the first elastic member 2, of the seventh resilient arm 58 of the base 5.

As shown in FIG. 42 and FIG. 43, when the first elastic member 2 pushes the support 3 to move down, the hook of the seventh resilient arm 58 fits with an inclined side surface of the fastening boss 391, the fastening boss 391 pushes away the seventh resilient arm 58, and the hook of the seventh resilient arm 58 springs open.

As shown in FIG. 43 and FIG. 44a, when the support 3 reaches a tail end of a push position, the sensor assembly 20 abuts against the organism. In this case, the bonding member 28 of the sensor assembly 20 is in contact with skin of the user, and the hook of the seventh resilient arm 58 snaps into the fastening boss 391. For example, in the axial (Z-axis) direction, the hook of the seventh resilient arm 58 is located on a side, away from the abutting end 502 of the base 5, of the fastening boss 391. In this way, the hook of the seventh resilient arm 58 can perform position limiting for the support 3 in the axial direction, to prevent the support 3 from sliding in the axial direction. In addition, there are a plurality of seventh resilient arms 58, and the plurality of seventh resilient arms 58 are spaced apart around the axial direction. A quantity of seventh resilient arms 58 is the same as a quantity of fastening bosses 391, and the seventh resilient arms 58 and the fastening bosses are arranged in a one-to-one correspondence. In this embodiment, two seventh resilient arms 58 and two fastening bosses 391 are shown. The plurality of seventh resilient arms 58 fit with each other to perform position limiting for the support 3 in a horizontal direction, to prevent the support 3 from shaking upon interaction with skin, keep a relative position between the support 3 and the skin fixed, reduce shaking of the support 3 relative to the skin, and reduce wound damage.

It can be understood that, the seventh resilient arm 58 and the fastening boss 391 constitute a locking mechanism for preventing the push mechanism from retracting. This can effectively reduce a risk of sliding of the support 3, and reduce wound damage.

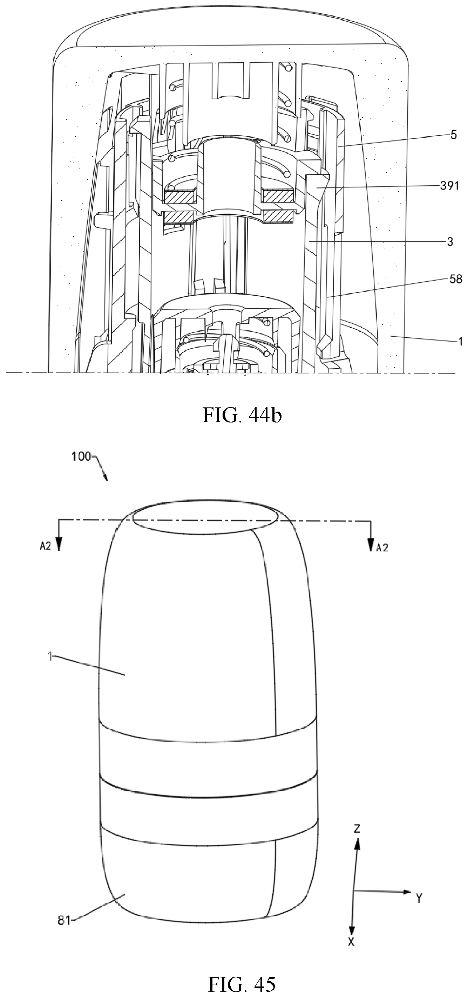

FIG. 44b is a partial cross-sectional view of the physiological sign monitoring apparatus 100 shown in FIG. 2 along K-K according to another implementation.

As shown in FIG. 44a and FIG. 44b, the seventh resilient arm 58 may alternatively be fastened to an end, away from the abutting end 502, of the upper side wall 51 of the base 5. A tail end of the seventh resilient arm 58 is provided with a hook. The tail end of the seventh resilient arm 58 is an end, close to the abutting end 502, of the seventh resilient arm 58. The hook of the seventh resilient arm 58 is located on the inner side of the base 5 and faces away from the abutting end 502. When the sensor assembly 20 abuts against the organism, in the axial (Z-axis) direction, the hook of the seventh resilient arm 58 snaps into a side, close to the abutting end 502 of the base 5, of the fastening boss 391.

It can be understood that an orientation of the hook of the seventh resilient arm 58 may be changed, and when the orientation of the hook of the seventh resilient arm 58 is changed, a joint surface between the hook of the seventh resilient arm 58 and the fastening boss 391 also correspondingly changes.

In the foregoing embodiments, the first elastic member 2, the spring seat 7, and the support 3 may constitute a push mechanism of the physiological sign monitoring apparatus 100. The lower housing 1, the base 5, and the support 3 may constitute a trigger mechanism of the physiological sign monitoring apparatus 100. The needle retraction holder 4, the second elastic member 6, the support 3, the needle holder 22, and the puncture needle 23 may constitute a needle retraction mechanism of the physiological sign monitoring apparatus 100. The trigger mechanism may serve as a switch for using the physiological sign monitoring apparatus 100, to control whether to deploy the sensor 24. The push mechanism may be configured to push the sensor assembly 20, and the puncture needle 23 carries the sensor 24 into tissue of a human body.

It can be understood that the foregoing specifically describes an implementation (the first stop structure 18 and the second stop structure 816) of preventing triggering upon falling with reference to the accompanying drawings. A limiting lock (the stopper 91) for preventing accidental triggering is disposed, to avoid accidental triggering of the apparatus in scenarios such as falling, transportation, and vibration. Because a trigger device of the trigger mechanism needs to axially move by a specific stroke to complete triggering, an axial movement stroke of the device of the trigger mechanism may be limited through fitting between limiting features, so that a limited axial movement stroke is less than a trigger stroke, to prevent accidental triggering. During use, the limiting lock for preventing accidental triggering may be automatically or manually released. After the limiting lock is released, the sensor assembly 20 may be successfully triggered.

The limiting lock may be a limiting feature of the trigger device, or a limiting feature of a limiting device or a limiting module.

For example, the limiting device may be independent, or may be another component with a position limiting function.

For example, mutually locked limiting features may be single-start or multi-start threads, smooth lead features, inclined lead features, helical lead features, or a snap ring.

For example, the limiting lock may be released by removing another component. For example, when the bottle cap is unscrewed, the limiting lock is automatically disengaged. In this case, the limiting lock is automatically released. If an unlocking action needs to be specially performed, for example, removing a snap ring or manually rotating or pressing a limiting device for unlocking, the limiting lock is manually released.

The following further describes several types of limiting locks (stop structures) in detail with reference to the accompanying drawings.

In some implementations, a part that is the same as that in the foregoing embodiments is not described herein again.

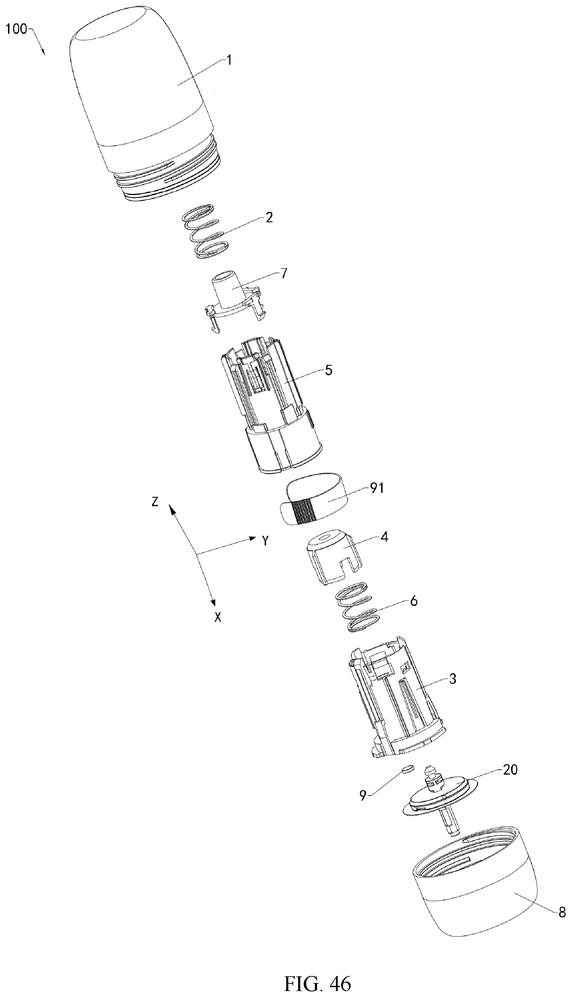

FIG. 45 is a diagram of the physiological sign monitoring apparatus 100 shown in FIG. 2 according to another implementation. FIG. 46 is a schematic exploded view of the physiological sign monitoring apparatus 100 shown in FIG. 45 according to an implementation.

As shown in FIG. 45 and FIG. 46, the physiological sign monitoring apparatus 100 may include an implantation apparatus 10 and a sensor assembly 20. The implantation apparatus 10 may include a lower housing 1, a first elastic member 2, a spring seat 7, a base 5, a needle retraction holder 4, a second elastic member 6, a support 3, a stopper 91, a first magnet 9, and a housing cap assembly 8. For a manner of disposing the first elastic member 2, the spring seat 7, the needle retraction holder 4, the second elastic member 6, the support 3, the first magnet 9, and the housing cap assembly 8, refer to the manner of disposing the first elastic member 2, the spring seat 7, the needle retraction holder 4, the second elastic member 6, the support 3, the first magnet 9, and the housing cap assembly 8 in the foregoing embodiments. Details are not described herein again.

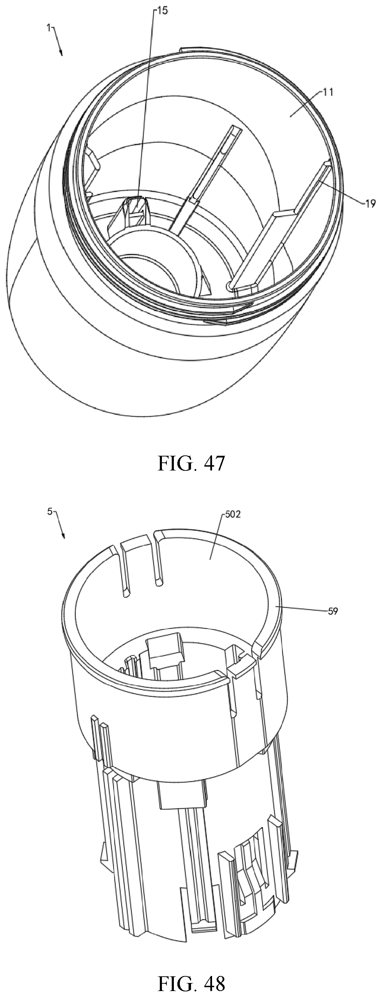

FIG. 47 is a diagram of a structure of the lower housing 1 shown in FIG. 46 according to an implementation.

As shown in FIG. 47, the lower housing 1 may further include a first limiting block 19. The first limiting block 19 is fastened to an inner side of a side wall 11 of the lower housing 1, and is exposed from an opening 101 of the lower housing 1. There may be one or more first limiting blocks 19. When there are a plurality of first limiting blocks 19, the plurality of first limiting blocks 19 are spaced apart around a central axis of the lower housing 1.

FIG. 48 is a diagram of a structure of the base 5 shown in FIG. 46 according to an implementation.

As shown in FIG. 48, the base 5 further includes a second limiting block 5083, and the second limiting block 5083 is fastened to an outer side of the abutting end 502. For example, the second limiting block 5083 may be a surrounding edge.

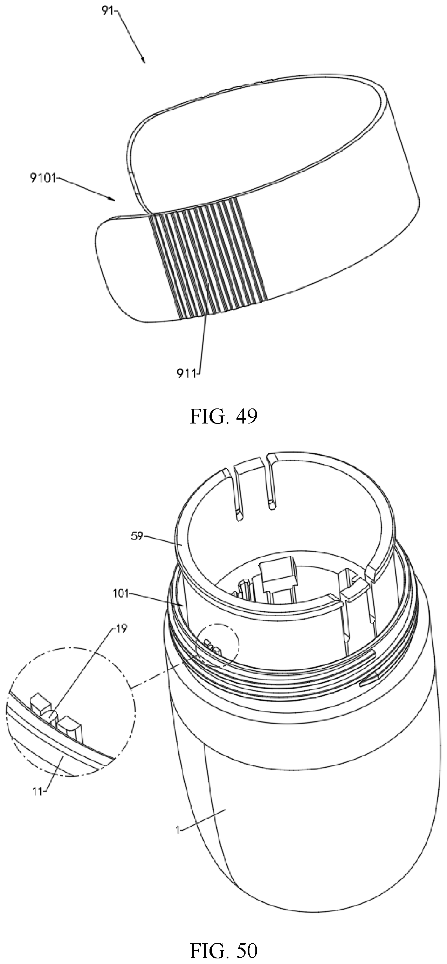

FIG. 49 is a diagram of a structure of the stopper 91 shown in FIG. 46 according to an implementation.

As shown in FIG. 49, the stopper 91 may be of a semi-ring structure. The stopper 91 may have an opening 9101. In this way, when using the physiological sign monitoring apparatus 100, the user may remove the stopper 91 for unlocking after removing the housing cap assembly 8, and a trigger mechanism can properly operate.

For example, an outer side surface of the stopper 91 may be provided with an anti-skid pattern 911. This provides convenience for the user to take the stopper 91.

FIG. 50 is a diagram of assembly of the base 5 and the lower housing 1 shown in FIG. 46 according to an implementation.

As shown in FIG. 50, when the base 5 is mounted to the lower housing 1, an axial direction of the base 5 may coincide with an axial direction of the lower housing 1. The second limiting block 5083 of the base 5 extends out of the housing. In the axial direction of the physiological sign monitoring apparatus 100, the first limiting block 19 and the second limiting block 5083 may be arranged opposite to each other.

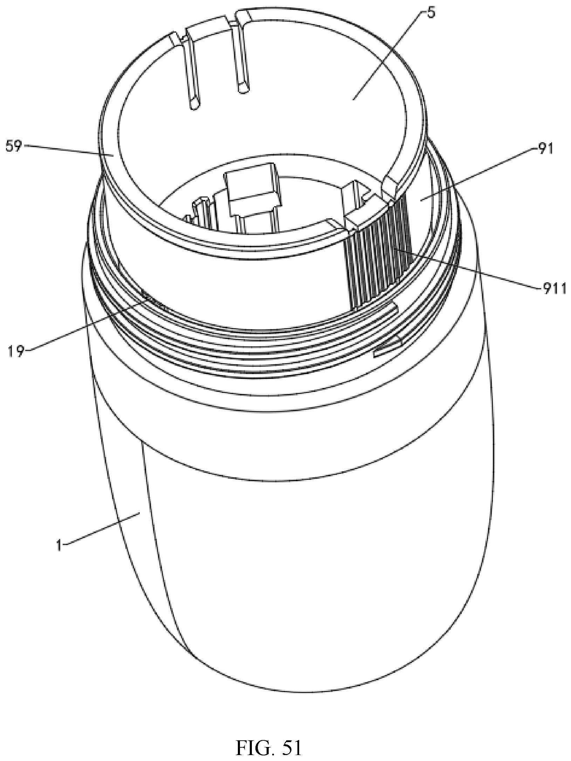

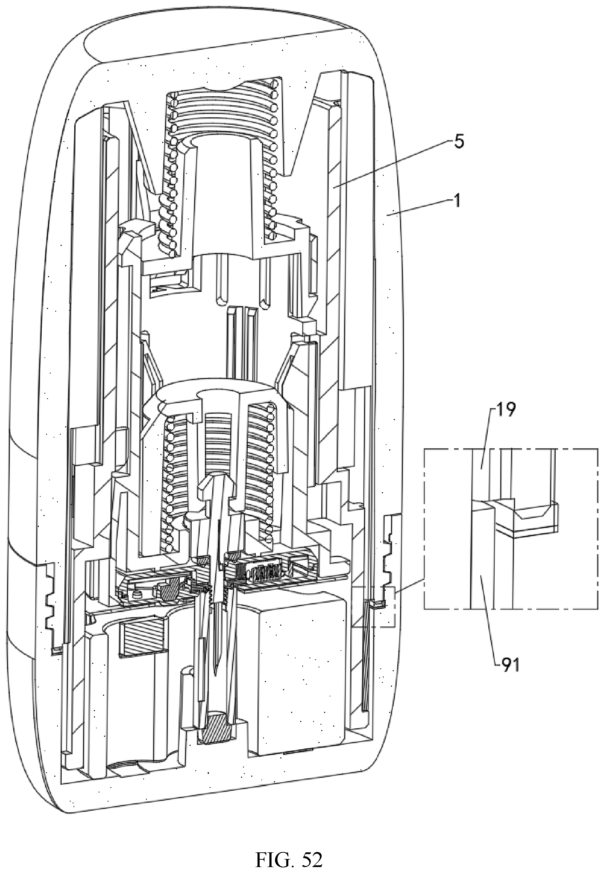

FIG. 51 is a diagram of assembly of a snap ring, the base 5, and the lower housing 1 shown in FIG. 46 according to an implementation. FIG. 52 is a schematic cross-sectional view of the physiological sign monitoring apparatus 100 shown in FIG. 45 along A2-A2 according to an implementation.

As shown in FIG. 51 and FIG. 52, the stopper 91 is detachably connected to the base 5. In the axial direction of the physiological sign monitoring apparatus 100, the stopper 91 is disposed between the first limiting block 19 of the lower housing 1 and the second limiting block 5083 of the base 5. When the physiological sign monitoring apparatus 100 is subject to collision, the stopper 91 may abut against the first limiting block 19 and the second limiting block 5083, and the stopper 91 may limit a trend of relative movement between the base 5 and the lower housing in the axial direction, to prevent the base 5 from moving relative to the lower housing 1 in the axial direction of the physiological sign monitoring apparatus 100, in other words, limit an axial movement stroke of the base 5 in a trigger direction. In this way, a toggle 15 of the lower housing does not spread open a second resilient arm 56 of the base 5, to prevent accidental triggering of the support 3.

When the physiological sign monitoring apparatus 100 switches from an unused state to an in-use state, the upper housing 81 is detached from the lower housing 1, the stopper 91 is detached from the base 5, the abutting end 502 may abut against the organism, and the user may apply, to the lower housing 1, a force toward the organism, to trigger the support 3.

In some implementations, the stopper 91 may be of a semi-ring structure. A central angle of the semi-ring structure is greater than 180°. In this way, the stopper 91 can snap into the base 5 through a structure of the stopper 91, without a need to additionally dispose a mechanical part for fastening.

In another implementation, the stopper 91 may alternatively be of a plate-like structure other than the semi-ring structure, or the like.

In some implementations, a part that is the same as that in the foregoing embodiments is not described herein again.

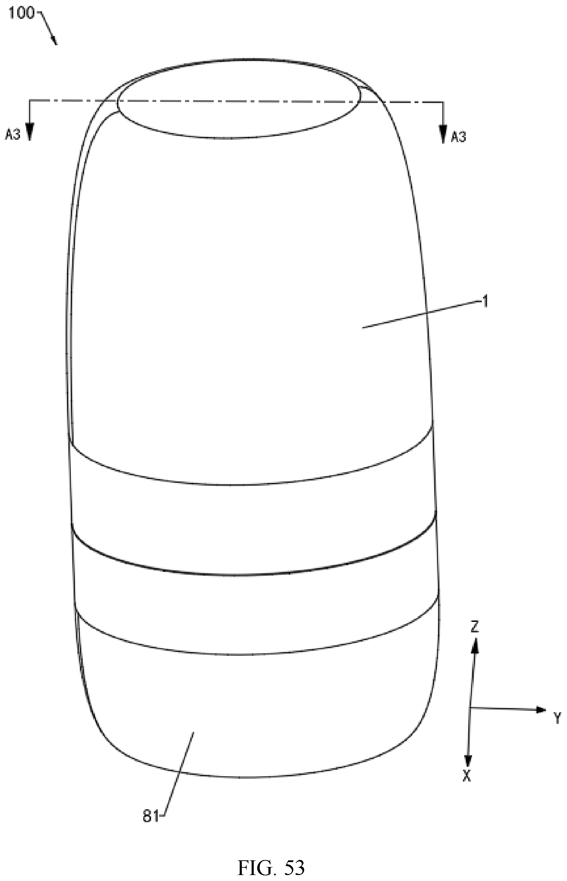

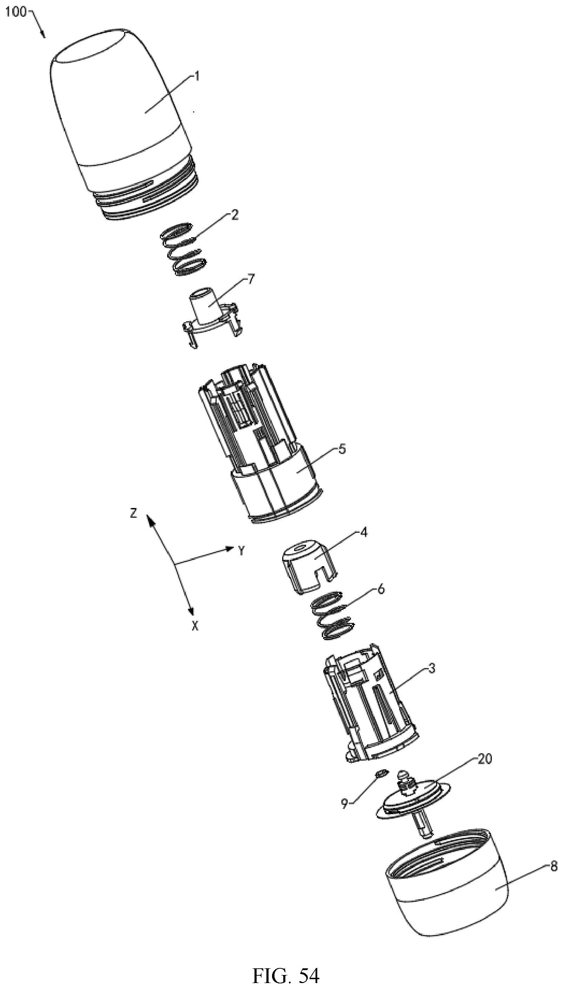

FIG. 53 is a diagram of the physiological sign monitoring apparatus 100 shown in FIG. 2 according to another implementation. FIG. 54 is a schematic exploded view of the physiological sign monitoring apparatus 100 shown in FIG. 53 according to an implementation.

As shown in FIG. 53 and FIG. 54, the physiological sign monitoring apparatus 100 may include an implantation apparatus 10 and a sensor assembly 20. The implantation apparatus 10 may include a lower housing 1, a first elastic member 2, a spring seat 7, a base 5, a needle retraction holder 4, a second elastic member 6, a support 3, a first magnet 9, and a housing cap assembly 8. For a manner of disposing the lower housing 1, the first elastic member 2, the spring seat 7, the needle retraction holder 4, the second elastic member 6, the support 3, and the first magnet 9, refer to the manner of disposing the lower housing 1, the first elastic member 2, the spring seat 7, the needle retraction holder 4, the second elastic member 6, the support 3, and the first magnet 9 in the foregoing embodiments. Details are not described herein again. The housing cap assembly 8 may include an upper housing 81, a drying member 82, and a second magnet 83. For a manner of disposing the drying member 82 and the second magnet 83, refer to the manner of disposing the drying member 82 and the second magnet 83 in the foregoing embodiments.

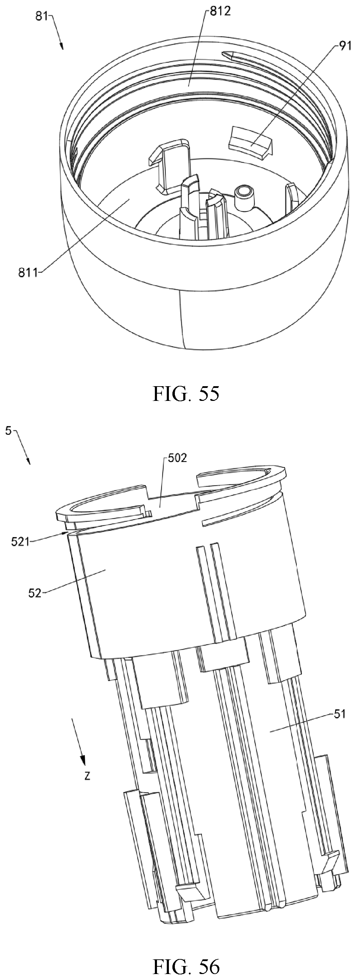

FIG. 55 is a diagram of a structure of the upper housing 81 shown in FIG. 54 according to an implementation.

As shown in FIG. 55, a stopper 91 (a short limiting rib) may be fastened to an inner side of a side wall 812 of the upper housing 81. For example, the stopper 91 may be located on a side, close to a bottom wall 811, of engaging threads of the upper housing 81, and is spaced away from the engaging threads of the upper housing 81.

In some implementations, there may be one or more stoppers 91. For example, the stopper 91 may be a continuous turn of threads, or may be several sections in a turn of threads. When there are a plurality of stoppers 91, the plurality of stoppers 91 may be spaced apart, and the plurality of stoppers 91 are evenly arranged around the axial direction of the physiological sign monitoring apparatus 100.

It can be understood that, when the stopper 91 is threads, a thread pitch of the threads of the stopper 91 is the same as a thread pitch of engaging threads of the upper housing 81.

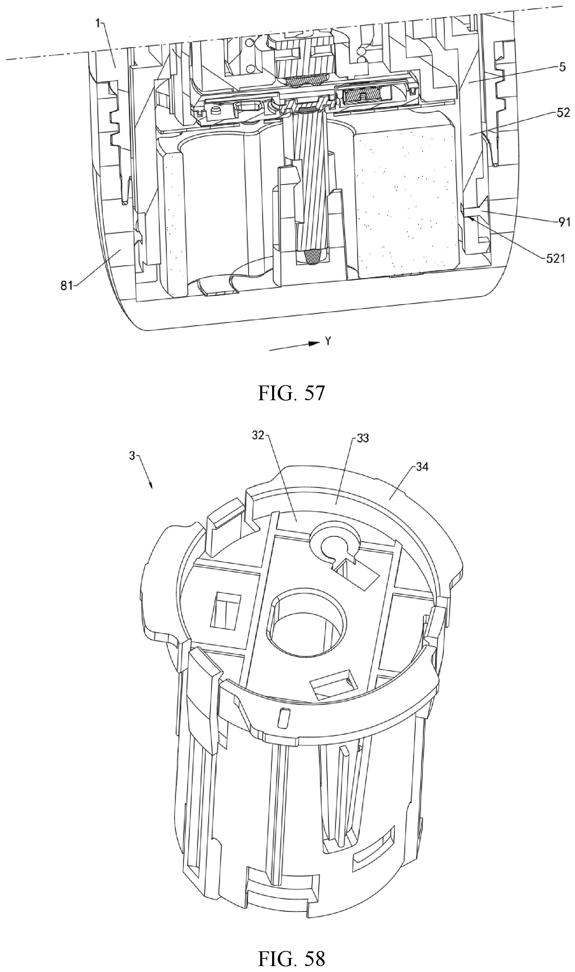

FIG. 56 is a diagram of a structure of the base 5 shown in FIG. 54 according to an implementation. FIG. 57 is a schematic cross-sectional view of the physiological sign monitoring apparatus 100 shown in FIG. 53 along A3-A3 according to an implementation.

As shown in FIG. 56 and FIG. 57, a part (for example, an abutting end 502) of a lower side wall 52 of the base 5 is exposed from the lower housing 1. The lower side wall 52 of the base 5 may be provided with a limiting groove 521. For example, the abutting end 502 may be provided with a limiting groove 521.

In some implementations, the limiting groove 521 may be a threaded groove. A thread pitch of the limiting groove 521 may be the same as the thread pitch of the stopper. In this way, the stopper 91 can well fit with the limiting groove 521. The stopper 91 may alternatively be designed to be long in an axial direction of the upper housing 81. A contact area between the stopper 91 and the base 5 may be large, to well prevent the base from moving relative to the lower housing in the axial direction of the physiological sign monitoring apparatus.

In some implementations, there are a plurality of stoppers 91. It can be understood that, in comparison with a solution in which there is one stopper, a large quantity of stoppers is disposed, to help increase a contact area between the stopper 91 and the base 5, and well prevent the base from moving relative to the lower housing in the axial direction of the physiological sign monitoring apparatus.

In some implementations, the limiting groove 521 is a threaded groove, and a thread pitch of the threaded groove may be the same as the thread pitch of the engaging threads of the upper housing 81.

It can be understood that, when the upper housing 81 is mounted to the lower housing 1, the stopper 91 is partially located in the limiting groove 521. For example, when the upper housing 81 and the lower housing 1 are fastened through the engaging threads, the stopper 91 may slide into the threaded limiting groove 521 on the lower side wall 52 of the base 5. In a direction parallel to the axial direction (a Z axis) of the physiological sign monitoring apparatus 100, the stopper 91 is arranged opposite to the base 5. When the physiological sign monitoring apparatus 100 is subject to collision, the stopper 91 may limit a trend of relative movement between the base 5 and the upper housing 81 in the axial direction, and the lower housing 1 is fastened to the upper housing 81. Therefore, the stopper 91 may limit a trend of relative movement between the base 5 and the lower housing 1 in the axial direction, to prevent the base 5 from moving relative to the lower housing 1 in the axial direction of the physiological sign monitoring apparatus 100, in other words, limit an axial movement stroke of the base 5 in a trigger direction. In this way, a toggle 15 of the lower housing 1 does not spread open a second resilient arm 56 of the base 5 (as shown in FIG. 39), to prevent accidental triggering of the support 3.

The following further describes an anti-oscillation design with reference to the accompanying drawings. In some implementations, a part that is the same as that in the foregoing embodiments is not described herein again. FIG. 58 is a diagram of a structure of the support 3 shown in FIG. 3 according to another implementation.

As shown in FIG. 58, the support 3 may have a flange 34. The flange 34 of the support 3 may be protrudingly disposed on an outer side surface of the support 3.

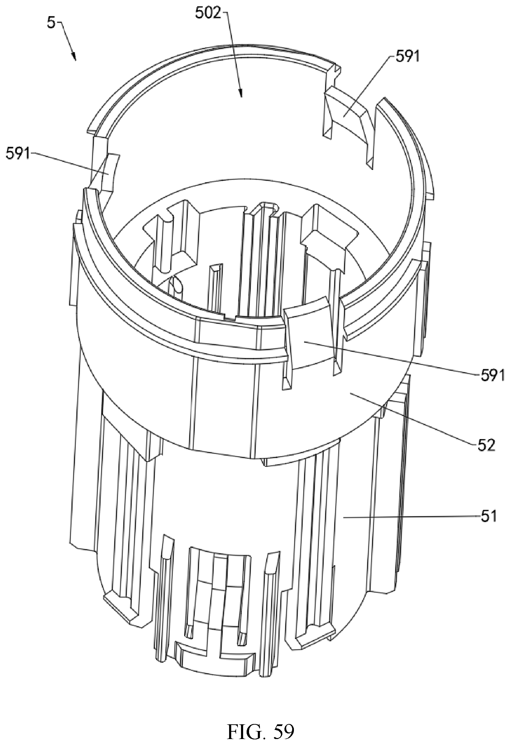

FIG. 59 is a diagram of a structure of the base 5 shown in FIG. 3 according to another implementation.

As shown in FIG. 59, the base 5 may be further provided with an elastic latch 591. An end, facing an upper side wall 51 of the base 5, of the elastic latch 591 is fastened to the lower side wall 52 of the base 5. The other end of the elastic latch 591 is spaced away from the lower side wall 52 of the base 5. An end, away from a top wall 12 of the lower housing 1, of the elastic latch 591 is spaced away from the abutting end 502, and the elastic latch 591 protrudes from an inner side of the base 5. For example, the other end of the elastic latch 591 protrudes from space enclosed by the lower side wall 52 of the base 5.

For example, there may be a plurality of elastic latches 591, and the plurality of elastic latches 591 may be spaced apart around an axial direction of the base 5. For example, there are three elastic latches 591, and the three elastic latches 591 are spaced apart.

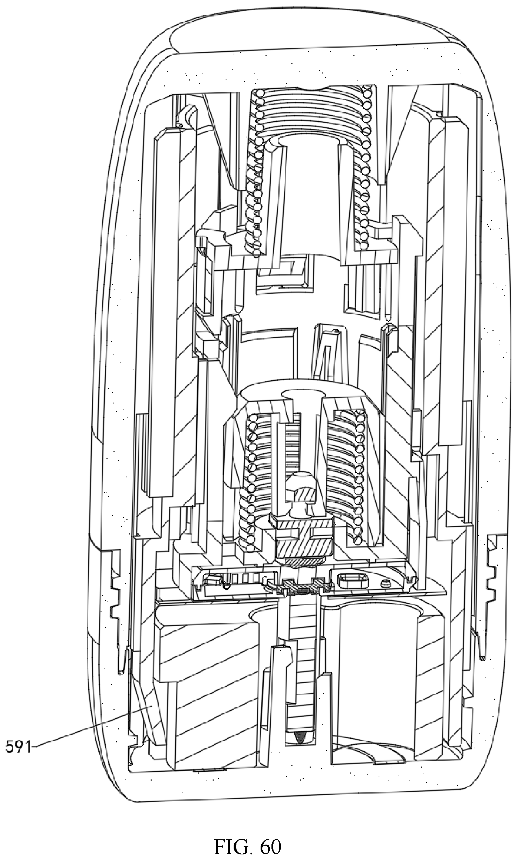

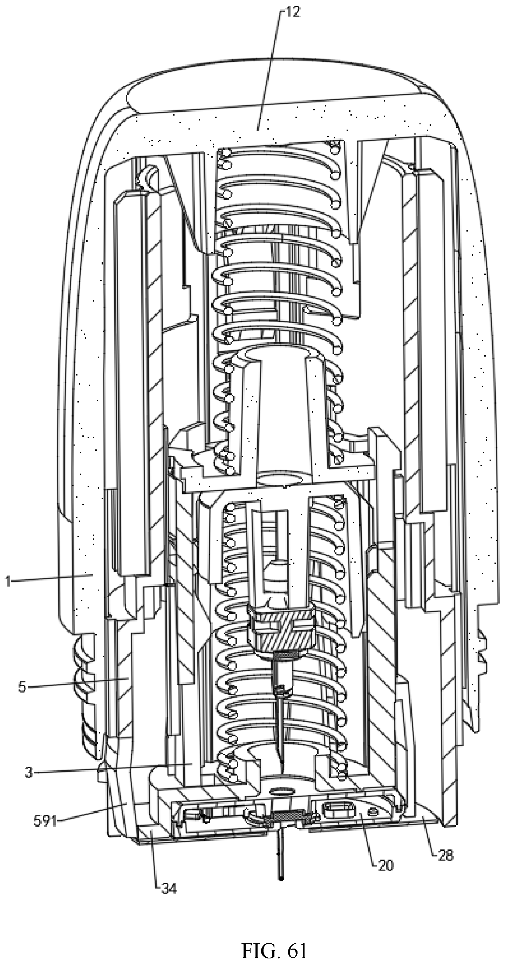

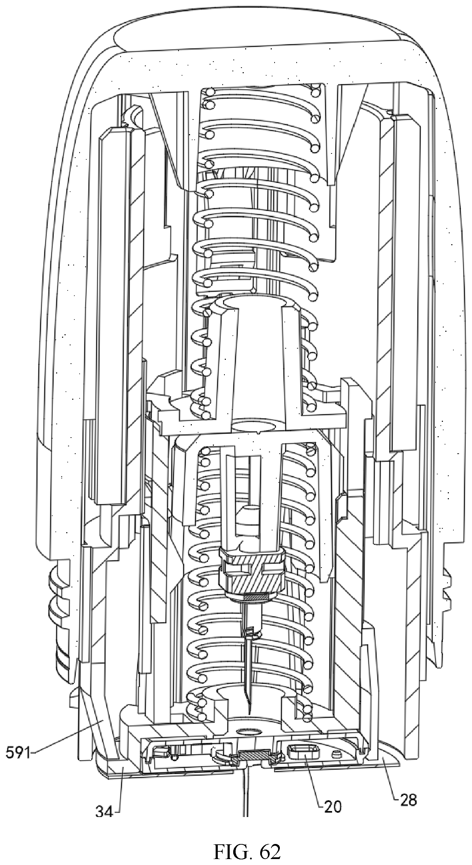

FIG. 60 is a partial cross-sectional view of the physiological sign monitoring apparatus 100 shown in FIG. 2 along F-F according to another implementation. FIG. 61 is a partial cross-sectional view of the physiological sign monitoring apparatus 100 shown in FIG. 2 along F-F during use according to an implementation. FIG. 62 is a partial cross-sectional view of the physiological sign monitoring apparatus 100 shown in FIG. 2 along F-F during use according to an implementation.

As shown in FIG. 60 to FIG. 62, when the physiological sign monitoring apparatus 100 switches from an unused state to an in-use state, the upper housing 81 is detached from the lower housing 1, the support 3 moves in a direction toward the organism (an opposite direction of a Z axis), and the sensor assembly 20 abuts against the organism, the elastic latch 591 abuts against a side, close to the top wall 12 of the lower housing 1, of the support 3. For example, the elastic latch 591 abuts against a side, close to the top wall 12 of the lower housing 1, of the flange 34.

For example, after the support 3 is unlocked, in a process in which the support 3 moves down under the action of the first elastic member 2, the flange 34 of the support 3 may spread open the elastic latch 591. Then, when the support 3 pushes the bonding member 28 of the sensor assembly 20 into position, to be specific, the bonding member 28 is approximately flush with an end face of the abutting end 502 of the base 5, the elastic latch 591 rebounds and abuts against the side, close to the top wall 12 of the lower housing 1, of the flange 34 of the support 3, to implement position limiting for the support 3 in an axial direction, and prevent the support 3 from sliding in the axial direction. The plurality of elastic latches 591 abut against the support 3 around the axial direction of the support 3, and perform position limiting for the support 3 in a horizontal direction, to prevent the support 3 from shaking upon interaction with skin, keep a relative position between the support 3 and the skin fixed, reduce shaking of the support 3 relative to the skin, and reduce wound damage.

The foregoing embodiments describe fitting of a double-headed ratchet wheel, so that when the housing cap assembly 8 is removed, the sealed cartridge 29 may also be loosened correspondingly and is removed together with the housing cap assembly 8. However, when a relative position between the upper housing 81 and the sealed cartridge 29 is abnormal (for example, a mounting error occurs), the double-headed ratchet wheel has no tolerance capability. When the position is abnormal, the upper housing 81 needs to rotate by a doubled stroke to loosen the sealed cartridge 29. When a stroke, for loosening, of the upper housing 81 is insufficient, it cannot be necessarily ensured that the sealed cartridge 29 is detached from a guide needle along with the removal of the housing cap assembly 8. The following describes several ratchet wheel structures with a specific tolerance capability with reference to the accompanying drawings.

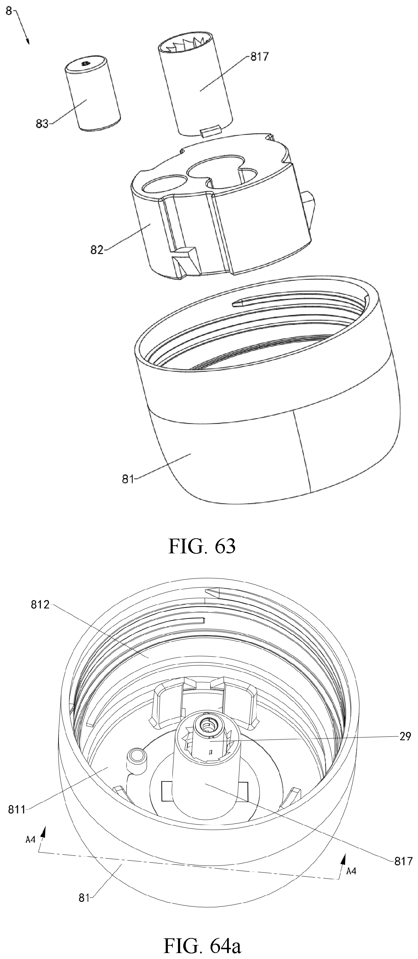

In some implementations, a part that is the same as that in the foregoing embodiments is not described herein again. FIG. 63 is a schematic exploded view of the housing cap assembly 8 shown in FIG. 3 according to another implementation. FIG. 64a is a diagram of assembly of an unlock ratchet wheel 817, an upper housing 81, and a sealed cartridge 29 shown in FIG. 63 according to an implementation.

As shown in FIG. 63 and FIG. 64a, the housing cap assembly 8 may include the upper housing 81, a drying member 82, and a second magnet 83. The upper housing 81 may include a bottom wall 811, a side wall 812, and the unlock ratchet wheel 817. The side wall 812 of the upper housing 81 is connected around a periphery of the bottom wall 811 of the upper housing 81. For an assembly relationship between the upper housing 81, the drying member 82, and the second magnet 83, refer to the assembly manner in the foregoing embodiments. Details are not described herein again.

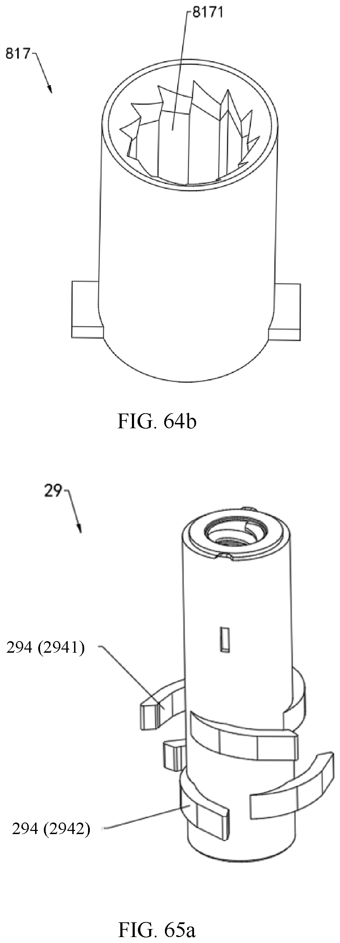

FIG. 64b is a diagram of a structure of the unlock ratchet wheel 817 shown in FIG. 63 according to an implementation. FIG. 65a is a diagram of a structure of the sealed cartridge 29 shown in FIG. 64a according to another implementation.

As shown in FIG. 64a to FIG. 65a, the unlock ratchet wheel 817 is fastened to the bottom wall 811 of the upper housing 81. An inner wall surface of the unlock ratchet wheel 817 is provided with a plurality of inclined ratchet wheel teeth 8171. An outer side surface of the sealed cartridge 29 may be provided with an elastic arm 294. The elastic arm 294 may be configured to fit with the inclined ratchet wheel teeth 8171 of the unlock ratchet wheel 817, to detach the sealed cartridge 29 from the needle holder 22. For example, in a process in which the upper housing 81 is tightened relative to the lower housing 1, the elastic arm 294 may be slidably connected to the unlock ratchet wheel 817. In a process in which the upper housing 81 is detached from the lower housing 1, the elastic arm 294 may snap into the unlock ratchet wheel 817, and push the sealed cartridge 29 to detach from the needle holder 22.

For example, an opening portion of the unlock ratchet wheel 817 may be designed for reverse interlock. In this way, in a length direction of the sealed cartridge 29, the ratchet wheel 293 and the elastic arm 294 may perform reverse interlock with each other. After the sealed cartridge 29 and the needle holder 22 are unlocked, the upper housing 81 may hook the sealed cartridge 29, so that the sealed cartridge 29 can be removed together with the lower housing 1.

For example, there are a plurality of inclined ratchet wheel teeth 8171. For example, in the accompanying drawings of this embodiment, an example in which a quantity n of inclined ratchet wheel teeth 8171 is 12 is used for description.

In some implementations, there may be a plurality of groups of elastic arms 294 on the outer side surface of the sealed cartridge 29, and the plurality of groups of elastic arms 294 may be located at one height or different heights. In FIG. 65a in this embodiment, different groups of elastic arms 294 are located at different heights.

For example, one group of elastic arms 294 includes a plurality of elastic arms 294. In the accompanying drawings, an example in which one group of elastic arms includes three elastic arms is used for description.

For example, the sealed cartridge 29 includes a plurality of first elastic arms 2941, and the first elastic arms 2941 are protrudingly disposed on the outer side surface of the sealed cartridge 29. The plurality of first elastic arms 2941 constitute a first elastic arm group. The sealed cartridge 29 further includes a plurality of second elastic arms 2942, and the second elastic arms 2942 are protrudingly disposed on the outer side surface of the sealed cartridge 29. The ratchet wheel 293 fits with the plurality of second elastic arms 2942. The plurality of second elastic arms 2942 constitute a second elastic arm group. The first elastic arm group and the second elastic arm group are disposed in a staggered manner in the axial direction of the physiological sign monitoring apparatus 100.

It can be understood that different groups of elastic arms 294 have different rotation angles, and a rotation angle a exists between two groups of elastic arms 294. To be specific, any one of the two groups of elastic arms rotates relative to the other group of elastic arms by the angle a, and the two groups of elastic arms 294 coincide in the axial direction. For example, when the sealed cartridge 29 is mounted to the unlock ratchet wheel 817, end faces of a first group of elastic arms 294 may be engaged with short edges of the inclined ratchet wheel teeth 8171, and end faces of a second group of elastic arms 294 are spaced away from the short edges of the inclined ratchet wheel teeth 8171. In this case, the sealed cartridge 29 needs to rotate relative to the unlock ratchet wheel 817 by an angle a1, so that the end faces of the second group of elastic arms 294 can be engaged with the short edges of the inclined ratchet wheel teeth 8171. a may be equal to C×(360°/n)+a1, where C is a non-zero integer. a1 may be greater than 0° and less than (360°/n). For example, a1=(360°/2n). When n=12, a may be (C×30°+15°), and a1 may be (360°/24)=15°. In this way, regardless of rotating forward or reversely, the second group of elastic arms need to rotate relative to the unlock ratchet wheel 817 by only 15° to be engaged with the short edges of the inclined ratchet wheel teeth 8171.

It can be understood that, when a pawl is disposed on the upper housing, during molding of the upper housing, forcible removal is needed at a position of the pawl, and the pawl is prone to expansion and deformation; or when a pawl and other devices of the upper housing are separately prepared and then assembled, a process is complex, and costs are high. In comparison, in this application, the elastic arm 294 is placed on the outer side surface of the sealed cartridge 29, and because a position of the elastic arm 294 is on an outer surface, no forcible removal is needed.

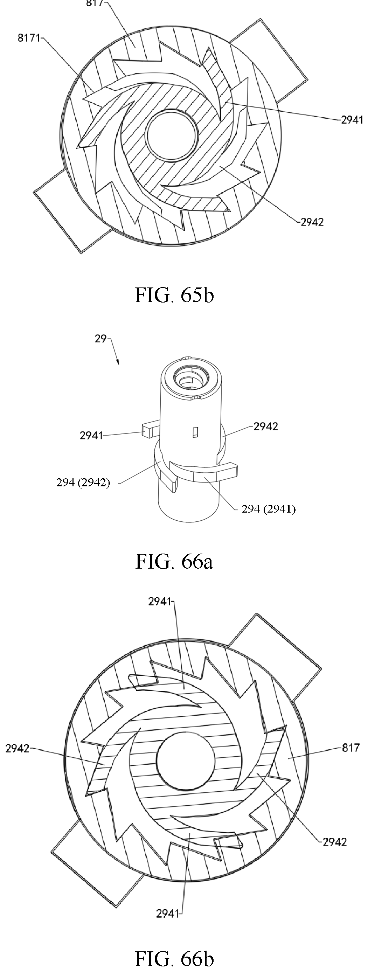

FIG. 65b is a partial cross-sectional view of the structure shown in FIG. 64a along A4-A4 according to an implementation.

As shown in FIG. 65b, when the sealed cartridge 29 is mounted in the unlock ratchet wheel 817, an end face of the elastic arm 294 may be engaged with a short edge of the inclined ratchet wheel tooth 8171. When the housing cap assembly 8 is removed, the short edge of the inclined tooth abuts against the end face of the elastic arm 294, to drive the sealed cartridge 29 to rotate, so that the sealed cartridge 29 can also be loosened correspondingly and is removed together with the housing cap assembly 8.

For example, when mounting positions of the sealed cartridge 29 and the unlock ratchet wheel 817 have an error, to be specific, when the sealed cartridge 29 and the unlock ratchet wheel 817 are arranged opposite to each other with a deflection angle, if there is only one group of elastic arms 294, a rotation angle of (360°/n) is needed to enable end faces of the elastic arms 294 to be correctly engaged with the short edges of the inclined ratchet wheel teeth 8171. When there are two groups of elastic arms 294, only a rotation angle of (360°/2n) is needed to enable one of the two groups of elastic arms 294 to be correctly engaged with the short edges of the inclined ratchet wheel teeth 8171. It can be understood that there may alternatively be more than two groups of elastic arms 294. In this way, when mounting positions of the sealed cartridge 29 and the unlock ratchet wheel 817 have a deflection angle, the upper housing 81 only needs to rotate by a smaller angle to implement correct engagement between the elastic arms 294 and the short edges of the inclined ratchet wheel teeth 8171.

It can be understood that, in this embodiment, a plurality of groups of elastic arms 294 are disposed to fit with the unlock ratchet wheel 817, to achieve a tolerance capability. When the upper housing 81 and the sealed cartridge 29 have a deflection angle during assembly, the elastic arms 294 can be correctly engaged with the ratchet wheel 293 only through rotation by a smaller angle. This avoids a case in which the engaging threads of the upper housing 81 are fully run down but unlock threads between the sealed cartridge 29 and the guide needle are not fully run down, and consequently, the sealed cartridge 29 cannot be removed together with the housing cap assembly 8.

It can be understood that, in this embodiment, the elastic arm 294 is disposed on the outer side surface of the sealed cartridge 29, and the unlock ratchet wheel 817 is disposed on the upper housing 81. In another implementation, a position of the elastic arm 294 and a position of the unlock ratchet wheel 817 may alternatively be interchanged.

In some implementations, a part that is the same as that in the foregoing embodiments is not described herein again. FIG. 66a is a diagram of a structure of the sealed cartridge 29 shown in FIG. 64a according to still another implementation. FIG. 66b is a partial cross-sectional view of the structure shown in FIG. 64a along A4-A4 according to another implementation.

As shown in FIG. 66a and FIG. 66b, in this embodiment, a difference from the foregoing embodiment lies in that a plurality of groups of elastic arms 294 included in the sealed cartridge 29 are at a same height.

For example, the sealed cartridge 29 includes two groups of elastic arms 294: a first group of elastic arms and a second group of elastic arms. Each group of elastic arms includes two elastic arms. A plurality of elastic arms 294 in the two groups may be spaced apart around an axial direction of the sealed cartridge 29. Any elastic arm 294 in one group is located between two elastic arms 294 in the other group. For example, any first elastic arm 2941 may be located between two second elastic arms 2942.

For example, different groups of elastic arms 294 have different rotation angles, and a rotation angle a exists between two groups of elastic arms 294. For example, when the sealed cartridge 29 is mounted to the unlock ratchet wheel 817, end faces of the first group of elastic arms 294 may be engaged with short edges of the inclined ratchet wheel teeth 8171, and end faces of the second group of elastic arms 294 are spaced away from the short edges of the inclined ratchet wheel teeth 8171, without engagement. It is assumed that, when the sealed cartridge 29 rotates relative to the unlock ratchet wheel 817 by an angle a1 again, the end faces of the second group of elastic arms 294 may also be engaged with the short edges of the inclined teeth. a may be equal to 90°+a1 or 90°-a1 and C is a non-zero integer. a1 may be greater than 0° and less than (360°/n). For example, a1(360°/2n). When n=12, a may be (C×30°+15°), and a1 may be (360°/24)=15°. In this way, when the sealed cartridge 29 and the upper housing 81 are not correctly engaged due to a mounting error, the sealed cartridge 29 needs to rotate forward or reversely relative to the unlock ratchet wheel 817 by at most 15° to implement engagement with the short edges of the inclined ratchet wheel teeth 8171.

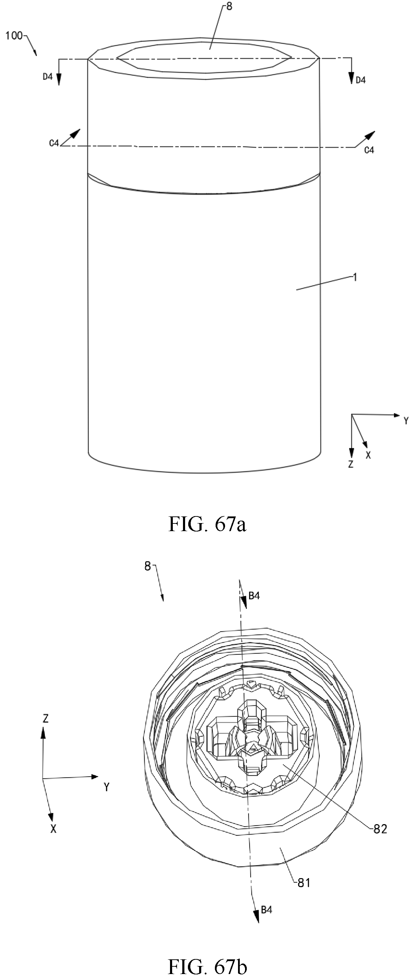

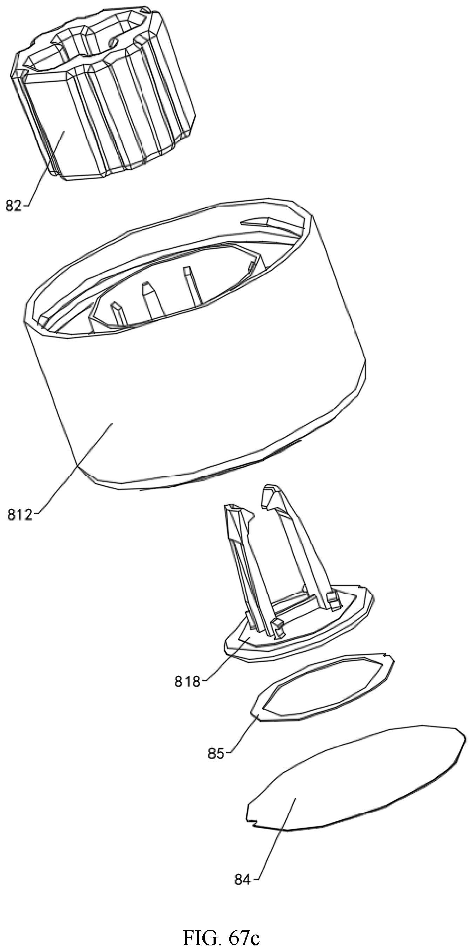

In some implementations, a part that is the same as that in the foregoing embodiments is not described herein again. FIG. 67a is a diagram of a structure of the physiological sign monitoring apparatus 100 shown in FIG. 2 according to still another implementation. FIG. 67b is a diagram of a partial structure of the housing cap assembly 8 shown in FIG. 67a according to an implementation. FIG. 67c is a diagram of a structure of the housing cap assembly 8 shown in FIG. 67b according to an implementation.

As shown in FIG. 67a and FIG. 67b, the housing cap assembly 8 may include an upper housing 81, a drying member 82, a decoration member 84, and a sealing member 85.

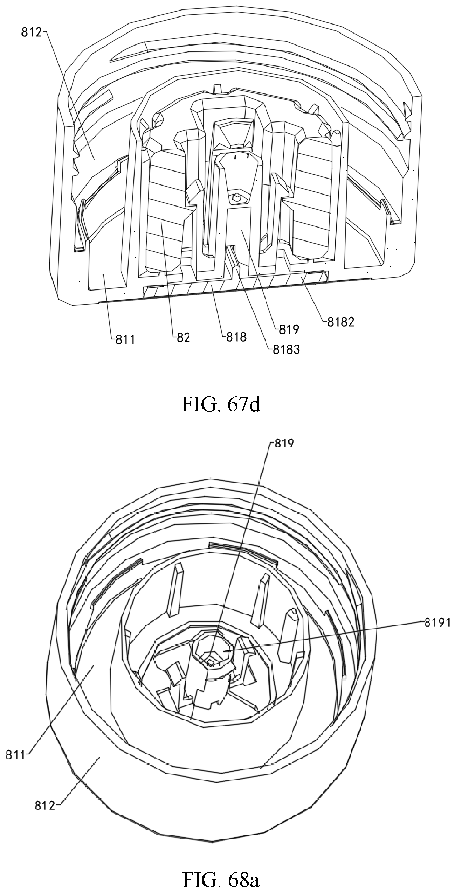

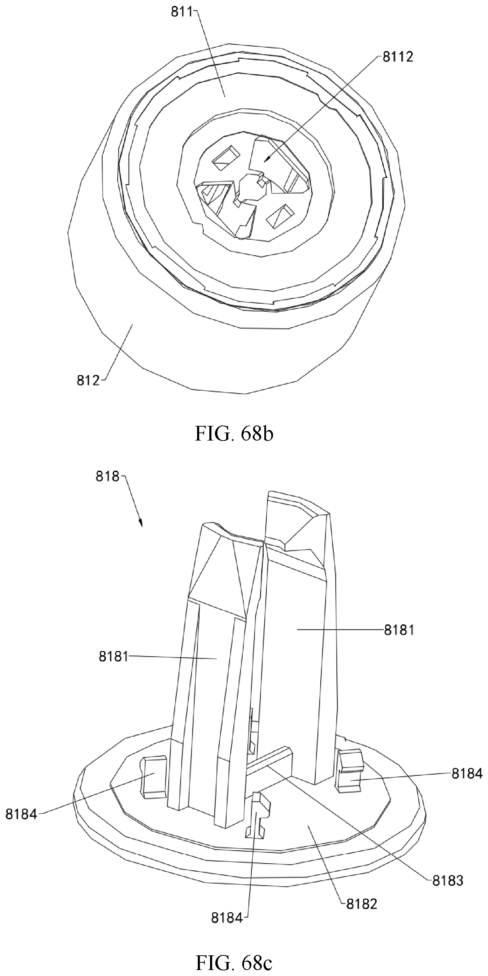

FIG. 67d is a partial cross-sectional view of the housing cap assembly 8 shown in FIG. 67a along B4-B4 according to an implementation. FIG. 68a is a diagram of a partial structure of the upper housing 81 shown in FIG. 67b according to an implementation. FIG. 68b is a diagram of a partial structure of the upper housing 81 shown in FIG. 68a from another perspective according to an implementation.

As shown in FIG. 67c to FIG. 68b, the upper housing 81 may include a bottom wall 811, a side wall 812, a clamping arm assembly 818, and a snap-fit boss 819. The side wall 812 of the upper housing 81 is connected around a periphery of the bottom wall 811 of the upper housing 81. The snap-fit boss 819 is fastened to a side, facing the top wall 12 of the lower housing 1, of the bottom wall 811 of the upper housing 81. The snap-fit boss 819 is provided with a snap-fit slot 8191. An opening of the snap-fit slot 8191 may face the top wall 12 of the lower housing 1 (face away from the bottom wall 811 of the upper housing).

In some implementations, a positioning groove may be provided on a side, away from the side wall 11, of the bottom wall 811 of the upper housing 81.

FIG. 68c is a diagram of a structure of the clamping arm assembly 818 shown in FIG. 67c according to an implementation.

As shown in FIG. 68c, the clamping arm assembly 818 may include a clamping arm 8181, a bottom plate 8182, a positioning rod 8183, and a snap-fit jaw 8184. The clamping arm 8181, the positioning rod 8183, and the snap-fit jaw 8184 may be fastened to a same side of the bottom plate 8182 of the clamping arm assembly 818. One end of the clamping arm 8181 is fastened to the bottom plate 8182 of the clamping arm assembly 818. The other end of the clamping arm 8181 may be provided with a hook. One end of the snap-fit jaw 8184 may be fastened to the bottom plate 8182 of the clamping arm assembly 818. The other end of the snap-fit jaw 8184 may be provided with a hook.

For example, there may be one or more clamping arms 8181. For example, there may be a plurality of clamping arms 8181, and the plurality of clamping arms 8181 may be arranged around a central axis of the physiological sign monitoring apparatus 100. Two clamping arms 8181 are shown in the figure.

For example, there may be one or more snap-fit jaws 8184. For example, there may be a plurality of snap-fit jaws 8184, and the plurality of snap-fit jaws 8184 may be arranged around the central axis of the physiological sign monitoring apparatus 100. Four snap-fit jaws 8184 are shown in the figure.

In some implementations, the positioning rod 8183 may be fastened between two clamping arms 8181.

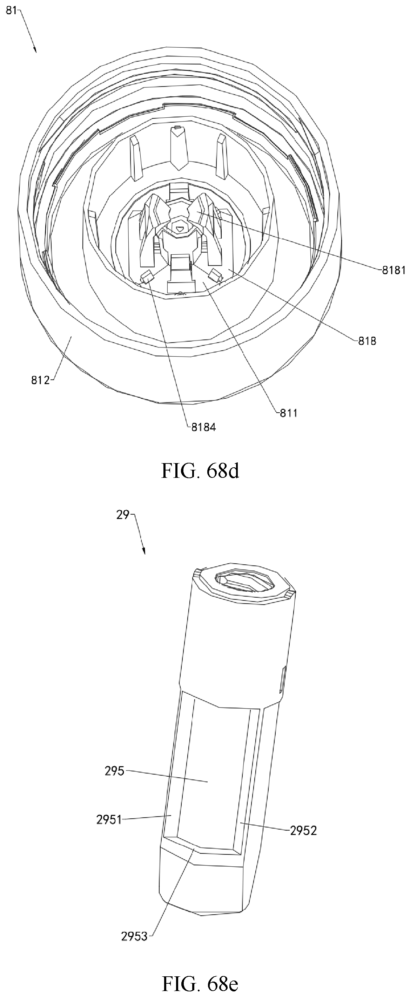

FIG. 68d is a diagram of assembly of the clamping arm assembly 818 and the upper housing 81 shown in FIG. 67b according to an implementation.

As shown in FIG. 67d and FIG. 68d, the clamping arm assembly 818 may be detachably fastened to the upper housing 81. For example, the bottom wall 811 of the upper housing 81 is provided with a mounting hole 8112 (as shown in FIG. 68b), and the mounting hole 8112 passes through the bottom wall 811 of the upper housing 81. The bottom plate 8182 of the clamping arm assembly 818 may be located on a side, away from the side wall 812 of the upper housing 81, of the bottom wall 811 of the upper housing 81. One end of the clamping arm 8181 is detachably fastened to the bottom wall 811 of the upper housing 81. The other end of the clamping arm 8181 extends, through the mounting hole 8112, into a side, facing the top wall 12 of the lower housing 1, of the bottom wall 811 of the upper housing 81. For example, one end of the clamping arm 8181 is fastened to the bottom plate 8182 of the clamping arm assembly 818.

In some implementations, the snap-fit jaw 8184 of the clamping arm assembly 818 may be snap into the bottom wall 811 of the upper housing 81.

FIG. 68e is a diagram of a structure of the sealed cartridge 29 provided in embodiments according to still another implementation.

As shown in FIG. 68e, the sealed cartridge 29 has a limiting groove 295, and an opening of the limiting groove 295 may be located on a side surface of the sealed cartridge 29. A groove wall of the limiting groove 295 includes a first limiting surface 2951 and a second limiting surface 2952. The first limiting surface 2951 and the second limiting surface 2952 may be spaced apart in a circumferential direction of the sealed cartridge 29.

In some implementations, the groove wall of the limiting groove 295 may further include a third limiting surface 2953, and the third limiting surface 2953 is connected between the first limiting surface 2951 and the second limiting surface 2952.

In some implementations, there may be one or more limiting grooves 295. When there are a plurality of limiting grooves 295, the plurality of limiting grooves 295 are spaced apart in the circumferential direction of the sealed cartridge 29. For example, there are two limiting grooves 295, and the two limiting grooves 295 are symmetric with respect to a central axis of the sealed cartridge 29.

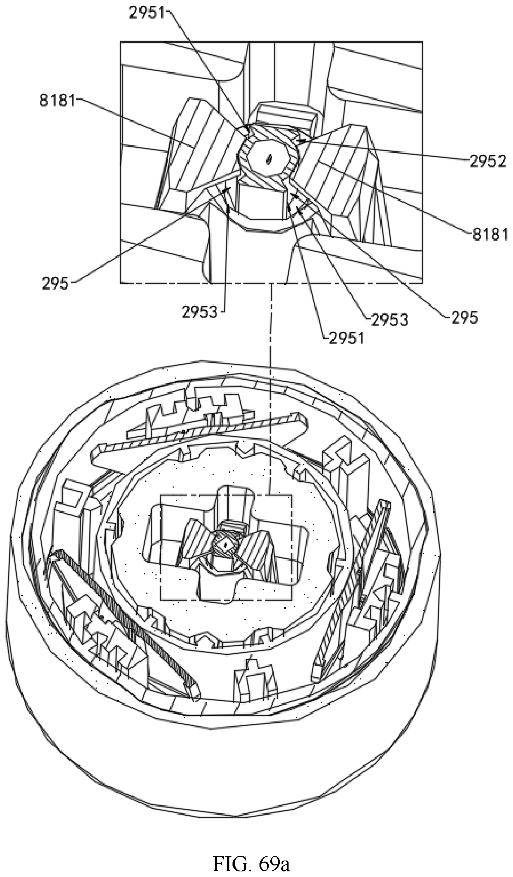

FIG. 69a is a partial cross-sectional view of the physiological sign monitoring apparatus 100 shown in FIG. 67a along C4-C4 according to an implementation. FIG. 69a is a diagram of assembly of the sealed cartridge 29 and the housing cap assembly 8 according to an implementation.

As shown in FIG. 69a, when the physiological sign monitoring apparatus 100 is in an unused state, an end, away from the needle holder 22, of the sealed cartridge 29 abuts against the snap-fit slot 8191. The clamping arm 8181 may be detachably connected to the sealed cartridge 29. For example, the hook of the clamping arm 8181 faces the sealing member 85, and is located at an end, away from the bottom wall 811 of the upper housing 81, of the clamping arm 8181. The hook of the clamping arm 8181 is at least partially located in the limiting groove 295. In the circumferential direction of the sealed cartridge 29, the hook of the clamping arm 8181 may be located between the first limiting surface 2951 and the second limiting surface 2952.

In some implementations, there may be a plurality of clamping arms 8181, and there may also be a plurality of limiting grooves 295. A quantity of clamping arms 8181 is equal to a quantity of limiting grooves 295. The plurality of clamping arms 8181 and the plurality of limiting grooves 295 are arranged in a one-to-one correspondence.

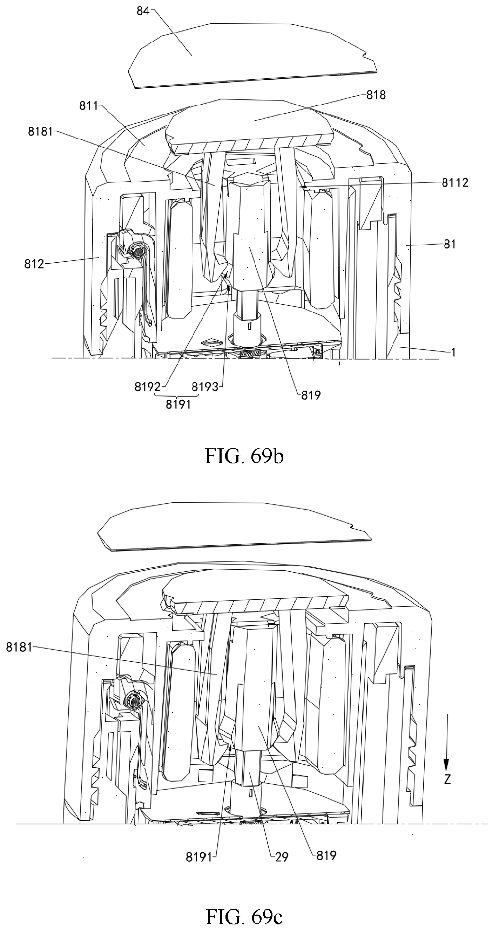

FIG. 69b is a partial cross-sectional view of the physiological sign monitoring apparatus 100 shown in FIG. 67a along D4-D4 during assembly according to an implementation.

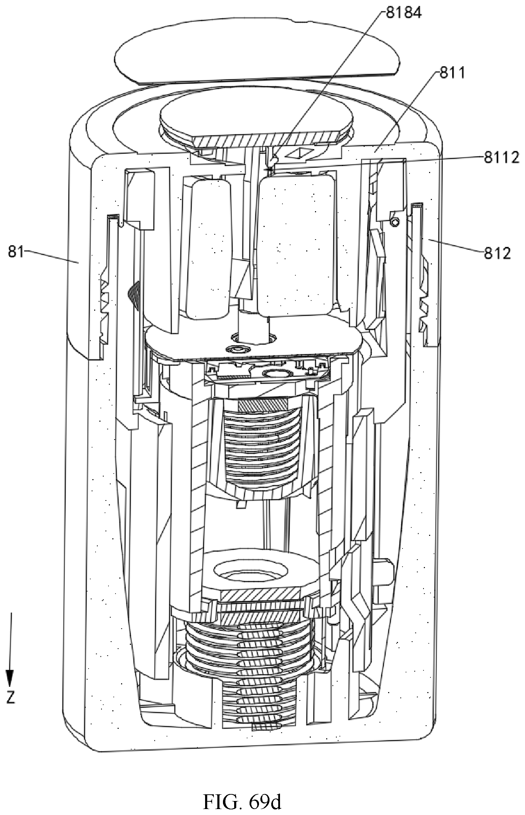

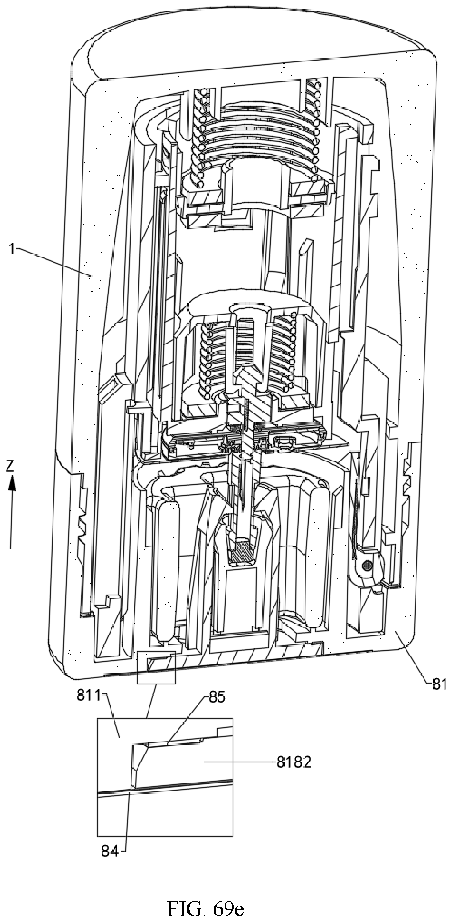

FIG. 69c is a partial cross-sectional view of the physiological sign monitoring apparatus 100 shown in FIG. 67a along D4-D4 during assembly according to an implementation. FIG. 69d is a partial cross-sectional view of the physiological sign monitoring apparatus 100 shown in FIG. 67a along D4-D4 during assembly according to an implementation. FIG. 69e is a partial cross-sectional view of the physiological sign monitoring apparatus 100 shown in FIG. 67a along D4-D4 during assembly according to an implementation.

FIG. 69b to FIG. 69e show a process of mounting components of the housing cap assembly 8 during assembly of the physiological sign monitoring apparatus 100 in this embodiment. The clamping arm assembly 818 of the upper housing 81 may be assembled after other components of the physiological sign monitoring apparatus 100 are mounted. It can be understood that, in comparison with the solutions in the foregoing embodiments, in this embodiment, the clamping arm 8181 is an independent detachable mechanical part, and the sealed cartridge 29 is first assembled, to avoid displacement of the sealed cartridge 29 during assembly of the clamping arm 8181 and therefore avoid difficulty in subsequent removal of the sealed cartridge 29.

As shown in FIG. 69b and FIG. 69c, an end, away from the bottom wall 811 of the upper housing 81, of the side wall 812 of the upper housing 81 is detachably fastened to the lower housing 1. The clamping arm assembly 818 may enter the physiological sign monitoring apparatus 100 through the mounting hole 8112 on the bottom wall 811 of the upper housing 81. An end, away from the bottom wall 811 of the upper housing 81, of a side surface of the snap-fit boss 819 may protrude toward the side wall 812 of the upper housing 81, to form an inclined guide surface 8191. The inclined guide surface 8191 may be arranged at an angle to an axial direction of the upper housing 81. The clamping arm 8181 may fit with the inclined guide surface 8191, and the plurality of clamping arms 8181 may be spread open by the snap-fit boss 819.

In some implementations, the inclined guide surface 8191 may include a first inclined surface 8192 and a second inclined surface 8193, the first inclined surface 8192 faces the bottom wall 811 of the upper housing 81, and the second inclined surface 8193 faces away from the bottom wall 811 of the upper housing 81. In a process in which the clamping arm assembly 818 moves toward 8184 the sealed cartridge 29, the clamping arm 8181 is first spread open and then gradually converges under the action of the inclined guide surface 8191.

FIG. 69d shows a mounting relationship between the snap-fit jaw 8184 of the clamping arm assembly 818 and the bottom wall 811 of the upper housing 81. During mounting of the clamping arm assembly 818, the snap-fit jaw 8184 may first abut against a wall surface of the mounting hole 8112, and an end, away from the bottom plate 32 of the clamping arm assembly 818, of the snap-fit jaw 8184 moves toward a central axis of the clamping arm 8181 assembly. After a hook of the snap-fit jaw 8184 enters a side, close to the side wall 812 of the upper housing 81, of the bottom wall 811 of the upper housing 81, the snap-fit jaw 8184 no longer abuts against a hole wall of the mounting hole 8112, and the snap-fit jaw 8184 is restored to an original shape and snaps into the bottom wall 811 of the upper housing 81.

In some implementations, as shown in FIG. 69e, in a direction parallel to an axial direction of the sealed cartridge 29, the third limiting surface 2953 and the hook of the clamping arm 8181 are arranged opposite to each other. In this way, when the physiological sign monitoring apparatus 100 switches from the unused state to an in-use state, in a process in which the upper housing 81 is detached from the lower housing 1, the clamping arm 8181 may clamp the sealed cartridge 29 through the third limiting surface 2953, and push the sealed cartridge 29 to detach from the needle holder 22.

In some implementations, an outer diameter of the snap-fit boss 819 is greater than an outer diameter of the sealed cartridge 29. In this way, the clamping arm assembly 818 is not likely to collide with the sealed cartridge 29 during mounting, to reduce a risk of damage to the sealed cartridge 29, and avoid a case that the sealed cartridge 29 cannot be removed together with the upper housing 81 when the upper housing 81 is detached from the lower housing 1.

In some implementations, after the clamping arm assembly 818 is mounted, there may be a gap between the clamping arm 8181 and the sealed cartridge 29 in a radial direction of the sealed cartridge 29. It can be understood that the gap may be reserved for rotation that occurs when the upper housing 81 is detached from the lower housing 1.

It can be understood that, in this embodiment, in the circumferential direction of the sealed cartridge 29, the limiting groove 295 on the side surface of the sealed cartridge 29 has two limiting surfaces (the first limiting surface 2951 and the second limiting surface 2952). In a process in which the upper housing 81 is detached from the lower housing 1 and the upper housing 81 drives the sealed cartridge 29 to rotate, a contact surface between the clamping arm 8181 and the sealed cartridge 29 is large, and there are limiting surfaces on both sides of the hook of the clamping arm 8181, so that the sealed cartridge 29 is not likely to detach from the upper housing 81. This reduces a risk that the sealed cartridge 29 falls off during removal of the sealed cartridge 29, causing damage to the puncture needle 23 and the bonding member 28.

In some implementations, the sealing member 85 may be fastened between the bottom wall 811 of the upper housing 81 and the bottom plate 8182 of the clamping arm assembly 818. The sealing member 85 may be configured to seal a gap between the clamping arm assembly 818 and the bottom wall 811 of the upper housing 81, to improve sealing performance of the physiological sign monitoring apparatus 100. For example, the sealing member 85 may be glue, for example, waterproof glue.

In some implementations, the decoration member 84 may be fastened to a side, away from the lower housing 1, of the bottom plate 8182 of the clamping arm assembly 818. In other words, the decoration member 84 may be fastened to a surface on a side, away from the lower housing 1, of the housing cap assembly 8. In this way, the decoration member 84 may be configured to cover a gap between the clamping arm assembly 818 and the upper housing 81, to improve appearance fineness of the physiological sign monitoring apparatus 100.

It can be understood that the foregoing embodiments show an example in which two-start threads are used for a fool-proofing design between the upper housing 81 and the lower housing 1. The following specifically describes a fool-proofing design with multi-start threads with reference to the accompanying drawings.

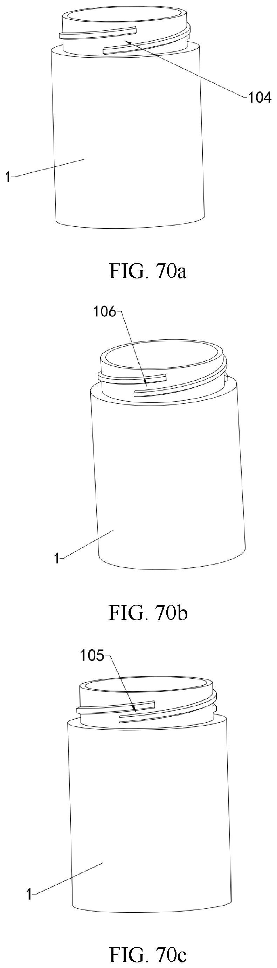

FIG. 70a is a diagram of a structure of the lower housing 1 provided in this application according to another implementation. FIG. 70b is a diagram of a structure of the lower housing 1 shown in FIG. 70a from another perspective. FIG. 70c is a diagram of a structure of the lower housing 1 shown in FIG. 70a from still another perspective.

As shown in FIG. 70a to FIG. 70c, engaging threads of the lower housing 1 include three threads with a same thread pitch and a same thread thickness. Different threads have different starting heights, and starting positions are arranged around an axial direction of the lower housing 1. In addition, distances between any two adjacent threads are different. In this way, the three threads can form three engagement positions with different sizes. For example, the three threads may form three engagement positions with different sizes: a coarse-pitch thread engagement opening 104, a fine-pitch thread engagement opening 105, and a medium-pitch thread engagement opening 106.

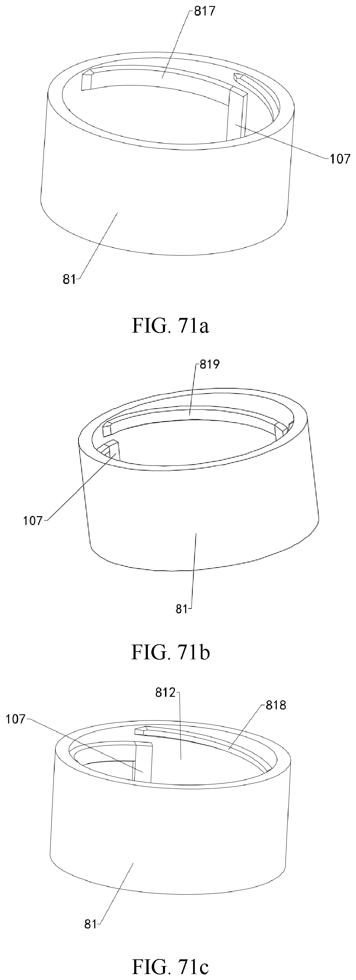

FIG. 71a is a diagram of a structure of the upper housing 81 provided in this application according to another implementation. FIG. 71b is a diagram of a structure of the upper housing 81 shown in FIG. 71a from another perspective. FIG. 71c is a diagram of a structure of the upper housing 81 shown in FIG. 71a from still another perspective.

As shown in FIG. 71a to FIG. 71c, three threads with different widths are correspondingly arranged on an inner side surface of the side wall 812 of the upper housing 81. The three threads with different widths have different starting positions in a circumferential direction on the inner side surface of the side wall 812 of the upper housing 81. When the upper housing 81 and the lower housing 1 are tightened through fitting between threads, the three threads with different widths on the upper housing 81 correspond to the three engagement positions with different sizes on the lower housing 1. The three threads can be properly engaged and the upper housing 81 can be tightened only when the upper housing 81 and the lower housing 1 are screwed at a specific initial angle. For example, engaging threads of the upper housing 81 may include a coarse-pitch thread 817, a fine-pitch thread 818, and a medium-pitch thread 819.

It can be understood that, when the upper housing 81 is detachably connected to the lower housing 1, the coarse-pitch thread 817 is engaged from the coarse-pitch thread engagement opening 104, the fine-pitch thread 818 is engaged from the fine-pitch thread engagement opening 105, and the medium-pitch thread 819 is engaged from the medium-pitch thread engagement opening 106.

For example, the threads of the upper housing 81 may have limiting features 107 that abut against each other, to ensure that the upper housing 81 and the lower housing 1 do not rotate after being screwed into position, and a relative position is fixed. In addition, limiting features are added at a starting position of the threads of the lower housing 1 and an ending position of the threads of the upper housing 81. After the upper housing 81 and the lower housing 1 are tightened, the two limiting features are in a zero-clearance fit, so that a position of the upper housing 81 relative to the lower housing 1 does not deflect toward a tightening direction.

It can be understood that, in embodiments of this application, several fool-proofing designs with multi-start threads are described by using the accompanying drawings. In the multi-start thread structure, different threads have a same thread pitch and different starting heights. This can avoid reverse mounting of the upper housing 81 and the lower housing 1 during mounting. Specifically, a plurality of threads are designed for the lower housing 1, and a starting height of one or more threads is designed to be different, to form thread engagement openings with different widths. A plurality of threads with different widths are provided on the upper housing 81. The upper housing 81 can be screwed and tightened only when a thread with each width is screwed from a corresponding engagement openings. If the thread is screwed from a different engagement opening, interference occurs between a coarse-pitch thread and a fine-pitch thread engagement opening, and the upper housing 81 cannot be tightened.

In addition, a position limiting function is further provided, so that a relative position between the upper housing 81 and the lower housing 1 is unique after they are tightened. This can meet some structural designs that can be implemented only at a specific position. For example, the upper housing 81 has a magnet for sensing power-on/off, and the magnet needs to be at a fixed position after assembly.

For example, the multi-start threads may be two-start threads, three-start threads, or four-start threads.

For example, a cross section of threads may be in a trapezoidal shape, a triangular shape, or a parallelogram shape.

For example, the limiting features may fit with each other through planar fit, spherical fit, or curved-surface fit.

It can be understood that, in the foregoing embodiments, the base 5, the needle retraction holder 4, the second elastic member 6, the support 3, the needle holder 22, and the puncture needle 23 may constitute a needle retraction mechanism of the physiological sign monitoring apparatus 100. The needle retraction mechanism is configured to implement a function of retracting the puncture needle 23 after the puncture needle 23 is pushed to puncture into skin. For example, after the sensor 24 punctures into and is implanted into the organism, the second elastic member 6 is configured to enable the needle retraction holder 4 to move away from the organism, the needle retraction holder 4 and the puncture needle 23 move toward a side away from the organism, and the puncture needle 23 is detached from the organism.

The needle retraction mechanism may include the needle retraction holder 4, a passive mechanical drive device for the puncture needle 23, a guide needle, and one or more snap-fit devices. Alternatively, the needle retraction mechanism may include the needle retraction holder 4, a passive mechanical drive device for the puncture needle 23, a guide needle, and a snap-fit feature. The guide needle may include the needle holder 22 and the puncture needle 23.

A needle retraction principle of the needle retraction mechanism includes: The needle retraction holder 4 and the needle holder 22 of the guide needle snap-fit with each other through a gripper, so that the guide needle can move together with a guide needle fastening base. At an initial position, the snap-fit device or the snap-fit feature fastens the needle retraction holder 4 to keep it at a specified position. In this case, the passive mechanical drive device stores potential energy needed for driving. After pushing is triggered, pushing is performed to a specific position, and pre-retraction or multi-stage retraction is triggered. In this case, the snap-fit device or the snap-fit feature partially releases the needle retraction holder 4. Under the action of the passive mechanical drive device, the needle retraction holder 4 is retracted by a specific distance. When pushing is performed to a position for triggering complete retraction, the snap-fit device or the snap-fit feature completely releases the needle retraction holder 4. The passive mechanical drive device pushes the guide needle and the needle retraction holder 4 to a specified position, to complete a needle retraction action.

For example, the passive mechanical drive device for the puncture needle 23 may provide kinetic energy for needle retraction by using potential energy stored by a passive energy storage device. For example, the passive mechanical drive device for the puncture needle 23 may include a compression spring, a tension spring, a torsion spring, a mainspring, a high-pressure nitrogen cylinder, or a vacuum cylinder. For example, the second elastic member 6 in the foregoing embodiments is the passive mechanical drive device for the puncture needle 23, and a compression spring is used as an example for description.

For example, the gripper for snap-fit between the needle retraction holder 4 and the guide needle may be on the needle holder 22 of the guide needle, or may be on the needle retraction holder 4. There may be two or more grippers. A type of the gripper may be a horizontal gripper or a vertical gripper. For example, the first cantilever 43 shown in FIG. 16 and FIG. 17 is the gripper for snap-fit between the needle retraction holder 4 and the guide needle. The gripper for snap-fit between the needle retraction holder 4 and the guide needle is disposed on the needle retraction holder 4, and is a vertical gripper.

For example, a releasable snap-fit device may be an elastic fastener, a lever latch, a threaded latch, a helical gear, or an inclined-surface ratchet wheel, so that the guide needle and the needle retraction holder 4 can be kept at a predetermined designed position, and the needle retraction holder 4 is released after pushing is completed.

In some implementations, multi-stage retraction may be implemented through multi-stage fitting features, for example, a stepped groove, a stepped limiting rib 961, or a stepped limiting rod.

The following further describes several needle retraction mechanisms with reference to the accompanying drawings.

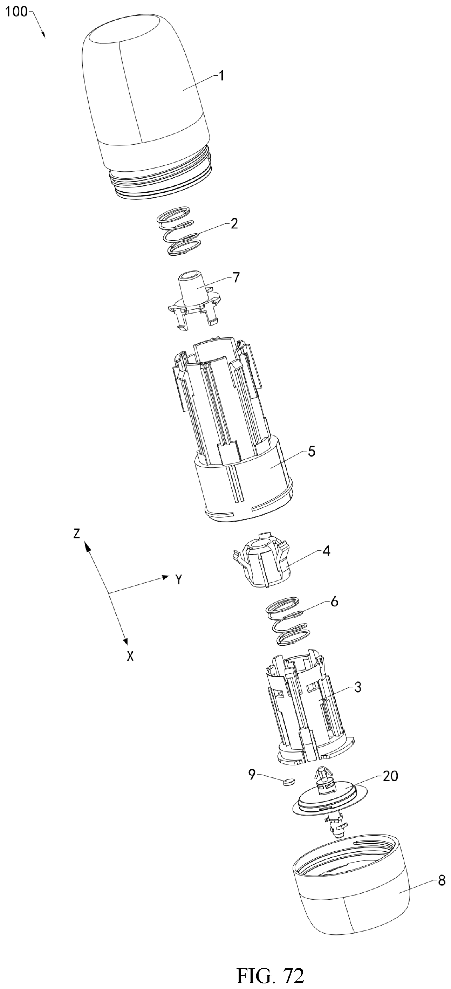

In some implementations, a part that is the same as that in the foregoing embodiments is not described again. FIG. 72 is a schematic exploded view of the physiological sign monitoring apparatus 100 shown in FIG. 2 according to yet another implementation.

As shown in FIG. 72, the physiological sign monitoring apparatus 100 may include an implantation apparatus 10 and a sensor assembly 20. The implantation apparatus 10 may include a lower housing 1, a first elastic member 2, a spring seat 7, a base 5, a needle retraction holder 4, a second elastic member 6, a support 3, a first magnet 9, and a housing cap assembly 8. For a manner of disposing the lower housing 1, the first elastic member 2, the spring seat 7, the second elastic member 6, the first magnet 9, and the housing cap assembly 8, refer to the manner of disposing the lower housing 1, the first elastic member 2, the spring seat 7, the second elastic member 6, the first magnet 9, and the housing cap assembly 8 in the foregoing embodiments. Details are not described herein again.

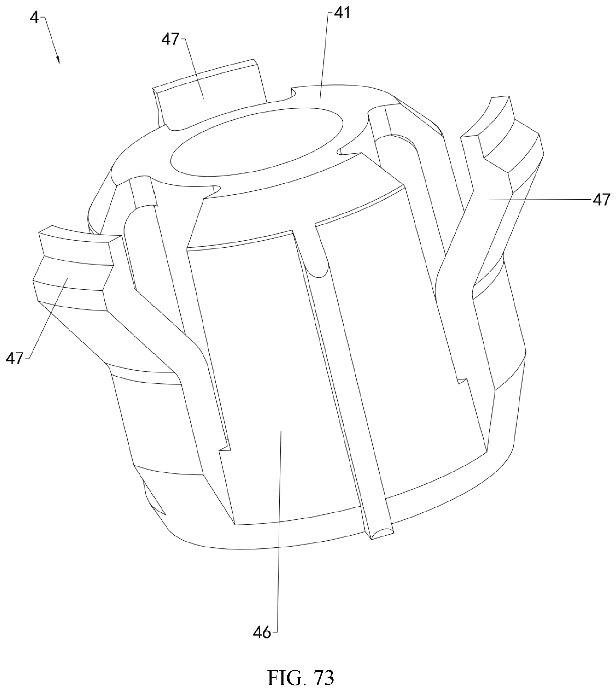

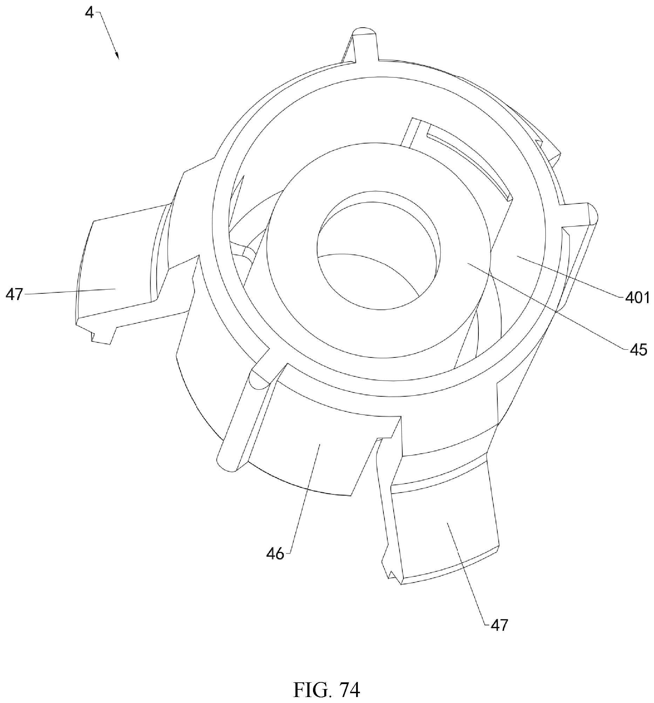

FIG. 73 is a diagram of a structure of the needle retraction holder 4 shown in FIG. 72 according to an implementation. FIG. 74 is a diagram of a structure of the needle retraction holder 4 shown in FIG. 73 from another perspective.

As shown in FIG. 73 and FIG. 74, the needle retraction holder 4 may include a top plate 41, an inner side plate 45, and an outer side plate 46. The outer side plate 46 is fastened to a periphery of the top plate 41. The inner side plate 45 is located on an inner side of the outer side plate 46. The inner side plate 45 and the outer side plate 46 are fastened to a same side of the top plate 41. The inner side plate 45 and the outer side plate 46 enclose third accommodation space 401.

For example, the needle retraction holder 4 may further include an elastic fastener 47. One end of the elastic fastener 47 is fastened to a side, away from the top plate 41, of the outer side plate 46 of the needle retraction holder 4. The other end of the elastic fastener 47 is spaced away from the inner side plate 45 of the needle retraction holder 4. There are a plurality of elastic fasteners 47, and the plurality of elastic fasteners 47 are spaced apart around an axial direction of the needle retraction holder 4. Three elastic fasteners 47 are shown in the figure.

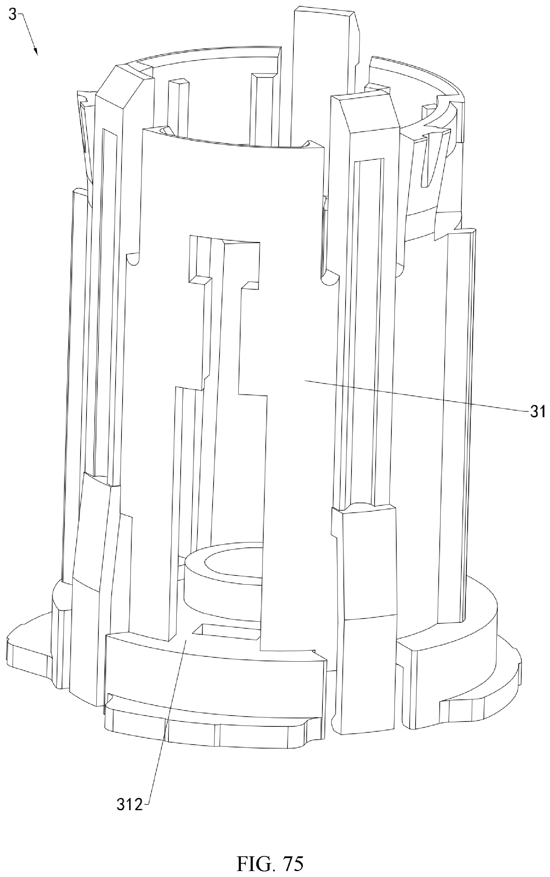

FIG. 75 is a diagram of a structure of the support 3 shown in FIG. 72 according to an implementation.

As shown in FIG. 75, a side plate 31 of the support 3 may be provided with a snap-fit hole 312. The snap-fit hole 312 passes through the side plate 31 of the support 3.

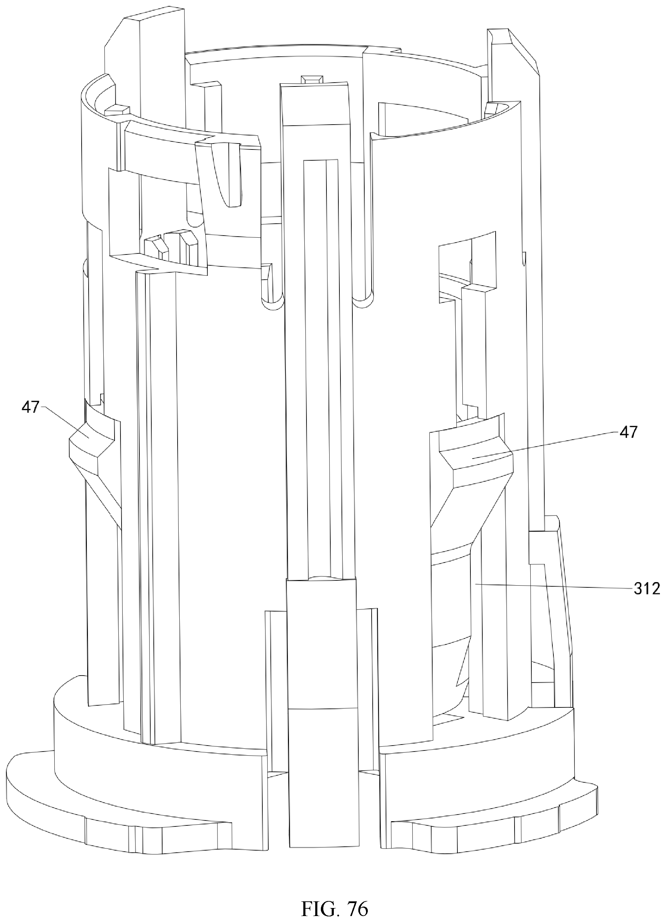

FIG. 76 is a diagram of assembly of the support 3 and the needle retraction holder 4 shown in FIG. 72 according to an implementation.

As shown in FIG. 76, the needle retraction holder 4 may be mounted in first accommodation space 301 of the support 3. The elastic fastener 47 of the needle retraction holder 4 snaps into the snap-fit hole 312. For example, the other end of the elastic fastener 47 snaps into the snap-fit hole 312.

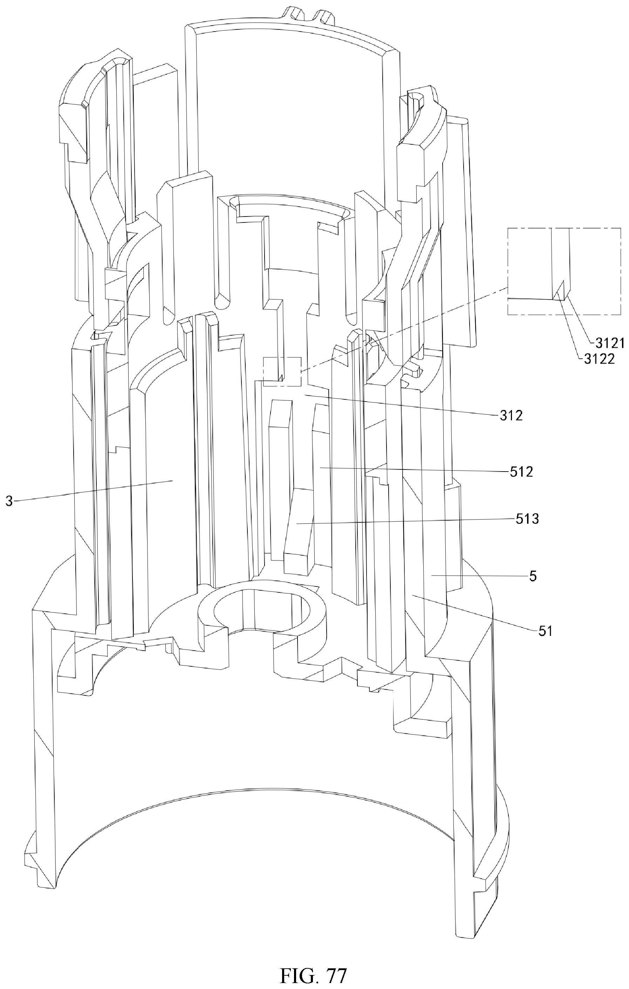

FIG. 77 is a schematic cross-sectional view of assembly of the support 3 and the base 5 shown in FIG. 72 according to an implementation.

As shown in FIG. 77, a pre-retraction pressure rib 512 and a retraction pressure rib 513 may be disposed on an inner side surface of an upper side wall 51 of the base 5. A hole wall of the snap-fit hole 312 includes a first stepped surface 3121 and a second stepped surface 3122.

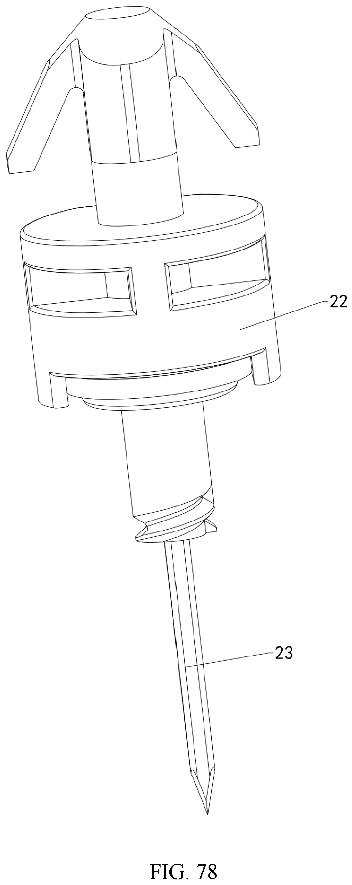

FIG. 78 is a diagram of a structure of a needle holder 22 and a puncture needle 23 of the sensor assembly 20 shown in FIG. 72 according to an implementation. The needle holder 22 and the puncture needle 23 constitute a guide needle.

As shown in FIG. 78, in this embodiment, a limiting fastener 221 of the needle holder 22 may serve as a gripper for snap-fit between the needle retraction holder 4 and the guide needle. To be specific, the gripper for snap-fit between the needle retraction holder 4 and the guide needle may be on the needle holder 22 of the guide needle. The limiting fastener 221 of the needle holder 22 is a vertical gripper.

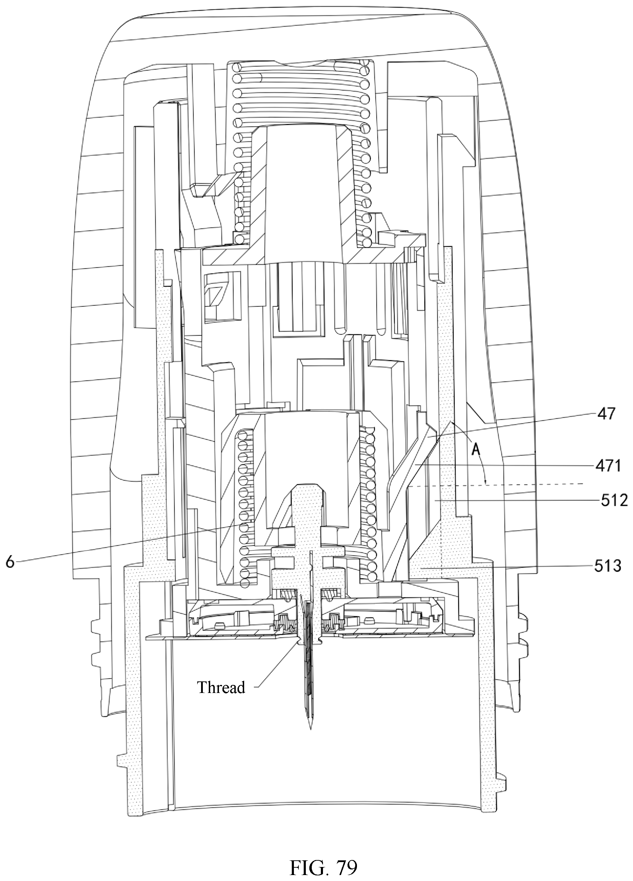

FIG. 79 to FIG. 82 are partial cross-sectional views of the physiological sign monitoring apparatus 100 in a process of deploying the sensor assembly 20 according to an embodiment. FIG. 79 to FIG. 82 show an operating process of a needle retraction mechanism in this embodiment.

FIG. 79 shows a state in which the housing cap assembly 8 of the physiological sign monitoring apparatus 100 is just removed. An end, away from a lower side wall 943 of the base 5, of the elastic fastener 47 abuts in the snap-fit hole 312. The elastic fastener 47 extends out of the snap-fit hole 312, and is arranged opposite to both the pre-retraction pressure rib 512 and the retraction pressure rib 513 of the base 5 in an axial direction of the base 5. In this case, an end (an end connected to a sealed cartridge 29), provided with threads, of the needle holder 22 protrudes from a bonding member 28 of the sensor assembly 20. The gripper of the guide needle snap-fits with the needle retraction holder 4.

For example, the elastic fastener 47 may include a tilt section 471, and the tilt section 471 and a bottom plate 32 of the support 3 form an included angle. In some implementations, the included angle between the tilt section 471 and the bottom plate 32 of the support 3 is A. A is an acute angle. For example, A may range from 30° to 80°. It can be understood that a larger value of A indicates a smaller force needed for pushing the elastic fastener 47 to move inward, and smaller space occupied by the elastic fastener 47.

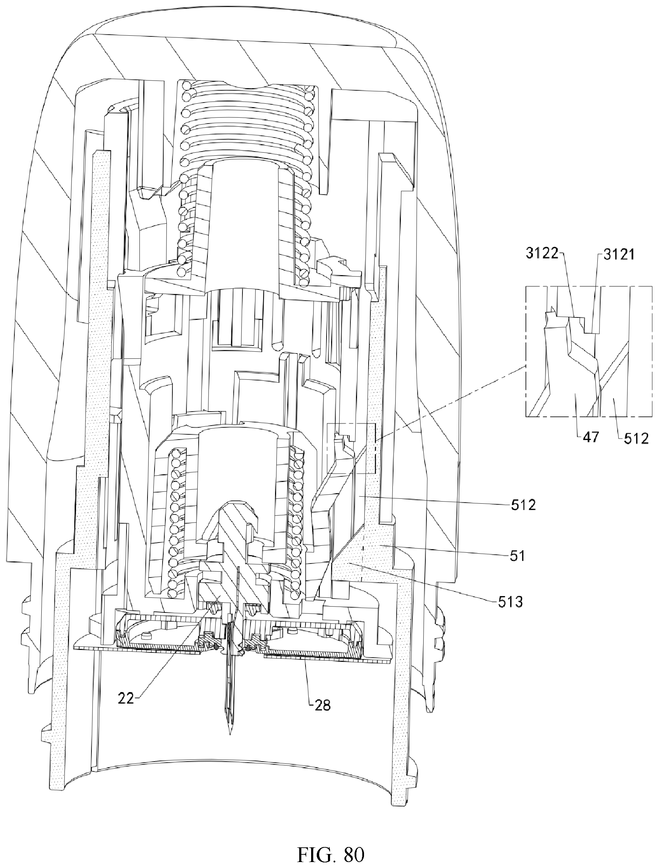

As shown in FIG. 79 and FIG. 80, when the first elastic member 2 applies a downward force to the support 3, the support 3 drives the needle retraction holder 4 to move down. When the support 3 moves to a first position, a middle part of the elastic fastener 47 abuts against the pre-retraction pressure rib 512, and is pressed against and deformed. The elastic fastener 47 abuts against the second stepped surface 3122. There is a height difference between the first stepped surface 3121 and the second stepped surface 3122 in the axial direction. In this case, the needle retraction holder 4 is released from space of the height difference, and the needle retraction holder 4 moves up relative to the support 3 under the action of the second elastic member 6. The gripper of the guide needle snap-fits with the needle retraction holder 4, and the needle retraction holder 4 drives the needle holder 22 to move up relative to the support 3 by a specific distance, so that the end, provided with the threads, of the needle holder 22 can move up relative to the bonding member 28 of the sensor assembly 20. In this case, the end, provided with the threads, of the needle holder 22 moves up, and is approximately flush with the bonding member 28 or is located on a side, close to the needle retraction holder 4, of the bonding member 28. In this way, a pre-retraction action is implemented.

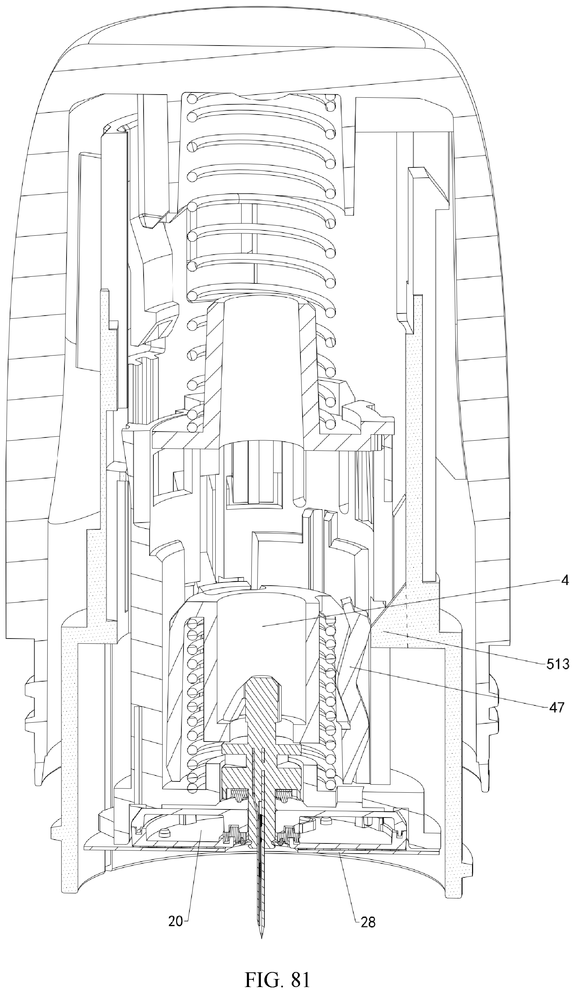

As shown in FIG. 80 and FIG. 81, under the action of the first elastic member 2, the support 3 continues to move down. When the support 3 moves to a second position, the middle part of the elastic fastener 47 abuts against the retraction pressure rib 513, and the retraction pressure rib 513 on the base 5 further presses against the elastic fastener 47, so that the elastic fastener 47 is completely detached from the second stepped surface 3122. The elastic fastener 47 is fully located on an inner side of the side plate of the support, and the support 3 continues to move down. The support 3 moves to a third position, the sensor assembly 20 abuts against the organism 30, and the bonding member 28 of the sensor assembly 20 is in contact with skin of the user. In this way, an implantation action is completed.

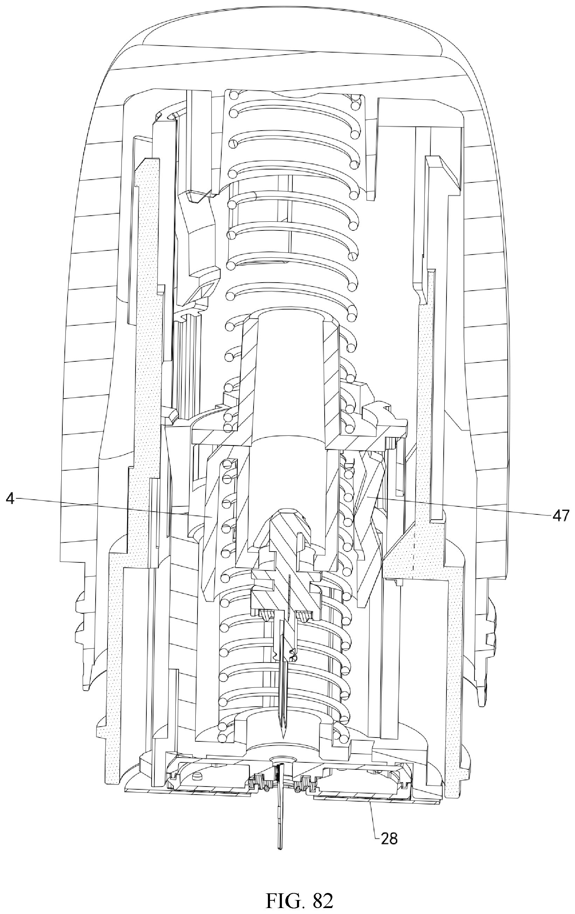

As shown in FIG. 81 and FIG. 82, the bonding member 28 of the sensor assembly 20 may be fastened to the skin of the user. The guide needle is completely retracted under an acting force of the second elastic member 6, to complete a needle retraction function.

It can be understood that this embodiment shows a design of staged needle retraction. The puncture needle 23 may be pulled for a specific distance through pre-extraction, so that threaded plastic on the needle holder 22 pre-retracts during implantation, to prevent a plastic part from getting in contact with skin and pressing against a wound.

In this embodiment, an example in which a passive mechanical drive device (namely, the second elastic member 6) for the puncture needle 23 is a compression spring is used as an example for description.

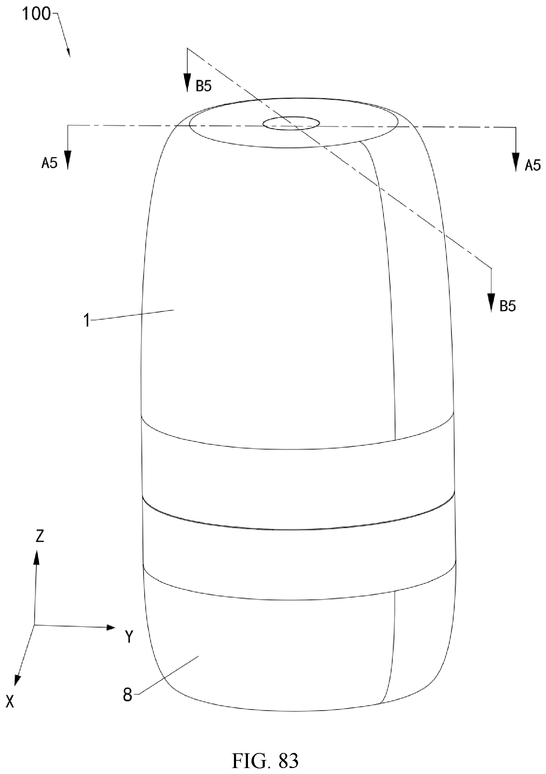

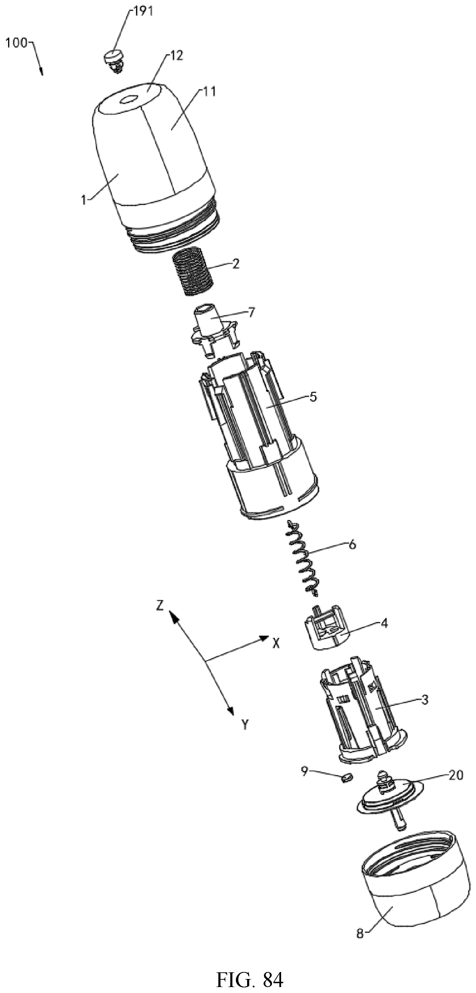

In some implementations, a part that is the same as that in the foregoing embodiments is not described again. FIG. 83 is a diagram of the physiological sign monitoring apparatus 100 shown in FIG. 2 according to yet another implementation. FIG. 84 is a schematic exploded view of the physiological sign monitoring apparatus 100 shown in FIG. 83 according to an implementation.

As shown in FIG. 83 and FIG. 84, the physiological sign monitoring apparatus 100 may include an implantation apparatus 10 and a sensor assembly 20. The implantation apparatus 10 may include a fastening stub 191, a lower housing 1, a first elastic member 2, a spring seat 7, a base 5, a needle retraction holder 4, a second elastic member 6, a support 3, a first magnet 9, and a housing cap assembly 8. For a manner of disposing the first elastic member 2, the spring seat 7, the base 5, the support 3, the first magnet 9, and the housing cap assembly 8, refer to the manner of disposing the first elastic member 2, the spring seat 7, the base 5, the support 3, the first magnet 9, and the housing cap assembly 8 in the foregoing embodiments. Details are not described herein again.

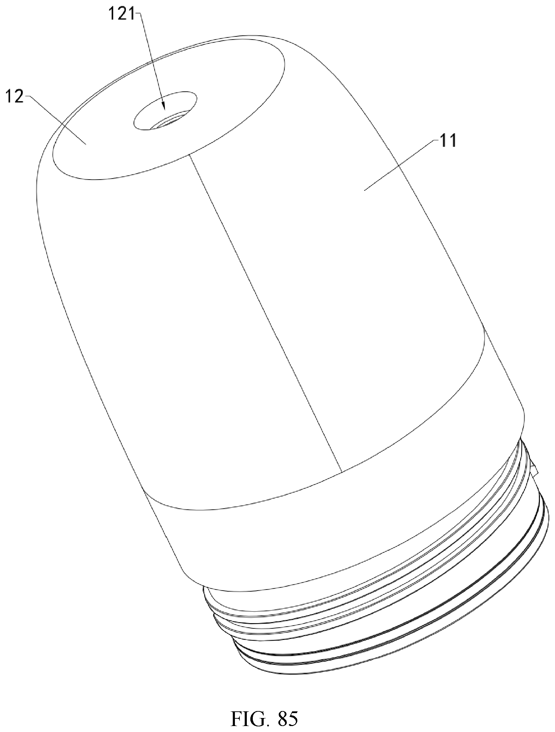

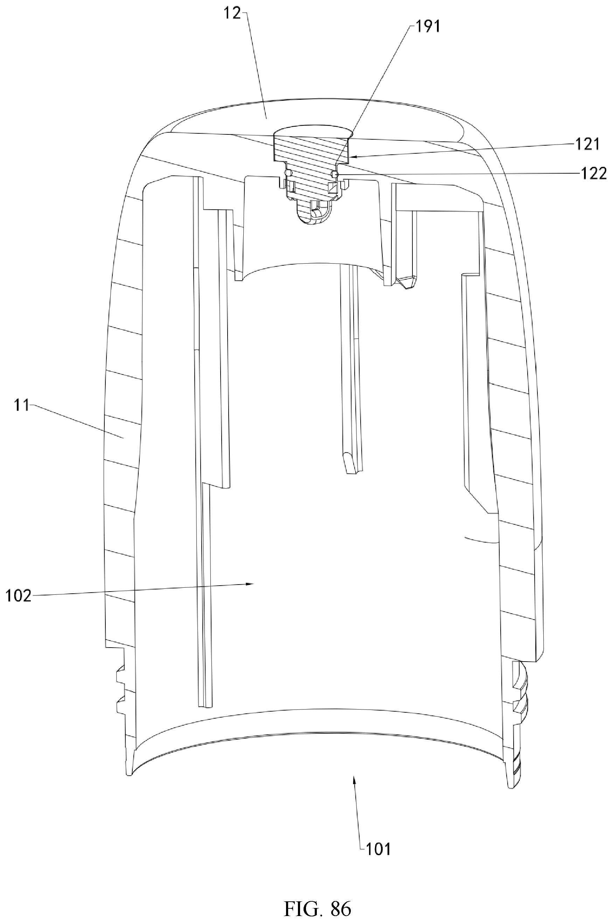

FIG. 85 is a diagram of a structure of the lower housing 1 shown in FIG. 84 according to an implementation. FIG. 86 is a partial cross-sectional view of the structure shown in FIG. 83 along A5-A5 according to an implementation.

As shown in FIG. 85 and FIG. 86, the lower housing 1 may include a top wall 12, a side wall 11, and the fastening stub 191, and the side wall 11 of the lower housing 1 is connected around a periphery of the top wall 12 of the lower housing 1. An opening 101 is formed on a side, away from the top wall 12 of the lower housing 1, of the side wall 11 of the lower housing 1. The lower housing 1 has an internal cavity 102, and the internal cavity 102 is in communication with the opening 101.

For example, the top wall 12 of the lower housing 1 is provided with a through hole 121. The through hole 121 may be in communication with an internal cavity 102. The fastening stub 191 is fastened in the through hole 121, and is partially exposed in the internal cavity 102. For example, a sealing O-ring abuts between the fastening stub 191 and a hole wall of the through hole 121. It can be understood that a gap between the hole wall of the through hole 121 and the fastening stub 191 is sealed by an O-ring 122.

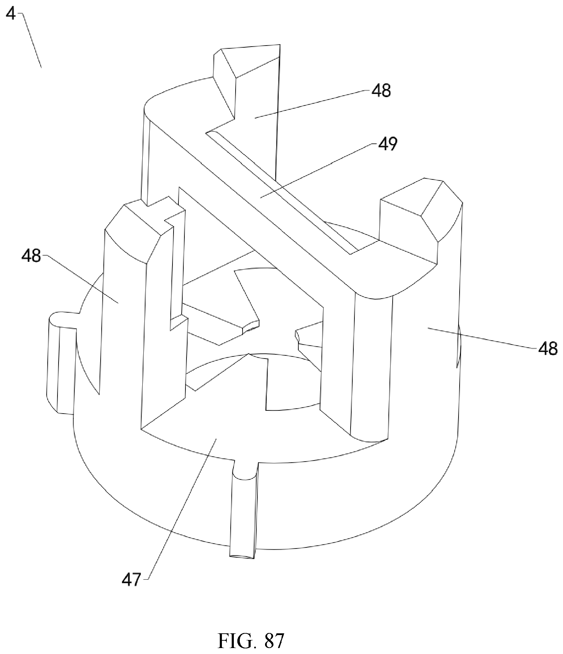

FIG. 87 is a diagram of a structure of the needle retraction holder 4 shown in FIG. 84 according to an implementation.

As shown in FIG. 87, the needle retraction holder 4 may include a bottom plate 47, a plurality of fastening pillars 48, and a beam 49. The plurality of fastening pillars 48 are fastened to a periphery of the bottom plate 47 at spacings. Two ends of the beam 49 are fastened between any two fastening pillars 48. For example, the plurality of fastening pillars 48 include two fastening pillars 48 that are symmetric with respect to a center of the bottom plate 47 of the needle retraction holder 4, and the two ends of the beam 49 are fastened to the two fastening pillars 48 that are symmetric with respect to the center of the bottom plate 47 of the needle retraction holder 4.

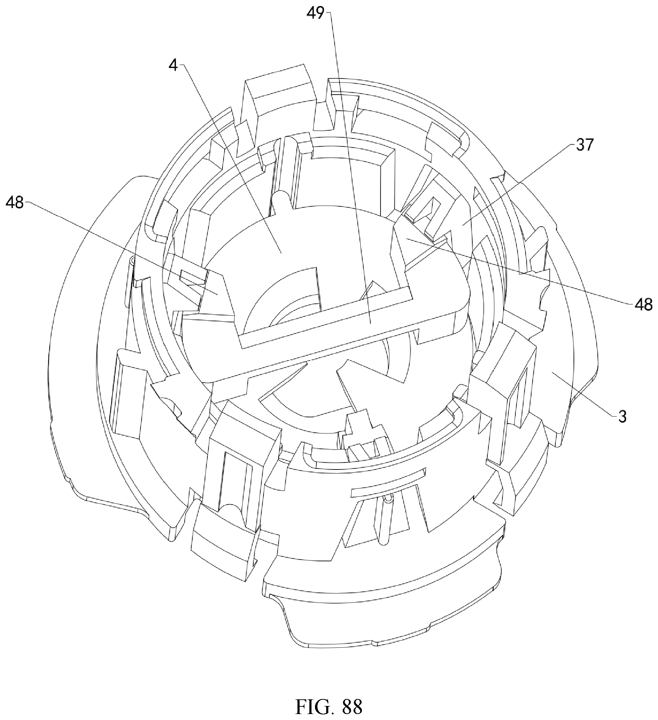

FIG. 88 is a diagram of assembly of the needle retraction holder 4 and the support 3 in FIG. 84 according to an implementation.

As shown in FIG. 88, the needle retraction holder 4 is mounted on an inner side of the support 3. The needle retraction holder 4 is mounted between a bottom plate 32 of the support 3 and a hook of a fourth resilient arm 37. The hook of the fourth resilient arm 37 snaps into the fastening pillar 48 of the needle retraction holder 4. There may be three fourth resilient arms 37 and three fastening pillars, so that the needle retraction holder 4 in snap-fit is subject to balanced forces. In this embodiment, the needle retraction holder 4 is located between the bottom plate 32 of the support 3 and the hook of the fourth resilient arm 37, so that a stable positional relationship is maintained between the needle retraction holder 4 and the support 3, and the fourth resilient arm 37 may enable the needle retraction holder 4 to move together with the support 3.

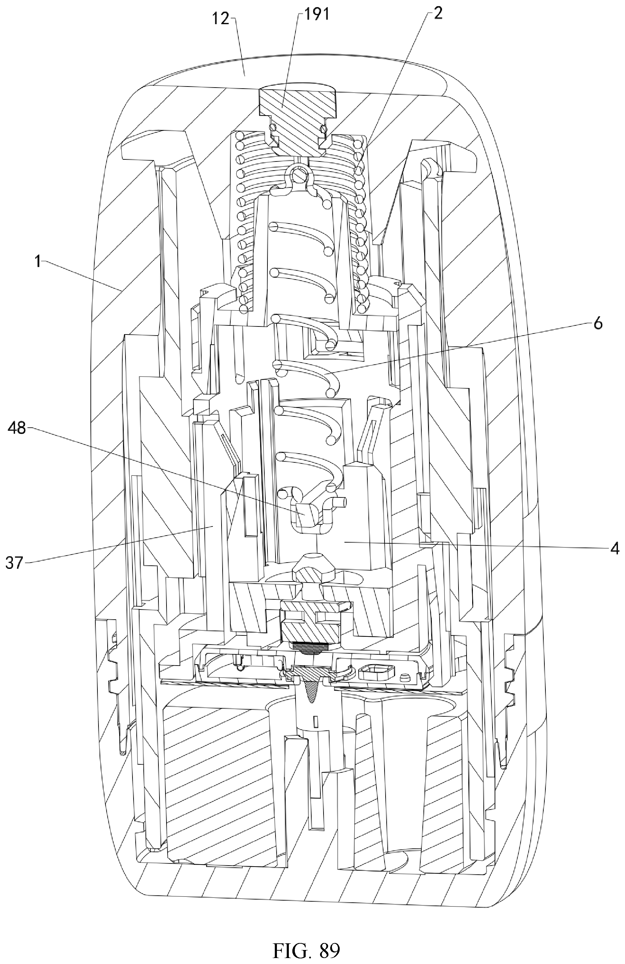

FIG. 89 is a partial cross-sectional view of the structure shown in FIG. 83 along A5-A5 according to an implementation.

As shown in FIG. 89, one end of the second elastic member 6 is fastened to the top wall 12 of the lower housing 1, and the other end of the second elastic member 6 is connected to the needle retraction holder 4. The second elastic member 6 may be located on a side, close to the top wall 12 of the lower housing 1, of the needle retraction holder 44. For example, one end of the second elastic member 6 is fastened to the fastening stub 191 on the top wall 12 of the lower housing 1, and the other end of the second elastic member 6 is connected to the beam 49 of the needle retraction holder 4. The second elastic member 6 may be a tension spring. In the state shown in FIG. 89, the second elastic member 6 is in a stretched state, and applies an upward pulling force to the needle retraction holder 4.

When the apparatus is not in use, the fourth resilient arm 37 of the support 3 abuts against the base 5, so that the hook of the fourth resilient arm 37 can press against the needle retraction holder 4. The needle retraction holder 4 is located between the bottom plate 32 of the support 3 and the hook of the fourth resilient arm 37, so that a stable positional relationship is maintained between the needle retraction holder 4 and the support 3.

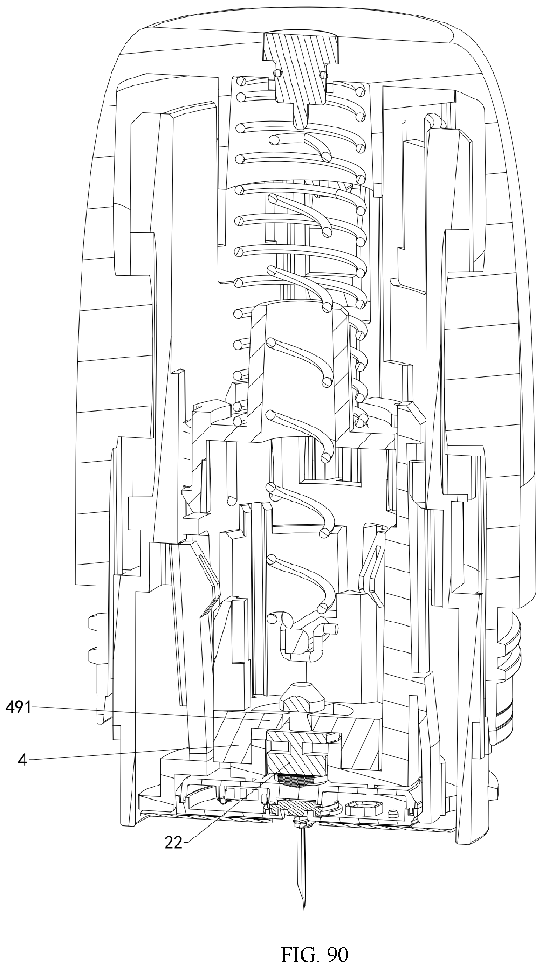

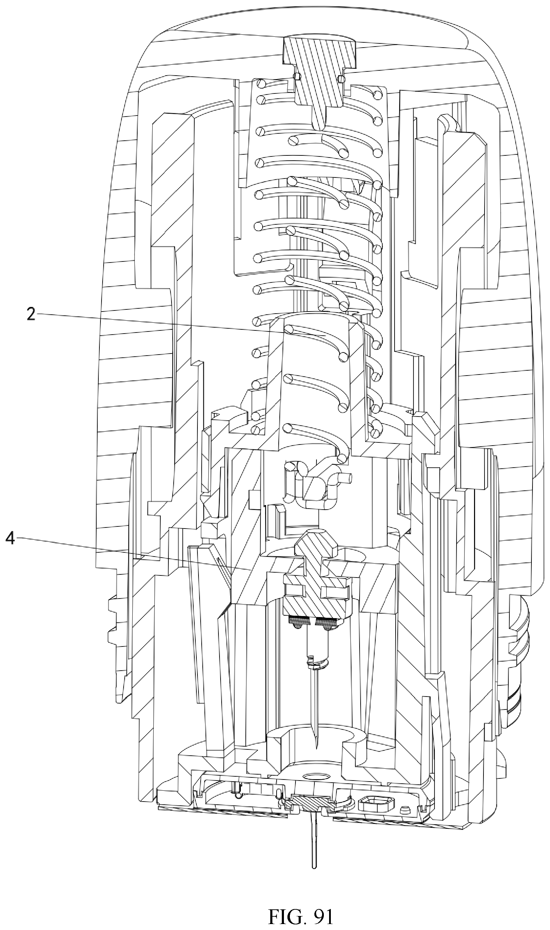

FIG. 90 is a partial cross-sectional view of the physiological sign monitoring apparatus 100 shown in FIG. 83 along B5-B5 during use according to an implementation. FIG. 91 is a partial cross-sectional view of the physiological sign monitoring apparatus 100 shown in FIG. 83 along B5-B5 during use according to an implementation.

As shown in FIG. 89, FIG. 90, and FIG. 91, the needle retraction holder 4 and the needle holder 22 of the guide needle are fastened through a gripper 491, and the guide needle may move together with the needle retraction holder 4. In this implementation, for a manner of disposing the guide needle, refer to the structure of the guide needle shown in FIG. 25b. Details are not described herein again. In this embodiment, the gripper 491 is disposed on the needle retraction holder 4, and is a horizontal gripper.

It can be understood that a type of the gripper 491 may be designed based on a type of the second elastic member 6. The horizontal gripper is disposed in a horizontal direction, so that the guide needle is not likely to be detached. The horizontal gripper facilitates disposing of a mainspring or a tension spring.

When the first elastic member 2 abuts against the support 3 through the spring seat 7, a downward pushing force is applied to the support 3. After a limitation between the base 5 and the support 3 is released (for details, refer to the first embodiment), the support 3 moves down under an acting force of the first elastic member 2. The fourth resilient arm 37 of the support 3 moves to a trigger slot 511, and a limitation on the needle retraction holder 4 is released (for details, refer to the first embodiment). The second elastic member 6 is a tension spring remaining in a stretched state, and applies an upward pulling force to the needle retraction holder 4. Under the action of the upward pulling force of the second elastic member 6, the needle retraction holder 4 drives the guide needle to move up, to complete needle retraction.

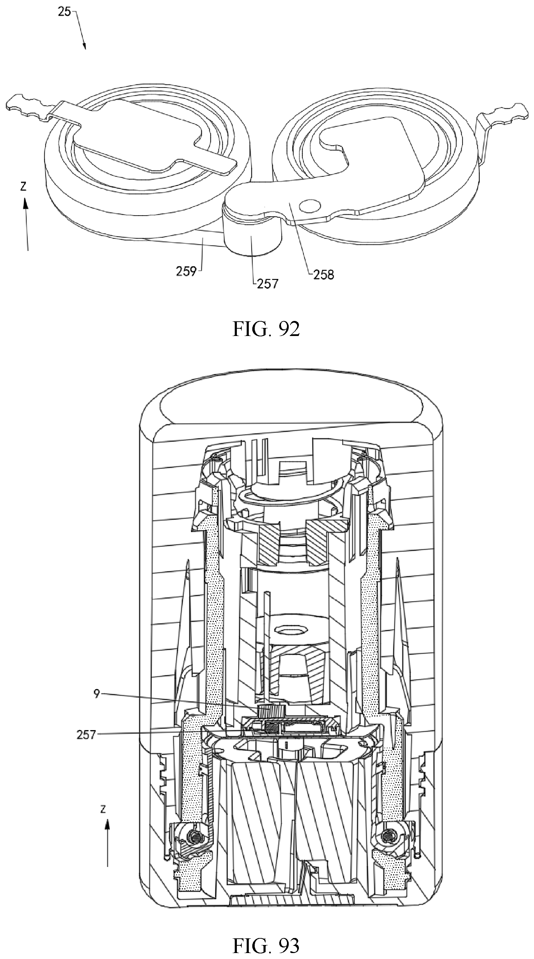

In some implementations, a part that is the same as that in the foregoing embodiments is not described again. FIG. 92 is a diagram of assembly of the battery assembly 25 shown in FIG. 24 according to an implementation. FIG. 93 is a partial cross-sectional view of the physiological sign monitoring apparatus 100 shown in FIG. 2 along B-B according to another implementation.

As shown in FIG. 92 and FIG. 93, the sensor assembly 20 may include a first electrode plate 258 and a second electrode plate 259. The first electrode plate 258 and the second electrode plate 259 are connected to opposite polarities of a battery. To be specific, the first electrode plate 258 and the second electrode plate 259 are electrically connected to a positive electrode and a negative electrode respectively. For example, the first electrode plate 258 may be the second positive electrode plate 255, and the second electrode plate 259 may be the first negative electrode plate 254.

For example, in the axial direction of the physiological sign monitoring apparatus 100, a switch magnet 257 may be located between the first electrode plate 258 and the second electrode plate 259. The switch magnet 257 is fastened to either of the electrode plates. One of the first electrode plate 258 and the second electrode plate 259 that is not fastened to the switch magnet 257 is deformable. For example, the switch magnet 257 may be fastened to the first negative electrode plate 254 and spaced away from the second positive electrode plate 255.

It can be understood that, in the axial direction of the physiological sign monitoring apparatus 100, the switch magnet 257 is located between the first electrode plate 258 and the second electrode plate 259, and the switch magnet 257 is fastened to either of the electrode plates. When the physiological sign monitoring apparatus 100 is in an unused state, the first magnet 9 and/or a second magnet 83 act/acts on the switch magnet 257, so that the switch magnet 257 is spaced away from an unconnected electrode plate, the first electrode plate 258 and the second electrode plate 259 are disconnected (spaced apart), and a sensor 24 is de-energized. When the physiological sign monitoring apparatus 100 is in an in-use state, the switch magnet 257 may be in contact with and electrically connected to the other unconnected electrode plate, to implement conduction between the first electrode plate 258 and the second electrode plate 259, and energize the sensor 24. In comparison with a case in which the switch magnet 257 is located on a same side of the first electrode plate 258 and the second electrode plate 259 and the switch magnet 257 needs to be in contact with both the first electrode plate 258 and the second electrode plate 259 to energize the sensor 24, in this embodiment, the switch magnet 257 needs to be in contact with only an unconnected electrode plate to energize the sensor 24. This reduces a risk of failing to properly energize the sensor 24.

In some implementations, a thickness of an electrode plate fastened to the switch magnet 257 may be less than a thickness of an electrode plate that is not in contact with the switch magnet 257. For example, a thickness of the first negative electrode plate 254 may be less than a thickness of the second positive electrode plate 255. In this way, the switch magnet 257 is more likely to drive the electrode plate to deform, and a magnetic force of the switch magnet 257 may be set to be small. This helps reduce a size of the switch magnet 257.

For example, an ultra-thin steel sheet may be used as an electrode plate fastened to the switch magnet 257. For example, the first electrode plate 258 is fastened to the switch magnet 257, and the first electrode plate 258 may include an ultra-thin steel sheet. The entire first electrode plate 258 may be the ultra-thin steel sheet, or the ultra-thin steel sheet may be used as an insert. It can be understood that the ultra-thin steel sheet is conductive and has high strength.

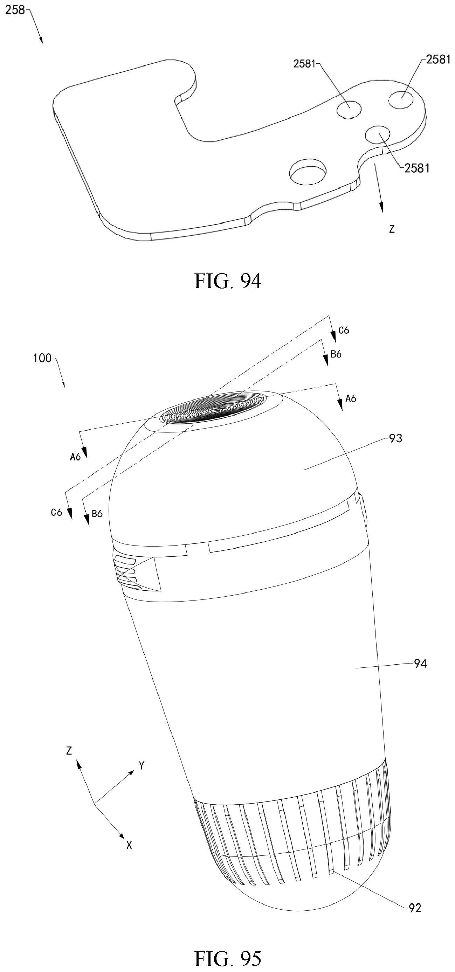

FIG. 94 is a diagram of a structure of the first electrode plate 258 shown in FIG. 91 from another perspective according to an implementation.

As shown in FIG. 92 and FIG. 94, when the first electrode plate 258 is movably connected to the switch magnet 257 and the second electrode plate 259 is fastened to the switch magnet 257, a bump 2581 may be provided on a surface on a side, facing the switch magnet 257, of the first electrode plate 258. In this way, in comparison with a solution in which no bump 2581 is provided, in this embodiment, the bump 2581 is provided on the surface, facing the switch magnet 257, of the first electrode plate 258. When the switch magnet 257 is in contact with the first electrode plate 258 under the action of the first magnet 9 and/or the second magnet 83, in the solution in which the bump 2581 is provided, under a force with a same magnitude, contact pressure between the switch magnet 257 and the first electrode plate 258 is higher, and an electrical connection is reliable.

In some implementations, a part that is the same as that in the foregoing embodiments is not described again. For ease of understanding, in this embodiment, an axial direction of the physiological sign monitoring apparatus 100 is a Z-axis direction, a length direction of the physiological sign monitoring apparatus 100 is an X direction, and a width direction of the physiological sign monitoring apparatus 100 is a Y direction. A direction of a connection line between two buttons 951 of the physiological sign monitoring apparatus 100 is the width direction.

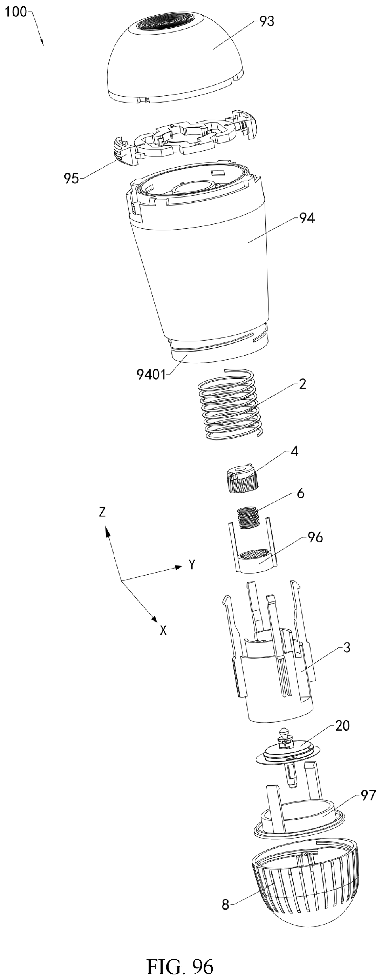

FIG. 95 is a diagram of a structure of the physiological sign monitoring apparatus 100 shown in FIG. 2 according to yet another implementation. FIG. 96 is a schematic exploded view of a partial structure of the physiological sign monitoring apparatus 100 shown in FIG. 95 according to an implementation.

As shown in FIG. 95 and FIG. 96, the physiological sign monitoring apparatus 100 may include an implantation apparatus 10 and a sensor assembly 20. The implantation apparatus 10 may include a front sealing cover 92, a rear sealing cover 93, a housing 94, a trigger button module 95, a first elastic member 2, a needle retraction holder 4, a helical fastener 96, a second elastic member 6, a support 3, a drying member 82, and a concealing cover 97. The drying member 82 may be fastened to the front sealing cover 92 to form a housing cap assembly 8.

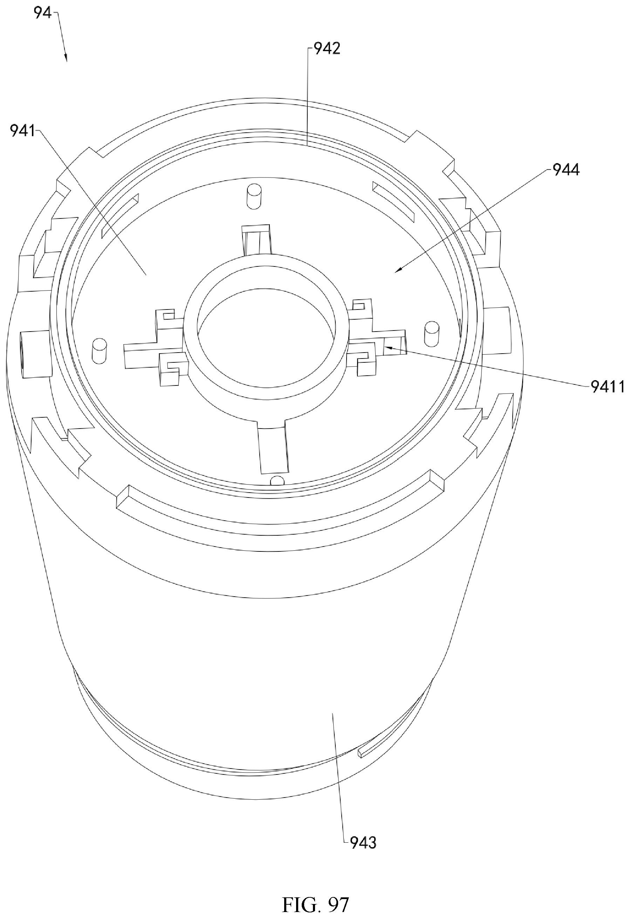

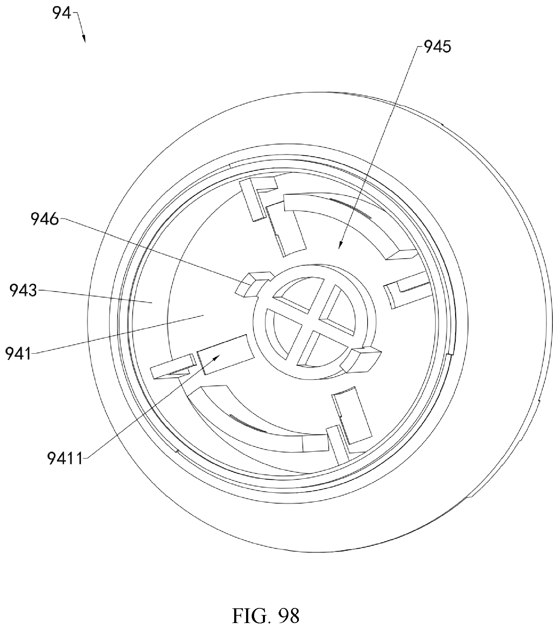

FIG. 97 is a diagram of a structure of the housing 94 shown in FIG. 96 according to an implementation. FIG. 98 is a diagram of a structure of the housing 94 shown in FIG. 97 from another perspective.

As shown in FIG. 97 and FIG. 98, the housing 94 includes a middle plate 941, an upper side wall 942, and a lower side wall 943. The middle plate 941 is connected between the upper side wall 942 and the lower side wall 943. The upper side wall 942 is connected to a periphery of the middle plate 941. The lower side wall 943 is connected to the periphery of the middle plate 941. The upper side wall 942 and the middle plate 941 enclose first space 944. The lower side wall 943 and the middle plate 941 enclose second space 945.

In some implementations, the middle plate 941 may be provided with a first snap-fit hole 9411. The first snap-fit hole 9411 is in communication with the first space 944 and the second space 945.

In some implementations, the housing 94 has an abutting end 9401 (as shown in FIG. 96). The abutting end 9401 is an end, close to the front sealing cover 92, of the housing 94.

In some implementations, the housing 94 may further include a limiting rib 946 (as shown in FIG. 98).

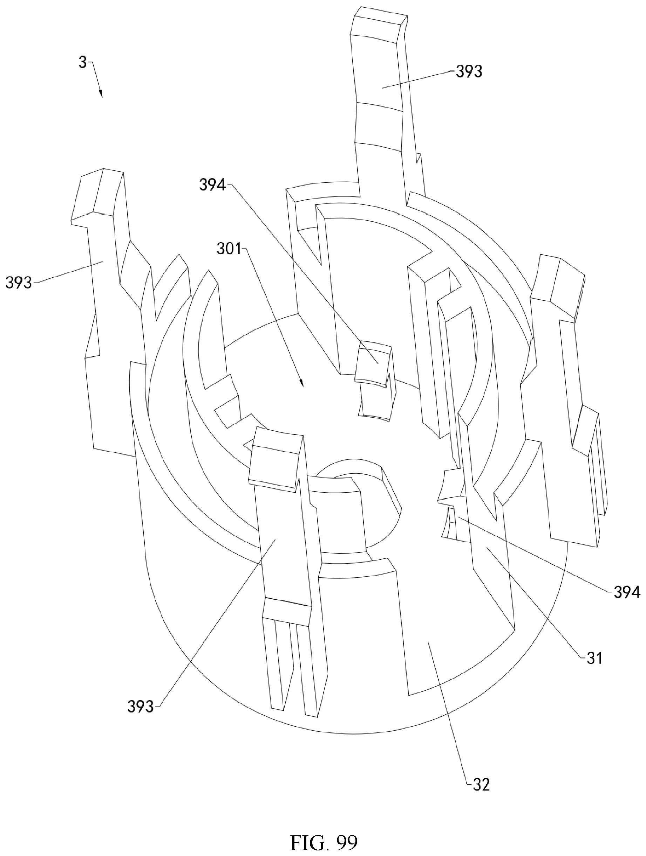

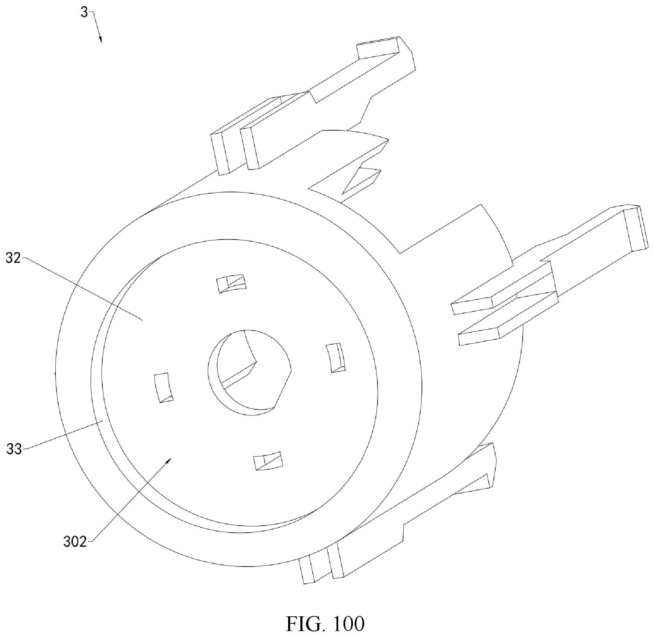

FIG. 99 is a diagram of a structure of the support 3 shown in FIG. 96 according to an implementation. FIG. 100 is a diagram of a structure of the support 3 shown in FIG. 99 from another perspective.

As shown in FIG. 99 and FIG. 100, the support 3 may include an upper side plate 31, a bottom plate 32, and a lower side plate 33. The bottom plate 32 of the support 3 is connected to the upper side plate 31 of the support 3 and the lower side plate 33 of the support 3. A periphery of the upper side plate 31 of the support 3 is fastened to the bottom plate 32 of the support 3. The upper side plate 31 of the support 3 encloses first accommodation space 301. The lower side plate 33 of the support 3 is connected around a periphery of the bottom plate 32 of the support 3. The lower side plate 33 of the support 3 encloses second accommodation space 302.

In some implementations, the support 3 may further include a plurality of snap-fit arms 393. One end of the snap-fit arm 393 is fastened to an end, away from the lower side plate 33, of the upper side plate 31 of the support 3. For example, the upper side plate 31, the bottom plate 32, and the lower side plate 33 of the support 3 may constitute a body portion of the support.

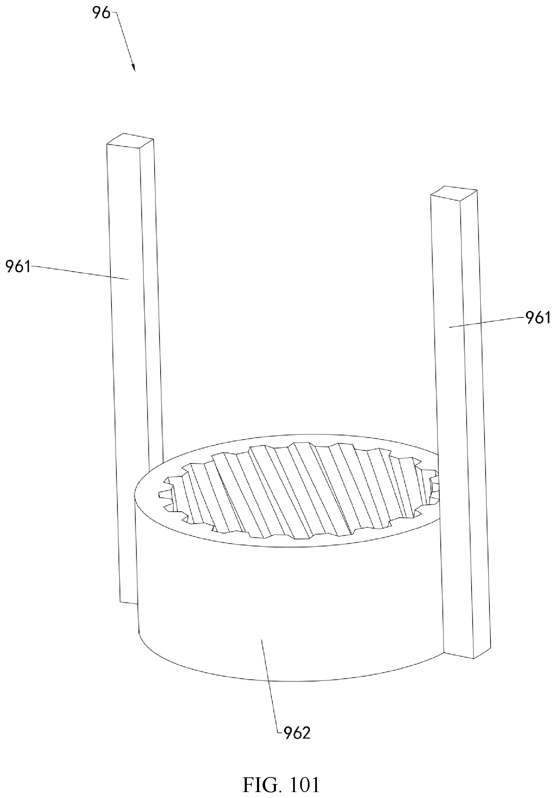

FIG. 101 is a diagram of a structure of the helical fastener 96 shown in FIG. 96 according to an implementation.

As shown in FIG. 101, the helical fastener 96 includes a limiting rib 961 and a rotation portion 962. The rotation portion 962 is approximately in a cylindrical shape. An inner side surface of the rotation portion 962 is provided with inclined teeth for fitting with inclined teeth on an outer side surface of the needle retraction holder 4. The needle retraction holder 4 is mounted on an inner side of the rotation portion 962. The inclined teeth of the needle retraction holder 4 fit with the inclined teeth of the rotation portion 962.

For example, the limiting rib 961 is fastened to a periphery of the rotation portion 962. There are a plurality of limiting ribs 961, and the plurality of limiting ribs 961 are spaced apart around an axial direction of the rotation portion 962. For example, there are two limiting ribs 961, and the two limiting ribs 961 are symmetrically arranged at 180°. For ease of distinguishing, in the following descriptions, the limiting rib 961 of the helical fastener 96 is referred to as a first limiting rib 961, and the limiting rib 946 of the housing 94 is referred to as a second limiting rib 946.

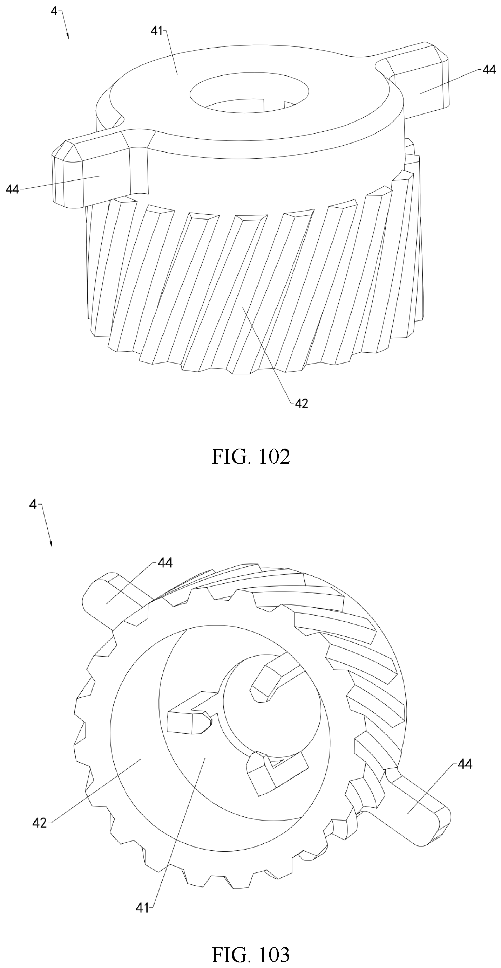

FIG. 102 is a diagram of a structure of the needle retraction holder 4 shown in FIG. 96 according to an implementation. FIG. 103 is a diagram of a structure of the needle retraction holder 4 shown in FIG. 102 from another perspective.

As shown in FIG. 102 and FIG. 103, the outer side surface of the needle retraction holder 4 may be provided with inclined teeth. An extension direction of the inclined teeth is different from an axial direction of the needle retraction holder 4. For example, the needle retraction holder 4 may include a top plate 41 and a side plate 42. The side plate 42 is fastened around a periphery of the top plate 41. An outer side surface of the side plate 42 may be provided with inclined teeth.

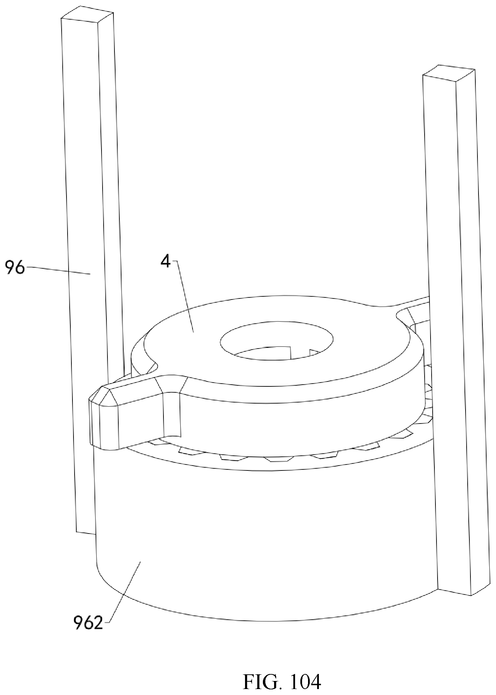

FIG. 104 is a diagram of assembly of the needle retraction holder 4 and the helical fastener 96 shown in FIG. 96 according to an implementation.

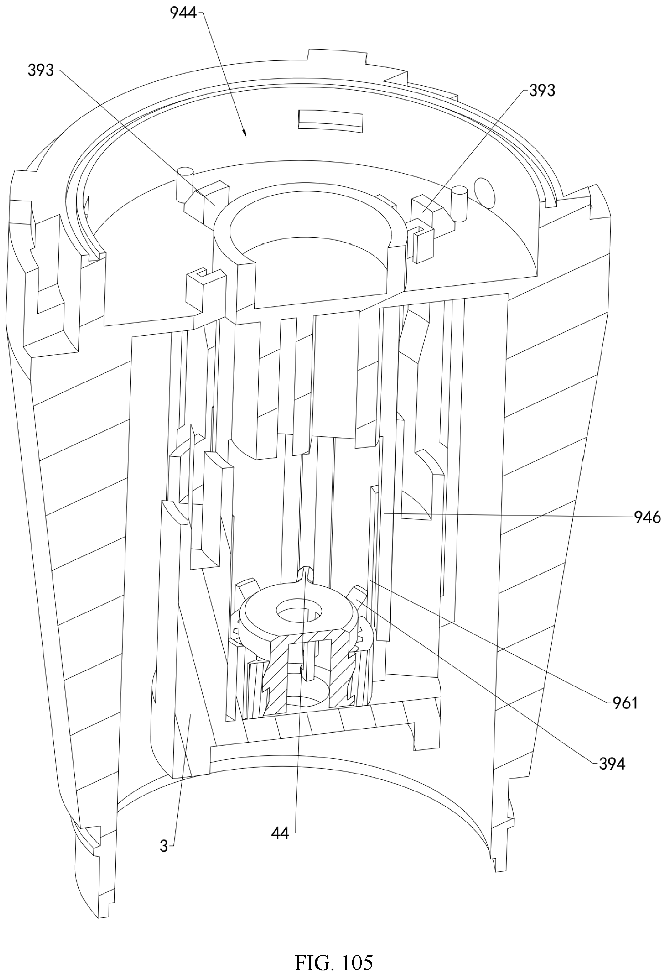

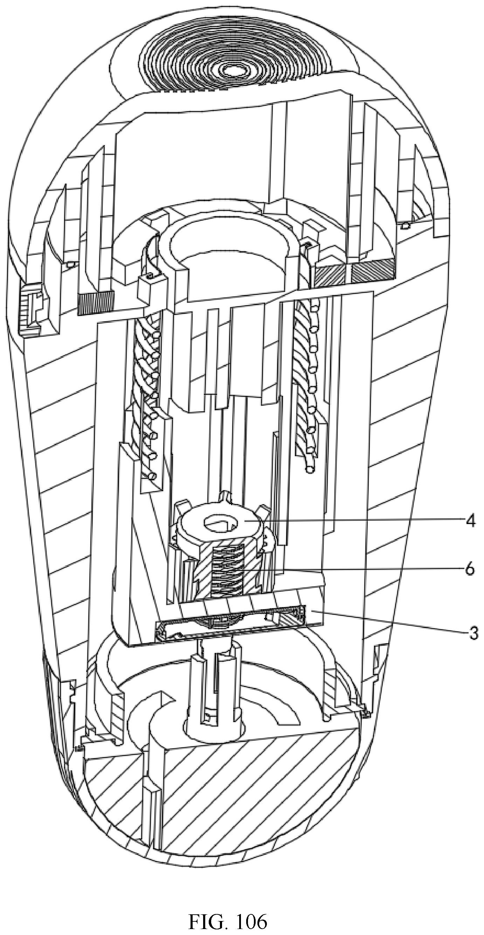

FIG. 105 is a partial cross-sectional view of the physiological sign monitoring apparatus 100 shown in FIG. 95 along A6-A6 according to an implementation. FIG. 106 is a partial cross-sectional view of the physiological sign monitoring apparatus 100 shown in FIG. 95 along B6-B6 according to an implementation.

As shown in FIG. 104 to FIG. 106, the rotation portion 962 may be fastened to the support 3. The needle retraction holder 4 may be rotatably connected to an inner side of the rotation portion 962. The second elastic member 6 abuts between the needle retraction holder 4 and the support 3. The second elastic member 6 may be a compression spring, and the second elastic member 6 may apply an upward force to the needle retraction holder 4. The needle retraction holder 4 applies a circumferential rotation force to the helical fastener 96 through fitting between inclined teeth. The first limiting rib 961 abuts against the second limiting rib 946 in a circumferential direction. Therefore, a relative position between the needle retraction holder 4 and the support 3 is fixed.

For example, the other end of the snap-fit arm 393 enters the first space 944 through the first snap-fit hole 9411. The other end of the snap-fit arm 393 is provided with a hook. The hook is located in the first space 944, and snaps into the middle plate 941 of the housing 94.

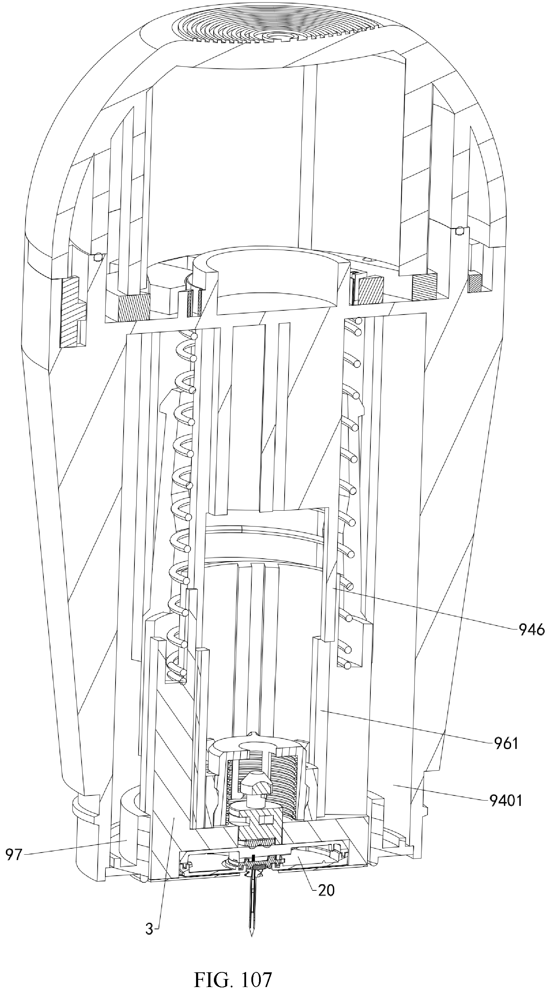

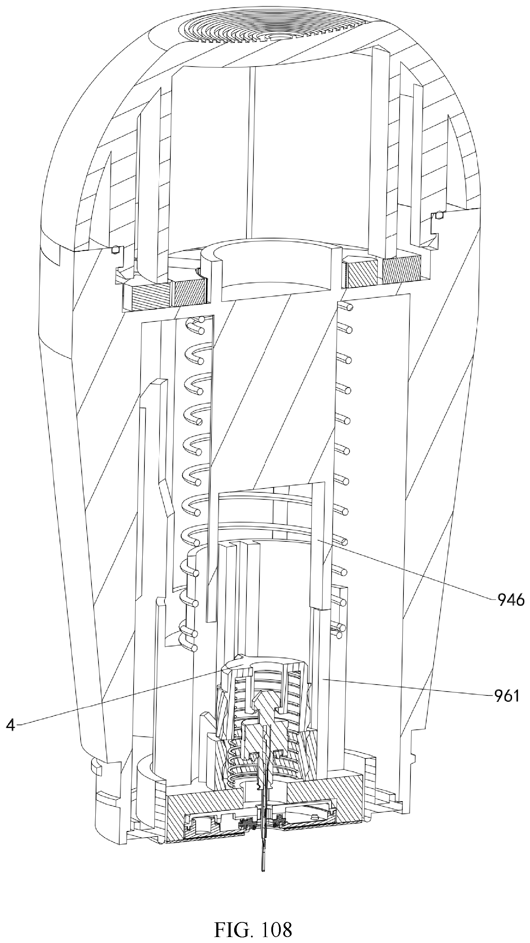

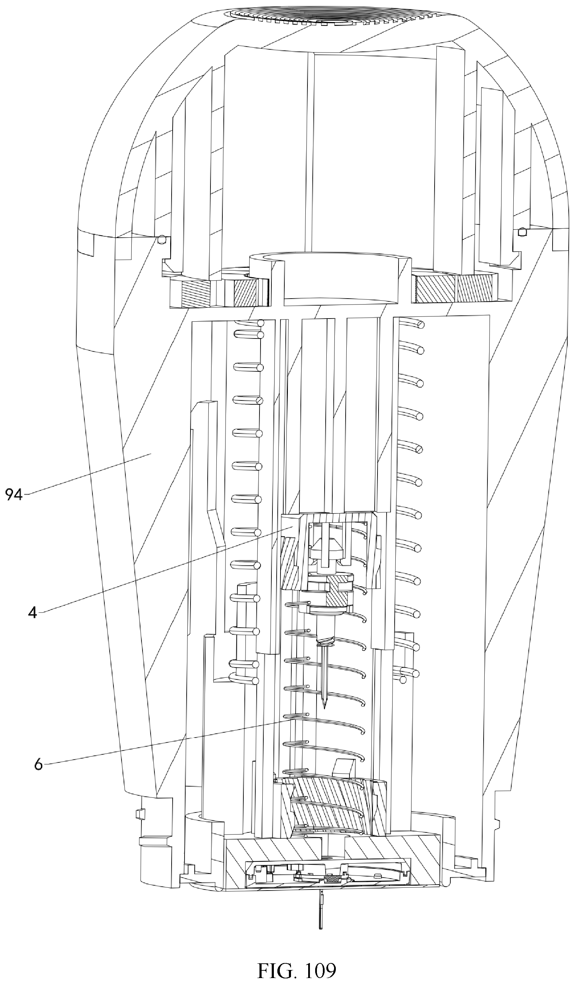

FIG. 107 to FIG. 109 are partial cross-sectional views of the physiological sign monitoring apparatus 100 shown in FIG. 95 along B6-B6 during use according to an implementation.

As shown in FIG. 107 to FIG. 109, the following describes deployment of the sensor 24 in this embodiment. The housing cap assembly 8 is removed, and the abutting end 9401 of the housing 94 abuts against tissue of the user. When the trigger button module 95 releases a snap-fit constraint between the snap-fit arm 393 of the support 3 and the housing 94, the snap-fit arm 393 of the support 3 exits the first snap-fit hole 9411, the support 3 is subject to an acting force of the first elastic member 2, and the support 3 moves in a direction toward the organism 30, to drive the sensor assembly 20 to move down. When a bonding member 28 of the sensor assembly 20 is approximately flush with an end face (a trigger position of a needle retraction action) of the abutting end 9401 of the housing 94, a constraint between the first limiting rib 961 and the second limiting rib 946 is released, and the first limiting rib 961 is detached from the second limiting rib 946. The helical fastener 96 may be driven, by an acting force applied by the needle retraction holder 4 through the inclined teeth, to rotate. A constraint on the needle retraction holder 4 is released, and the needle retraction holder 4 is unlocked. Under the action of an abutting force of the second elastic member 6, the needle retraction holder 4 may move up together with a guide needle, to complete needle retraction.

For example, the outer side surface of the needle retraction holder 4 may be provided with a guide bar 44, and an inner side surface of the lower side wall 943 of the housing 94 may be provided with a guide slot 947. An extension direction of the guide slot 947 may be used to provide guidance for a slide direction of the needle retraction holder 4. In addition, the needle retraction holder 4 may be further limited in the circumferential direction through fitting between the guide bar 44 and the guide slot 947, to avoid rotation of the needle retraction holder 4.

For example, the support 3 may further include a limiting arm 394. One end of the limiting arm 394 is fastened to a side, facing the upper side plate 31 of the support 3, of the bottom plate 32 of the support 3. The other end of the limiting arm 394 may be provided with a hook. The hook faces the bottom plate 32 of the support 3. The rotation portion 962 of the helical fastener 96 snap fits between the bottom plate 32 of the support 3 and the hook of the limiting arm 394, to implement position limiting for the helical fastener 96 in the axial direction, and prevent the helical fastener 96 from directly sliding in the axial direction.

In some implementations, the concealing cover 97 snaps into an inner side of the lower side wall 943 of the housing 94, and is partially exposed from the housing 94. The concealing cover 97 may be configured to cover mechanism devices, inside the housing 94, of the physiological sign monitoring apparatus 100. When the user uses the physiological sign monitoring apparatus 100, the user cannot observe most internal mechanism devices from an external perspective, and overall appearance is tidy and appealing.

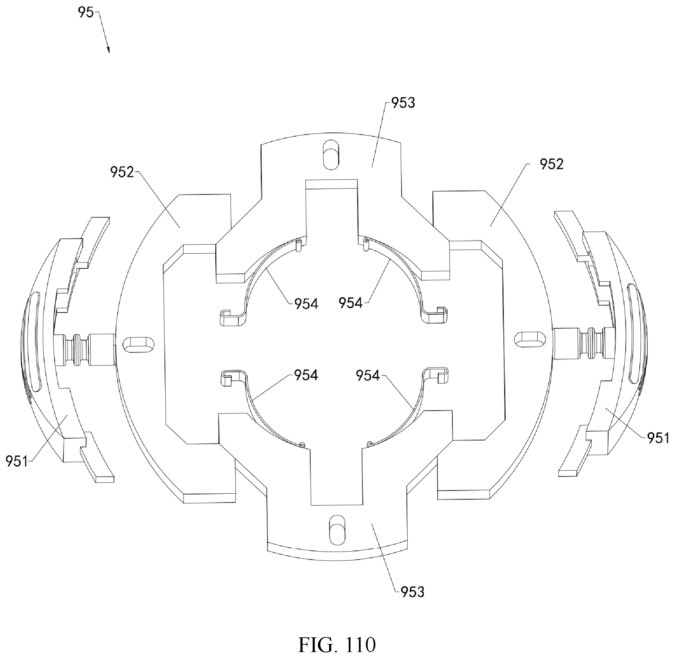

With reference to FIG. 110 to FIG. 112, the following specifically describes a process of releasing, by the trigger button module 95, a constraint on the support 3.

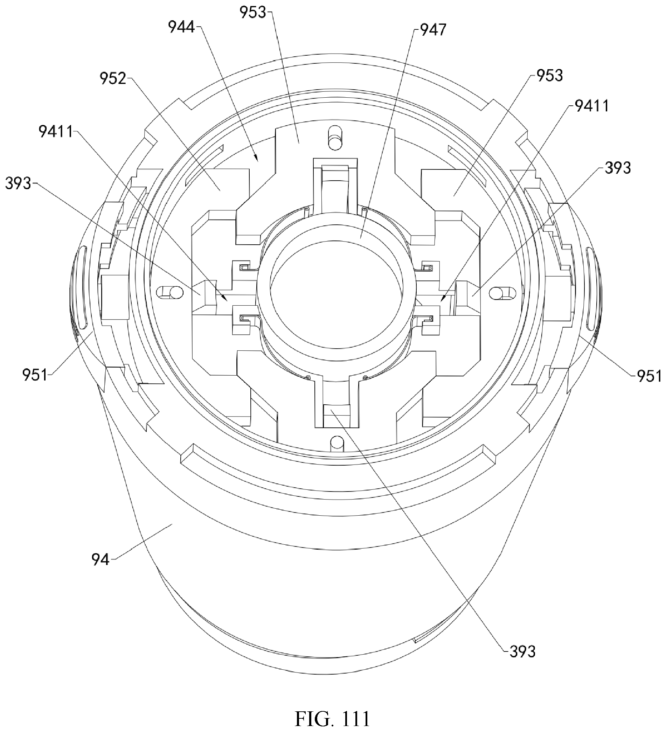

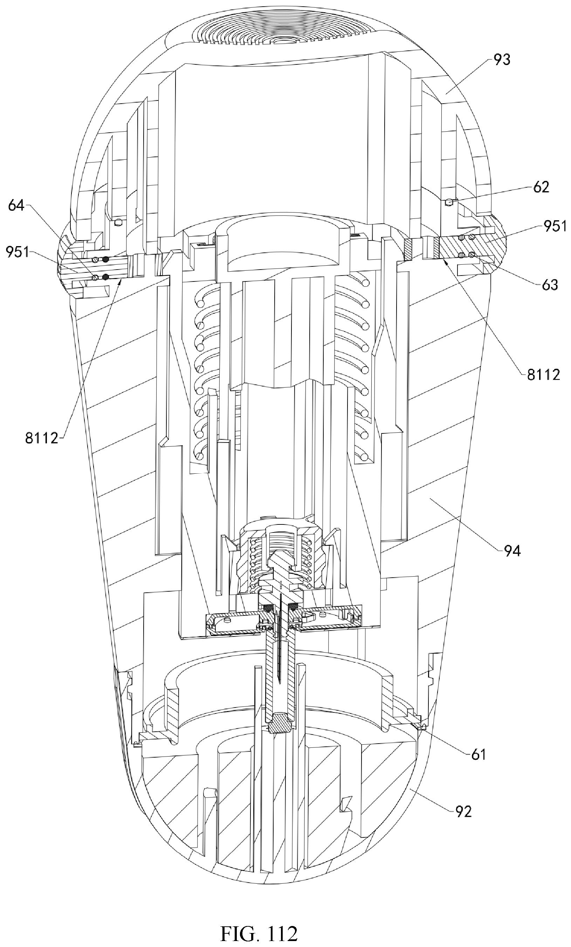

FIG. 110 is a diagram of a structure of the trigger button module 95 shown in FIG. 96 according to an implementation. FIG. 111 is a diagram of assembly of the trigger button module 95, the housing 94, and the support 3 shown in FIG. 96 according to an implementation. FIG. 112 is a partial cross-sectional view of the physiological sign monitoring apparatus 100 shown in FIG. 95 along C6-C6 according to an implementation.

As shown in FIG. 110 to FIG. 112, the trigger button module 95 may include two buttons 951, two first push blocks 952, at least one second push block 953, and an elastic member 954. For example, there may be two second push blocks 953. The two second push blocks 953 are spaced apart in a second direction, and are respectively located on two sides of the central axis of the physiological sign monitoring apparatus 100.

For example, the elastic member 954 may be a spring.

For example, the upper side wall 942 of the housing 94 is provided with two mounting holes 8112, and the two mounting holes 8112 are arranged opposite to each other and are symmetric with respect to an axis of the housing 94. The button 951 is slidably connected to the mounting hole 8112. One end of the button 951 is exposed to the first space 944 of the housing 94. The other end of the button 951 is exposed to an outer side surface of the housing 94.

For example, the two first push blocks 952 are spaced apart in a first direction, and the two second push blocks 953 are spaced apart in the second direction. The first direction is different from the second direction, and the first direction is a direction in which the two buttons 951 are connected. The two first push blocks 952 and the two second push blocks 953 surround the first snap-fit hole 9411. The second push block 953 is partially located between the two first push blocks 952. The two first push blocks 952 respectively press against outer sides of two ends of the second push block 953.

For example, two ends of each second push block 953 abut against the two first push blocks 952. When the user simultaneously presses the two buttons 951, the two first push blocks 952 move toward the middle, and press against outer sides of the two second push blocks 953. For example, a contact surface between the second push block 953 and the first push block 952 is an inclined surface. On the inclined surface, the second push block 953 is subject to an abutting force of the first push block 952, and the two second push blocks 953 also move toward the middle.

For example, the support 3 may include four snap-fit arms 393, and each snap-fit arm 393 corresponds to one first push block 952 or second push block 953. A snap-fit arm 393 arranged opposite to the first push block 952 in the first direction is a first snap-fit arm. A snap-fit arm 292 arranged opposite to the second push block 953 in the second direction is a second snap-fit arm. The second direction and the first direction form an included angle. An end, away from the body portion of the support 3, of the first snap-fit arm has a first hook. An end, away from the body portion of the support 3, of the second snap-fit arm has a second hook. Both the first snap-fit arm and the second snap-fit arm are partially located in the first snap-fit hole 9411. The first hook is located in the first space 944 and snaps into the middle plate. The second hook is located in the first space 944 and snaps into the middle plate. For example, the first direction is a direction of a connection line between the two buttons 951.

In some implementations, the front sealing cover 92 may be detachably fastened to an end, away from the middle plate, of the lower side wall 943. The front sealing cover 92 may cover the end, away from the middle plate, of the lower side wall 943, and close the second space 945.

In some implementations, when the front sealing cover 92 is detached from the lower side wall 943 and the user simultaneously presses the two buttons 951, the first push block 952 or the second push block 953 moves toward the middle, and presses against a corresponding snap-fit arm 393, so that the snap-fit arm 393 exits the first space 944 through the first snap-fit hole 9411, to release a limitation on the support 3. Under the action of the first elastic member 2, the support 3 moves down. For example, both of the two buttons 951 are subject to pressure toward the central axis of the physiological sign monitoring apparatus 100 and push the two first push blocks 952 to the central axis of the physiological sign monitoring apparatus. The two first push blocks 952 push the second push block 953 to the central axis of the physiological sign monitoring apparatus 100. The first hook and the second hook enter the second space 945 from the first space 944. The first elastic member 2 pushes the support 3 toward the organism 30. The sensor assembly 20 abuts against the organism 30, and the sensor 24 is implanted into the organism 30 under guidance of the puncture needle 23.

For example, the two ends of the second push block 953 are respectively located on sides, close to the central axis of the physiological sign monitoring apparatus 100, of the two first push blocks 952. In this way, the first push block 952 can push the second push block 953 to move toward the central axis of the physiological sign monitoring apparatus 100.

For example, the first snap-fit arm may be partially located on a side, close to the central axis of the physiological sign monitoring apparatus 100, of the first push block 952. In this way, the first push block 952 can push the first snap-fit arm to move toward the central axis of the physiological sign monitoring apparatus 100.

For example, the second snap-fit arm may be partially located on a side, close to the central axis of the physiological sign monitoring apparatus 100, of the second push block 953. In this way, the second push block 953 can push the second snap-fit arm to move toward the central axis of the physiological sign monitoring apparatus 100.

It can be understood that there may be one or more first snap-fit holes 9411. When there is one first snap-fit hole 9411, a plurality of snap-fit arms 393 are located in one first snap-fit hole 9411. When there are a plurality of first snap-fit holes 9411, a quantity of first snap-fit holes 9411 may be the same as a quantity of snap-fit arms 393, and a plurality of snap-fit arms 393 may be arranged in a one-to-one correspondence with a plurality of first snap-fit holes 9411.

In some implementations, the elastic member 954 may be configured to enable the first push block 952 or the second push block 953 to reset after the limitation on the support 3 is released. For example, there are four elastic members 954, with two elastic members 954 in one group. The housing 94 may be further provided with a boss 947. The boss 947 is fastened to a side, facing the upper side wall 942, of the middle plate 941 of the housing 94. For example, the boss 947 is fastened to a middle part of the middle plate 941 of the housing 94. The two first push blocks 952 and the two second push blocks 953 are arranged around the boss 947, and may be located between the boss 947 and the upper side wall 942. The four elastic members 954 are arranged around the boss 947. The four elastic members 954 are located between the second push block 953 and the boss 947. Two elastic members 954 in a same group abut against inner side surfaces of two ends of a second push block 953 and an outer side of the boss 947 of the housing 94, and are spaced away from the first push block 952.

It can be understood that, when the user simultaneously presses the two buttons 951, the first push block 952 presses against the second push block 953 toward the middle, and the elastic member 954 is pressed against. After the user releases pressure on a first button 951 and a second button 951, a restoring force of the elastic member 954 may push the second push block 953 to return to an original initial position. In addition, the second push block 953 abuts against the first push block 952. In a process in which the second push block 953 resets under a force of the elastic member 954, the first push block 952 may also be pushed back to an initial position. The first push block 952 moves outward to further push the two buttons 951 back to positions at which the buttons 951 are located before being pressed.

In some implementations, when there is one second push block 953, there may be two elastic members 954, and the second push block 953 includes a first abutting end and a second abutting end. The first abutting end abuts between one first push block 952 and one elastic member 954. The second abutting end abuts between the other first push block 952 and the other elastic member 954.

It can be understood that a quantity of elastic members 954 may be twice a quantity of second push blocks 953, and two abutting ends (the first abutting end and the second abutting end) of the second push block 953 that abut against the first push block 952 each correspond to one elastic member. In this way, the second push block 953 is subject to uniform forces.

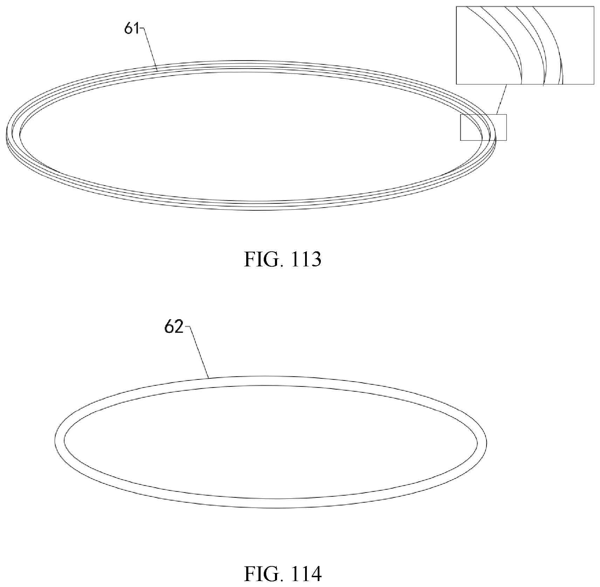

FIG. 113 is a diagram of a structure of a sealing pad 61 shown in FIG. 112 according to an implementation.

As shown in FIG. 112 and FIG. 113, the implantation apparatus 10 may further include the sealing pad 61 (as shown in FIG. 113), and the sealing pad 61 may be used for sealing between the front sealing cover 92 and the housing 94. The sealing pad 61 may be fastened to the front sealing cover 92 or the housing 94. When the front sealing cover 92 is tightened with the housing 94 through threads, the sealing pad 61 may abut between the front sealing cover 92 and the housing 94, to implement sealing. The sealing pad 61 may be a silicone pad or a rubber pad.

FIG. 114 is a diagram of a structure of a sealing ring 62 shown in FIG. 112 according to an implementation.

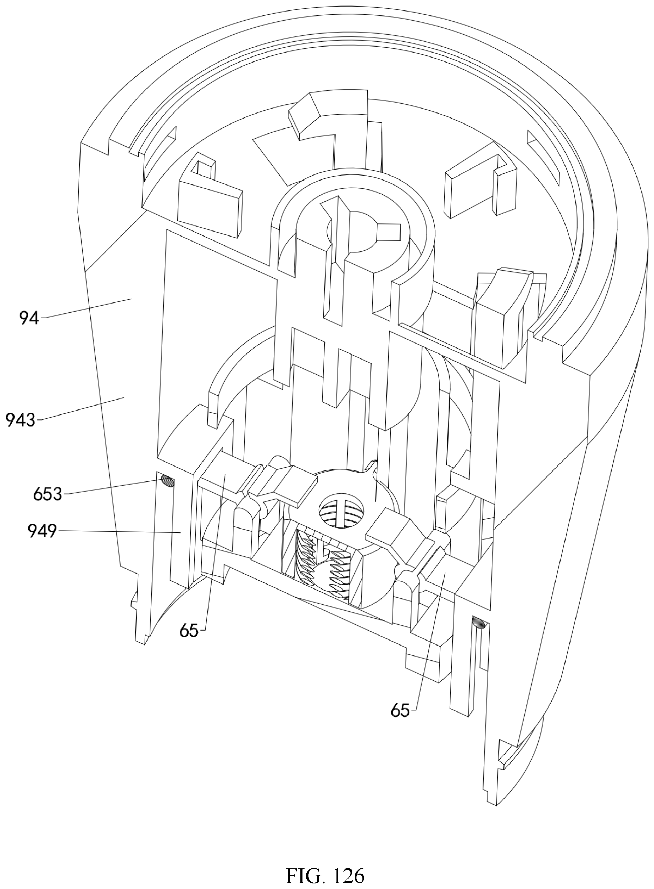

As shown in FIG. 112 and FIG. 114, the implantation apparatus 10 may further include the sealing ring 62 (as shown in FIG. 126), and the sealing ring 62 may be used for sealing between the rear sealing cover 93 and the housing 94. The sealing ring 62 may be fastened to the upper side wall 942 of the housing 94 or the rear sealing cover 93. The rear sealing cover 93 may snap into the upper side wall 942 of the housing 94 through a fastener, and the sealing ring 62 may abut between the rear sealing cover 93 and the housing 94, to implement sealing. The sealing ring 62 may be a silicone ring or a rubber ring.

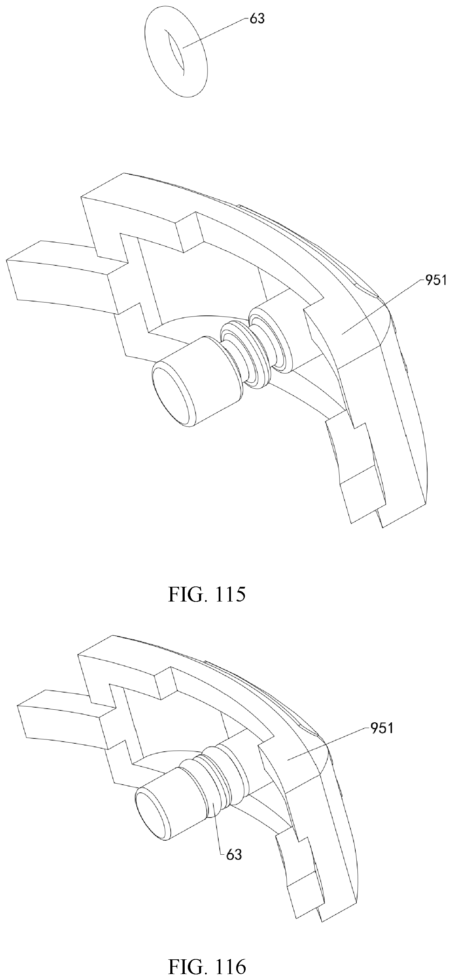

FIG. 115 is a diagram of a structure of a first "O"-shaped sealing ring 63 and the button 951 shown in FIG. 112 according to an implementation. FIG. 116 is a diagram of an assembly structure of the first "O"-shaped sealing ring 63 and the button 951 shown in FIG. 115 according to an implementation.

As shown in FIG. 112, FIG. 115, and FIG. 116, the implantation apparatus 10 may further include the first "O"-shaped sealing ring 63, and the first "O"-shaped sealing ring 63 may be used for sealing between the button 951 and the housing 94. The first "O"-shaped sealing ring 62 is sleeved on one of the two buttons 951. When the button 951 slides in a first mounting hole 8112, the button 951 and a hole wall of the first mounting hole 8112 of the housing 94 clamp the first "O"-shaped sealing ring 63, so that the first "O"-shaped sealing ring 63 abuts between the button 951 and the hole wall of the first mounting hole 8112, to implement sealing. The first "O"-shaped sealing ring 63 may be a silicone ring or a rubber ring. For example, there may be a plurality of first "O"-shaped sealing rings 63. This can achieve better sealing effect.

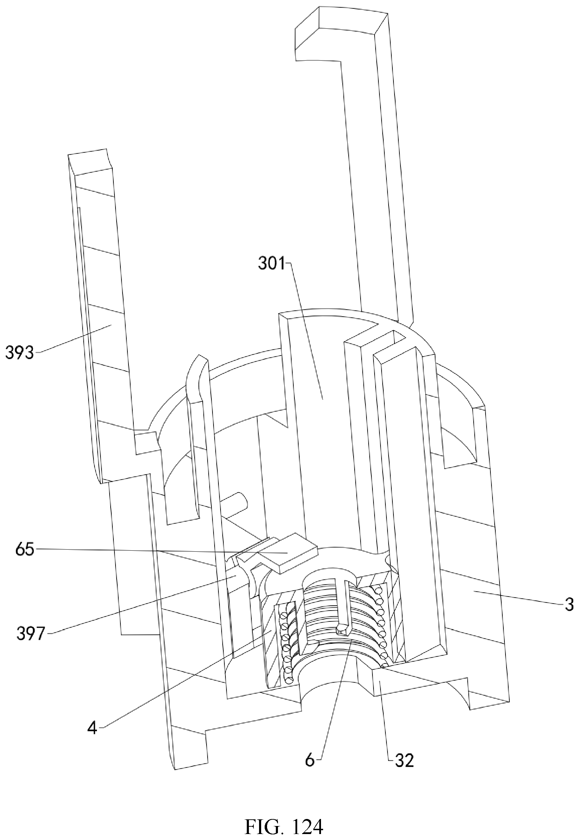

As shown in FIG. 124, the implantation apparatus 10 may further include a second "O"-shaped sealing ring 64, and the second "O"-shaped sealing ring 64 may be used for sealing between the button 951 and the housing 94. The second "O"-shaped sealing ring 62 is sleeved on the other button 951. When the other button 951 slides in a second mounting hole 8112, the button 951 and a hole wall of the mounting hole 8112 of the housing 94 clamps the second "O"-shaped sealing ring 64, so that the second "O"-shaped sealing ring 64 abuts between the other button 951 rod and the hole wall of the mounting hole 8112, to implement sealing. The second "O"-shaped sealing ring 64 may be a silicone ring or a rubber ring.

It can be understood that the button module in this embodiment includes two buttons 951 that are symmetrically distributed at 180°. When the physiological sign monitoring apparatus 100 falls or is accidentally triggered, a button 951 on one side is pressed against, and only one first push block 952 is pushed to move, without simultaneously driving the two second push blocks 953 to move. Therefore, at most one snap-fit arm 393 is detached, and the other three snap-fit arms 393 can still remain in snap-fit with the housing 94, to prevent falling or accidental triggering. In this way, in the physiological sign monitoring apparatus 100 in this embodiment, pushing is not accidentally triggered when only one button 951 is pressed, or when the button 951 touches the ground upon falling, or during vibration. In addition, a sealing function is retained while the button 951 is allowed to move. The implantation apparatus 10 of the physiological sign monitoring apparatus 100 has a function of carrying the sensor 24 into a human body, and also has a function of retracting a guide needle.

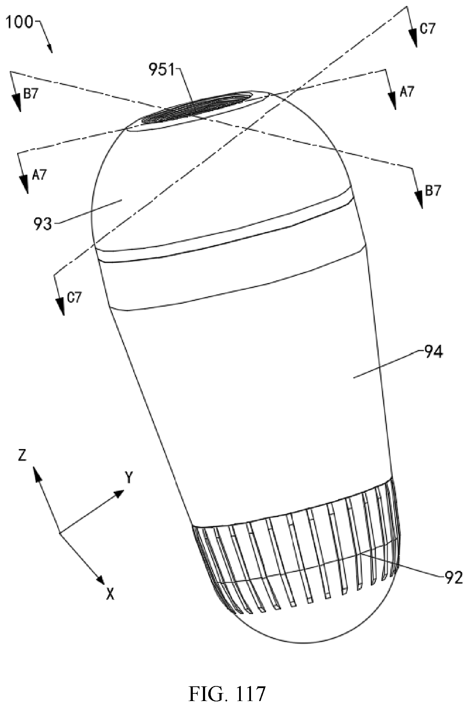

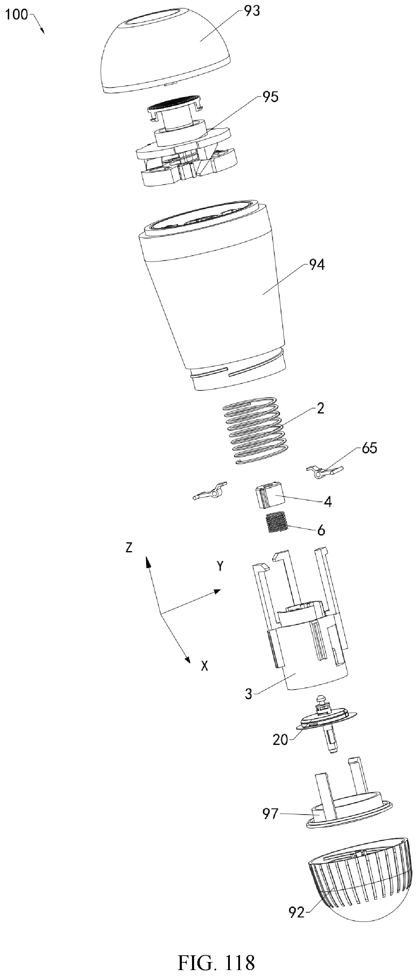

In some implementations, a part that is the same as that in the foregoing embodiments is not described again. FIG. 117 is a diagram of a structure of the physiological sign monitoring apparatus 100 shown in FIG. 2 according to yet another implementation. FIG. 118 is a schematic exploded view of a partial structure of the physiological sign monitoring apparatus 100 shown in FIG. 117 according to an implementation.

As shown in FIG. 117 and FIG. 118, the physiological sign monitoring apparatus 100 may include an implantation apparatus 10 and a sensor assembly 20. The implantation apparatus 10 may include a front sealing cover 92, a rear sealing cover 93, a housing 94, a trigger button module 95, a first elastic member 2, a needle retraction holder 4, a lever fastener 65, a second elastic member 6, a support 3, a drying member 82, and a concealing cover 97. The drying member 82 may be fastened to the front sealing cover 92 to form a housing cap assembly 8. The trigger button module 95 may include a button 951, a push block 955, a first reset spring 956, a slide block 957, and a second reset spring 958.

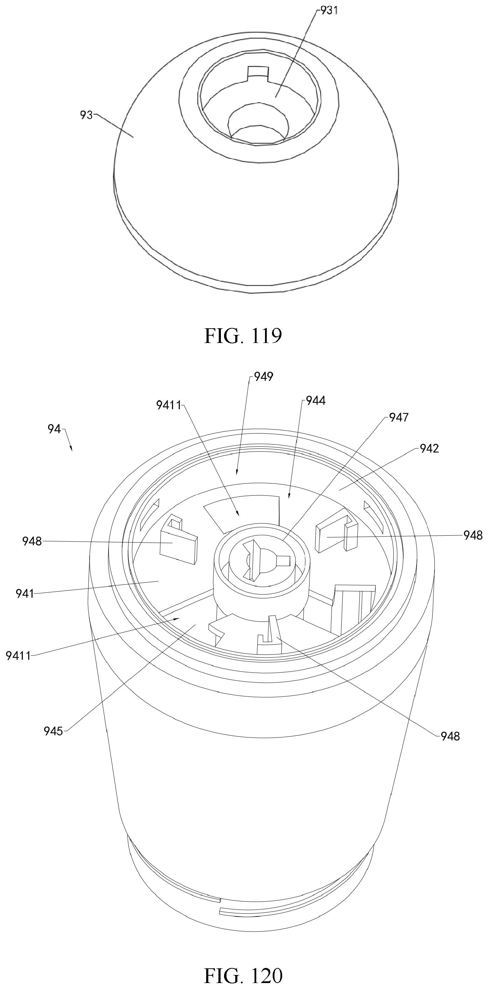

FIG. 119 is a diagram of a structure of the rear sealing cover 93 shown in FIG. 118 according to an implementation.

As shown in FIG. 119, the top of the rear sealing cover 93 may be provided with a mounting hole 931. The mounting hole 931 may run through the rear sealing cover 93 to be in communication with an inner side of the rear sealing cover 93 and an outer side of the implantation apparatus 10.

FIG. 120 is a diagram of a structure of the housing 94 shown in FIG. 118 according to an implementation.

As shown in FIG. 120, the housing 94 may include a middle plate 941, an upper side wall 942, a lower side wall 943, a boss 947, and a plurality of limiting blocks 948. The upper side wall 942 and the middle plate 941 enclose first space 944. The lower side wall 943 and the middle plate 941 enclose second space 945. Both the boss 947 and the plurality of limiting blocks 948 are located in the first space 944 of the housing 94, and are fastened to the middle plate 941 of the housing 94. For example, an axial direction of the boss 947 may coincide with an axial direction of the housing 94. The plurality of limiting blocks 948 and the boss 947 are spaced apart, and are arranged around the boss 947 in a circumferential direction at spacings. For example, there are three limiting blocks 948, and the three limiting blocks 948 are evenly spaced apart in the circumferential direction of the boss 947. The boss 947 and the upper side wall 942 are spaced apart, and enclose rotation space 949.

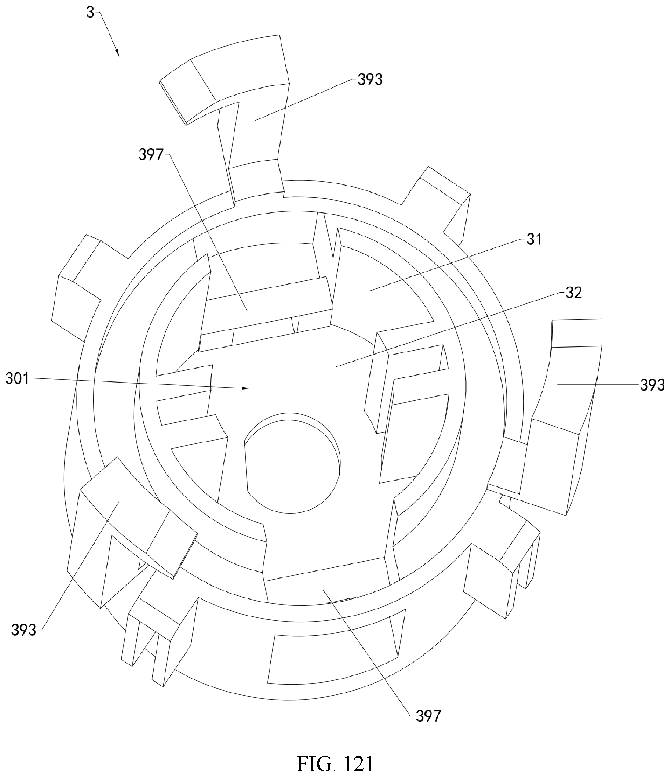

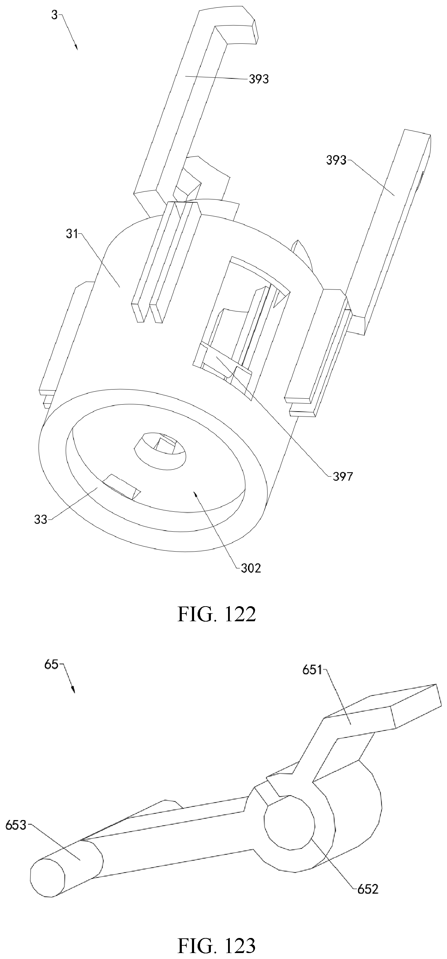

FIG. 121 is a diagram of a structure of the support 3 shown in FIG. 118 according to an implementation. FIG. 122 is a diagram of a structure of the support 3 shown in FIG. 121 from another perspective according to an implementation.

As shown in FIG. 121 and FIG. 122, the support 3 may include a bottom plate 32, an upper side plate 31, and a lower side plate 33. The bottom plate 32 of the support 3 is connected to the upper side plate 31 of the support 3 and the lower side plate 33 of the support 3. A periphery of the upper side plate 31 of the support 3 is fastened to the bottom plate 32 of the support 3. The upper side plate 31 of the support 3 encloses first accommodation space 301. The lower side plate 33 of the support 3 is connected around a periphery of the bottom plate 32 of the support 3. The lower side plate 33 of the support 3 encloses second accommodation space 302.

In some implementations, the support 3 may further include a plurality of snap-fit arms 393. One end of the snap-fit arm 393 is fastened to an end, away from the lower side plate 33, of the upper side plate 31 of the support 3.

In some implementations, the support 3 may further include a rotating rod 397. The rotating rod 397 is fastened to the bottom plate 32 of the support 3, and is spaced away from the bottom plate 32 of the support 3.

FIG. 123 is a diagram of a structure of the lever fastener 65 shown in FIG. 118 according to an implementation.

As shown in FIG. 123, the lever fastener 65 may include a head portion 651, a middle portion 652, and a tail portion 653, and the middle portion 652 is connected between the head portion 651 and the tail portion 653.

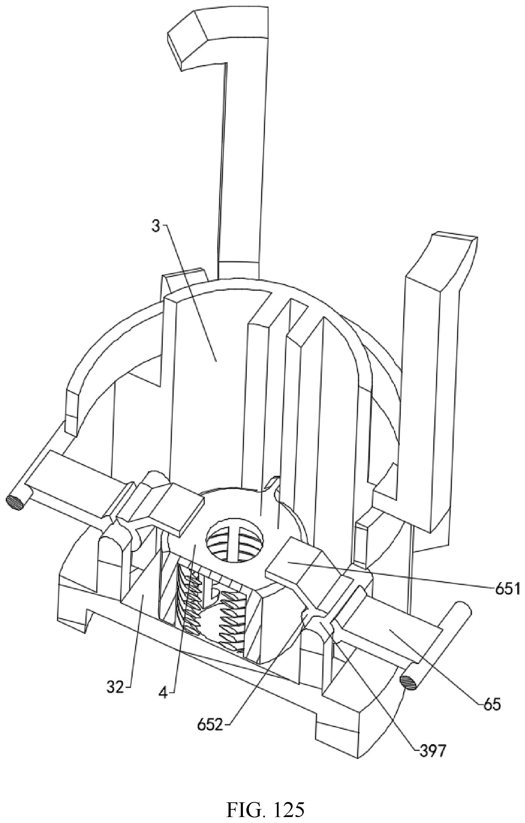

FIG. 124 is a partial cross-sectional view of the physiological sign monitoring apparatus 100 shown in FIG. 117 along A7-A7 according to an implementation. FIG. 125 is a partial cross-sectional view of the physiological sign monitoring apparatus 100 shown in FIG. 117 along B7-B7 according to an implementation.

As shown in FIG. 124 and FIG. 125, the needle retraction holder 4 is located in the first accommodation space 301 of the support 3. The needle retraction holder 4 is mounted to the bottom plate 32 of the support 3. The second elastic member 6 abuts between the needle retraction holder 4 and the support 3. The second elastic member 6 may be a compression spring, and the second elastic member 6 may apply an upward force to the needle retraction holder 4.

For example, there are a plurality of lever fasteners 65. The housing 94 may further include a limiting rod 949. One end of the limiting rod 949 may be fastened to the lower side wall 943 of the housing 94, and other parts of the limiting rod 949 are spaced away from the lower side wall 943. A quantity of limiting rods 949 may be the same as a quantity of lever fasteners 65. A plurality of limiting rods 949 and a plurality of fasteners are arranged in a one-to-one correspondence. The lever fastener 65 may be configured to perform position limiting for the needle retraction holder 4 in an axial direction.

For example, the middle portion 652 is rotatably connected to the support 3. The head portion 651 of the lever fastener 65 is partially located on a side, away from the bottom plate 32 of the support 3, of the needle retraction holder 4.

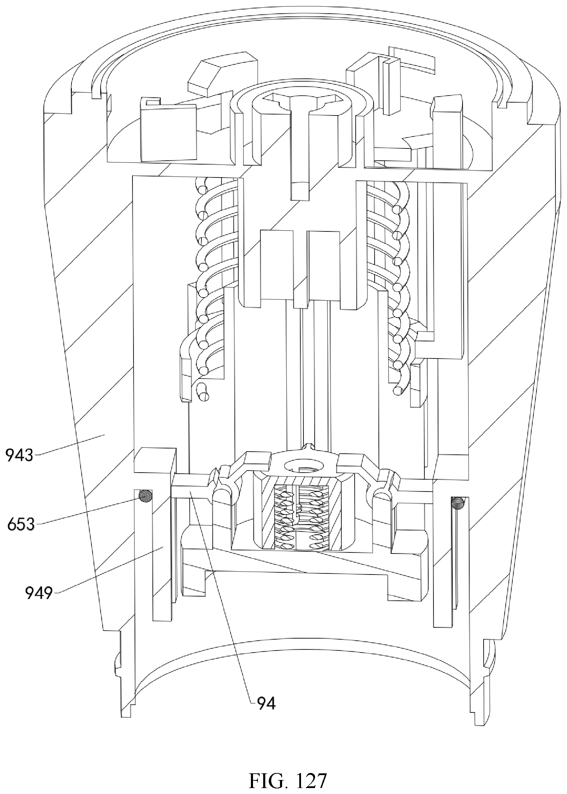

FIG. 126 is a partial cross-sectional view of the physiological sign monitoring apparatus 100 shown in FIG. 117 along B7-B7 according to an implementation. FIG. 127 is a partial cross-sectional view of the physiological sign monitoring apparatus 100 shown in FIG. 117 along B7-B7 according to an implementation.

As shown in FIG. 126 and FIG. 127, one end of the first elastic member 2 abuts against the middle plate 941 of the housing 94, and the other end of the first elastic member 2 abuts against the support 3.

For example, the tail portion 653 of the lever fastener 65 snaps into the limiting rod 949. For example, the lever fastener 65 is sleeved on one end of the limiting rod 949, and the limiting rod 949 may limit the lever fastener 65, to prevent the tail portion of the first fastener from rotating downward. The head portion 651 abut against the needle retraction holder 4, so that the needle retraction holder 4 and the support 3 are fastened relative to each other. For example, the head portion 651 may abut against a side, close to the middle plate of the housing 94, of the needle retraction holder 4. The second elastic member 6 fits with the lever fastener 65 and the limiting rod 949, so that the needle retraction holder 4 is fastened relative to the support 3.

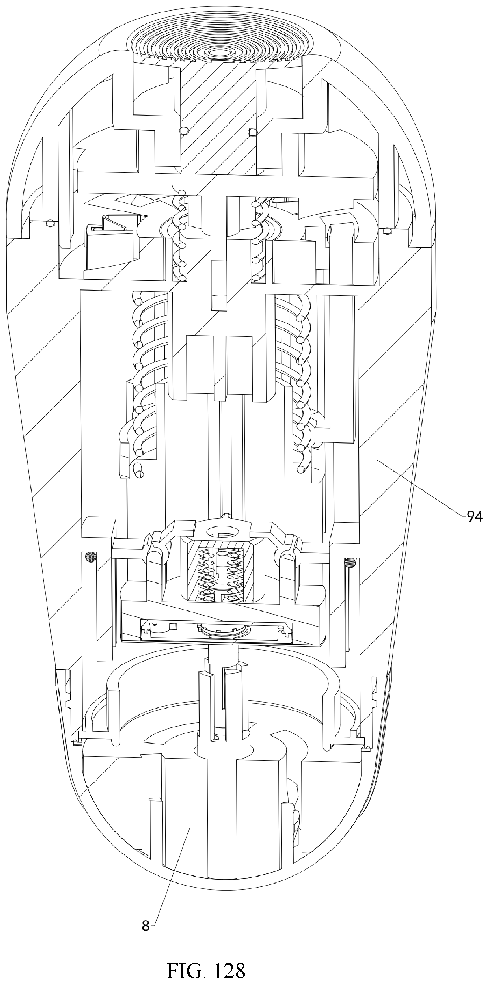

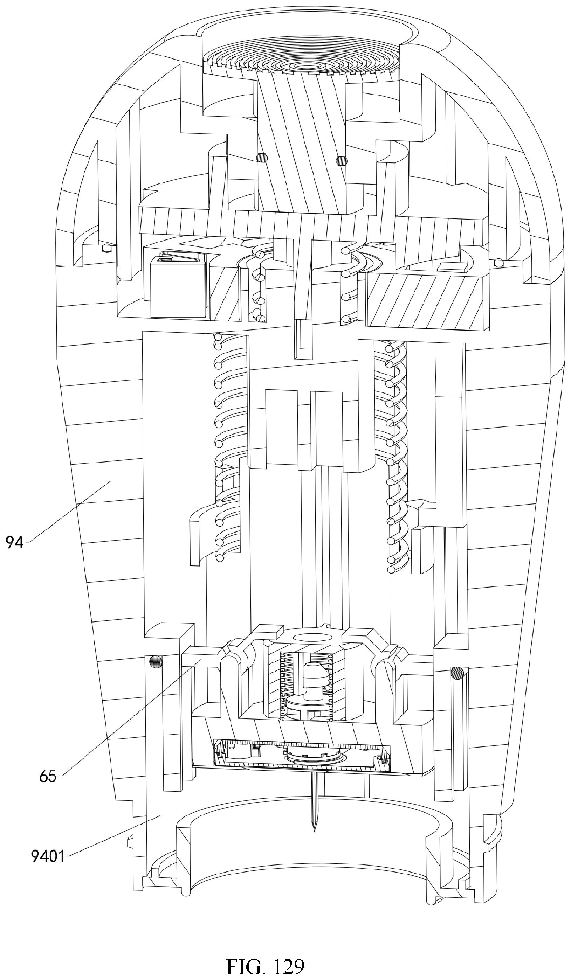

FIG. 128 is a partial cross-sectional view of the physiological sign monitoring apparatus 100 shown in FIG. 117 along B7-B7 according to an implementation. FIG. 129 to FIG. 132 are partial cross-sectional views of the physiological sign monitoring apparatus 100 shown in FIG. 117 along B7-B7 during use according to an implementation.

As shown in FIG. 128, the button 951 is mounted in the mounting hole 931. One end of the button 951 is exposed from the rear sealing cover 93, to be touched and pressed by the user. The other end of the button 951 is located on an inner side of the rear sealing cover 93.

The sensor assembly 20 may be mounted in the second accommodation space 302 of the support 3. The trigger button module 95 may be configured to release snap-fit between the snap-fit arm 393 of the support 3 and the housing 94. The following provides detailed descriptions with reference to the accompanying drawings.

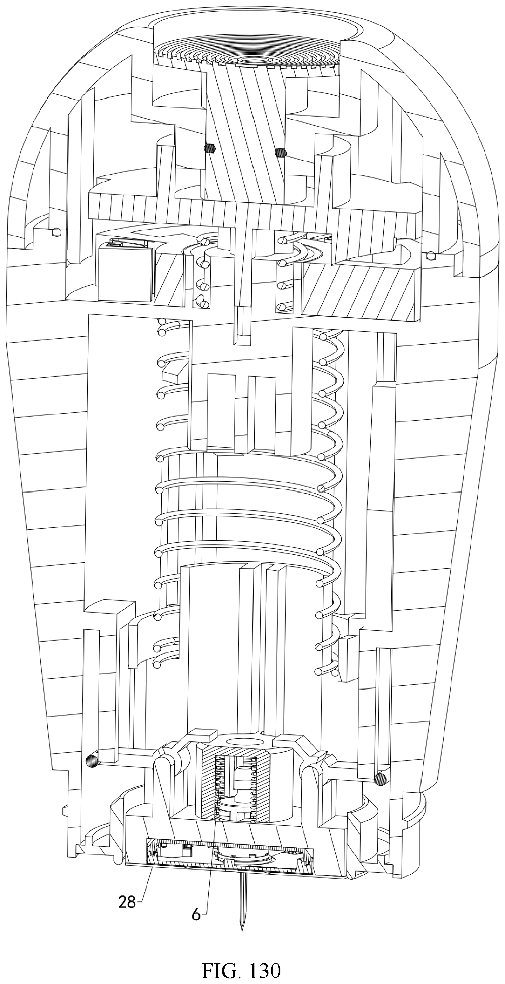

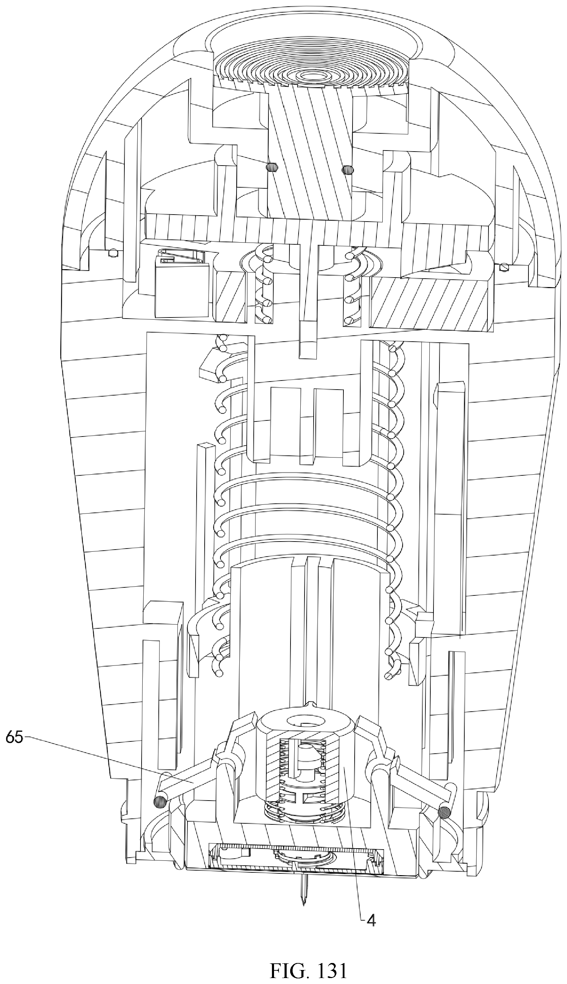

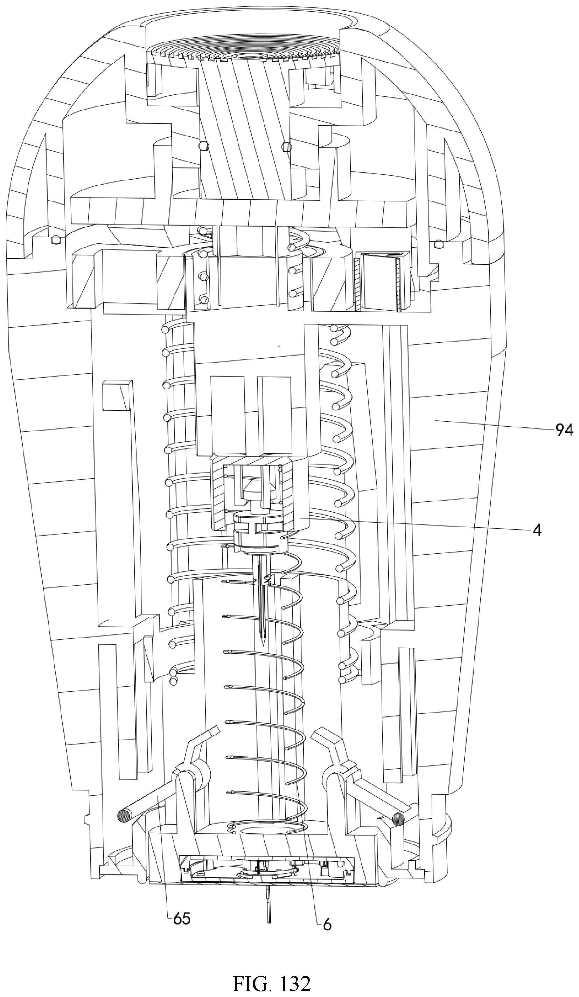

The following describes a process of deploying the sensor 24 in this embodiment. The housing cap assembly 8 is removed, and the abutting end 9401 of the housing 94 abuts against tissue of the user. When the trigger button module 95 (as shown in FIG. 129) releases a snap-fit constraint between the snap-fit arm 393 of the support 3 and the housing 94, the snap-fit arm 393 of the support 3 exits the first snap-fit hole 9411, the support 3 is subject to an acting force of the first elastic member 2, the support 3 moves in a direction toward the organism 30, to drive the sensor assembly 20 to move down, and the lever fastener 65 also moves down correspondingly. When the lever fastener 65 moves to an end, close to the first accommodation space 301, of the limiting rod 949, the bonding member 28 of the sensor assembly 20 is approximately flush with an end face (a trigger position of a needle retraction action, as shown in FIG. 130) of the abutting end 9401 of the housing 94. The second elastic member 6 applies an upward pulling force to the needle retraction holder 4. The needle retraction holder 4 may apply an upward pushing force to the head portion 651 of the lever fastener 65. The limiting rod 949 releases a limitation on the lever fastener 65. The second elastic member 6 may drive the needle retraction holder 4 to enable the tail portion of the lever fastener 65 to rotate downward (as shown in FIG. 131), and the head portion 651 of the lever fastener 65 is detached from the needle retraction holder 4. Under the action of the pulling force of the second elastic member 6, the needle retraction holder 4 may move up together with a guide needle, and the puncture needle 23 is detached from the organism 30, to complete needle retraction (as shown in FIG. 132).

With reference to the accompanying drawings, the following specifically describes a process of releasing, by the trigger button module 95, a constraint on the support 3.

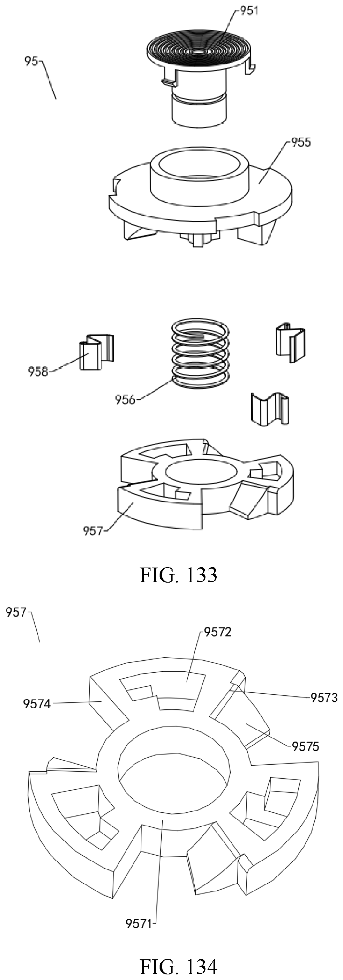

FIG. 133 is a schematic exploded cross-sectional view of the trigger button module 95 shown in FIG. 118 according to an implementation.

As shown in FIG. 133, the trigger button module 95 may include a button 951, a push block 955, a first reset spring 956, a slide block 957, and a second reset spring 958.

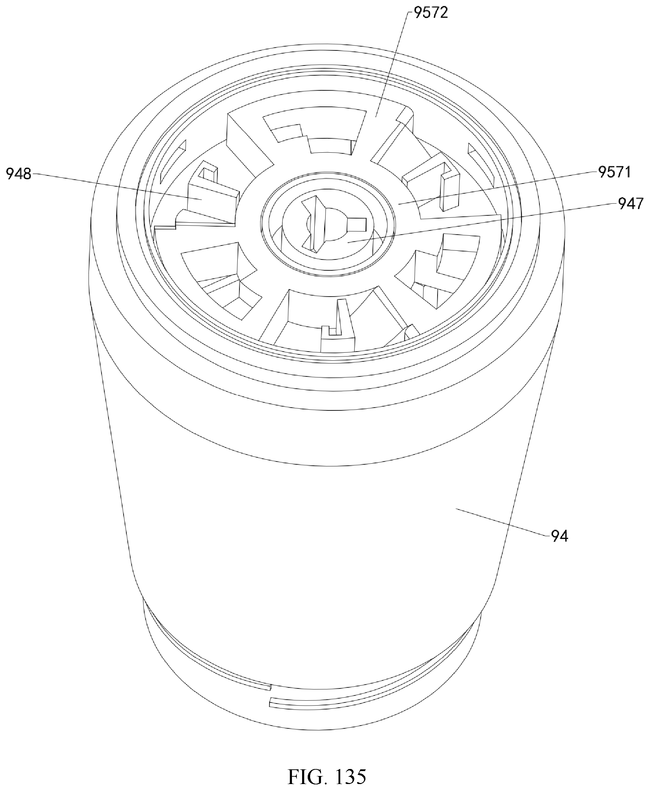

FIG. 134 is a diagram of the slide block 957 shown in FIG. 133 according to an implementation. FIG. 135 is a diagram of assembly of the slide block 957 and the housing 94 according to an implementation.

As shown in FIG. 134 and FIG. 135, the slide block 957 is located in the rotation space 949. For example, the slide block 957 may include a ring portion 9571 and a plurality of snap-fit portions 9572 connected to an outer side of the ring portion 9571. The ring portion 9571 is sleeved on an outer side of the boss 947 of the housing 94. The plurality of snap-fit portions 9572 are spaced apart in a circumferential direction of the ring portion 9571. For example, there are three snap-fit portions 9572, and the three snap-fit portions 9572 are evenly spaced apart in the circumferential direction of the ring portion 9571. For example, a quantity of snap-fit portions 9572 may be the same as a quantity of limiting blocks 948, and a plurality of snap-fit portions 9572 and a plurality of limiting blocks 948 are alternately arranged at spacings. To be specific, one snap-fit portion 9572 may be located between two adjacent limiting blocks 948.

In some implementations, in the circumferential direction of the ring portion 9571, the snap-fit portion 9572 includes a first side 9573 and a second side 9574. The slide block 957 may further include a plurality of abutting ramps 9575. A quantity of abutting ramps 9575 may be the same as a quantity of snap-fit portions 9572. The abutting ramp 9575 may be fastened to the first side 9573 of the snap-fit portion 9572.

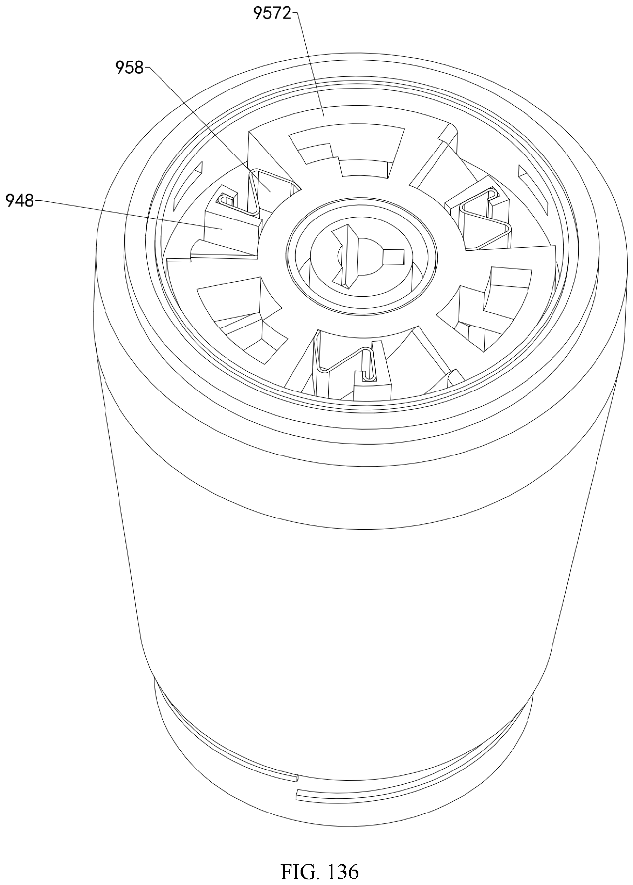

FIG. 136 is a diagram of assembly of the second reset spring 958, the slide block 957, and the housing 94 according to an implementation.

As shown in FIG. 136, the second reset spring 958 abuts between the middle plate 941 and the button 951, for example, between the limiting block 948 and the second side 9574 of the snap-fit portion 9572. A quantity of second reset springs 958 is the same as the quantity of snap-fit portions 9572, and the second reset springs 958 and the snap-fit portions 9572 are arranged in a one-to-one correspondence. The second reset spring is disposed 958, to avoid a case that the slide block 957 rotates in the housing 94 due to impact of an external force when the apparatus is not in use, causing accidental triggering of the support 3.

For example, the push block 955 is located between the slide block 957 and the button 951. A push block reset spring 954 abuts between the push block 955 and the middle plate 941 of the housing 94, so that the push block 955 abuts against the rear sealing cover 93 and can be fastened relative to the rear sealing cover 93.

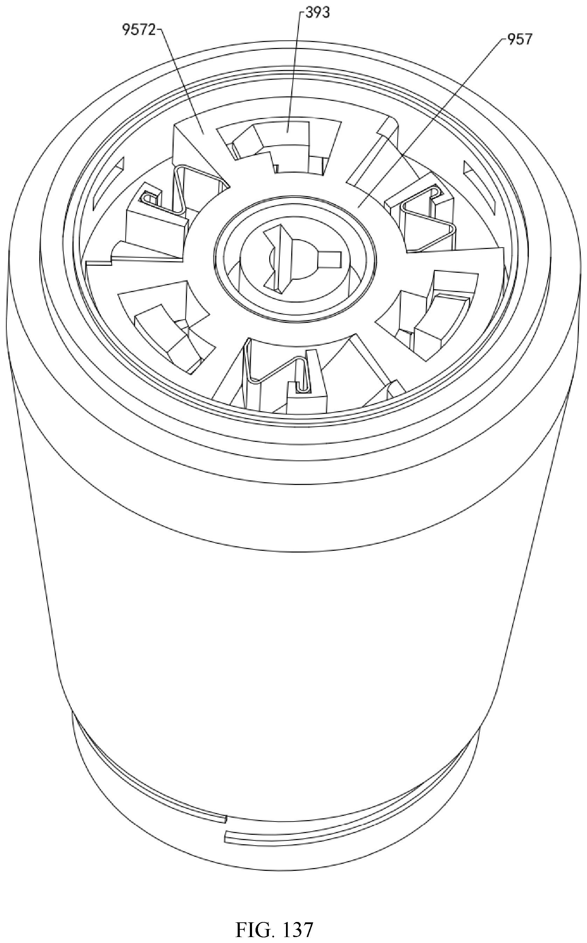

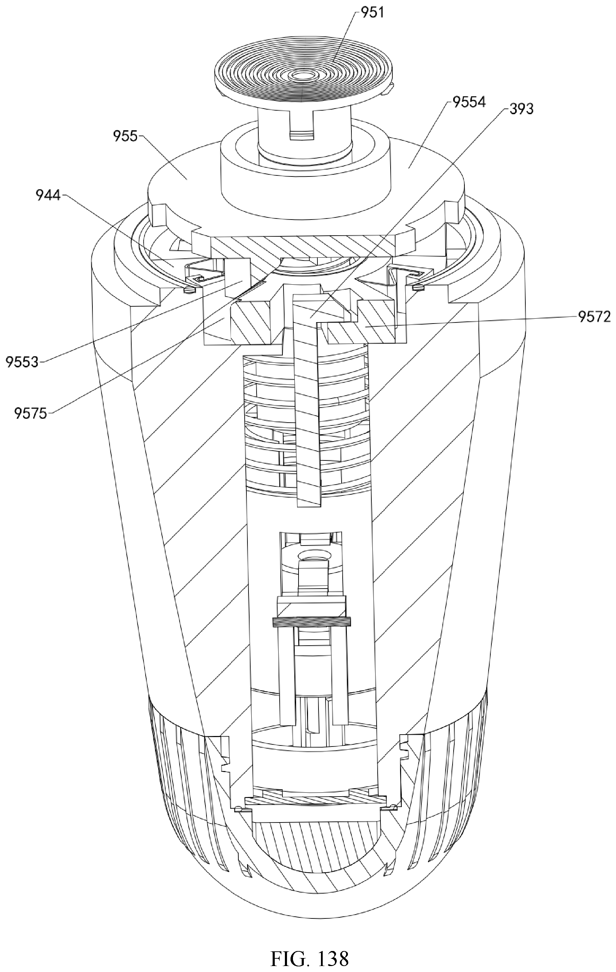

FIG. 137 is a diagram of assembly of the support 3, the second reset spring 958, the slide block 957, and the housing 94 according to an implementation. FIG. 138 is a partial cross-sectional view of the physiological sign monitoring apparatus 100 shown in FIG. 117 along C7-C7 according to an implementation.

For example, a hook of the snap-fit arm 393 of the support 3 may snap into the snap-fit portion 9572 of the slide block 957.

It can be understood that, when the user presses the button 951, the button 951 may abut against the push block 955, and the push block 955 abuts against the abutting ramp 9575 of the slide block 957. Through fitting between inclined surfaces, the slide block 957 is pushed to axially rotate relative to the housing 94. During rotation of the slide block 957, the support 3 may remain stationary relative to the housing 94. The snap-fit portion 9572 of the slide block 957 rotates relative to the snap-fit arm 393 of the support 3 until the snap-fit portion 9572 and the snap-fit arm 393 are staggered. A constraint on the snap-fit arm 393 is released, and the snap-fit arm 393 exits the first space 944 from the first snap-fit hole 9411, to release a limitation on the support 3. Under the action of the first elastic member 2, the support 3 moves down. In comparison with a conventional button solution, in this embodiment, a force applied to the button 951 in an axial direction is converted into a rotation force in a horizontal direction, to avoid a case that the support is detached due to collision caused by the force in the axial direction to the physiological sign monitoring apparatus 100 during falling.

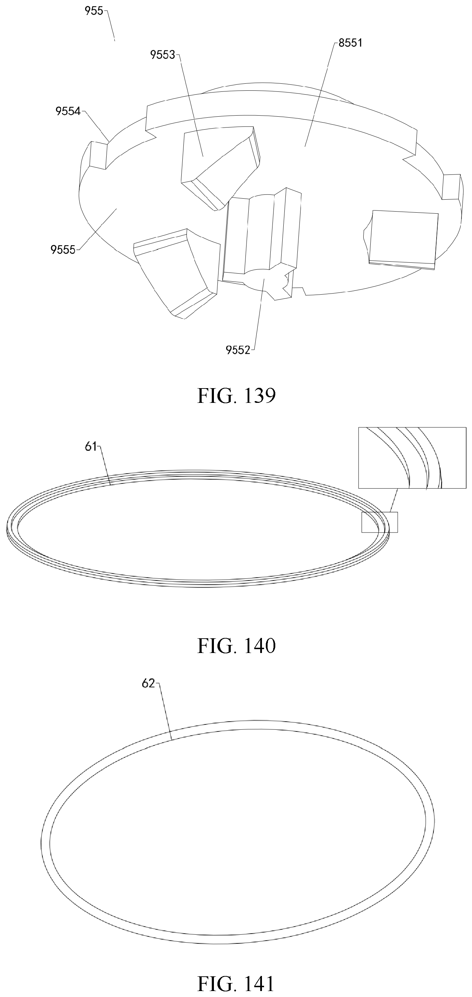

FIG. 139 is a diagram of the push block 955 shown in FIG. 133 according to an implementation.

As shown in FIG. 139, the push block 955 may include a plate portion 8551, a limiting portion 9552, and a second ramp 9553. The limiting portion 9552 and the second ramp 9553 are fastened to a second surface 9555 of the plate portion 8551. The button 951 abuts against a first surface 9554 of the plate portion 8551. The limiting portion 9552 may be configured to snap into the boss 947 of the housing 94, to limit rotation of a bump, so that the push block 955 can move in the axial direction. The second ramp 9553 is configured to fit with an inclined surface of the ramp of the slide block 957 to push the slide block 957 to rotate.

FIG. 140 is a diagram of a structure of a sealing pad 61 shown in FIG. 128 according to an implementation.

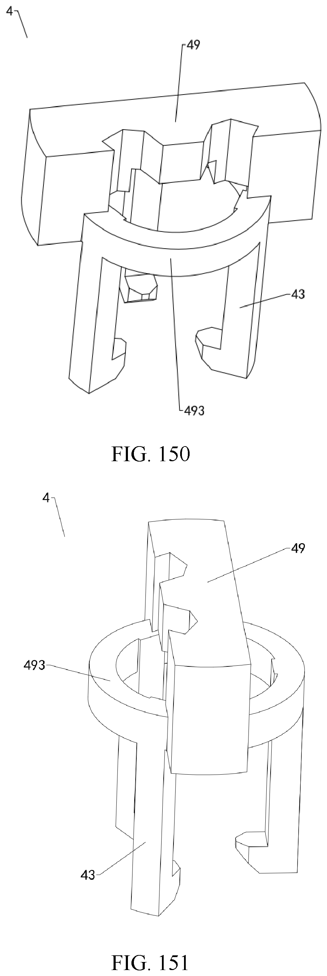

In some implementations, the physiological sign monitoring apparatus 100 may further include the sealing pad 61 (as shown in FIG. 151), and the sealing pad 61 may be used for sealing between the front sealing cover 92 and the housing 94. The sealing pad 61 may be fastened to the front sealing cover 92 or the housing 94. When the front sealing cover 92 is tightened with the housing 94 through threads, the sealing pad 61 may abut between the front sealing cover 92 and the housing 94 (as shown in FIG. 128), to implement sealing. The sealing pad 61 may be a silicone pad or a rubber pad.

FIG. 141 is a diagram of a structure of a sealing ring 62 shown in FIG. 128 according to an implementation.

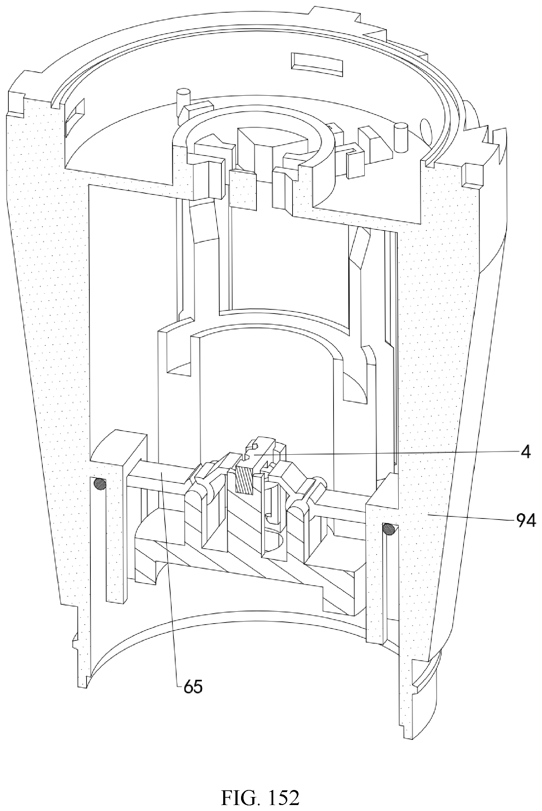

In some implementations, the physiological sign monitoring apparatus 100 may further include the sealing ring 62 (as shown in FIG. 152), and the sealing ring 62 may be used for sealing between the rear sealing cover 93 and the housing 94. The sealing ring 62 may be fastened to the upper side wall 942 of the housing 94 or the rear sealing cover 93. The rear sealing cover 93 may snap into the upper side wall 942 of the housing 94 through a fastener, and the sealing ring 62 may abut between the rear sealing cover 93 and the housing 94 (as shown in FIG. 140), to implement sealing. The sealing ring 62 may be a silicone ring or a rubber ring.

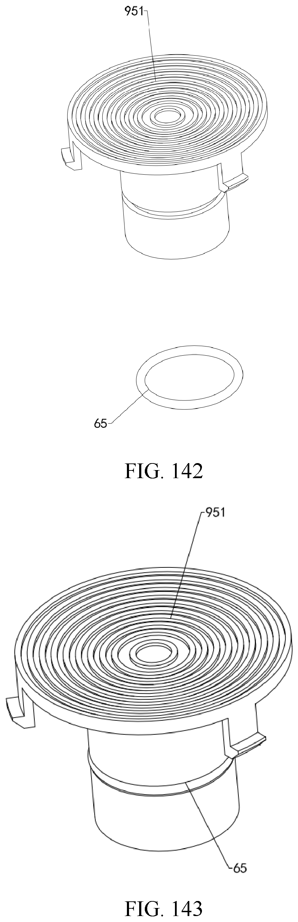

FIG. 142 is a diagram of a structure of an "O"-shaped sealing ring 65 and the button 951 shown in FIG. 128 according to an implementation. FIG. 143 is a diagram of an assembly structure of the "O"-shaped sealing ring 65 and the button 951 shown in FIG. 141 according to an implementation.

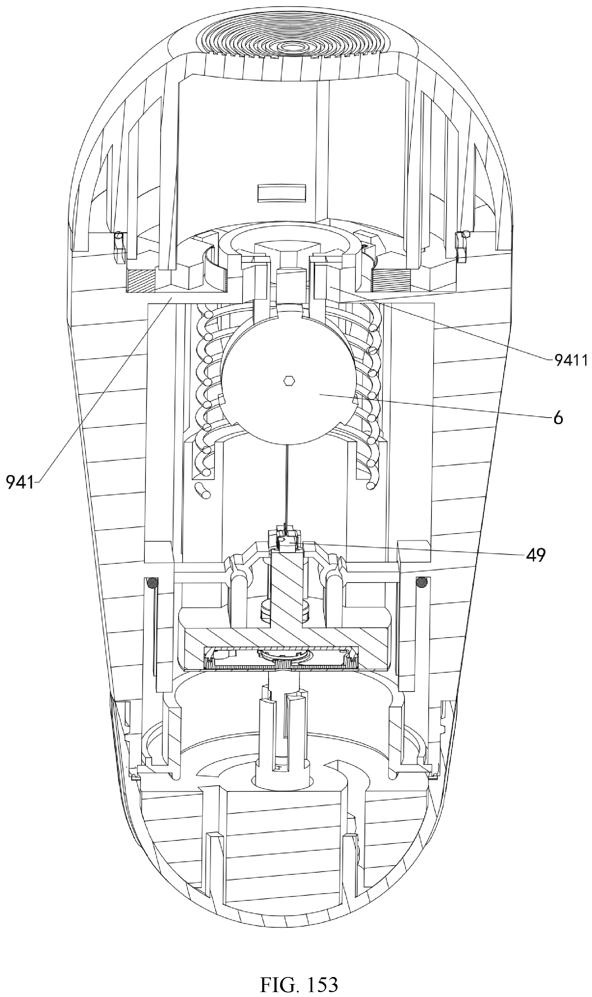

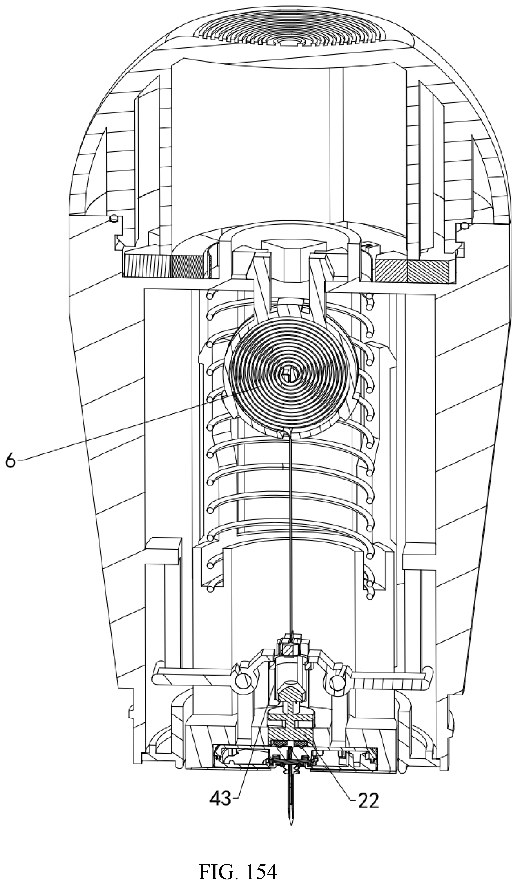

In some implementations, the physiological sign monitoring apparatus 100 may further include the "O"-shaped sealing ring 65, and the "O"-shaped sealing ring 65 may be used for sealing between the button 951 and the housing 94. The "O"-shaped sealing ring 65 is sleeved on the button 951 (as shown in FIG. 153 and FIG. 154). When the button 951 slides in the mounting hole 931, the button 951 and a hole wall of the mounting hole 931 of the housing 94 clamps the "O"-shaped sealing ring 65, so that the "O"-shaped sealing ring 65 abuts between the button 951 rod and the hole wall of the mounting hole 931 (as shown in FIG. 140), to implement sealing. The "O"-shaped sealing ring 65 may be a silicone ring or a rubber ring.

In some implementations, an end face, exposed from the rear sealing cover 93, of the button 951 is located in the mounting hole 931, and is recessed (at a concave position shown in FIG. 128) toward a side (the first space 944) of the housing 94, so that the rear sealing cover 93 protrudes above the end face of the button 951. In this way, during falling, after the apparatus is in contact with the ground, the rear sealing cover 93 is first deformed, and an amount of deformation of the button 951 is much less than a stroke needed for triggering (a distance by which the button 951 needs to move in the axial direction to release a limitation on the support 3 in a normal state), to prevent accidental triggering of the physiological sign monitoring apparatus 100 in this embodiment during falling.

In some implementations, a part that is the same as that in the foregoing embodiments is not described again.

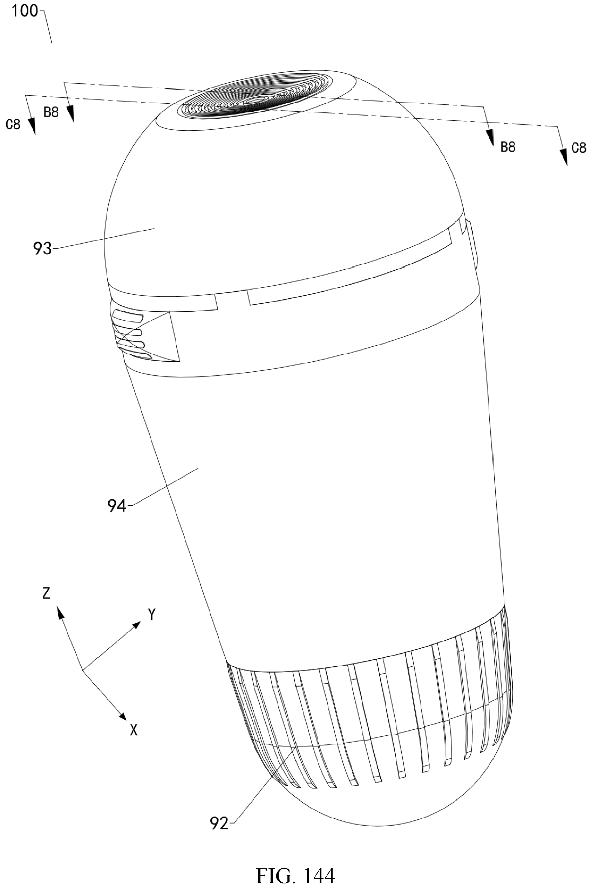

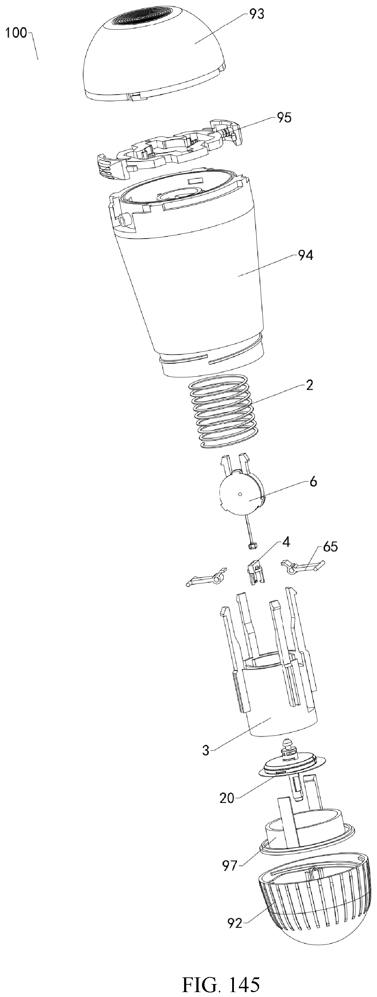

FIG. 144 is a diagram of a structure of the physiological sign monitoring apparatus 100 shown in FIG. 2 according to yet another implementation. FIG. 145 is a schematic exploded view of a partial structure of the physiological sign monitoring apparatus 100 shown in FIG. 144 according to an implementation. In this embodiment, the lever fastener 65 shown in FIG. 127 and the trigger button module 95 shown in FIG. 96 are used.

In this embodiment, a difference from the foregoing embodiments (as shown in FIG. 96 and FIG. 127) lies in that a passive energy storage device (the second elastic member 6) for driving the needle retraction holder 4 to perform needle retraction changes from a compression spring to a needle retraction mainspring.

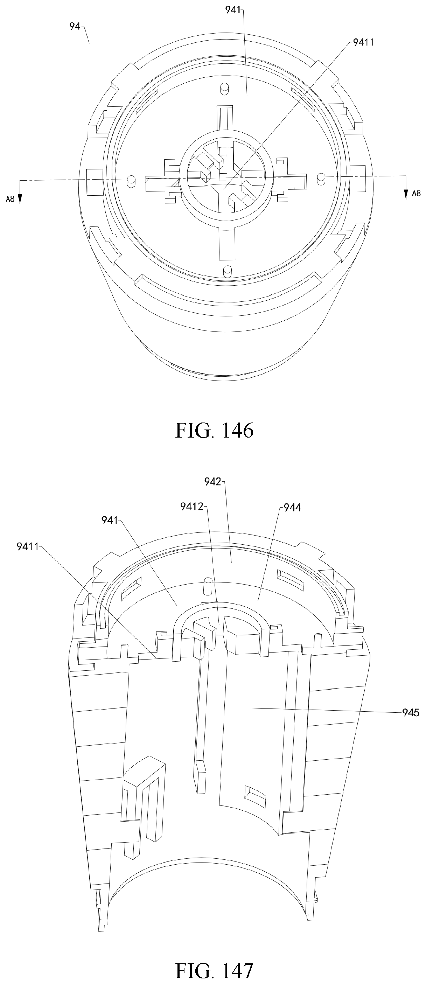

FIG. 146 is a diagram of a structure of a housing 94 shown in FIG. 145 according to an implementation. FIG. 147 is a schematic cross-sectional view of the housing 94 shown in FIG. 146 along A8 to A8 according to an implementation.

As shown in FIG. 146 and FIG. 147, a middle plate 941 of the housing 94 may be further provided with a second snap-fit hole 9411.

For example, one end of the second elastic member 6 may snap into the second snap-fit hole 9411 of the housing 94 through a snap-fit member, to fasten the second elastic member 6 to the housing 94. The second elastic member 6 may be a tension spring. In this case, the second elastic member 6 is in a stretched state, and applies an upward pulling force to the needle retraction holder 4.

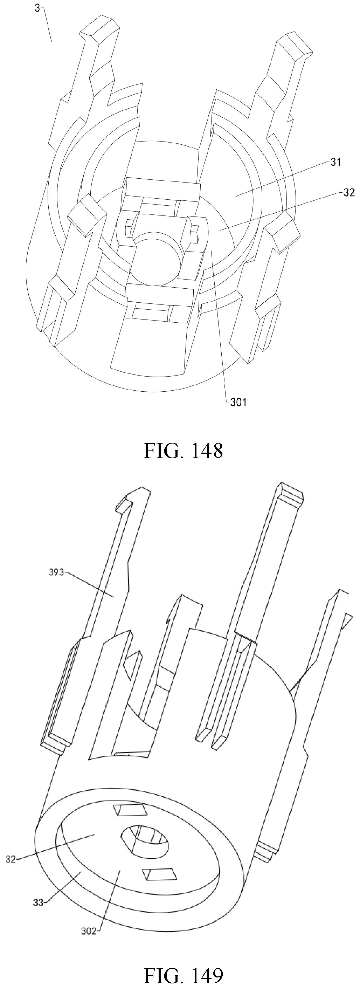

FIG. 148 is a diagram of a structure of the support 3 shown in FIG. 145 according to an implementation. FIG. 149 is a diagram of a structure of the support 3 shown in FIG. 148 from another perspective according to an implementation.

As shown in FIG. 148 and FIG. 149, the support 3 may include an upper side plate 31, a bottom plate 32, and a lower side plate 33. The bottom plate 32 of the support 3 is connected to the upper side plate 31 of the support 3 and the lower side plate 33 of the support 3. A periphery of the upper side plate 31 of the support 3 is fastened to the bottom plate 32 of the support 3. The upper side plate 31 of the support 3 encloses first accommodation space 301. The lower side plate 33 of the support 3 is connected around a periphery of the bottom plate 32 of the support 3. The lower side plate 33 of the support 3 encloses second accommodation space 302.

In some implementations, the support 3 may further include a plurality of snap-fit arms 393. One end of the snap-fit arm 393 is fastened to an end, away from the lower side plate 33, of the upper side plate 31 of the support 3.

FIG. 150 is a diagram of a structure of the needle retraction holder 4 shown in FIG. 148 according to an implementation. FIG. 151 is a diagram of a structure of the needle retraction holder 4 shown in FIG. 150 from another perspective according to an implementation.

As shown in FIG. 150 and FIG. 151, the needle retraction holder 4 may include a ring portion 493, a plurality of first cantilevers 43, and a beam 49. The plurality of first cantilevers 43 are fastened to the ring portion 493. The beam 49 may be fastened to the ring portion 493.

FIG. 152 is a partial schematic cross-sectional view of the physiological sign monitoring apparatus 100 shown in FIG. 144 along B8-B8 according to an implementation. FIG. 152 shows a manner of assembling the second elastic member 6, the support 3, the housing 94, the lever fastener 65, and the needle retraction holder 4 in this embodiment.

As shown in FIG. 152, one end of the second elastic member 6 is fastened to the needle retraction holder 4, and the other end of the second elastic member 6 is connected to the middle plate 941 of the housing 94. For example, one end of the second elastic member 6 is fastened to the beam 49 of the needle retraction holder 4, and the other end of the second elastic member 6 snaps into the middle plate 941 of the housing 94 through the second snap-fit hole 9411.

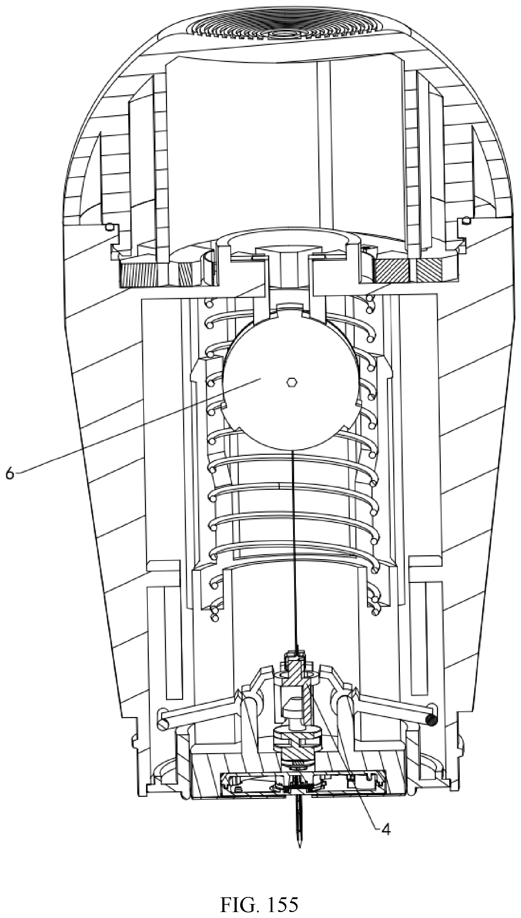

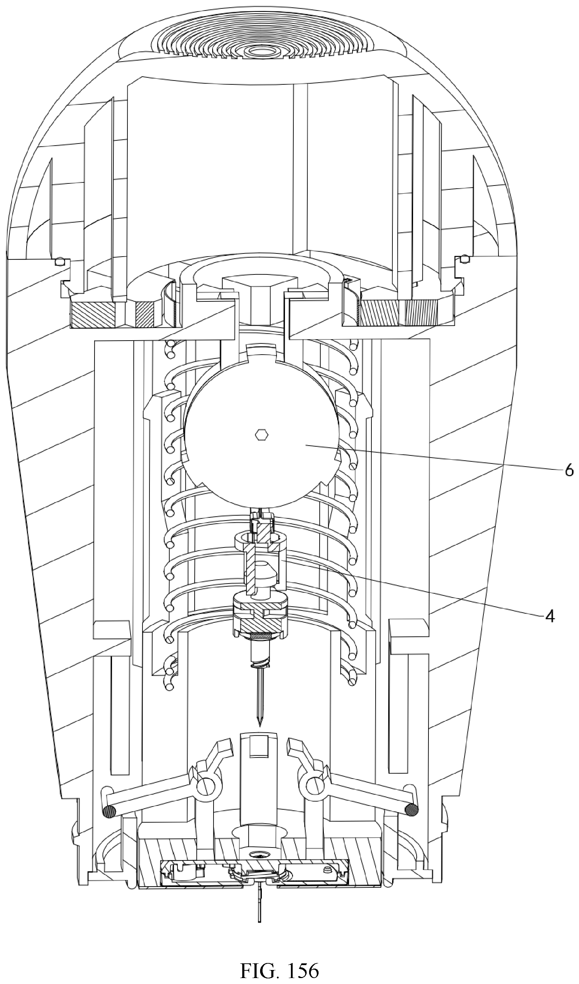

FIG. 153 is a partial schematic cross-sectional view of the physiological sign monitoring apparatus 100 shown in FIG. 144 along B8-B8 during use according to an implementation. FIG. 154 is a partial schematic cross-sectional view of the physiological sign monitoring apparatus 100 shown in FIG. 144 along C8-C8 during use according to an implementation. FIG. 155 is a partial schematic cross-sectional view of the physiological sign monitoring apparatus 100 shown in FIG. 144 along B8-B8 during use according to an implementation. FIG. 156 is a partial schematic cross-sectional view of the physiological sign monitoring apparatus 100 shown in FIG. 144 along B8-B8 during use according to an implementation.

FIG. 153 to FIG. 156 show a process of using the physiological sign monitoring apparatus 100. The second elastic member 6 applies an upward force to the needle retraction holder 4. A needle retraction process of the needle retraction holder 4 is approximately the same as that in the embodiment shown in FIG. 127. A difference lies in that an abutting and force application position of the second elastic member 6 is different. The second elastic member 6 pulls, from a side of the needle retraction holder 4 that is away from the support 3, the needle retraction holder 4 to move away from the support 3, in other words, away from the organism 30.

In some implementations, a part that is the same as that in the foregoing embodiments is not described again.

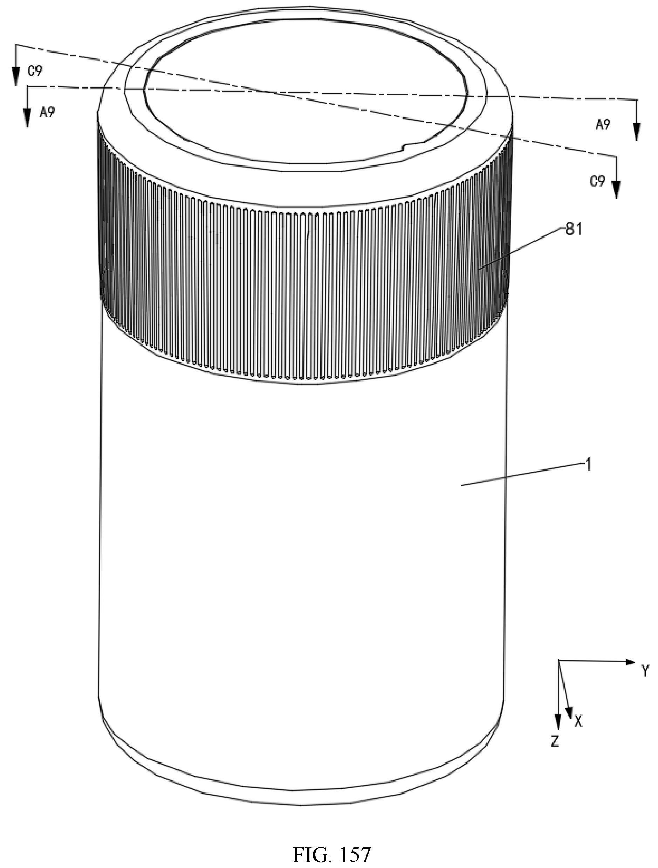

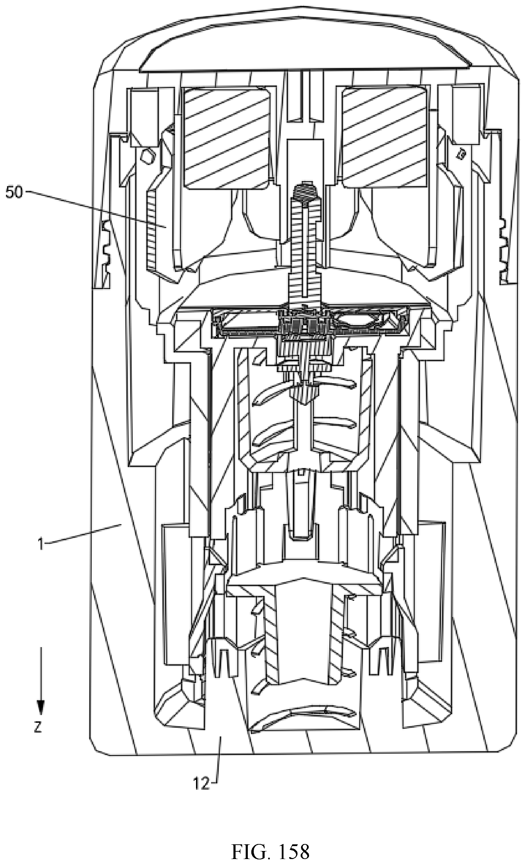

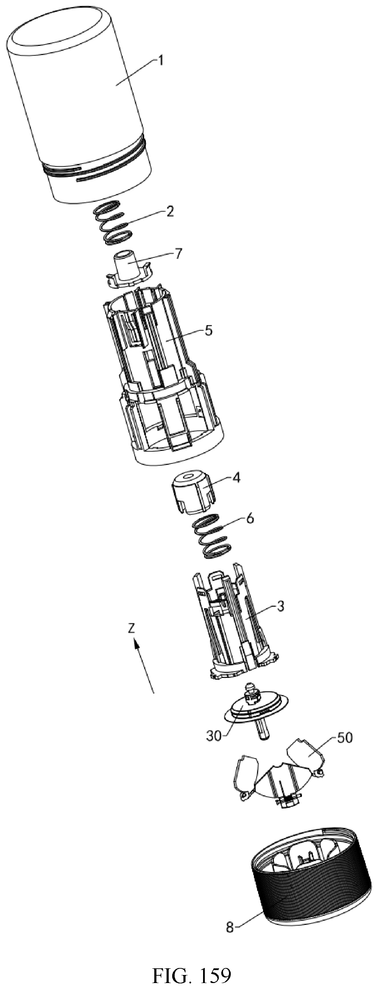

FIG. 157 is a diagram of a structure of the physiological sign monitoring apparatus 100 shown in FIG. 2 according to yet another implementation. FIG. 158 is a partial cross-sectional view of the physiological sign monitoring apparatus 100 shown in FIG. 157 along A9-A9 according to an implementation. FIG. 159 is a partial schematic exploded view of the physiological sign monitoring apparatus 100 shown in FIG. 157 according to an implementation.

As shown in FIG. 157 to FIG. 159, the physiological sign monitoring apparatus 100 may include an implantation apparatus 10, a concealing mechanism 50, and a sensor assembly 20. The implantation apparatus 10 may include a lower housing 1, a first elastic member 2, a spring seat 7, a base 5, a needle retraction holder 4, a second elastic member 6, a support 3, a first magnet 9, and a housing cap assembly 8. For a manner of disposing the sensor assembly 20, the first elastic member 2, the spring seat 7, the needle retraction holder 4, the second elastic member 6, the support 3, the first magnet 9, and the housing cap assembly 8, refer to the foregoing manners of disposing the sensor assembly 20, the first elastic member 2, the spring seat 7, the needle retraction holder 4, the second elastic member 6, the support 3, the first magnet 9, and the housing cap assembly 8. Details are not described herein again.

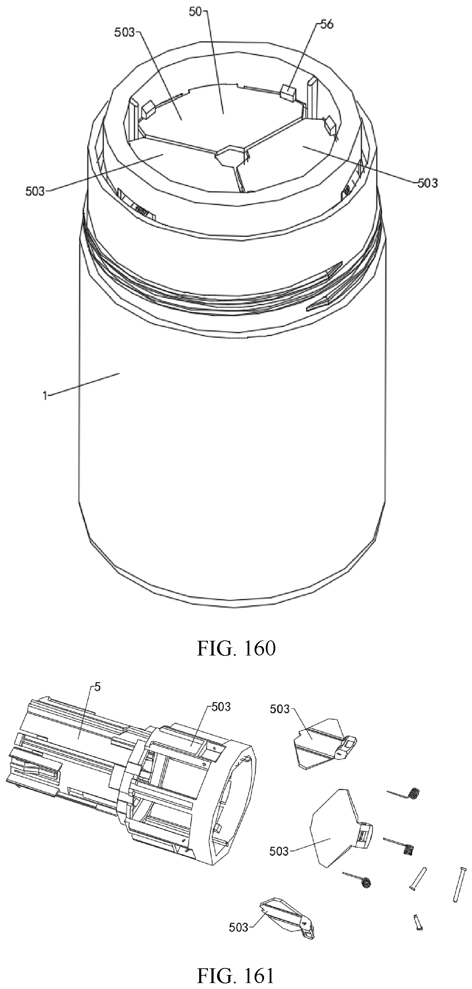

FIG. 160 is a diagram of a structure of the physiological sign monitoring apparatus 100 shown in FIG. 157 with the housing cap assembly 8 removed according to an implementation.

As shown in FIG. 158 and FIG. 160, the concealing mechanism 50 may be configured to conceal a puncture needle 23. The concealing mechanism 50 is fastened to a side, away from a top wall 12 of the lower housing 1, of the base 5, and a part of the concealing mechanism 50 protrudes from the lower housing 1 through an opening 101. For example, the concealing mechanism 50 includes a plurality of hinges 503. When the concealing mechanism 50 is in a closed state, the plurality of hinges 503 are located on a side, away from the top wall 12 of the lower housing 1, of the puncture needle 23, to conceal the puncture needle 23. For example, the plurality of hinges 503 may be arranged around a central axis of the physiological sign monitoring apparatus 100.

As shown in FIG. 158, when the physiological sign monitoring apparatus 100 is in an unused state, the upper housing 81 is mounted to the lower housing 1, and the concealing mechanism 50 is in an open state. As shown in FIG. 160, after the upper housing 81 is detached from the lower housing 1, the concealing mechanism 50 switches from the open state to the closed state, and the concealing mechanism 50 conceals the puncture needle 23. This helps eliminate a fear of the user for the puncture needle 23 when the user uses the physiological sign monitoring apparatus 100.

It can be understood that, during use of the physiological sign monitoring apparatus 100, because the concealing mechanism 50 exists, a downward pressing stroke of the user during implantation is divided into two sections. A first section of stroke enables the concealing mechanism 50 to open. After the concealing mechanism 50 is fully open, a second section of stroke enables the support 3 to be triggered, to deploy the sensor assembly 20. Therefore, a pressing force for triggering the first section of stroke is less than a pressing force for triggering the second section of stroke. The following specifically describes several implementations of the concealing mechanism 50 with reference to the accompanying drawings.

FIG. 161 is a schematic exploded view of a partial structure shown in FIG. 159 according to an implementation.

As shown in FIG. 161, the concealing mechanism 50 further includes a fastening pillar 504, a hinge pin 505, and a torsion spring 506. A quantity of fastening pillars 504, a quantity of hinge pins 505, a quantity of torsion springs 506, and a quantity of hinges 503 are equal.

As shown in FIG. 160 and FIG. 161, there are three hinges 503, and the three hinges 503 may be sequentially arranged around the central axis of the physiological sign monitoring apparatus 100.

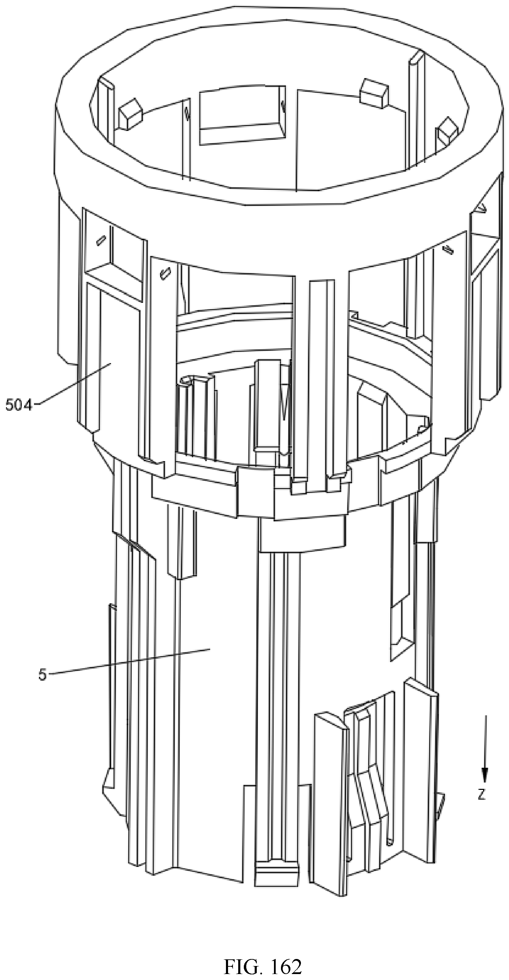

FIG. 162 is a diagram of a structure of the base 5 and the fastening pillar 504 shown in FIG. 161 according to an implementation.

As shown in FIG. 158 and FIG. 162, the fastening pillar 504 may be fastened to an end, away from the top wall 12 of the lower housing 1, of the base 5, and the plurality of fastening pillars 504 are connected around a periphery of the base 5.

In some implementations, the fastening pillar 504 and the base 5 may be an integrally formed mechanical part. For example, the fastening pillar 504 and the base 5 may be integrally formed by using a mold through an injection molding process.

In another implementation, the fastening pillar 504 may alternatively be fastened to the base 5 through bonding, welding, or the like.

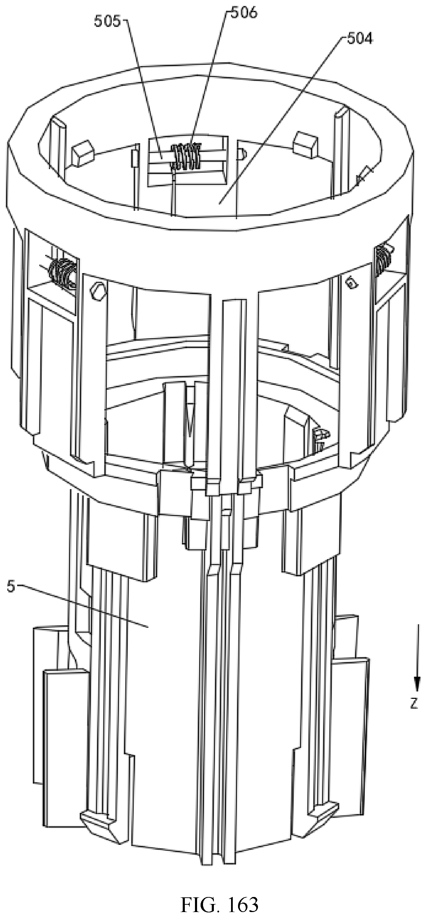

FIG. 163 is a diagram of assembly of the base 5, the fastening pillar 504, the hinge pin 505, and the torsion spring 506 shown in FIG. 161 according to an implementation.

As shown in FIG. 163, the hinge pin 505 is connected to an end, away from the base 5, of the fastening pillar 504, and the torsion spring 506 is sleeved on the hinge pin 505. The torsion spring 506 is rotatably connected to the fastening pillar 504.

In some implementations, the concealing mechanism 50 may further include a snap spring. The snap spring snaps into one end of the hinge pin 505. The hinge pin 505 is rotatably connected to a fastening base. It can be understood that the other end of the hinge pin 505 may be designed to be large, to prevent, through fitting with the snap spring, the hinge pin 505 from falling off from the fastening pillar 504 in an axial direction of the hinge pin 505.

In another implementation, the hinge pin 505 may be fastened to the fastening pillar 504 through interference fit.

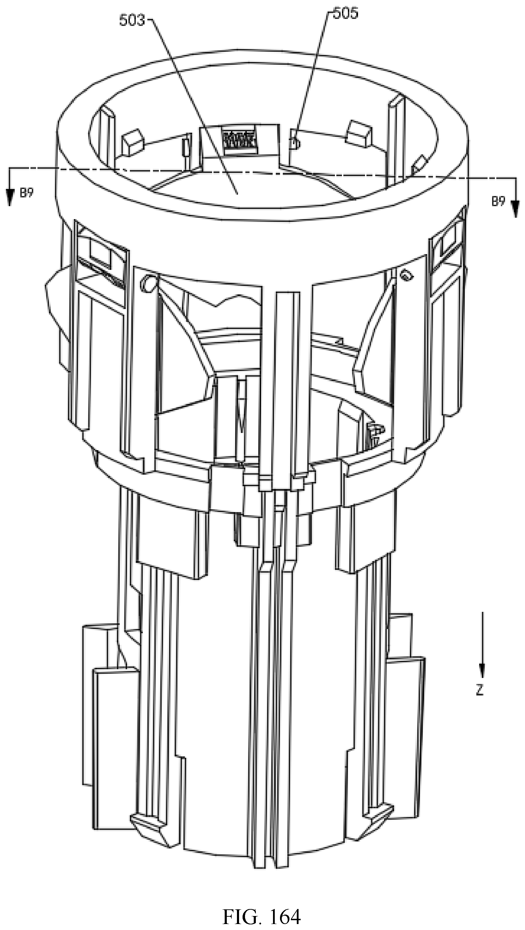

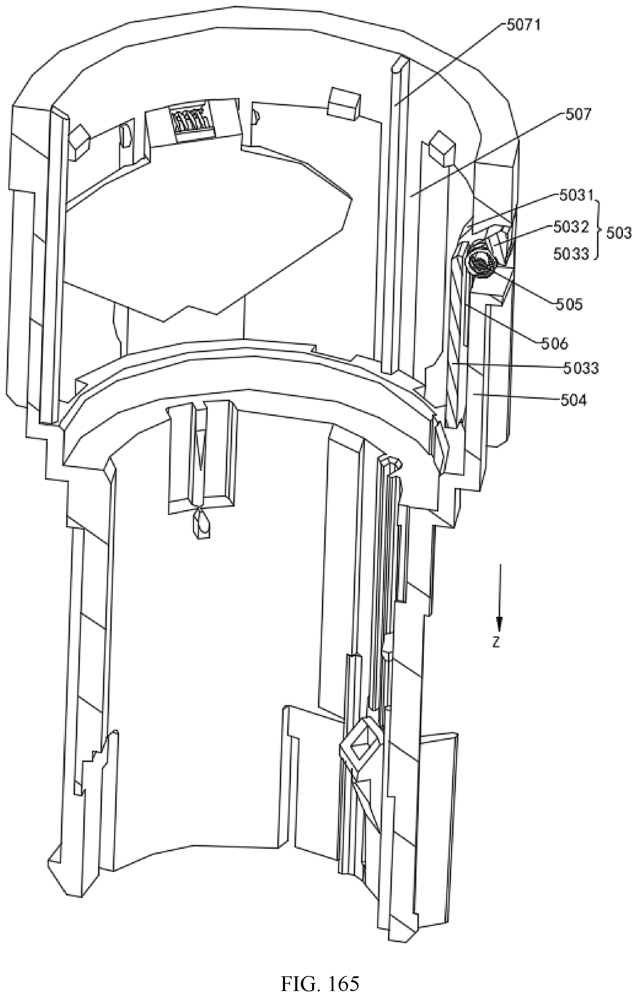

FIG. 164 is a diagram of assembly of the base 5, the fastening pillar 504, the hinge 503, the hinge pin 505, and the torsion spring 506 shown in FIG. 161 according to an implementation. FIG. 165 is a partial cross-sectional view of the structure shown in FIG. 164 along B9-B9 according to an implementation.

As shown in FIG. 164 and FIG. 165, the hinge 503 is rotatably connected to the fastening pillar 504. The torsion spring 506 abuts against a side, close to the base 5, of the hinge 503. When the concealing mechanism 50 switches from the open state to the closed state, the torsion spring 506 may be configured to provide a force for the hinge 503 to rotate. When the concealing mechanism 50 is in the closed state, the torsion spring 506 may be configured to support the hinge 503 to conceal the puncture needle 23. For example, the hinge 503 is slidably connected to the hinge pin 505, and is slidably connected to the fastening pillar 504 through the hinge pin 505.

For example, the hinge 503 includes a rotation portion 5031, a limiting portion 5032, and a concealing portion 5033, and the rotation portion 5031 is connected between the limiting portion 5032 and the concealing portion 5033. The rotation portion 5031 is rotatably connected to the fastening pillar 504. The concealing portion 5033 is in a plate-like shape.

In some implementations, when the concealing mechanism 50 is in the open state, an included angle between a plane on which the hinge 503 is located and the axial direction of the physiological sign monitoring apparatus 100 is less than or equal to 10°. It can be understood that the hinge 503 does not necessarily need to be completely parallel to the axial direction of the physiological sign monitoring apparatus 100, and the hinge 503 may alternatively be disposed to a larger angle, so that the hinge 503 can be open wider, to reduce interference to deployment of the support 3.

For example, the concealing mechanism 50 further includes a guide pillar 507. The guide pillar 507 is fastened to the end, away from the top wall 12 of the lower housing 1, of the base 5. The guide pillar 507 and the fastening pillar 504 are connected around the periphery of the base 5. A seventh guide structure 5071 is disposed on a side, facing third space, of the guide pillar 507.

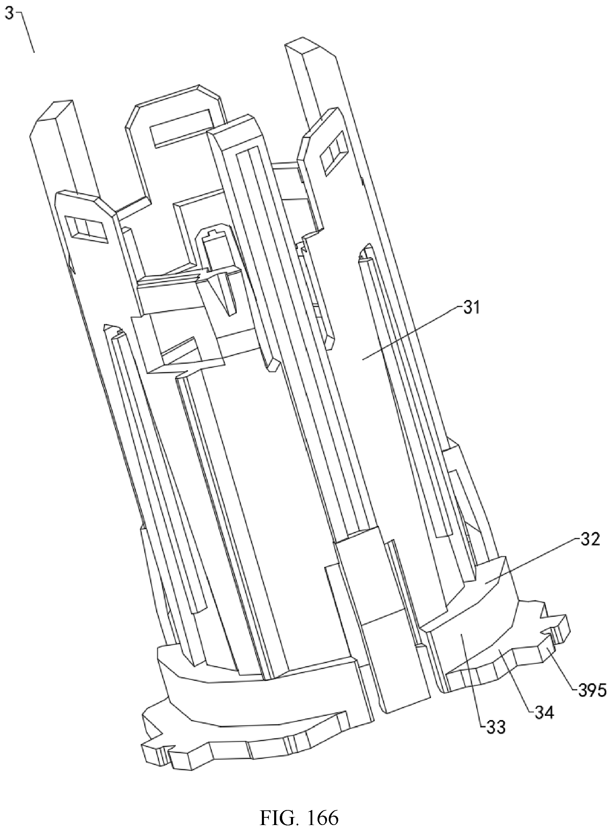

FIG. 166 is a diagram of a structure of the support 3 shown in FIG. 159 according to an implementation.

As shown in FIG. 158 and FIG. 166, the support 3 may include a side plate 31, a bottom plate 32, a lower side plate 33, a flange 34, and a sixth guide structure 395. The bottom plate 32 of the support 3 is connected to the side plate 31 of the support 3 and the lower side plate 33 of the support 3. The side plate 31 of the support 3 is fastened to a periphery of the bottom plate 32 of the support 3. The lower side plate 33 of the support 3 is connected around the periphery of the bottom plate 32 of the support 3. The flange 34 may be fastened to an outer side surface of the lower side plate 33 of the support 3. The sixth guide structure 395 may be fastened to the outer side surface of the lower side plate 33 of the support 3. For example, the sixth guide structure 395 may be fastened to an end, away from the top wall 12 of the lower housing 1, of the support 3 and is protrudingly disposed on an outer side of the support 3.

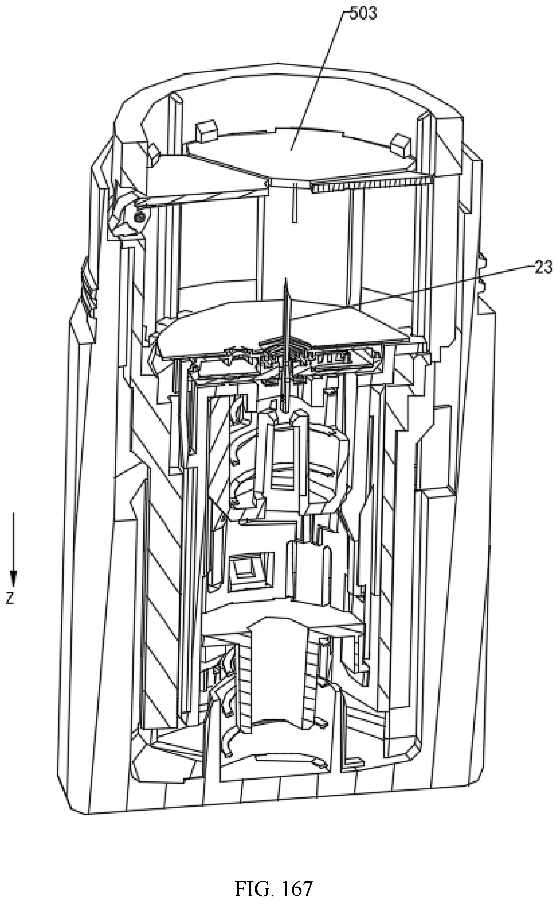

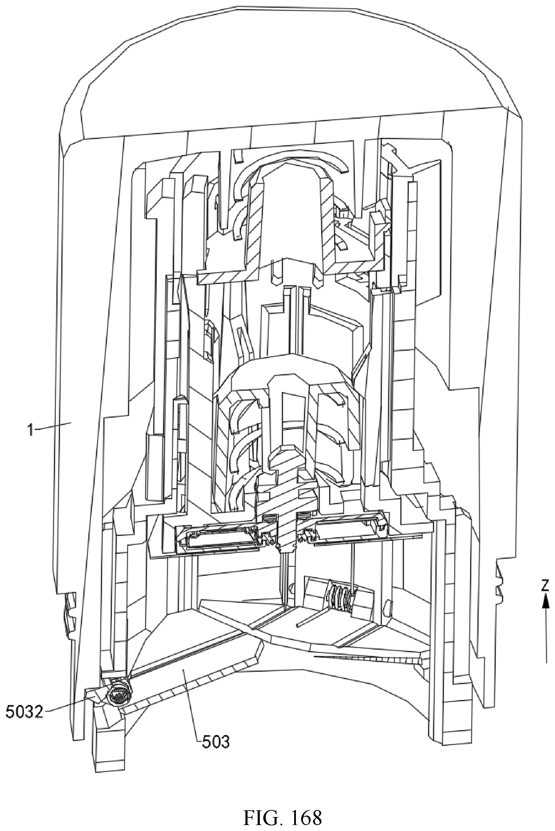

FIG. 167 to FIG. 170a are partial schematic cross-sectional views of the physiological sign monitoring apparatus 100 shown in FIG. 157 along C9-C9 during use according to an implementation.

As shown in FIG. 158 and FIG. 167, after the upper housing 81 is detached from the lower housing 1, the concealing mechanism 50 switches from the open state to the closed state, and the concealing mechanism 50 conceals the puncture needle 23.

In some implementations, the concealing mechanism 50 further includes a first limiting block 508. The first limiting block 508 is fastened to the fastening pillar 504 and is located on a side, away from the base 5, of the concealing portion 5033 of the hinge 503. In a direction parallel to the axial direction of the physiological sign monitoring apparatus 100, the first limiting block 508 is arranged opposite to the concealing portion 5033 of the hinge 503. When the concealing mechanism 50 is in the closed state, the first limiting block 508 abuts against the concealing portion 5033 of the hinge 503.

It can be understood that the first limiting block 508 is disposed, so that when the concealing mechanism 50 is in the closed state, the first limiting block 508 abuts against the concealing portion 5033 of the hinge 503, to prevent the hinge 503 from being subject to an abutting force of the torsion spring 506, and avoid exposing the puncture needle 23 due to an excessively large opening angle.

For example, there may be one or more first limiting blocks 508. A plurality of first limiting blocks 508 are spaced apart around the central axis of the physiological sign monitoring apparatus 100. In addition, at least one first limiting block 508 is correspondingly disposed for one hinge 503.

As shown in FIG. 167 to FIG. 169, in a process in which the lower housing 1 moves relative to the base 5 in a direction toward the organism, the lower housing 1 pushes the limiting portion 5032, an end, away from the concealing portion 5033, of the limiting portion 5032 moves toward the organism, the concealing portion 5033 moves toward a side away from the organism, the concealing structure switches from the closed state to the open state, and the puncture needle 23 is exposed.

In some implementations, in a process in which the first elastic member 2 pushes the support 3 toward the organism, the sixth guide structure 395 is slidably connected to the second guide structure 55, to implement directional movement of the support 3.

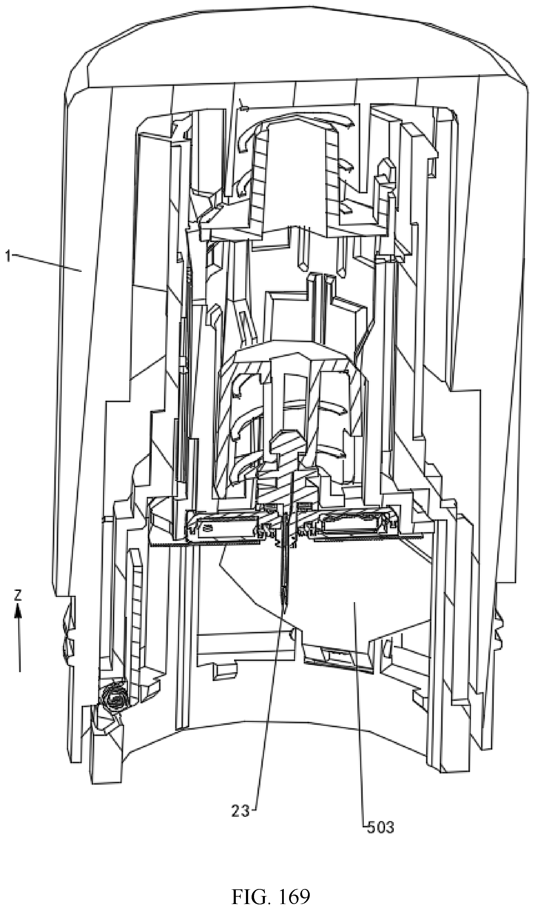

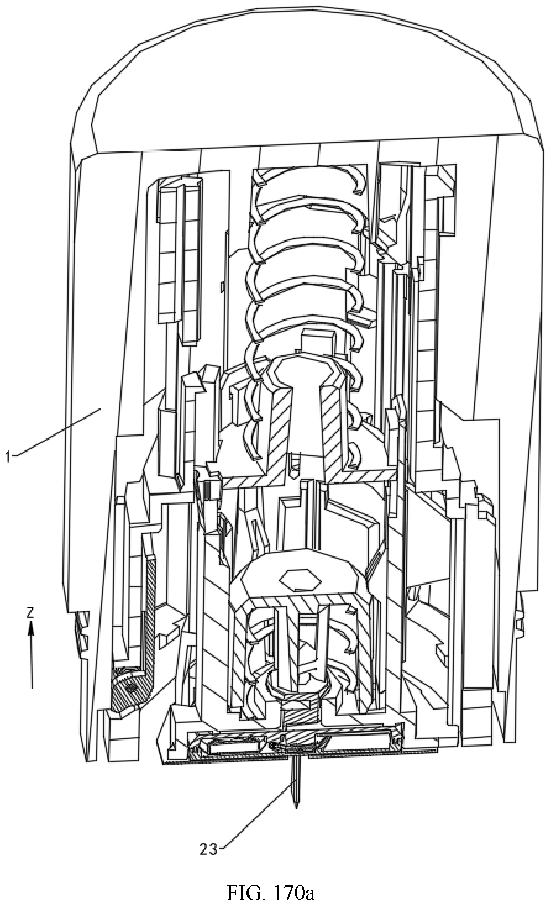

As shown in FIG. 169 and FIG. 170a, after the puncture needle 23 is exposed, the first elastic member 2 pushes the support 3 toward the organism, the sensor assembly 20 abuts against the organism, and the sensor 24 is implanted into the organism under guidance of the puncture needle 23. For subsequent needle retraction, refer to the foregoing descriptions.

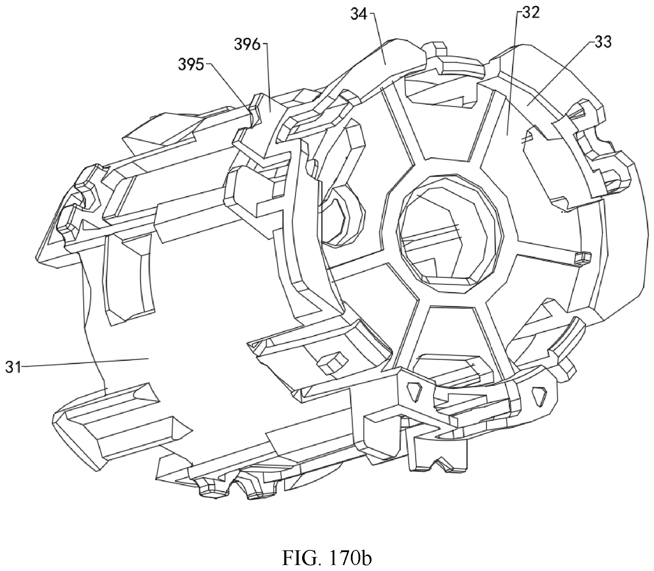

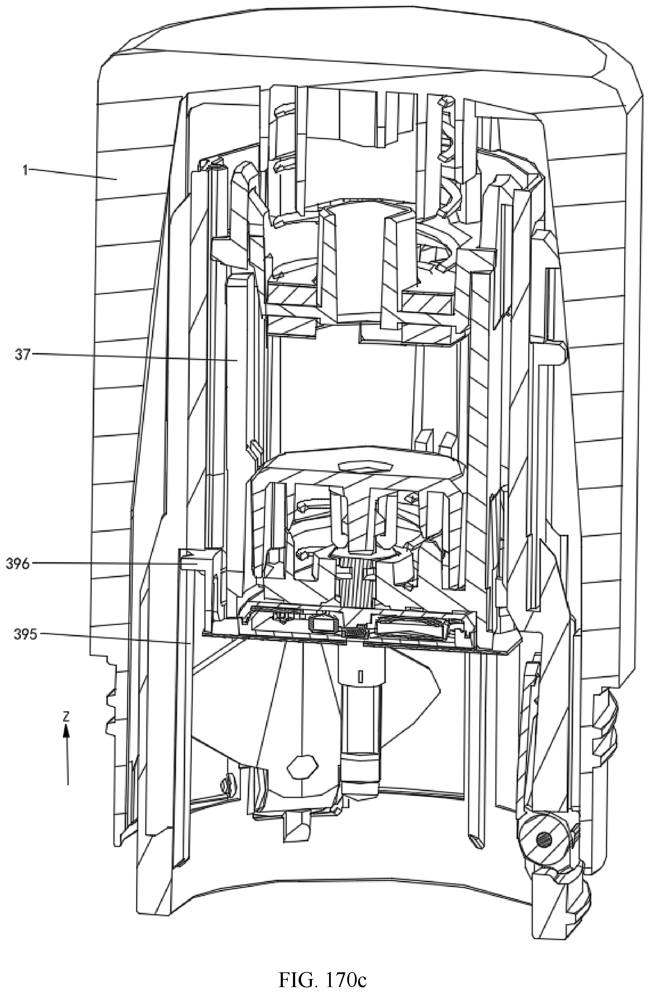

FIG. 170b is a diagram of a structure of the support 3 shown in FIG. 159 according to another implementation. FIG. 170c is a partial schematic cross-sectional view of the physiological sign monitoring apparatus shown in FIG. 157 along C9-C9 according to another implementation.

As shown in FIG. 170b and FIG. 170c, the support 3 may further include an elastic support arm 396. The elastic support arm 396 may be fastened to an end, away from the bottom plate 32 of the support 3, of the lower side plate 33 of the support 3. For example, the elastic support arm 396 may be fastened to the flange 34 and spaced away from the lower side plate 33 of the support 3. It can be understood that the elastic support arm 396 is elastically deformable.

In some implementations, the elastic support arm 396 may be provided with a sixth guide structure 395. The elastic support arm 396 abuts against an inner side of the guide pillar 507. In a process in which the first elastic member 2 pushes the support 3 toward the organism, the sixth guide structure 395 is slidably connected to the seventh guide structure 5071, to implement directional movement of the support 3. In this way, the elastic arm 294 is disposed between the base 5 and the support 3, so that there is no movement clearance between the support 3 and the base 5. Therefore, when the support 3 moves down, an amount of shaking of the support 3 in a horizontal direction is minimized, to minimize an amount of horizontal shaking of a hard needle during implantation, and minimize subcutaneous damage.

In some implementations, a hook of a fourth resilient arm 37 may be located in a middle section of the fourth resilient arm 37. It can be understood that a position of the hook of the fourth resilient arm 37 may be designed according to a requirement, and the hook of the fourth resilient arm 37 is configured to snap into the needle retraction holder 4.

In some implementations, foam, damping adhesive, or other shock-absorbent coatings may be added near the first elastic member 2 and the second elastic member 6. In this way, after the support 3 is deployed, vibration of the first elastic member 2 and the second elastic member 6 may be absorbed or reduced, to reduce subcutaneous damage caused by shaking. For example, two pieces of foam are shown in the figure. In another implementation, foam may be disposed between the first elastic member 2 and the base 5; and/or foam may be disposed between the second elastic member 6 and the needle retraction holder 4; and/or foam may be disposed between the second elastic member 6 and the support 3; and/or foam may be disposed on a side, away from the second elastic member 6, of the needle retraction holder 4.

In some implementations, a part that is the same as that in the foregoing embodiments is not described again.

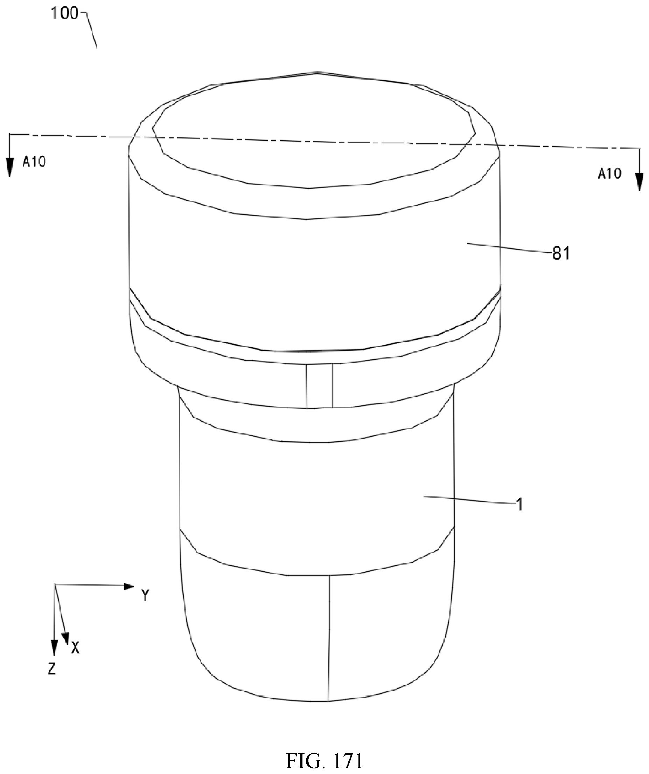

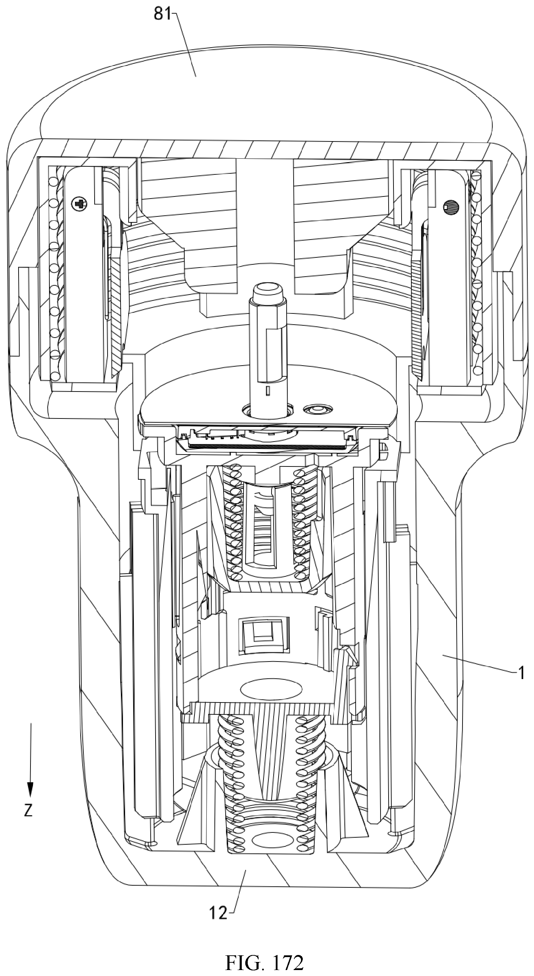

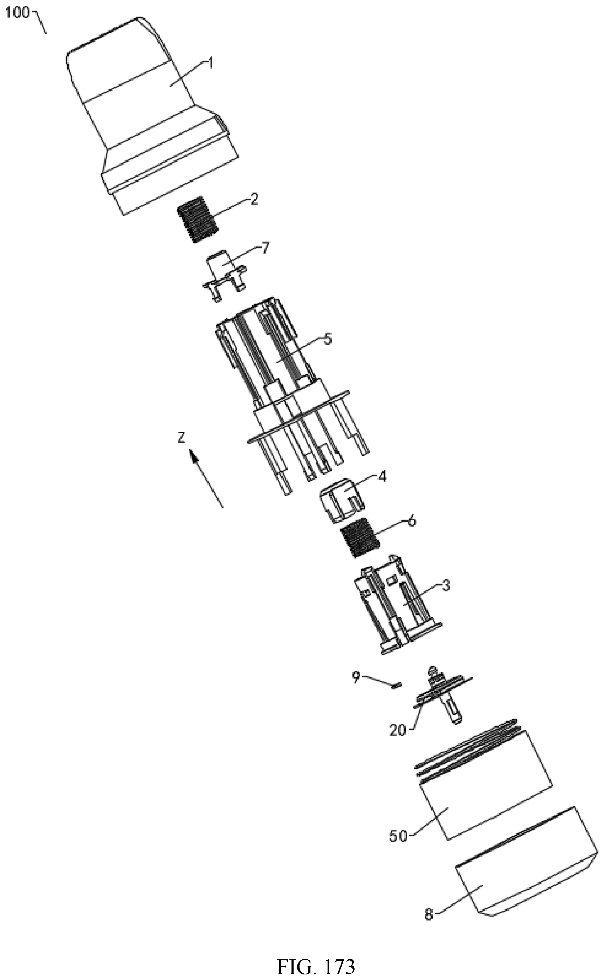

FIG. 171 is a diagram of a structure of the physiological sign monitoring apparatus 100 shown in FIG. 2 according to yet another implementation. FIG. 172 is a partial cross-sectional view of the physiological sign monitoring apparatus 100 shown in FIG. 171 along A10-A10 according to an implementation. FIG. 173 is a partial schematic exploded view of the physiological sign monitoring apparatus 100 shown in FIG. 171 according to an implementation.

As shown in FIG. 171 to FIG. 173, the physiological sign monitoring apparatus 100 may include an implantation apparatus 10, a concealing mechanism 50, and a sensor assembly 20. The implantation apparatus 10 may include a lower housing 1, a first elastic member 2, a spring seat 7, a base 5, a needle retraction holder 4, a second elastic member 6, a support 3, a first magnet 9, and a housing cap assembly 8. For a manner of disposing the sensor assembly 20, the first elastic member 2, the spring seat 7, the needle retraction holder 4, the second elastic member 6, the support 3, the first magnet 9, and the housing cap assembly 8, refer to the foregoing manners of disposing the sensor assembly 20, the first elastic member 2, the spring seat 7, the needle retraction holder 4, the second elastic member 6, the support 3, the first magnet 9, and the housing cap assembly 8. Details are not described herein again.

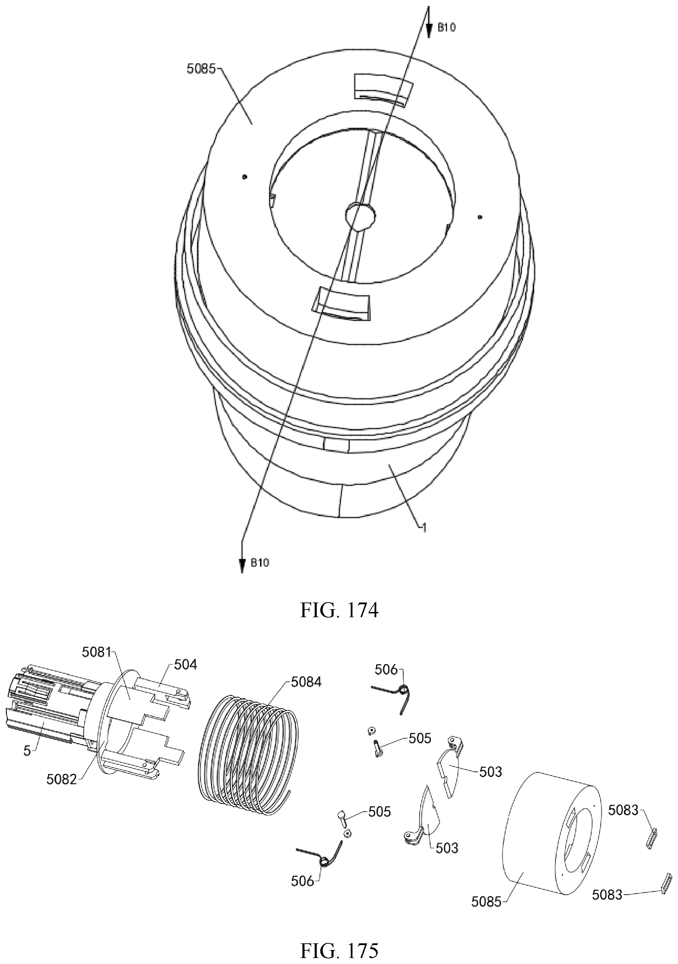

FIG. 174 is a diagram of a structure of the physiological sign monitoring apparatus 100 shown in FIG. 171 with the housing cap assembly 8 removed according to an implementation.

As shown in FIG. 172 and FIG. 174, when the physiological sign monitoring apparatus 100 is in a storage state and is not in use, the concealing mechanism 50 is fastened to the base 5, and an upper housing 81 of the housing cap assembly 8 is connected to the concealing mechanism 50. The concealing mechanism 50 is in an open state, and the sensor assembly 20 may be exposed inside the physiological sign monitoring apparatus 100. As shown in FIG. 48, when using the physiological sign monitoring apparatus 100, the user removes the housing cap assembly 8 and a sealed cartridge 29 of the sensor assembly 20. In this case, the concealing mechanism 50 is in a closed state, and the user cannot see a puncture needle 23 from the outside.

FIG. 175 is a schematic exploded view of the concealing mechanism 50 and the base 5 shown in FIG. 173 according to an implementation.

As shown in FIG. 175, the concealing mechanism 50 may include a hinge 503, a fastening pillar 504, a hinge pin 505, a torsion spring 506, a limiting pillar 5081, a bearing plate 5082, a second limiting block 5083, a first push block reset spring 5084, and a first push block 5085. There are a plurality of hinges 503, a plurality of fastening pillars 504, a plurality of hinge pins 505, and a plurality of torsion springs 506. A quantity of fastening pillars 504, a quantity of hinge pins 505, a quantity of torsion springs 506, and a quantity of hinges 503 are equal.

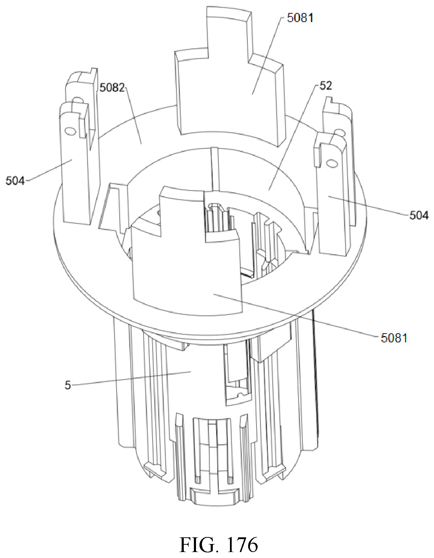

FIG. 176 is a partial diagram of assembly of the base 5 and the concealing mechanism 50 shown in FIG. 173.

As shown in FIG. 172 and FIG. 176, the bearing plate 5082 may be fastened to an end, away from a top wall 12 of the lower housing 1, of the base 5. For example, the bearing plate 5082 is fastened to an end, away from a connection arm 53, of a lower side wall 53 of the base 5. Both the limiting pillar 5081 and the fastening pillar 504 may be fastened to a side, away from the base 5, of the bearing plate 5082.

In some implementations, the fastening pillar 504 and the limiting pillar 5081 are spaced apart, there are a plurality of limiting pillars 5081, and a plurality of fastening pillars 504 are connected around a periphery of the base 5. All of the plurality of limiting pillars 5081 and the plurality of fastening pillars 504 are spaced apart around a central axis of the physiological sign monitoring apparatus 100. For example, there are two fastening pillars 504 and two limiting pillars 5081. All of the two limiting pillars 5081 and the two fastening pillars 504 are fastened to an end, away from a bottom plate 32 of the base 5, of the bearing plate 5082. The two limiting pillars 5081 and the two fastening pillars 504 are spaced apart. The two limiting pillars 5081 may be symmetrically arranged at 180°. The two fastening pillars 504 may be symmetrically arranged at 180°.

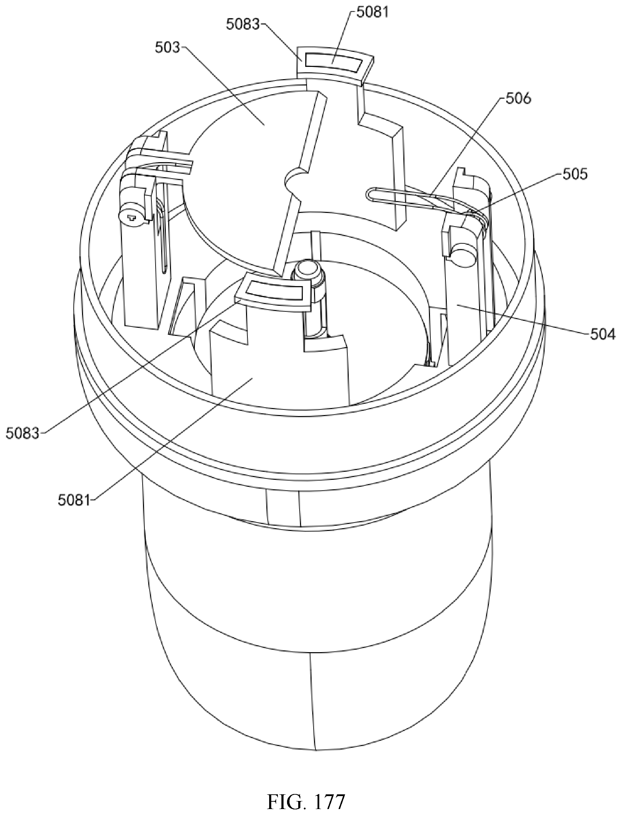

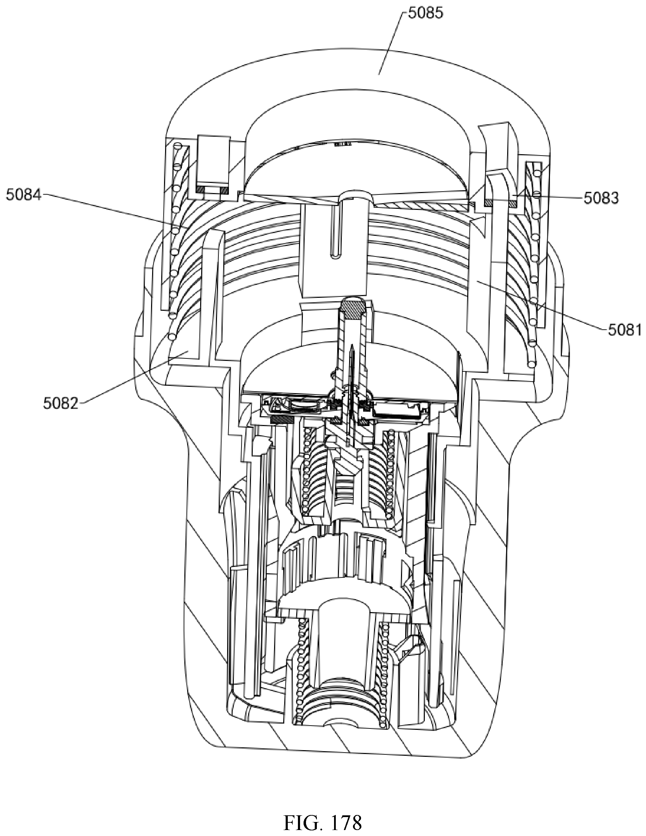

FIG. 177 is a diagram of a partial structure of the physiological sign monitoring apparatus 100 shown in FIG. 174 according to an implementation. FIG. 178 is a partial schematic cross-sectional view of the physiological sign monitoring apparatus 100 shown in FIG. 174 along B10-B10 according to an implementation.

As shown in FIG. 177 and FIG. 178, in some implementations, the hinge pin 505 is connected to an end, away from the base 5, of the fastening pillar 504, the torsion spring 506 is sleeved on the hinge pin 505, the hinge 503 is rotatably connected to the fastening pillar 504, and the torsion spring 506 abuts against a side, close to the base 5, of the hinge 503. For example, one end of the torsion spring 506 abuts against the hinge 503, and the other end of the torsion spring 506 abuts against the fastening pillar 504. The torsion spring 506 may be partially located between the hinge 503 and the bearing plate 5082.

In some implementations, the first push block 5085 is movably connected to the limiting pillar 5081, the first push block 5085 is partially located between the second limiting block 5083 and the bearing plate 5082, the first push block reset spring 5084 abuts between the first push block 5085 and the bearing plate 5082, the first push block 5085 includes an abutting end 9401, and the abutting end 9401 protrudes from the lower housing 1 through an opening 101. For example, the first push block 5085 is movably connected to the two limiting pillars 5081. One of two hinge pins 505 is rotatably connected to one fastening pillar 504, and the other hinge pin 505 is rotatably connected to the other fastening pillar 504. Two hinges 503 and the two hinge pins 505 are arranged in a one-to-one correspondence.

In some implementations, the second limiting block 5083 is fastened to the limiting pillar 5081, and is spaced away from the bearing plate 5082. For example, there may also be two second limiting blocks 5083. The two second limiting blocks 5083 and the two limiting pillars 5081 are arranged in a one-to-one correspondence. For example, the second limiting block 5083 may be fastened to an end, away from the bearing plate 5082, of the limiting pillar 5081. A part of the first push block 5085 may be located between the second limiting block 5083 and the bearing plate 5082.

In some implementations, the first push block reset spring 5084 abuts against the push block and the bearing plate 5082. The second limiting block 5083 may be configured to perform position limiting for the first push block 5085 in an axial direction, to prevent the first push block 5085 from detaching from the limiting pillar 5081 due to abutting against the first push block reset spring 5084.

For example, the concealing mechanism 50 may further include a limiting cap 5092. The limiting cap 5092 is fastened to one end of the hinge pin 505. The limiting cap 5092 includes an abutting portion. The abutting portion abuts against the fastening pillar 504, and is located between the fastening pillar 504 and the bearing plate 5082. The limiting cap 5092 may be configured to prevent the hinge 503 from over-rotating under the action of an upward abutting force of the torsion spring 506.

A movement process of the concealing mechanism 50 is as follows: During assembly of the physiological sign monitoring apparatus 100, after the sensor assembly 20 is mounted to the support 3, the concealing structure is mounted to the base 5, and then the housing cap assembly 8 is mounted. The housing cap assembly 8 may apply, to the push block of the concealing mechanism 50, a force for moving toward the base 5. The first push block 5085 moves toward the base 5, and the first push block 5085 abuts against the hinge 503, so that one end of the hinge 503 moves toward the bearing plate 5082 of the base 5, the torsion spring 506 and the first push block reset spring 5084 are compressed, and the housing cap assembly 8 is fastened to the lower housing 1 through threads. In this case, the hinge 503 of the concealing mechanism 50 remains in an open state.

When using the physiological sign monitoring apparatus 100, the user removes the housing cap assembly 8, the first push block reset spring 5084 applies, to the first push block 5085, a force for moving away from the base 5, the first push block 5085 moves in a direction away from the base 5, two torsion springs 506 respectively apply, to the two hinges 503, forces for moving away from the base 5, and the hinges 503 are closed to conceal the puncture needle 23 of the sensor assembly 20. In this case, sight of the user may be blocked by the hinges 503, and the user cannot see the puncture needle 23 from the outside. This helps eliminate a fear of the user for the puncture needle 23 when the user uses the physiological sign monitoring apparatus 100.

It can be understood that the bearing plate 5082, the two limiting pillars 5081, and the two fastening pillars 504 in this embodiment may constitute a bearing member of the concealing mechanism 50. The bearing member and a lower side plate 33 of the base 5 are an integrally formed mechanical part. In another implementation, the bearing member may alternatively be separately prepared, and then assembled with a lower side plate 33 of the base 5, or assembled with the housing. This is not limited in this application.

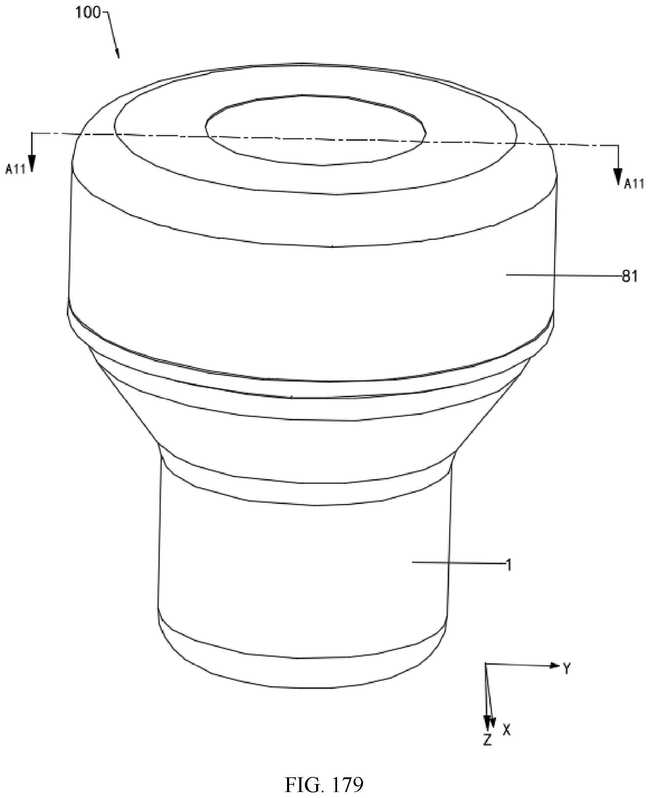

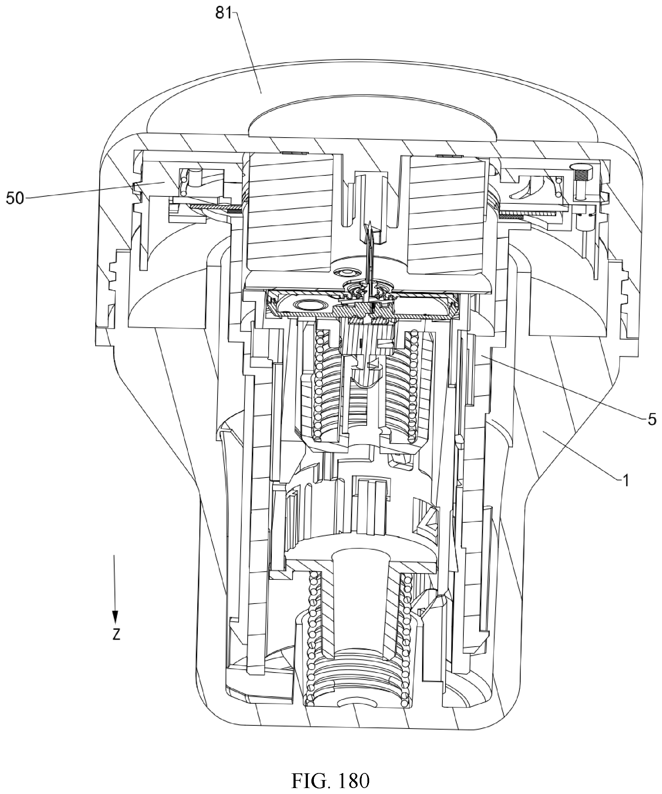

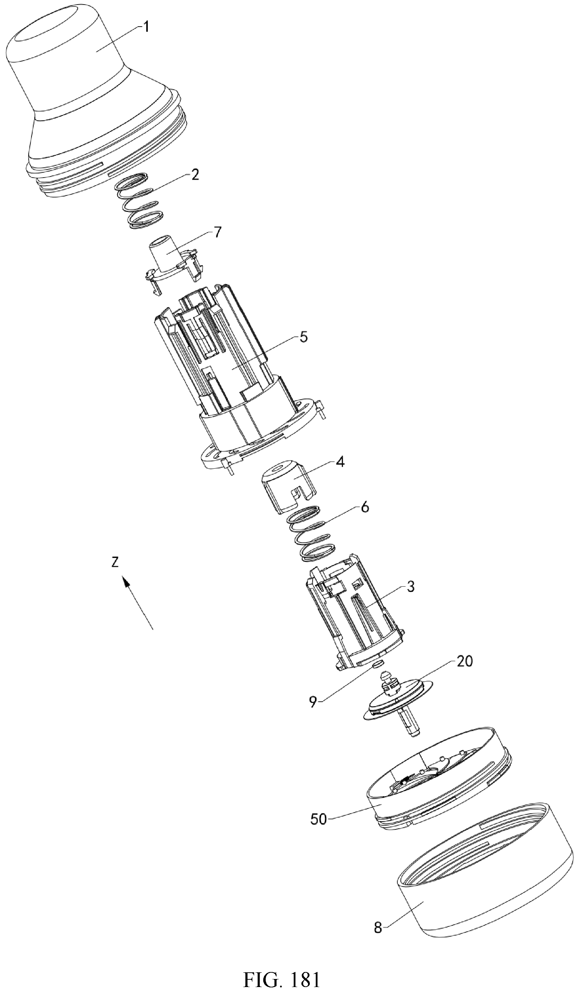

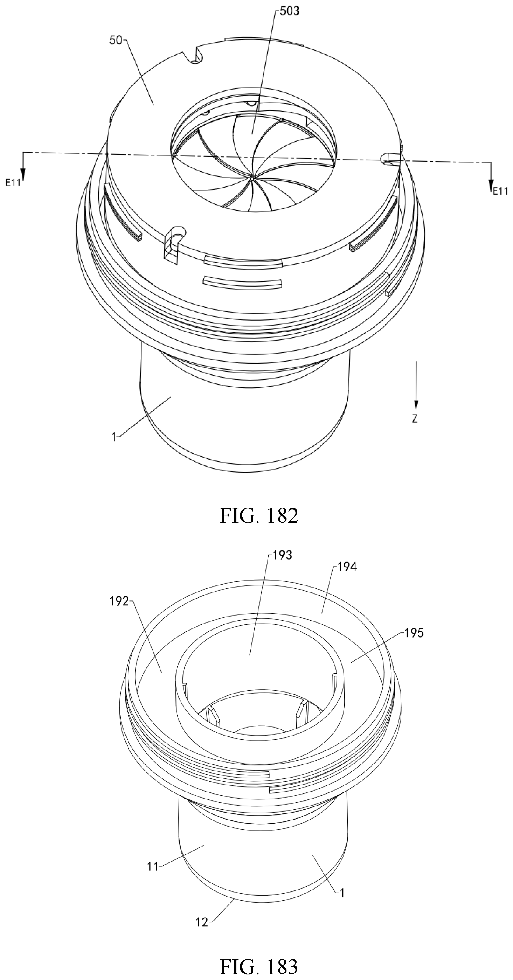

In some implementations, a part that is the same as that in the foregoing embodiments is not described again. FIG. 179 is a diagram of a structure of the physiological sign monitoring apparatus 100 shown in FIG. 2 according to yet another implementation. FIG. 180 is a partial cross-sectional view of the physiological sign monitoring apparatus 100 shown in FIG. 179 along A11-A11 according to an implementation. FIG. 181 is a partial schematic exploded view of the physiological sign monitoring apparatus 100 shown in FIG. 179 according to an implementation. FIG. 182 is a diagram of a structure of the physiological sign monitoring apparatus 100 shown in FIG. 179 with a housing cap assembly 8 removed according to an implementation.

As shown in FIG. 179 to FIG. 182, the physiological sign monitoring apparatus 100 may include an implantation apparatus 10, a concealing mechanism 50, and a sensor assembly 20. The implantation apparatus 10 may include a lower housing 1, a first elastic member 2, a spring seat 7, a base 5, a needle retraction holder 4, a second elastic member 6, a support 3, a first magnet 9, and the housing cap assembly 8. For a manner of disposing the sensor assembly 20, the first elastic member 2, the spring seat 7, the needle retraction holder 4, the second elastic member 6, the support 3, the first magnet 9, and the housing cap assembly 8, refer to the foregoing manners of disposing the sensor assembly 20, the first elastic member 2, the spring seat 7, the needle retraction holder 4, the second elastic member 6, the support 3, the first magnet 9, and the housing cap assembly 8. Details are not described herein again.

FIG. 183 is a diagram of the lower housing 1 shown in FIG. 180 according to an implementation. A part, the same as that in the foregoing embodiments, of the lower housing 1 shown in FIG. 183 is not described herein again.

As shown in FIG. 183, the lower housing 1 may include a top wall 12, a side wall 11, a bottom plate 192, a first side plate 193, and a second side plate 194. The bottom plate 192 is connected to an end, away from the top wall 12, of the side wall 11 of the lower housing 1. The bottom plate 192 is provided with a first through hole 1921, and the first through hole 1921 is in communication with an internal cavity 102 of the lower housing 1. Both the first side plate 193 and the second side plate 194 are fastened to a side, away from the side wall 11 of the lower housing 1, of the bottom plate 192 of the lower housing 1. Both the first side plate 193 and the second side plate 194 are arranged around a through hole 121. The first side plate 193 and the second side plate 194 are approximately in a ring shape. The first side plate 193 is located on an inner side of the second side plate 194. The bottom plate 192, the first side plate 193, and the second side plate 194 enclose movement space 195.

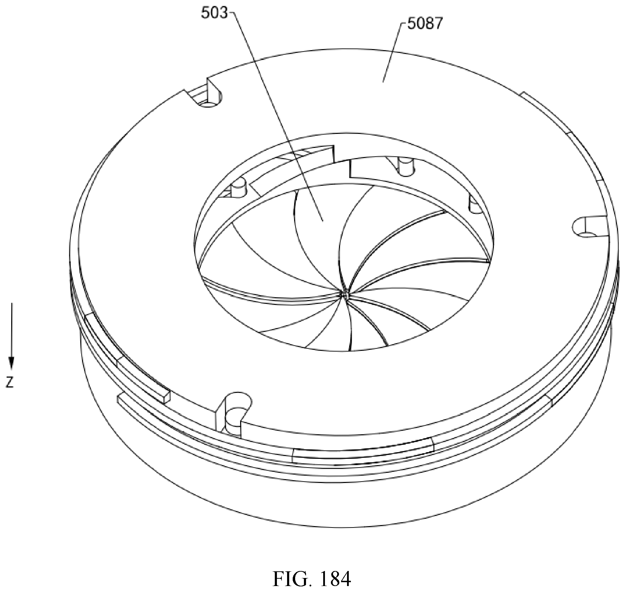

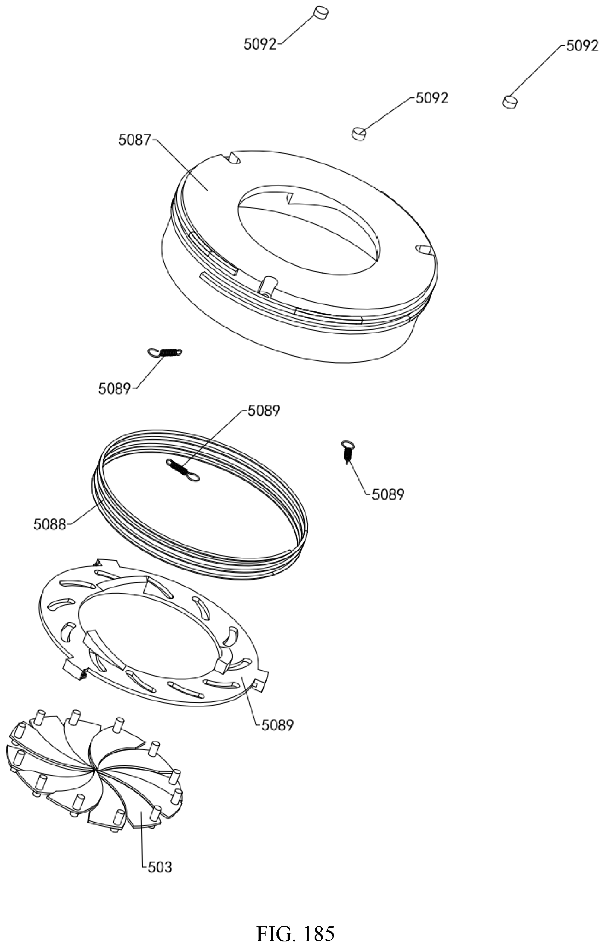

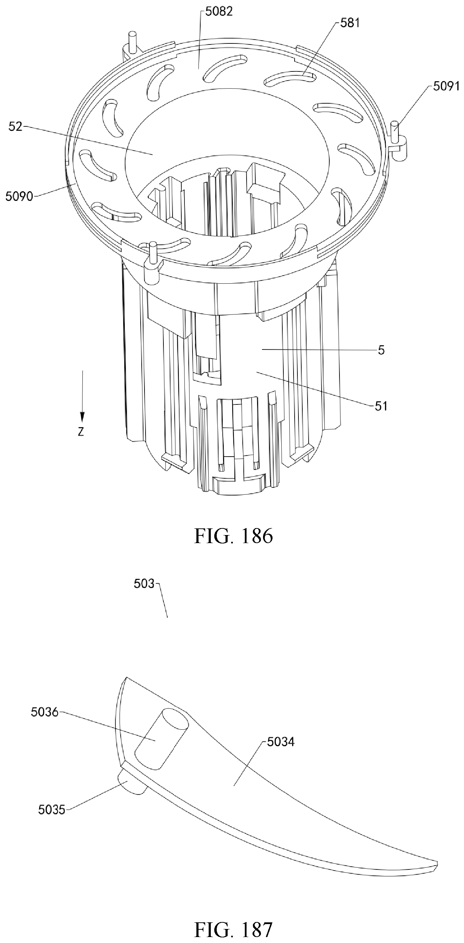

FIG. 184 is a diagram of a partial structure of the concealing mechanism 50 shown in FIG. 181 according to an implementation. FIG. 185 is a schematic exploded view of the concealing mechanism 50 shown in FIG. 184 according to an implementation. FIG. 186 is a diagram of the base 5 shown in FIG. 180 according to an implementation. A part, the same as that in the foregoing embodiments, of the base 5 shown in FIG. 186 is not described herein again.

As shown in FIG. 184 to FIG. 186, the concealing mechanism 50 may include a hinge 503, a bearing plate 5082, a drive wheel 5086, a second push block 5087, a second push block reset spring 5088, and a drive wheel reset spring 5089.

In some implementations, the bearing plate 5082 may be fastened to an end, away from the top wall 12 of the lower housing 1, of the base 5, and the bearing plate 5082 is provided with a first guide hole 581. For example, the bearing plate 5082 may be fastened to a side, away from an upper side wall 51, of a lower side wall 52 of the base 5.

For example, the concealing mechanism 50 may further include a side plate 5090, and the side plate 5090 may be fastened to a periphery of the bearing plate 5082. The bearing plate 5082 may be provided with a first guide hole 581.

In some implementations, the concealing mechanism 50 may further include a fastening stub 5091. The fastening stub 5091 may be fastened to an outer side of the bearing plate 5082.

FIG. 187 is a diagram of a structure of the hinge 503 shown in FIG. 185 according to an implementation.

As shown in FIG. 187, the hinge 503 includes a blade 5034, a first guide pillar 5035, and a second guide pillar 5036. The blade 5034 includes a first surface 5037 and a second surface 5038 that face away from each other. The first guide pillar 5035 is fastened to the first surface 5037, and the second guide pillar 5036 is fastened to the second surface 5038.

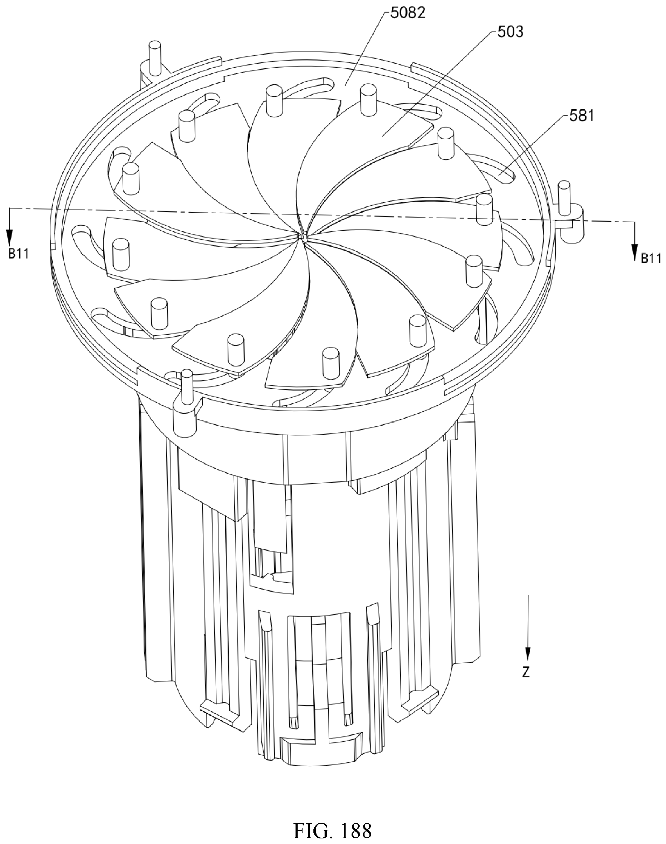

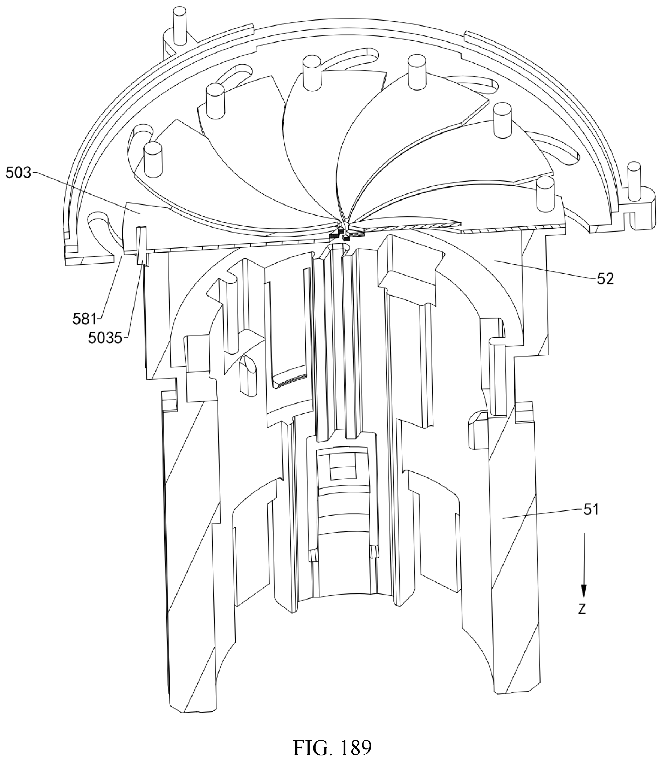

FIG. 188 is a diagram of a partial structure of the physiological sign monitoring apparatus 100 shown in FIG. 179. FIG. 189 is a partial cross-sectional view along B11-B11 in FIG. 188 according to an implementation.

As shown in FIG. 188 and FIG. 189, the first guide pillar 5035 of the hinge 503 is slidably connected to the first guide hole 581 of the base 5. For example, there may be a plurality of first guide holes 581, and a quantity of first guide holes 581 may match a quantity of hinges 503. A plurality of hinges 503 and a plurality of first guide holes 581 are arranged in a one-to-one correspondence.

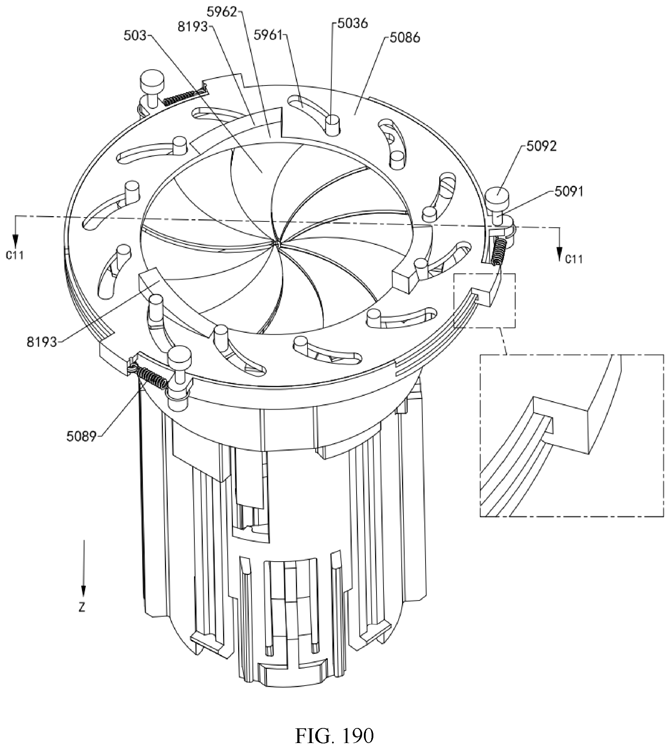

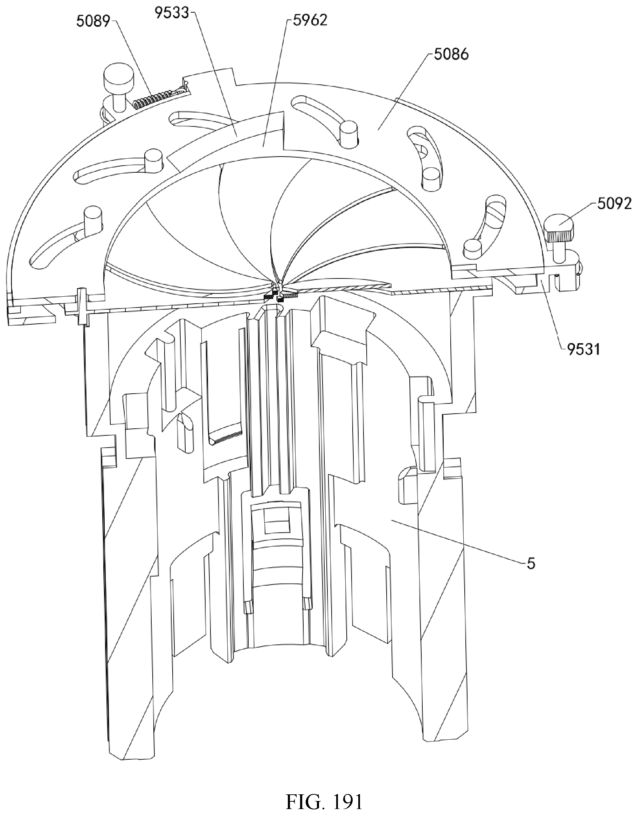

FIG. 190 is a diagram of a partial structure of the physiological sign monitoring apparatus 100 shown in FIG. 179. FIG. 191 is a partial cross-sectional view of the structure shown in FIG. 190 along C11-C11 according to an implementation.

As shown in FIG. 190 and FIG. 191, the drive wheel 5086 is slidably connected to the base 5. The drive wheel 5086 may be provided with a second guide hole 5961, and the second guide pillar 5036 of the hinge 503 is slidably connected to the second guide hole 5961.

For example, the drive wheel 5086 includes a second bump 5962, and the second bump 5962 has a second inclined surface 8193.

For example, a limiting cap 5092 may be fastened to an end, away from the side plate 5090, of the fastening stub 5091. The limiting cap 5092 and the side plate 5090 are spaced apart in the axial direction of the physiological sign monitoring apparatus 100. It can be understood that the limiting cap 5092 is disposed to perform position limiting for the second push block 5087 in the axial direction, to prevent the second push block 5087 from falling off under the action of the second push block reset spring 5088.

For example, one end of the drive wheel reset spring 5089 is fastened to the base 5, and the other end of the drive wheel reset spring 5089 is fastened to the drive wheel 5086. For example, one end of the drive wheel reset spring 5089 may be fastened to the fastening stub 5091.

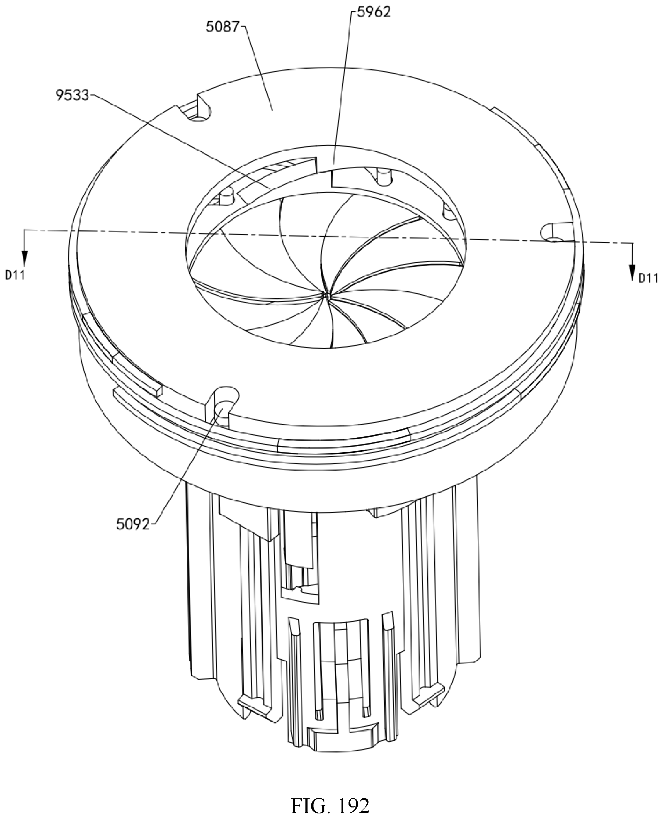

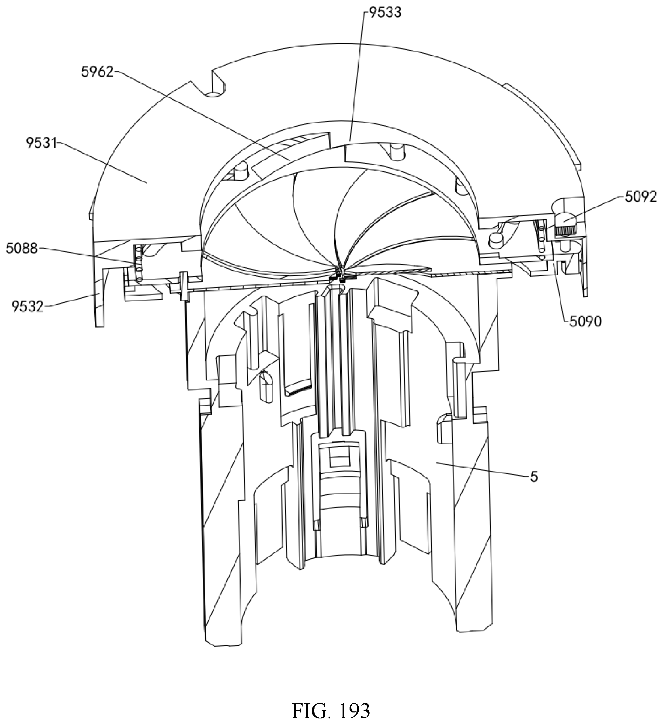

FIG. 192 is a diagram of a partial structure of the physiological sign monitoring apparatus 100 shown in FIG. 179. FIG. 193 is a partial cross-sectional view of the structure shown in FIG. 192 along D11-D11 according to an implementation.

As shown in FIG. 192 and FIG. 193, the second push block 5087 may include a top plate 9531, a side plate 9532, and a first bump 9533. The side plate 9532 of the second push block 5087 may be fastened to a side, facing the drive wheel 5086, of the top plate 9531 of the second push block 5087. The first bump 9533 may also be fastened to the side, facing the drive wheel 5086, of the top plate 9531 of the second push block 5087, and is spaced away from the side plate 9532 of the second push block 5087. The first bump 9533 of the second push block 5087 may be arranged opposite to the second bump 5962 of the drive wheel 5086.

It can be understood that, when the first bump 9533 has an inclined surface matching the inclined surface of the second bump 5962 and the second push block 5087 moves down under pressure, the first bump 9533 and the second bump 5962 may fit with each other through the inclined surfaces, to implement relative rotation between the second push block 5087 and the drive wheel 5086.

For example, the second push block reset spring 5088 may abut between the second push block 5087 and the drive wheel 5086. An axial direction of the second push block reset spring 5088 is approximately the same as an axial direction of the base 5. For example, the second push block 5087 includes an abutting end 9401, and the abutting end 9401 protrudes from the lower housing 1 through an opening 101.

For example, the top plate 9531 of the second push block 5087 is provided with a fastening hole. A part of the fastening stub 5091 is located in the fastening hole of the second push block 5087. One end of the fastening stub 5091 passes through the fastening hole, and the limiting cap 5092 is fastened to one end of the fastening stub 5091. A part of the second push block 5087 is located between the limiting cap 5092 and the base 5.

For example, there is a gap between the limiting cap 5092 and the side plate 5090 of the concealing mechanism 50. The second push block 5087 may move up and down in the gap.

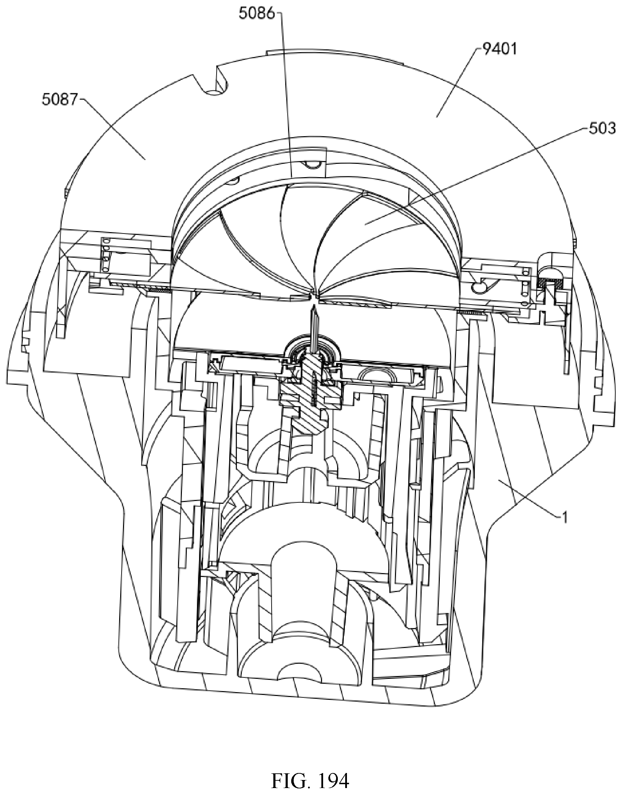

For example, there may be a plurality of fastening stubs 5091 and a plurality of limiting caps 5092, and the plurality of fastening stubs 5091 and the plurality of limiting caps 5092 are arranged in a one-to-one correspondence. The plurality of fastening stubs 5091 may be spaced apart around the hinge 503. In this way, position limiting can be performed for movement of the second push block 5087 in a circumferential direction. FIG. 194 is a partial cross-sectional view of the mechanism shown in FIG. 182 along E11-E11 according to an implementation.

As shown in FIG. 180 and FIG. 194, an outer side of the side plate 9532 of the second push block 5087 may be provided with threads (as shown in FIG. 184). Threads matching the threads on the outer side of the side plate 9532 of the second push block 5087 may be provided on an inner side of a side wall 812 of an upper housing 81. When the housing cap assembly 8 is mounted to the lower housing 1, the threads of the upper housing 81 may fit with the threads of the second push block 5087, to apply a pressing force to the second push block 5087. The first bump 9533 abuts against the second bump 5962, and the second push block 5087 may push, through the inclined surface, the drive wheel 5086 to rotate. When the drive wheel 5086 rotates, the drive wheel 5086 may drive, through fitting between the second guide hole 5961 and the second guide pillar 5036 and between the first guide hole 581 and the first guide pillar 5035, the hinge 503 to rotate. The concealing mechanism 50 is open, and the puncture needle 23 below the hinge 503 is exposed.

When the second push block 5087 is being pressed, the second push block 5087 is close to the drive wheel 5086, and the second push block reset spring 5088 is compressed. The drive wheel 5086 rotates under a force, and the drive wheel reset spring 5089 is stretched. When the housing cap assembly 8 is removed, the second push block reset spring 5088 abuts between the second push block 5087 and the drive wheel 5086, the second push block reset spring 5088 may apply an upward force to the second push block 5087, the second push block 5087 moves up, and a force between the first bump 9533 and the second bump 5962 gradually disappears. The drive wheel reset spring 5089 shrinks to apply a return force to the drive wheel 5086, so that the drive wheel 5086 can rotate back to a position at which the hinge 503 is not open. Driven by the drive wheel 5086, the plurality of hinges 503 rotate, so that the concealing mechanism 50 is closed, and the through hole 121 of the lower housing 1 is covered. In this case, the puncture needle 23 in the base 5 cannot be seen.

It can be understood that, when using the apparatus, the user removes the housing cap assembly 8, and the plurality of hinges 503 rotate, to enable the concealing mechanism 50 to be in the closed state. When the user presses the apparatus against skin, the second push block 5087 first pushes the drive wheel 5086 to rotate by a specific angle to enable the concealing mechanism 50 to open, without affecting pushing of the sensor assembly 20. During use, the user needs to first press the apparatus by a specific stroke. Then the concealing mechanism 50 is open, and the user continues to press the apparatus. After a second resilient arm 56 is spread open, the support 3 is pushed out.

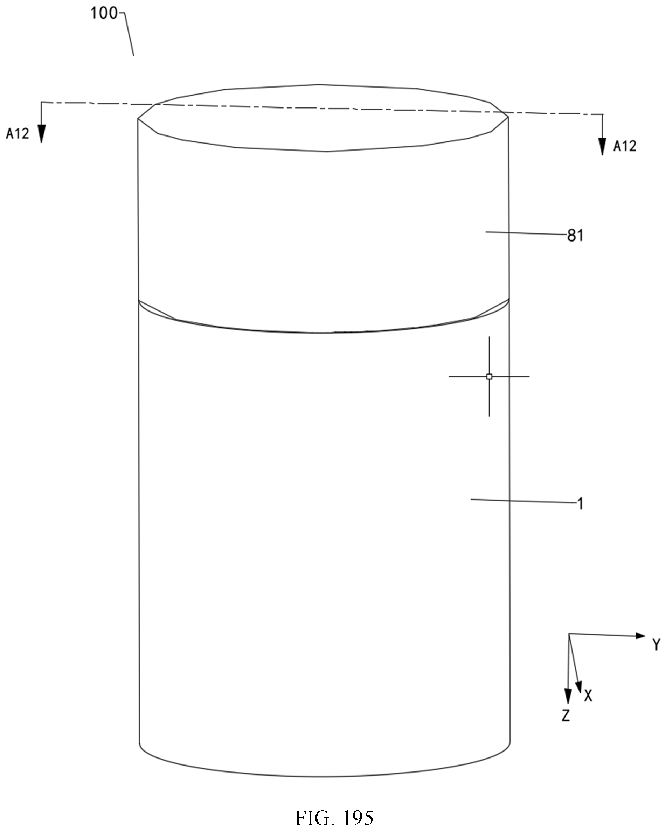

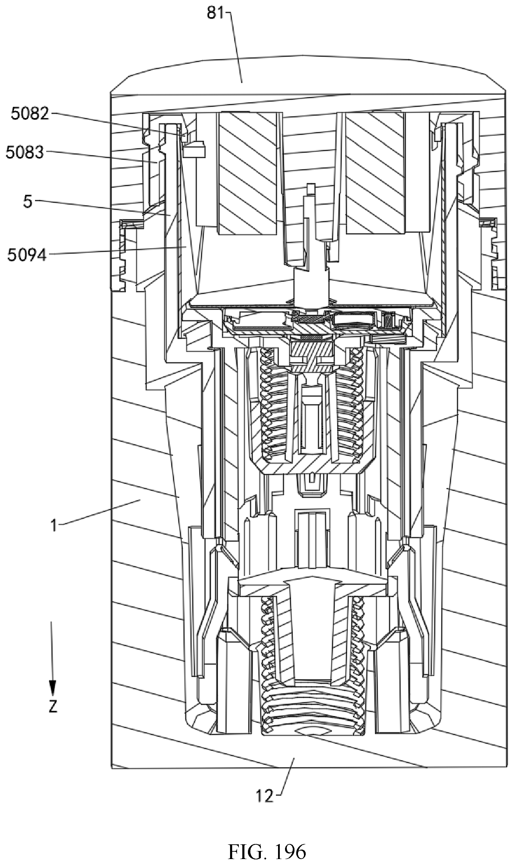

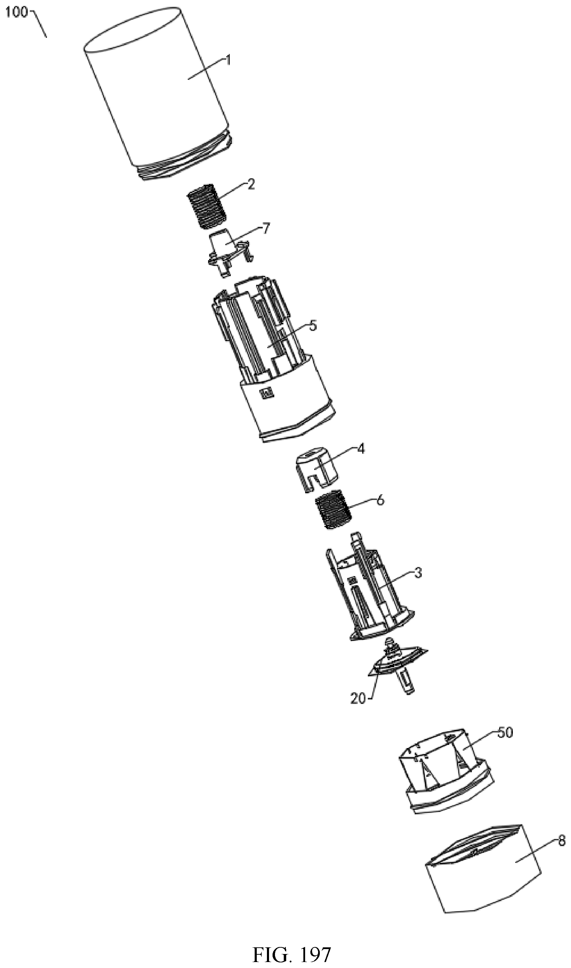

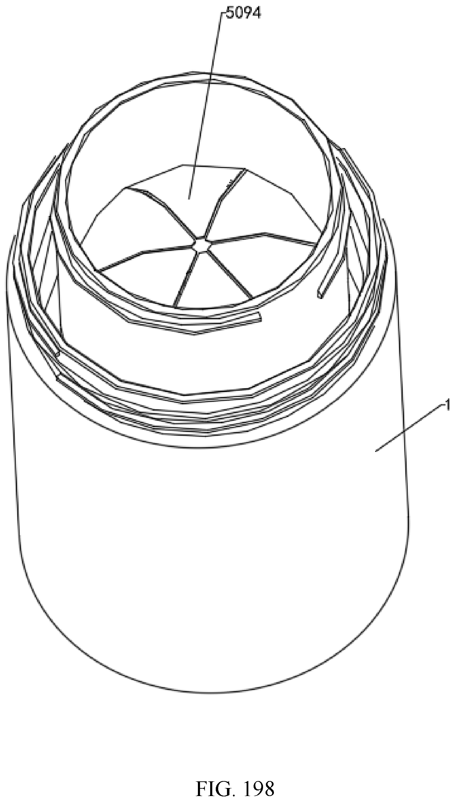

In some implementations, a part that is the same as that in the foregoing embodiments is not described again. FIG. 195 is a diagram of a structure of the physiological sign monitoring apparatus 100 shown in FIG. 2 according to yet another implementation. FIG. 196 is a partial cross-sectional view of the physiological sign monitoring apparatus 100 shown in FIG. 195 along A12-A12 according to an implementation. FIG. 197 is a partial schematic exploded view of the physiological sign monitoring apparatus 100 shown in FIG. 195 according to an implementation. FIG. 198 is a diagram of a structure of the physiological sign monitoring apparatus 100 shown in FIG. 195 with a housing cap assembly 8 removed according to an implementation.

As shown in FIG. 195 to FIG. 198, the physiological sign monitoring apparatus 100 may include an implantation apparatus 10, a concealing mechanism 50, and a sensor assembly 20. The implantation apparatus 10 may include a lower housing 1, a first elastic member 2, a spring seat 7, a base 5, a needle retraction holder 4, a second elastic member 6, a support 3, a first magnet 9, and the housing cap assembly 8. For a manner of disposing the sensor assembly 20, the first elastic member 2, the spring seat 7, the needle retraction holder 4, the second elastic member 6, the support 3, the first magnet 9, and the housing cap assembly 8, refer to the foregoing manners of disposing the sensor assembly 20, the first elastic member 2, the spring seat 7, the needle retraction holder 4, the second elastic member 6, the support 3, the first magnet 9, and the housing cap assembly 8. Details are not described herein again.

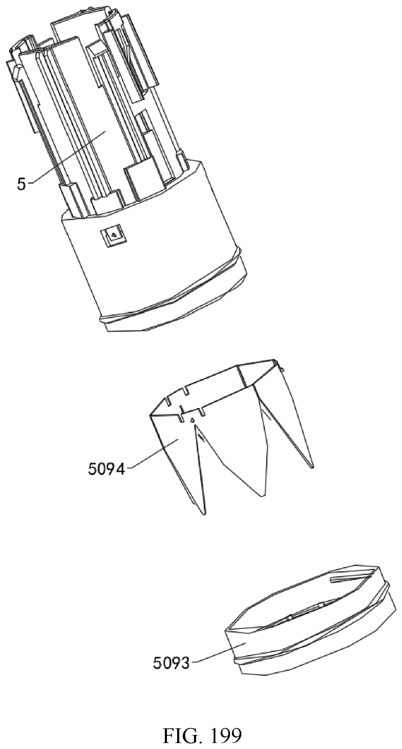

FIG. 199 is a diagram of a partial structure of the physiological sign monitoring apparatus 100 shown in FIG. 196.

As shown in FIG. 199, the concealing mechanism 50 may include an alloy limiting cap 5093 and a plurality of alloy sheets 5094. The alloy is in a curved-surface shape. For example, the alloy sheet 5094 may be made of a memory alloy.

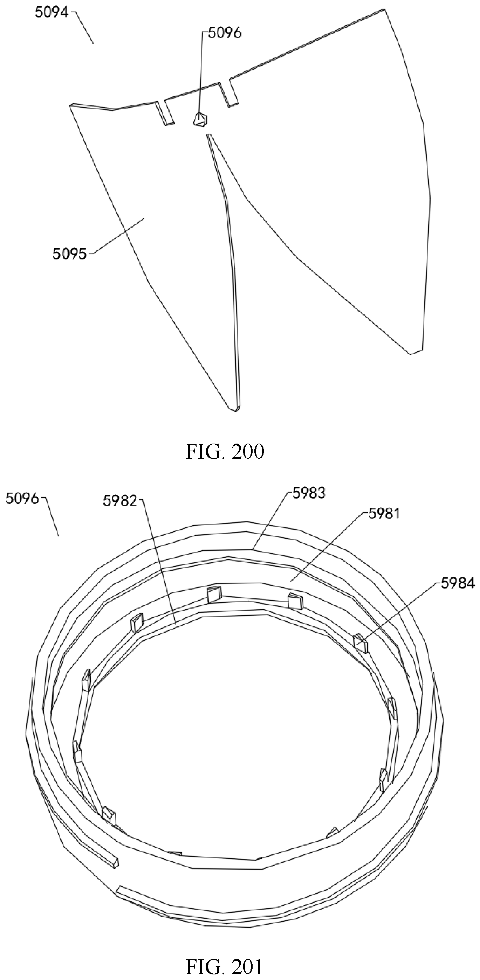

FIG. 200 is a diagram of a structure of the alloy sheet 5094 shown in FIG. 199 according to an implementation.

As shown in FIG. 200, the alloy sheet 5094 may include a body portion 5095 and a positioning portion 5096. The positioning portion 5096 is protrudingly disposed on an outer side of the body portion 5095. For example, the body portion 5095 may be approximately in a sheet-like shape. For example, the body portion 5095 may be approximately in a sheet-like shape similar to a petal, as shown in FIG. 200. In another implementation, the body portion 5095 may alternatively be in another shape. This is not limited in this application.

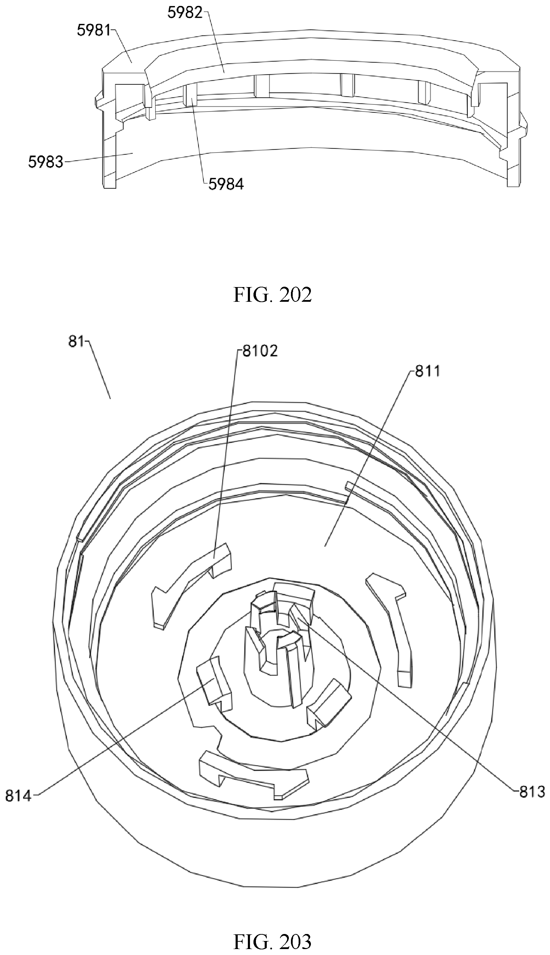

FIG. 201 is a diagram of a structure of the alloy limiting cap 5093 shown in FIG. 199 according to an implementation. FIG. 202 is a partial cross-sectional view of the physiological sign monitoring apparatus 100 shown in FIG. 195 along A12-A12 according to an implementation.

As shown in FIG. 201 and FIG. 202, the alloy limiting cap 5093 may include a bottom plate 5981, a first side plate 5982, and a second side plate 5983. The first side plate 5982 and the second side plate 5983 are fastened to a same side of the bottom plate 5981 at a spacing. The bottom plate 5981 is approximately in a ring shape. The first side plate 5982 is fastened to an inner periphery of the bottom plate 5981, and the second side plate 5983 is fastened to an outer periphery of the bottom plate 5981.

For example, the alloy limiting cap 5093 may further include a third limiting block 5984. The third limiting block 5984 may be fastened to an end, away from the bottom plate 5981, of the first side plate 5982. There may be one or more third limiting blocks 5984. When there are a plurality of third limiting blocks 5984, the plurality of third limiting blocks 5984 may be spaced apart around a central axis of the alloy limiting cap 5093.

For example, an inner side surface of the second side plate 5983 may be provided with a first thread, and an outer side surface of the second side plate 5983 may be provided with a second thread. The inner side surface of the second side plate 5983 is a surface on a side, facing the first side plate 5982, of the second side plate 5983. The outer side surface of the second side plate 5983 is a surface on a side, facing away from the first side plate 5982, of the second side plate 5983.

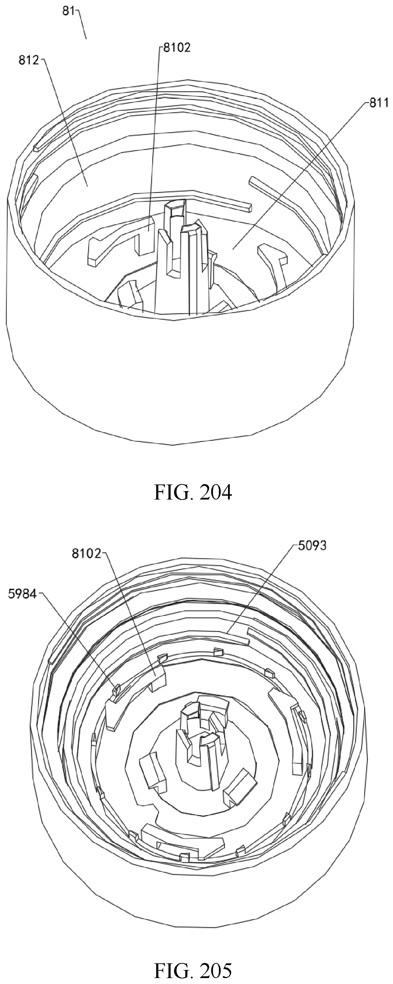

FIG. 203 is a diagram of a structure of an upper housing 81 shown in FIG. 197 according to an implementation. FIG. 204 is a diagram of a structure of the upper housing 81 shown in FIG. 203 from another perspective.

As shown in FIG. 203 and FIG. 204, the upper housing 81 may further include a second limiting arm 8102. One end of the second limiting arm 8102 is fastened to a bottom wall 811 of the upper housing 81, and is spaced away from a pawl 813 and a limiting member 814. The other end of the second limiting arm 8102 may be provided with a hook. The hook faces a side wall 812 of the upper housing 81.

For example, there may be one or more second limiting arms 8102. When there are a plurality of second limiting arms 8102, the plurality of second limiting arms 8102 are spaced apart around a central axis of the upper housing 81.

FIG. 205 is a diagram of assembly of a partial structure shown in FIG. 197 according to an implementation. FIG. 205 is a diagram of assembly of the upper housing 81 and the alloy limiting cap 5093 according to an implementation.

As shown in FIG. 196 and FIG. 205, when the physiological sign monitoring apparatus 100 is in an unused state, the first thread on the inner side surface of the second side plate 5983 may be configured to fit with threads on the base 5, to implement fastening to the base 5. The second thread on the outer side surface of the second side plate 5983 may be configured to fit with threads on the upper housing 81, to implement fastening to the upper housing 81.

For example, the base 5 may be partially located between the first side plate 5982 and the second side plate 5983 of the alloy limiting cap 5093. An end, away from the base 5, of the alloy sheet 5094 is located between the base 5 and the alloy limiting cap 5093.

For example, the alloy sheet 5094 may be fastened to an inner side of the base 5, and the alloy limiting cap 5093 is fastened to the lower housing 1.

For example, in an axial direction of the upper housing 81, the second limiting arm 8102 of the upper housing 81 may be arranged opposite to the alloy limiting cap 5093. In a length direction of the first side plate 5982 (namely, a circumferential direction of the first side plate 5982), the hook of the second limiting arm 8102 may be arranged opposite to the third limiting block 5984. It can be understood that, through fitting between the second limiting arm 8102 and the third limiting block 5984, when the physiological sign monitoring apparatus 100 switches from the unused state to an in-use state and the upper housing 81 is detached from the lower housing 1, the second limiting arm 8102 may push the alloy limiting cap 5093 to rotate, to disconnect from the base 5. Then the second limiting arm 8102 may be detached from the base 5 together with the alloy limiting cap 5093. Ends, away from the base 5, of the alloy converge to conceal a puncture needle 23 (as shown in FIG. 198).

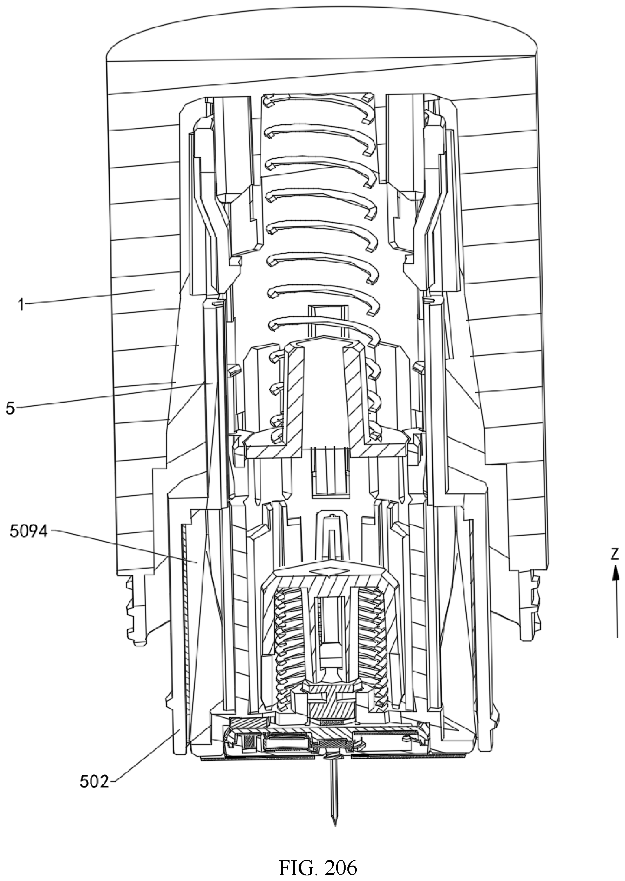

FIG. 206 is a partial cross-sectional view of the physiological sign monitoring apparatus 100 shown in FIG. 195 along A12-A12 in an in-use state according to an implementation.

As shown in FIG. 196 and FIG. 206, the base 5 includes an abutting end 502, and the abutting end 502 protrudes from the lower housing 1 through an opening 101. For example, when the physiological sign monitoring apparatus 100 is in an unused state, the first thread on the inner side surface of the second side plate 5983 may be configured to fit with threads on the base 5, to connect to the abutting end 502 of the base 5.

When the physiological sign monitoring apparatus 100 switches from the unused state to the in-use state, the alloy limiting cap 5093 is detached from the base 5, the abutting end 502 abuts against the organism, and the lower housing 1 moves relative to the base 5 in a direction toward the organism, the first elastic member 2 pushes the support 3 toward the organism, and the support 3 pushes the alloy sheets 5094 open, to enable the concealing mechanism 50 to be in an open state and the puncture needle 23 to be exposed. The first elastic member 2 continues to push the support 3, to deploy the sensor assembly 20, and the puncture needle 23 punctures into skin of the user.

It can be understood that, in comparison with the foregoing embodiments of the concealing mechanism 50, in this embodiment, during implantation of the sensor assembly 20, a hinge 503 of the concealing mechanism 50 opens while moving, without a need to first press a door plate to enable the hinge 503 to open and then trigger implantation. Therefore, a deployment stroke of pressing is small, and a length of the physiological sign monitoring apparatus 100 in the axial direction can be set to be small. In addition, there are a large quantity of hinges 503, and when the concealing mechanism 50 is in the closed state, the hinges 503 can better conceal the puncture needle 23, to ensure that the user cannot see the puncture needle 23, and eliminate a fear of the user for the needle.

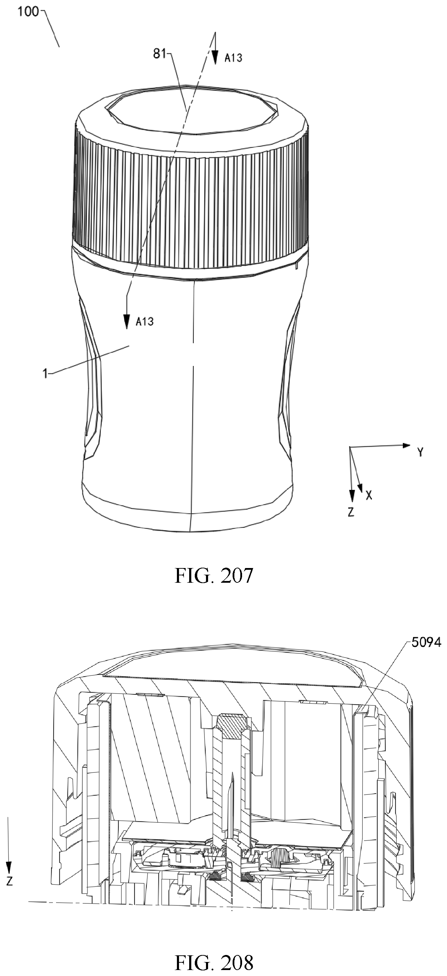

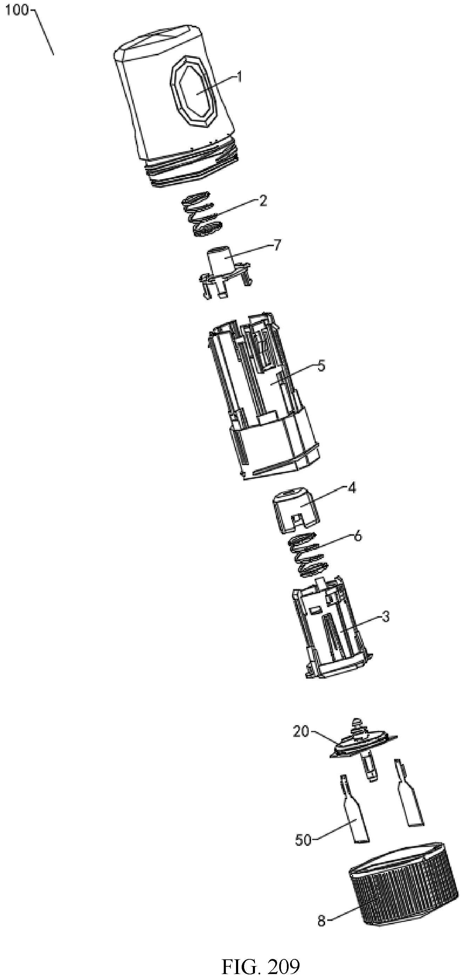

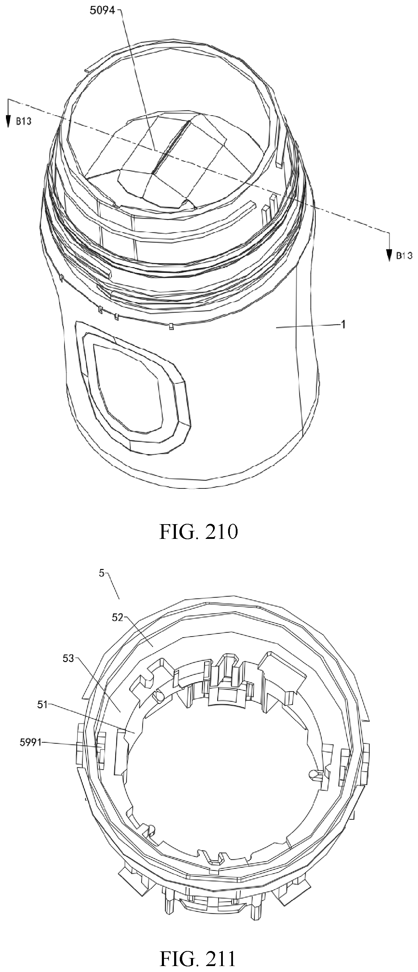

In some implementations, a part that is the same as that in the foregoing embodiments is not described again. FIG. 207 is a diagram of a structure of the physiological sign monitoring apparatus 100 shown in FIG. 2 according to yet another implementation. FIG. 208 is a partial cross-sectional view of the physiological sign monitoring apparatus 100 shown in FIG. 207 along A13-A13 according to an implementation. FIG. 209 is a partial schematic exploded view of the physiological sign monitoring apparatus 100 shown in FIG. 207 according to an implementation. FIG. 210 is a diagram of a structure of the physiological sign monitoring apparatus 100 shown in FIG. 207 with a housing cap assembly 8 removed according to an implementation.

As shown in FIG. 207 to FIG. 210, the physiological sign monitoring apparatus 100 may include an implantation apparatus 10, a concealing mechanism 50, and a sensor assembly 20. The implantation apparatus 10 may include a lower housing 1, a first elastic member 2, a spring seat 7, a base 5, a needle retraction holder 4, a second elastic member 6, a support 3, a first magnet 9, and the housing cap assembly 8. For a manner of disposing the sensor assembly 20, the first elastic member 2, the spring seat 7, the needle retraction holder 4, the second elastic member 6, the support 3, the first magnet 9, and the housing cap assembly 8, refer to the foregoing manners of disposing the sensor assembly 20, the first elastic member 2, the spring seat 7, the needle retraction holder 4, the second elastic member 6, the support 3, the first magnet 9, and the housing cap assembly 8. Details are not described herein again.

As shown in FIG. 209 and FIG. 210, the concealing mechanism 50 may include an alloy sheet 5094, and the alloy sheet 5094 may be in a curved-surface shape. For example, the alloy sheet 5094 may be made of a memory alloy.

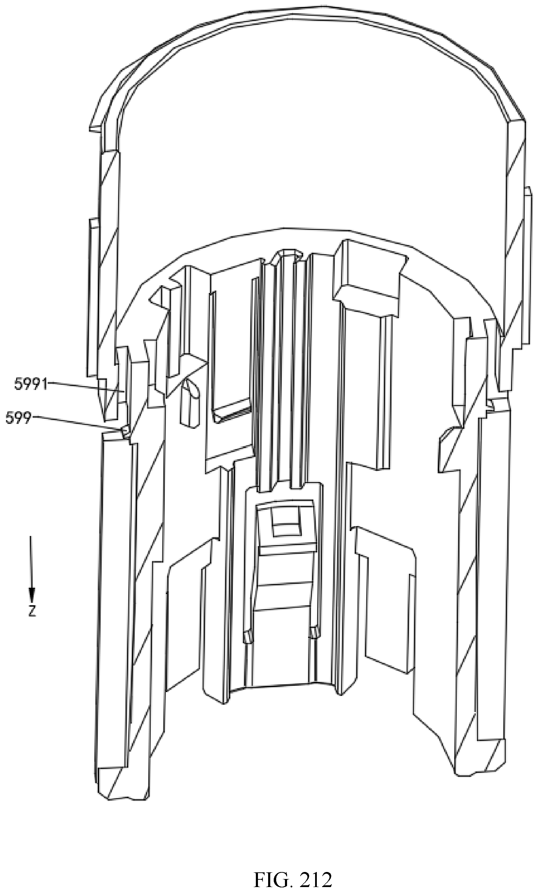

FIG. 211 is a diagram of a structure of the base 5 shown in FIG. 209 according to an implementation. FIG. 212 is a partial schematic cross-sectional view of the physiological sign monitoring apparatus 100 shown in FIG. 210 along B13-B13 according to an implementation.

As shown in FIG. 211 and FIG. 212, the base 5 may be provided with a positioning structure 599. The positioning structure 599 may be used for mounting the concealing mechanism 50. For example, the positioning structure 599 may be a bump, and the positioning structure 599 may be protrudingly disposed on an outer side surface of the base 5. The base 5 may be further provided with a second through hole 5991. The second through hole 5991 is in communication with an inner side and an outer side of the base 5. It can be understood that a specific position of the positioning structure 599 on the base 5 may be set according to a requirement.

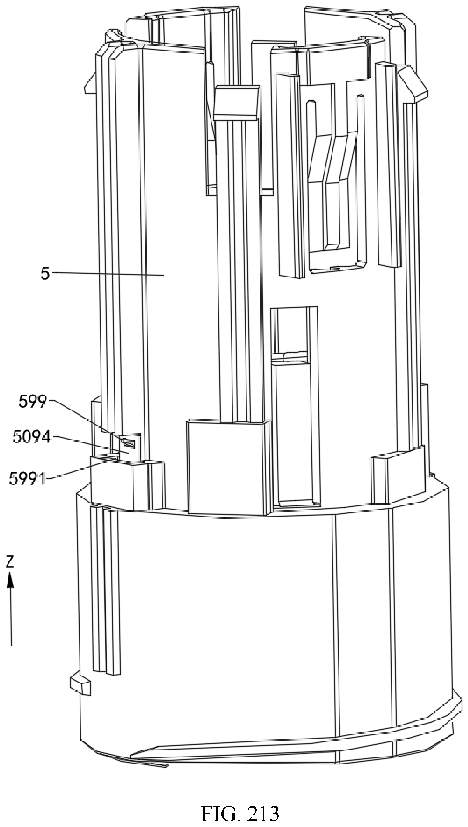

FIG. 213 is a diagram of a partial structure of the physiological sign monitoring apparatus 100 shown in FIG. 207 according to an implementation. FIG. 213 shows a manner of assembling the concealing structure and the base 5 according to an implementation.

As shown in FIG. 213, the concealing structure 50 may be fastened to the base 5. For example, the alloy sheet 5094 may be at least partially fastened to the inner side of the base 5.

In some implementations, the alloy sheet 5094 may be partially located in the second through hole 5991 and fastened to the positioning structure 599. It can be understood that the second through hole 5991 is provided, so that the alloy sheet 5094 can extend from the inner side of the base 5 to the outer side of the base 5, to be fastened to the positioning mechanism. In addition, the positioning structure 599 further facilitates positioning for the concealing mechanism 50 during mounting, so that the concealing mechanism 50 can smoothly conceal a puncture needle 23 in a closed state. For example, when the positioning structure 599 is a bump, a fastening hole/slot in a corresponding shape may be provided on the alloy sheet 5094, to fit with the positioning structure 599.

As shown in FIG. 208, when the physiological sign monitoring apparatus 100 is in an unused state, an end, away from the base 5, of the alloy sheet 5094 is located between the base 5 and the upper housing 81.

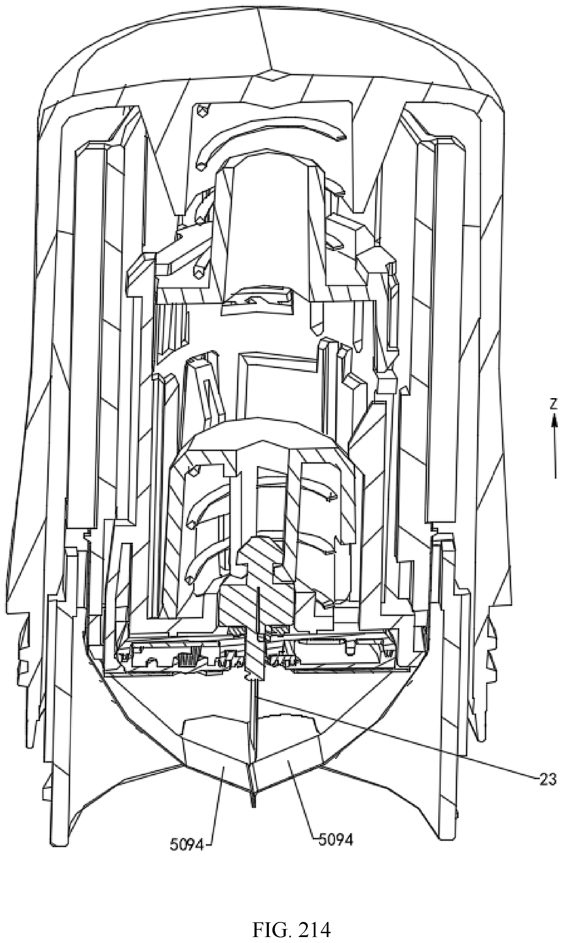

FIG. 214 is a partial cross-sectional view of the physiological sign monitoring apparatus 100 shown in FIG. 210 along B13-B13 according to an implementation.

As shown in FIG. 210 and FIG. 214, when the physiological sign monitoring apparatus 100 switches from the unused state to an in-use state, after the upper housing 81 is detached from the lower housing 1, ends, away from the base 5, of the alloy converge to conceal the puncture needle 23.

In some implementations, the end, away from the base 5, of the alloy sheet 5094 may be made of a magnetic material. In this way, during assembly of the physiological sign monitoring apparatus 100, ends, away from the base 5, of two alloy sheets 5094 may converge based on a magnetic attractive force, so that the concealing mechanism 50 is in the closed state.

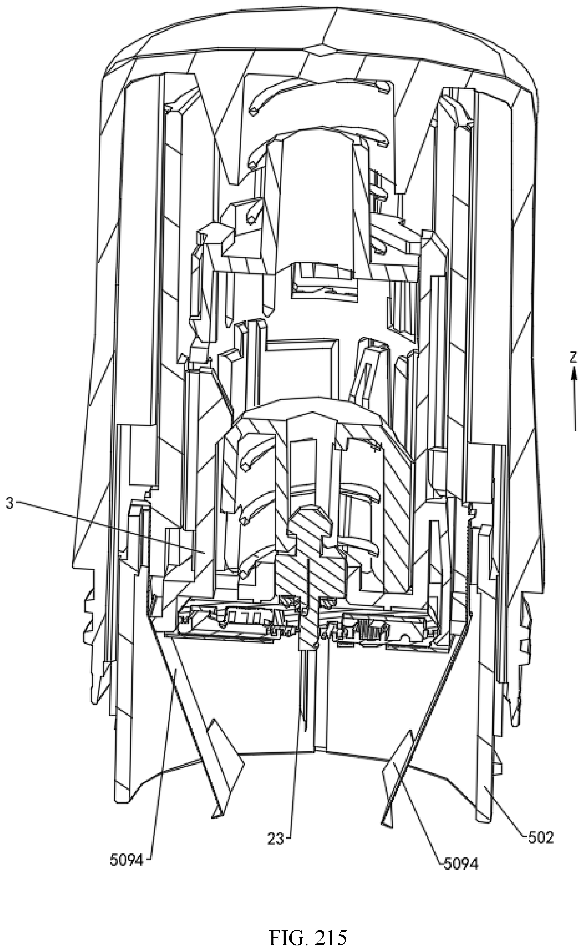

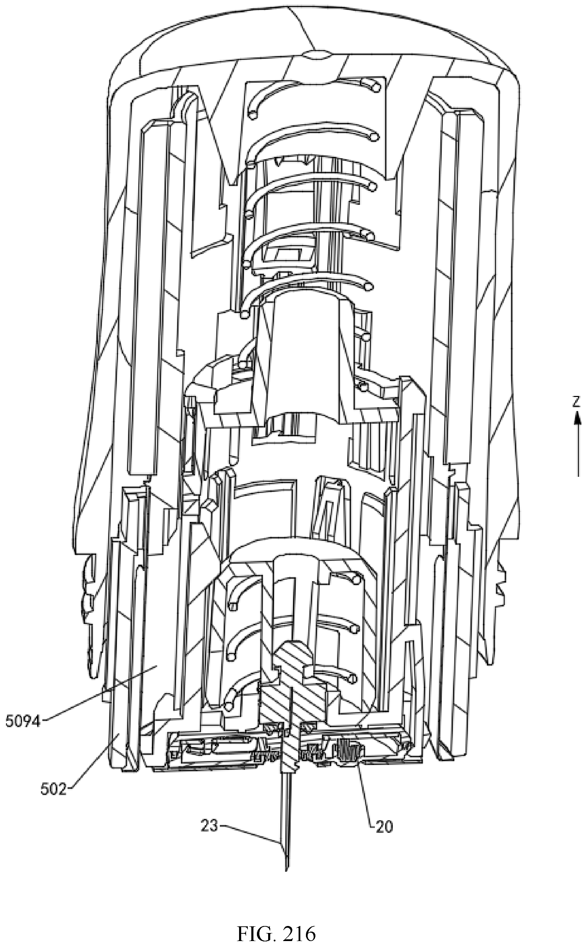

FIG. 215 is a partial cross-sectional view of the physiological sign monitoring apparatus 100 shown in FIG. 207 along A13-A13 in an in-use state according to an implementation. FIG. 216 is a partial cross-sectional view of the physiological sign monitoring apparatus 100 shown in FIG. 207 along A13-A13 in an in-use state according to an implementation.

As shown in FIG. 215 and FIG. 216, the base 5 includes an abutting end 502, and the abutting end 502 protrudes from the lower housing 1 through an opening 101. When the abutting end 502 abuts against the organism and the lower housing 1 moves relative to the base 5 in a direction toward the organism, the first elastic member 2 pushes the support 3 toward the organism, and the support 3 pushes the alloy sheets 5094 open, to enable the concealing mechanism 50 to be in an open state and the puncture needle 23 to be exposed. The first elastic member 2 continues to push the support 3, to deploy the sensor assembly 20, and the puncture needle 23 punctures into skin of the user.

It can be understood that, in comparison with the foregoing embodiments of the concealing mechanism 50, in this embodiment, the concealing mechanism 50 has two hinges 503, occupying small space. In addition, during implantation of the sensor assembly 20, the hinges 503 open while moving, without a need to first press a door plate to enable the hinges 503 to open and then trigger implantation. Therefore, a deployment stroke of pressing is small, and a length of the physiological sign monitoring apparatus 100 in the axial direction can be set to be small.

In some implementations, a part that is the same as that in the foregoing embodiments is not described again.

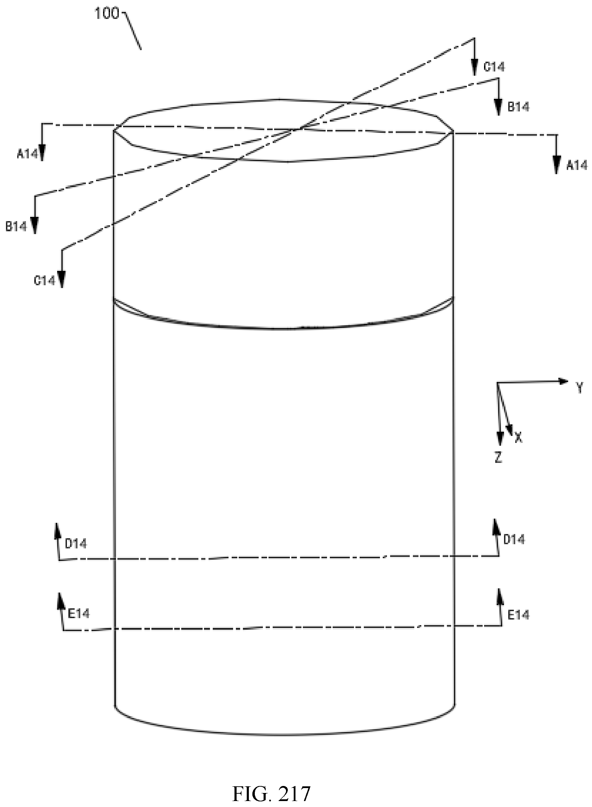

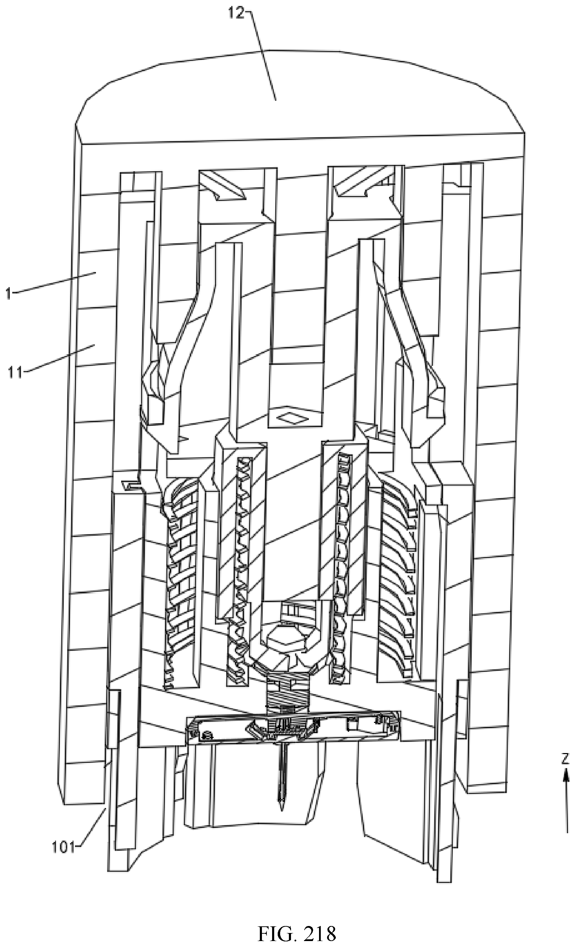

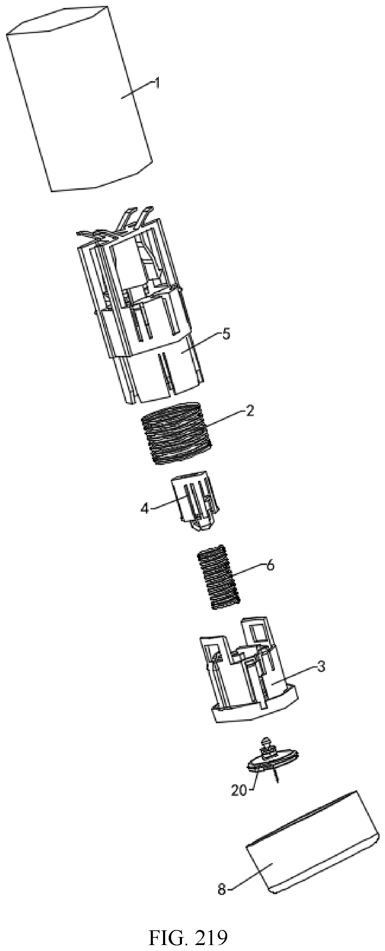

FIG. 217 is a diagram of a structure of the physiological sign monitoring apparatus 100 shown in FIG. 2 according to yet another implementation. FIG. 218 is a partial cross-sectional view of the physiological sign monitoring apparatus 100 shown in FIG. 217 along A14-A14 according to an implementation. FIG. 219 is a partial schematic exploded view of the physiological sign monitoring apparatus 100 shown in FIG. 217 according to an implementation.

As shown in FIG. 207 to FIG. 210, the physiological sign monitoring apparatus 100 may include an implantation apparatus 10 and a sensor assembly 20. The implantation apparatus 10 may include a lower housing 1, a first elastic member 2, a base 5, a needle retraction holder 4, a second elastic member 6, a support 3, a first magnet 9, and a housing cap assembly 8 (not shown in the figure). For a manner of disposing the sensor assembly 20, the first magnet 9, and the housing cap assembly 8, refer to the foregoing manners of disposing the sensor assembly 20, the first magnet 9, and the housing cap assembly 8. Details are not described herein again.

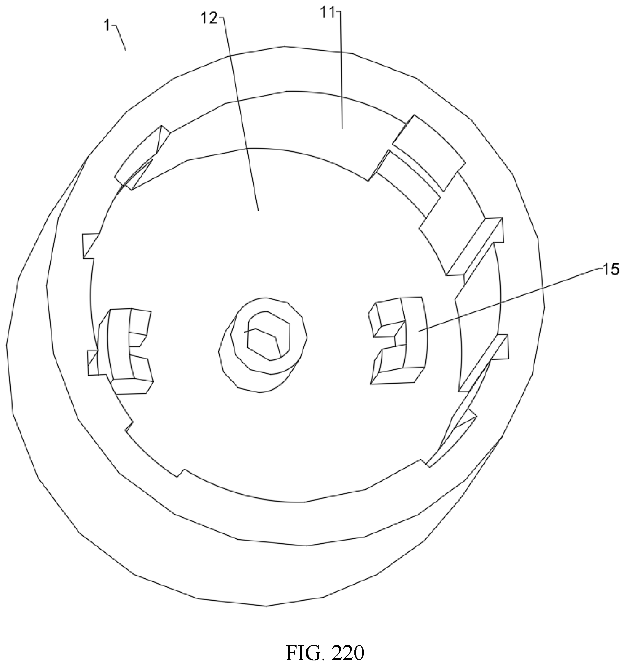

FIG. 220 is a diagram of a structure of the lower housing 1 shown in FIG. 219 according to an implementation.

As shown in FIG. 220, the lower housing 1 may include a top wall 12 and a side wall 11. The side wall 11 of the lower housing 1 is connected around a periphery of the top wall 12 of the lower housing 1. An opening 101 is formed on a side, away from the top wall 12 of the lower housing 1, of the side wall 11 of the lower housing 1.

In some implementations, the lower housing 1 may further include a toggle 15. The toggle 15 is fastened to the top wall 12. The toggle 15 may be located on an inner side of the side wall 111 of the lower housing 1. There may be one or more toggles 15. When there are a plurality of toggles 15, the plurality of toggles 15 may be spaced apart around a central axis of the lower housing 1. For example, in FIG. 220, there are two toggles 15.

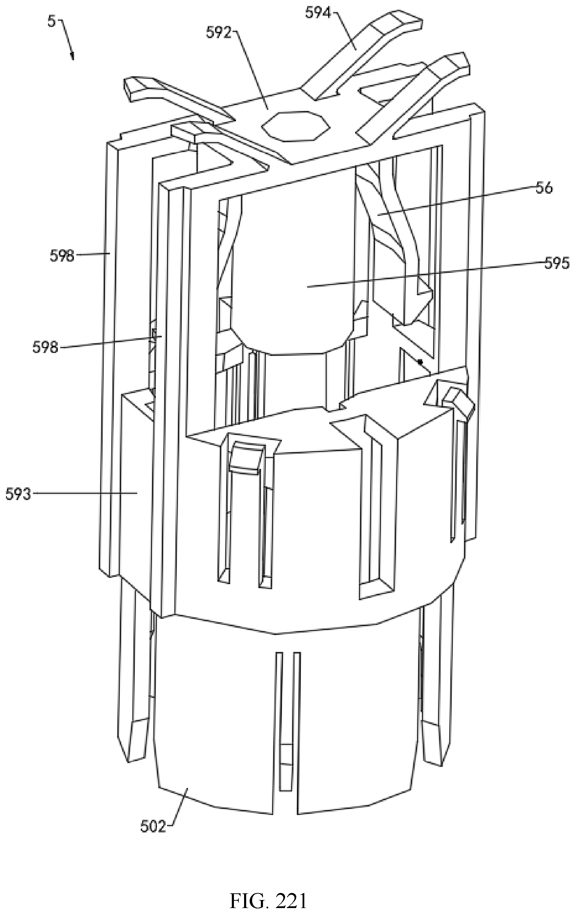

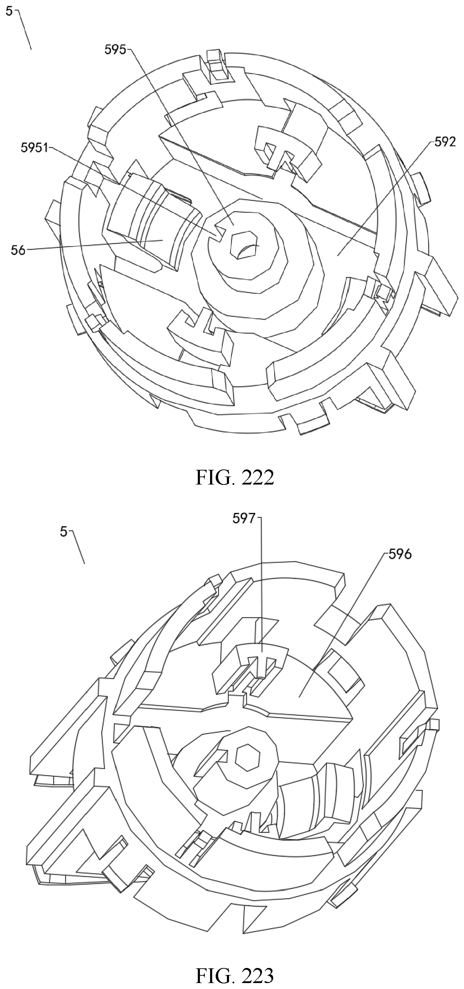

FIG. 221 is a diagram of a structure of the base 5 shown in FIG. 219 according to an implementation. FIG. 222 is a diagram of a structure of the base 5 shown in FIG. 221 from another perspective. FIG. 223 is a diagram of a structure of the base 5 shown in FIG. 221 from still another perspective.

As shown in FIG. 221, the base 5 may include a top plate 592, a side wall 593, and a rebound arm 594. The side wall 593 of the base 5 is connected around a periphery of the top plate 592. The rebound arm 594 is fastened to a side, away from the side wall 593 of the base 5, of the top plate 592. There may be one or more rebound arms 594. When there are a plurality of rebound arms 594, the plurality of rebound arms 594 are spaced apart around a central axis of the base 5. The rebound arm 594 is deformable. The base 5 has an abutting end 502. The abutting end 502 is an end, away from the top plate 592, of the base 5.

In some implementations, the base 5 may further include a positioning pillar 595, and the positioning pillar 595 may be fastened to a side, facing the side wall 593, of the top plate 592. The positioning pillar 595 may be spaced away from the side wall 593. For example, a side surface of the positioning pillar 595 may be provided with a first positioning structure 5951. For example, the first positioning structure 5951 may be a bump or a groove. For example, a central axis of the positioning pillar 595 may coincide with the central axis of the base 5.

In some implementations, the base 5 may further include a second resilient arm 56, and one end of the second resilient arm 56 may be fastened to the top plate 592 of the base 5. A tail end of the second resilient arm 56 is provided with a hook. The tail end of the second resilient arm 56 is an end, close to the abutting end 502, of the second resilient arm 56. The other end of the second resilient arm 56 is fastened to an upper side wall 942 of the base 5. The second resilient arm 56 is spaced away from the upper side wall 942 of the base 5 in a circumferential direction. The hook of the second resilient arm 56 faces an outer side of the base 5, and faces away from the abutting end 502. There may be one or more second resilient arms 56, and a plurality of second resilient arms 56 may be spaced apart around an axial direction of the base 5. For example, there are two second resilient arms 56, and the two second resilient arms 56 are arranged opposite to each other.

In some implementations, the base 5 may further include a middle plate 596 and a pressure rib 597. The middle plate 596 may be fastened to an inner side of the side wall 593 and spaced away from the top plate 592. One end of the pressure rib 597 may be fastened to the middle plate 596. The other end of the pressure rib 597 may be spaced away from the side wall 593 of the base 5. The pressure rib 597 may be located on the inner side of the side wall 593 of the base 5. The other end of the pressure rib 597 may be provided with a hook. The hook may face away from the side wall 593 of the base 5. For example, there may be one or more pressure ribs 597. When there are a plurality of pressure ribs 597, the plurality of pressure ribs 597 may be spaced apart around the central axis of the base 5.

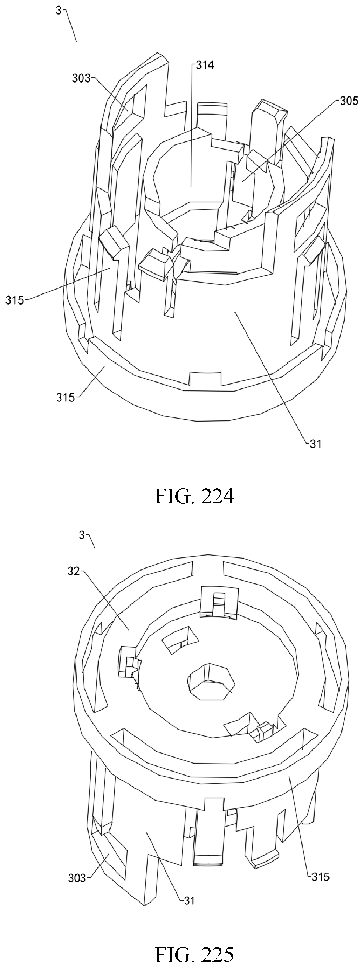

FIG. 224 is a diagram of a structure of the support 3 shown in FIG. 219 according to an implementation. FIG. 225 is a diagram of a structure of the support 3 shown in FIG. 224 from another perspective.

As shown in FIG. 224 and FIG. 225, the support 3 may include a side plate 31, a bottom plate 32, an outer side plate 315, and an inner side plate 314. All of the side plate 31, the outer side plate 315, and the inner side plate 314 may be fastened to a same side of the bottom plate 32. The side plate 31, the outer side plate 315, and the inner side plate 314 may be approximately in a tubular shape. The inner side plate 314 is located on an inner side of the side plate 31. The outer side plate 315 is arranged around the side plate 31.

In some implementations, the support 3 may be provided with first snap-fit space 303. The first snap-fit space 303 may be located at an end, away from the bottom plate 32 of the support 3, of the side plate 31 of the support 3. For example, the first snap-fit space 303 may be a snap-fit slot 8191. An opening 101 of the snap-fit slot 8191 faces an inner side of the support 3. In addition, the first snap-fit space 303 may alternatively be a snap-fit hole 312 that passes through the side plate 31 of the support 3.

In some implementations, the support 3 may be provided with third snap-fit space 305. The third snap-fit space 305 may be located on the inner side plate 314 of the support 3. For example, the third snap-fit space 305 may be a snap-fit slot. An opening of the snap-fit slot faces an inner side of the inner side plate 314 of the support 3. In addition, the third snap-fit space 305 may alternatively be a snap-fit hole that passes through the inner side plate 314 of the support 3.

In some implementations, the support 3 may further include a first abutting arm 315. One end of the first abutting arm 315 may be fastened to the bottom plate 32 of the support 3. The other end of the first abutting arm 315 is provided with a hook. The first abutting arm 315 may be spaced away from the outer side plate 315 of the support 3. The hook provided at the other end of the first abutting arm 315 may face an outer side of the support 3. There may be one or more first abutting arms 315. When there are a plurality of first abutting arms 315, the plurality of first abutting arms 315 are evenly spaced apart around a central axis of the support 3. For example, there are three first abutting arms 315, and the three first abutting arms 315 are evenly arranged at 120°.

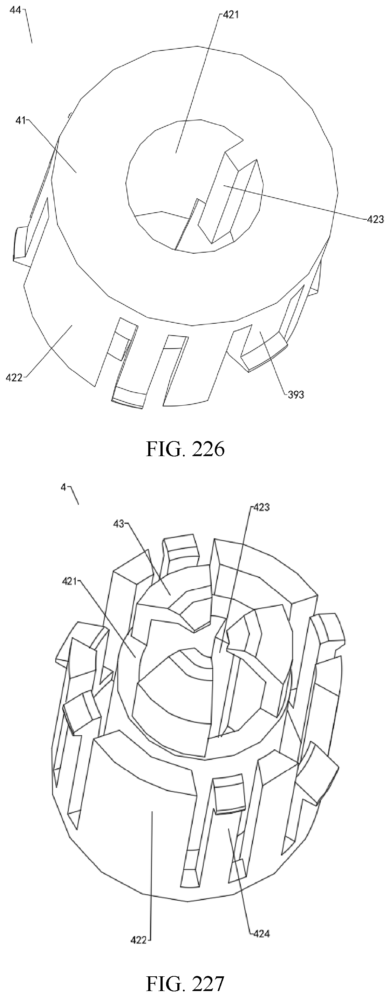

FIG. 226 is a diagram of a structure of the needle retraction holder 4 shown in FIG. 219 according to an implementation. FIG. 227 is a diagram of a structure of the needle retraction holder 4 shown in FIG. 226 from another perspective.

As shown in FIG. 226 and FIG. 227, the needle retraction holder 4 may include a top plate 41, a first side plate 421, and a second side plate 422. Both the first side plate 421 of the needle retraction holder 4 and the second side plate 422 of the needle retraction holder 4 may be arranged around the top plate 41 of the needle retraction holder 4. The second side plate 422 is arranged around the first side plate 421. The needle retraction holder 4 is approximately in a cylindrical shape.

In some implementations, the needle retraction holder 4 may further include a first cantilever 43. One end of the first cantilever 43 may be fastened to a side, away from the top plate 41 of the needle retraction holder 4, of the first side plate 421 of the needle retraction holder 4. The other end of the first cantilever 43 is provided with a hook. The other end of the first cantilever 43 is an end, away from the top plate 41 of the needle retraction holder 4, of the first cantilever 43. The hook of the first cantilever 43 faces the top plate 41 of the needle retraction holder 4. For example, there may be a plurality of first cantilevers 43, and the plurality of first cantilevers 43 are spaced apart around a central axis of the needle retraction holder 4. For example, there may be three first cantilevers 43.

In some implementations, the needle retraction holder 4 may further include 425. One end of the third snap-fit arm 425 may be fastened to the top plate 41 of the needle retraction holder 4. The other end of the third snap-fit arm 425 is provided with a hook. The hook faces an outer side of the needle retraction holder 4. For example, there may be a plurality of third snap-fit arms, and the plurality of third snap-fit arms may be spaced apart around the central axis of the needle retraction holder 4.

In some implementations, the needle retraction holder 4 may further include a second positioning structure 423. The second positioning structure 423 may be disposed on an inner side surface of the first side plate 421 of the needle retraction holder 4. The inner side surface of the first side plate 421 is a surface on a side, away from the second side plate 422, of the first side plate 421. For example, the second positioning structure 423 may be a bump or a groove.

In some implementations, the needle retraction holder 4 may further include a second abutting arm 424. One end of the second abutting arm 424 may be fastened to the top plate 41 of the needle retraction holder 4. The other end of the second abutting arm 424 is provided with a hook. The second abutting arm 424 may be spaced away from the second side plate 422 of the needle retraction holder 4. The hook provided at the other end of the second abutting arm 424 may face the outer side of the needle retraction holder 4. There may be one or more second abutting arms 424. When there are a plurality of second abutting arms 424, the plurality of second abutting arms 424 are evenly spaced around the central axis of the support 3. For example, there are four second abutting arms 424, and the four second abutting arms 424 are evenly arranged at 90°.

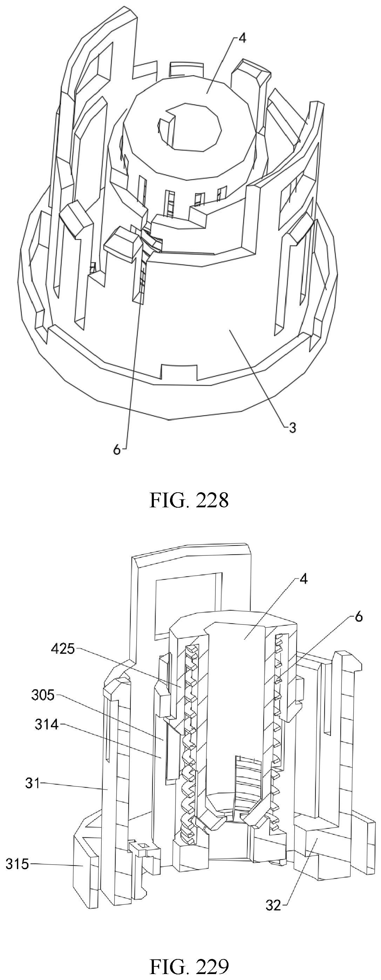

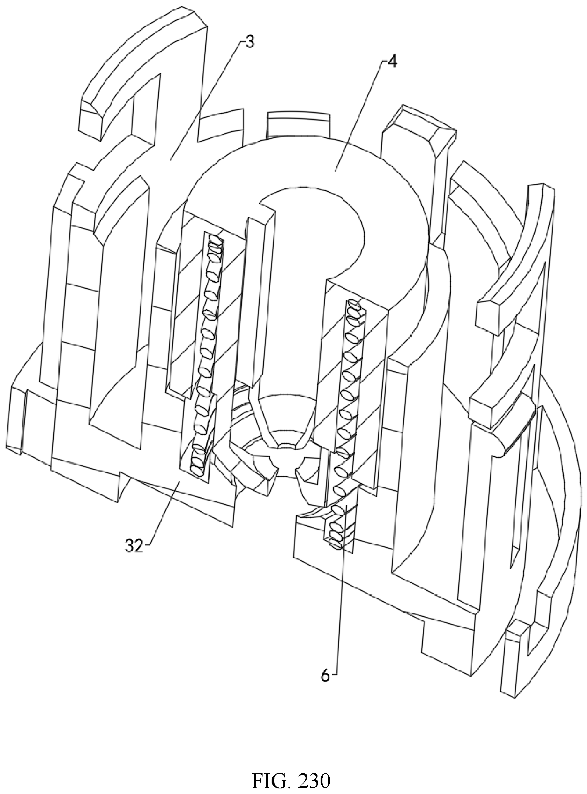

FIG. 228 is a diagram of assembly of the needle retraction holder 4, the support 3, and the second elastic member 6 shown in FIG. 219 according to an implementation. FIG. 229 is a partial cross-sectional view of the physiological sign monitoring apparatus 100 shown in FIG. 217 along C14-C14 according to an implementation. FIG. 230 is a partial cross-sectional view of the physiological sign monitoring apparatus 100 shown in FIG. 217 along B14-B14 according to an implementation.

As shown in FIG. 228 to FIG. 230, the needle retraction holder 4 may snap into the support 3, the lower housing 1, or the base 5. For example, the needle retraction holder 4 may be connected to the inner side plate 314 of the support 3. The third snap-fit arm 425 of the needle retraction holder 4 may snap into the third snap-fit space 305 of the support 3.

In some implementations, the second elastic member 6 is located between the support 3 and the needle retraction holder 4, or the second elastic member 6 is located on a side, close to the top wall 12 of the lower housing 1, of the needle retraction holder 4. The second elastic member 6 may be configured to apply, to the needle retraction holder 4, a force for moving from a side of the bottom plate 32 of the support 3. For example, the second elastic member 6 may be a compression spring. The second elastic member 6 may abut between the support 3 and the needle retraction holder 4.

It can be understood that the third snap-fit arm 425 snaps into the third snap-fit space 305, and may be configured to limit displacement of the needle retraction holder 4 in an axial direction, to prevent the needle retraction holder 4 from moving, under the action of the second elastic arm 2942, toward a side away from the bottom plate 32 of the support 3 when the support 3 has not deployed the sensor assembly 20.

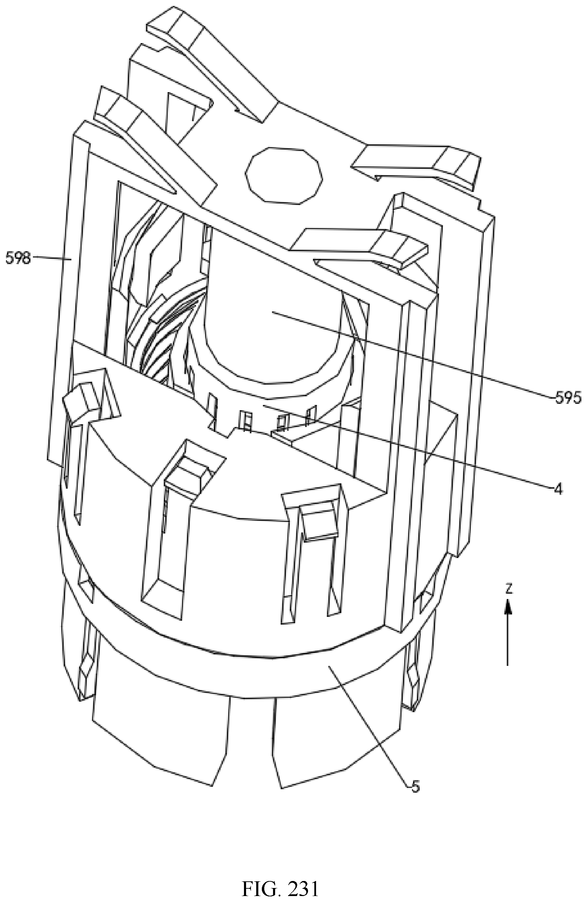

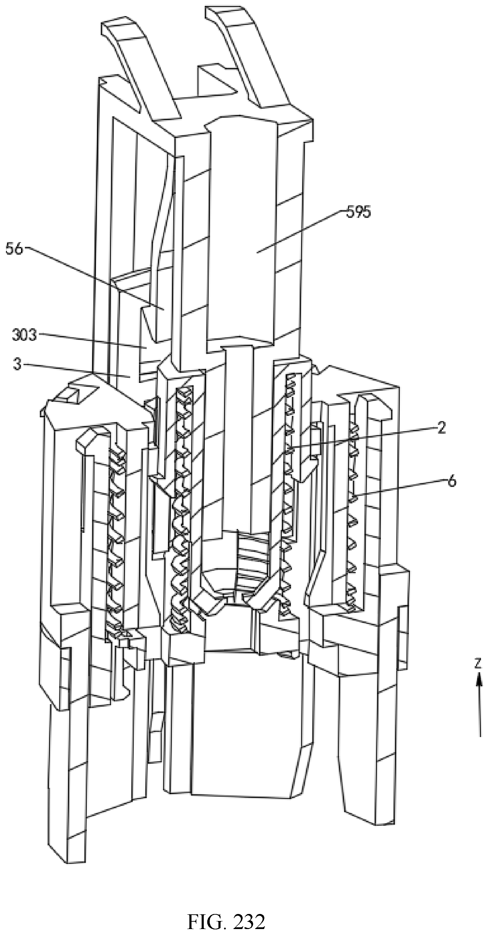

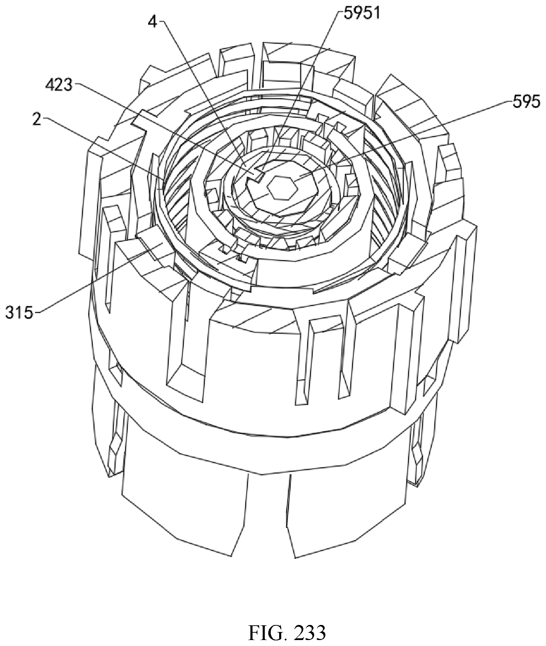

FIG. 231 is a diagram of assembly of the base 5, the first elastic member 2, the needle retraction holder 4, the support 3, and the second elastic member 6 shown in FIG. 219 according to an implementation. FIG. 232 is a partial cross-sectional view of the physiological sign monitoring apparatus 100 shown in FIG. 231 along C14-C14 according to an implementation. FIG. 233 is a partial cross-sectional view of the physiological sign monitoring apparatus 100 shown in FIG. 231 along D14-D14 according to an implementation.

As shown in FIG. 231 to FIG. 233, the support 3 may be mounted on an inner side of the base 5 and slidably connected to the base 5. For example, the second resilient arm 56 of the base 5 may snap into the first snap-fit space 303 of the support 3.

In some implementations, the positioning pillar 595 of the base 5 may be slidably connected to an inner side of the needle retraction holder 4. It can be understood that the first positioning structure 5951 may fit with the second positioning structure 423, to implement positioning for the base 5 and the needle retraction holder 4 in a circumferential direction during assembly. For example, the first positioning structure 5951 of the base 5 is a groove, and the second positioning structure 423 of the needle retraction holder 4 is a bump.

In some implementations, the first elastic member 2 may abut between the base 5 and the support 3. For example, the first elastic member 2 may be sleeved on an outer side of the second elastic member 6. It can be understood that, in comparison with the solution in which the first elastic member 2 and the second elastic member are arranged in the axial direction of the physiological sign monitoring apparatus 100 in the foregoing embodiments, in this embodiment, the first elastic member 2 and the second elastic member 6 are approximately located on one horizontal plane, and an overall position is near the abutting end 502 of the physiological sign monitoring apparatus 100. During use of the physiological sign monitoring apparatus 100, the first elastic member 2 and the second elastic member 6 are more stable with slight shaking. In addition, when the physiological sign monitoring apparatus 100 is started, the first elastic member 2 is not further compressed, a pressing force of the lower housing 1 is decoupled from a pushing force of the first elastic member 2, and the pressing force may be adjusted by using a size of an elastic arm of the base 5, to reduce the pressing force. For example, in the foregoing embodiments, a pressing force = a fastener spreading force + a compression force of the first elastic member 2; and in this embodiment, the pressing force = a fastener spreading force + a force of pressing the rebound arm 594.

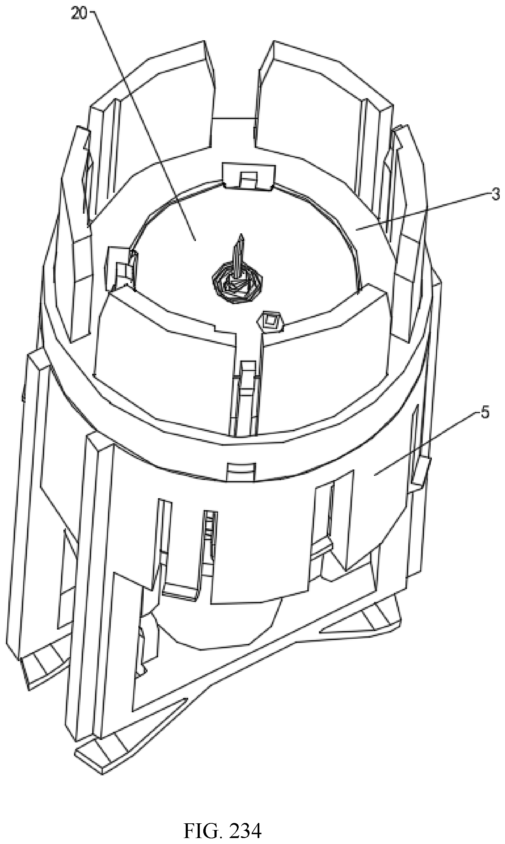

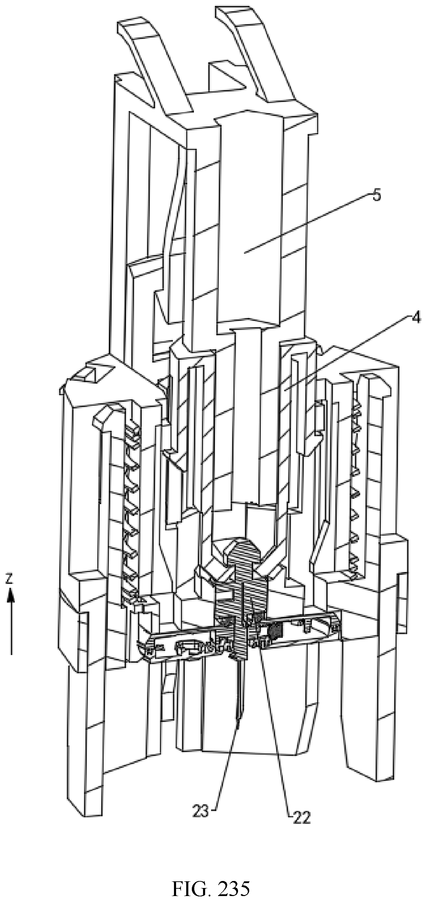

FIG. 234 is a diagram of assembly of the base 5, the first elastic member 2, the needle retraction holder 4, the support 3, the second elastic member 6, and the sensor assembly 20 shown in FIG. 219 according to an implementation. FIG. 235 is a partial cross-sectional view of the physiological sign monitoring apparatus shown in FIG. 217 along C14-C14 according to an implementation.

As shown in FIG. 234 and FIG. 235, the sensor assembly 20 may be fastened to the support 3. For example, the sensor assembly 20 may include a sensor 24, a puncture needle 23, and a needle holder 22. The puncture needle 23 is fastened to the needle holder 22. A puncture tip of the puncture needle 23 protrudes from the needle holder 22. The needle holder 22 snaps into the needle retraction holder 4.

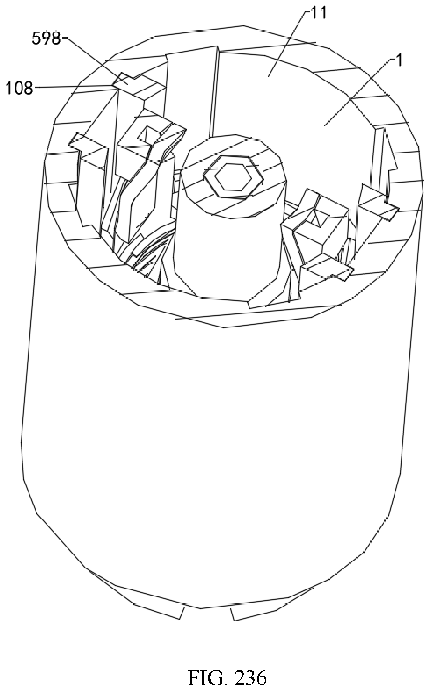

FIG. 236 is a partial cross-sectional view of the physiological sign monitoring apparatus 100 shown in FIG. 217 along E14-E14 according to an implementation.

As shown in FIG. 236, the base 5 may be mounted on an inner side of the lower housing 1 and slidably connected to the side wall 11 of the lower housing 1. For example, the side wall 11 of the lower housing 1 may be provided with a slide groove 108. An extension direction of the slide groove 108 is parallel to an axial direction of the lower housing 1. A guide bar 598 may be protrudingly disposed on an outer side surface of the base 5. The guide bar 598 is slidably connected to the slide groove 108. For example, there may be one or more slide grooves 108 and one or more guide bars 598. A quantity of slide grooves 108 is the same as a quantity of guide bars 598, and the slide grooves 108 and the guide bars 598 are arranged in a one-to-one correspondence. It can be understood that the slide groove 108 and the guide bar 598 are provided, and may be configured to limit a trend of the base 5 rotating relative to the lower housing 1 around the axial direction, so that the base 5 can slide in the axial direction of the lower housing 1.

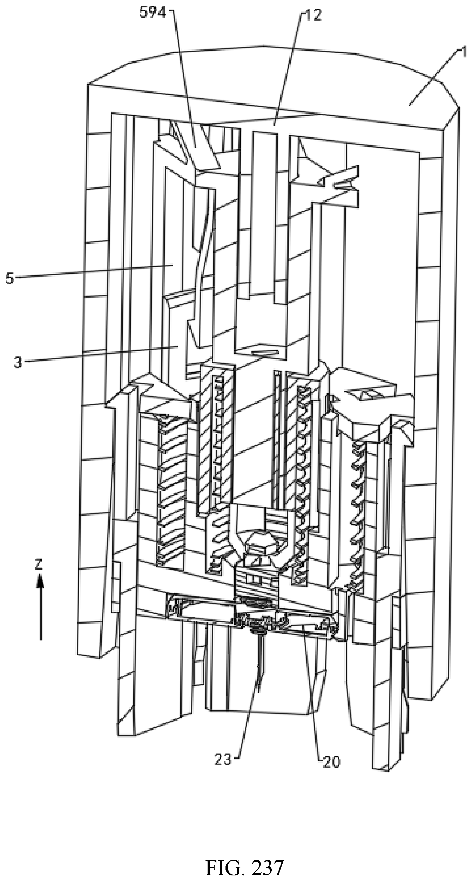

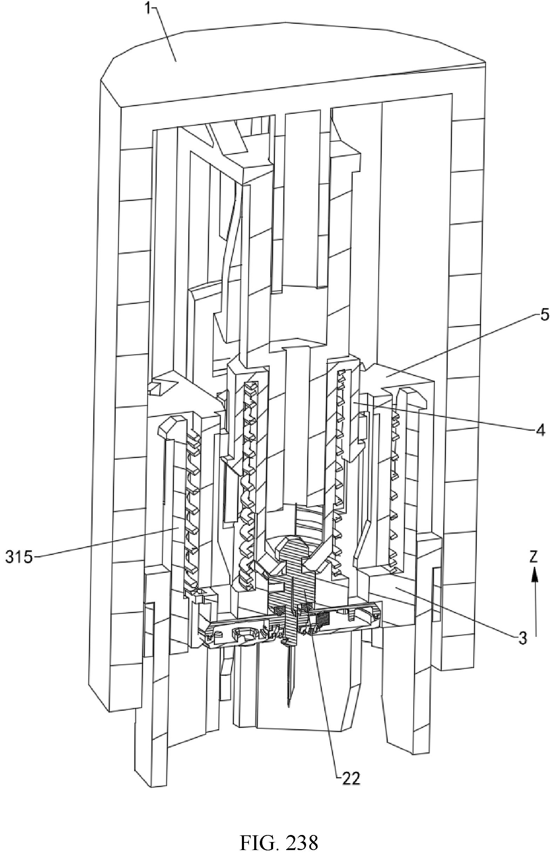

FIG. 237 is a partial cross-sectional view of the physiological sign monitoring apparatus 100 shown in FIG. 217 along B14-B14 according to an implementation. FIG. 238 is a partial cross-sectional view of the physiological sign monitoring apparatus 100 shown in FIG. 217 along C14-C14 according to an implementation.

As shown in FIG. 237 and FIG. 238, the rebound arm 594 of the base 5 may abut against the top wall 12 of the lower housing 1. The base 5 includes an abutting end 502, and the abutting end 502 protrudes from the lower housing 1 through the opening 101.

For example, the sensor assembly 20 may be located on a side, away from the top wall 12 of the lower housing 1, of the support 3.

As shown in FIG. 217 and FIG. 237, the upper housing 81 may be detachably fastened to an end, away from the top wall 12 of the housing, of the side wall 593 of the lower housing 1. The upper housing 81 covers the abutting end 502 and closes the opening 101.

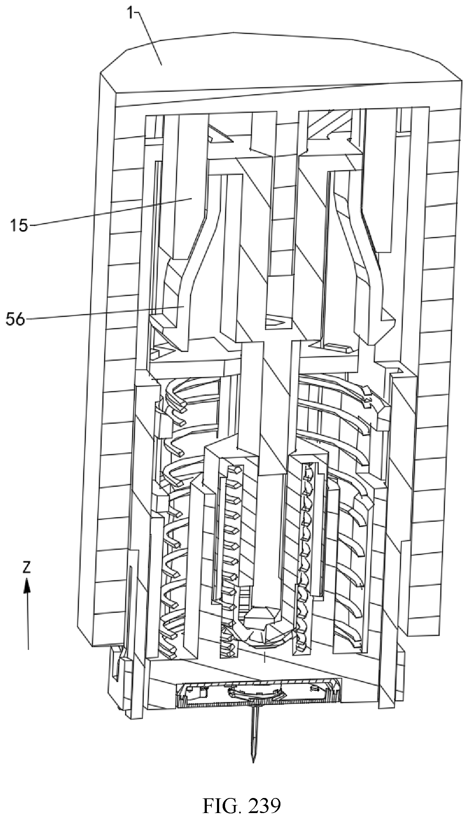

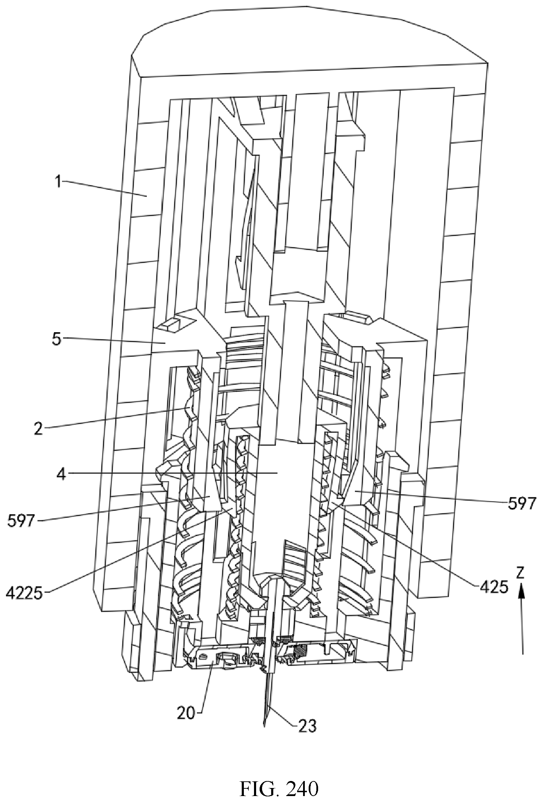

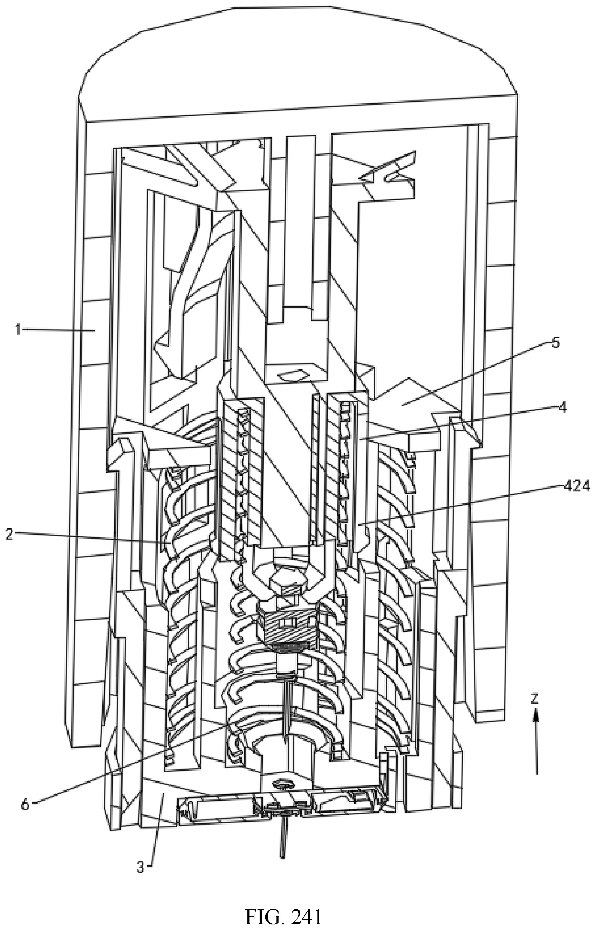

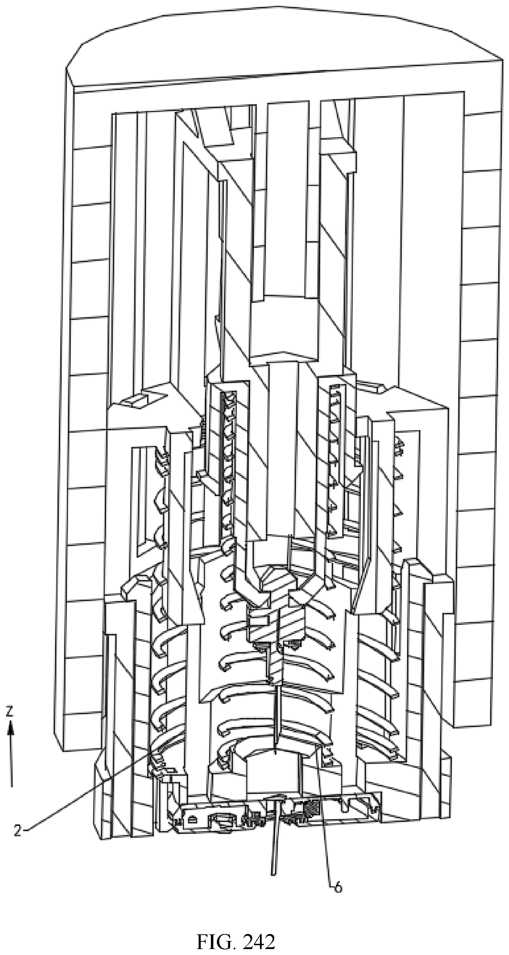

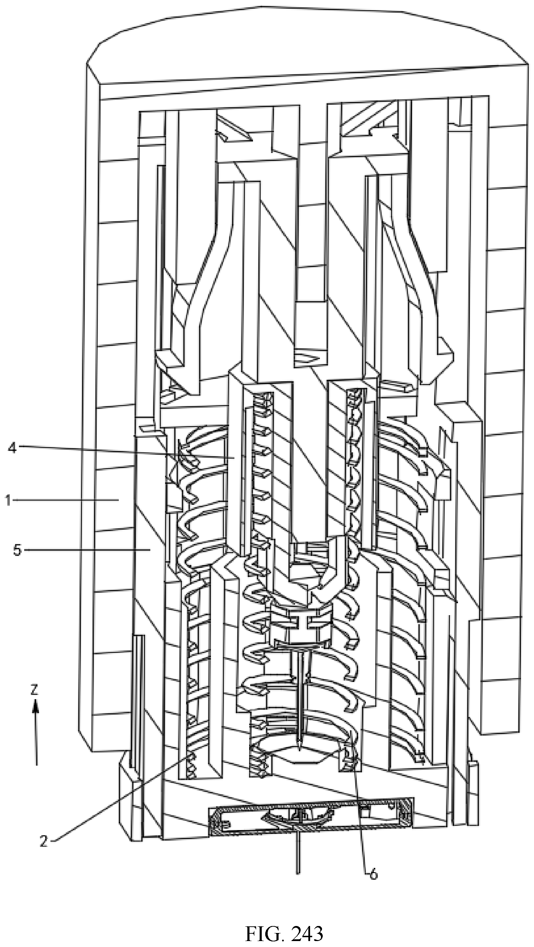

FIG. 239 is a partial cross-sectional view of the physiological sign monitoring apparatus 100 shown in FIG. 217 along A14-A14 in an in-use state according to an implementation. FIG. 240 is a partial cross-sectional view of the physiological sign monitoring apparatus 100 shown in FIG. 217 along C14-C14 in an in-use state according to an implementation. FIG. 241 is a partial cross-sectional view of the physiological sign monitoring apparatus 100 shown in FIG. 217 along B14-B14 in an in-use state according to an implementation. FIG. 242 is a partial cross-sectional view of the physiological sign monitoring apparatus 100 shown in FIG. 217 along C14-C14 in an in-use state according to an implementation. FIG. 243 is a partial cross-sectional view of the physiological sign monitoring apparatus 100 shown in FIG. 217 along A14-A14 in an in-use state according to an implementation.

As shown in FIG. 238 to FIG. 240, when the upper housing 81 is detached from the lower housing 1, the physiological sign monitoring apparatus 100 switches from an unused state to the in-use state, the abutting end 502 abuts against the organism, and the lower housing 1 moves relative to the base 5 in a direction toward the organism, the first elastic member 2 pushes the support 3 to the organism. The sensor assembly 20 abuts against the organism, and the sensor 24 is implanted into the organism under guidance of the puncture needle 23.

In some implementations, when the support 3 is being pushed to the organism, the first abutting arm 315 of the support 3 may remain in an elastically deformed state, and abut against the side wall 593 of the base 5. In this way, during movement of the support 3, elastic forces may be applied to the support 3 in a plurality of directions (three directions), so that an orientation of the support 3 relative to the base 5 is stable during pushing.

As shown in FIG. 240 to FIG. 243, when the support 3 drives the needle retraction holder 4 to move toward the organism to a specific position, the pressure rib 597 of the base 5 may abut against the third snap-fit arm 425 of the needle retraction holder 4, and position limiting between the needle retraction holder 4 and the support 3 is released. In this way, under the action of the second elastic member 6, the needle retraction holder 4 can move away from the organism. Then the needle retraction holder 4 and the puncture needle 23 move toward a side away from the organism, and the puncture needle 23 is detached from the organism.

As shown in FIG. 237 and FIG. 241, before the needle retraction holder 4 is detached from the support 3, the second abutting arm 424 of the needle retraction holder 4 remains in an elastically deformed state. For example, the second abutting arm 424 of the needle retraction holder 4 may abut against the inner side of the inner side plate 314 of the support 3. It can be understood that the second abutting arm 424 abuts against the support 3, so that elastic forces may be applied to the needle retraction holder 4 from all directions when the needle retraction holder 4 slides on the inner side plate 314 of the support 3. In this way, during needle retraction, an orientation of the needle retraction holder 4 sliding relative to the support 3 is stable, to reduce a risk of shaking of the needle retraction holder 4.

It can be understood that embodiments of this application or features in embodiments may be combined with each other if there is no conflict, and any combination of features in different embodiments also falls within the protection scope of this application. That is, the plurality of embodiments described above may alternatively be combined in any manner according to an actual requirement.

It can be understood that all of the foregoing accompanying drawings are example drawings of this application, and do not represent actual sizes of products. In addition, a size proportional relationship between components in the accompanying drawings is not intended to limit an actual product in this application.

The foregoing descriptions are merely specific implementations of this application, but are not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

## Claims

1. A physiological sign monitoring apparatus, comprising:
a lower housing, comprising a top wall and a side wall, wherein the side wall of the lower housing is connected around a periphery of the top wall of the lower housing, and an opening is formed on a side, away from the top wall of the lower housing, of the side wall of the lower housing;
a base, mounted on an inner side of the lower housing and slidably connected to the side wall of the lower housing, wherein the base comprises an abutting end, and the abutting end protrudes from the lower housing through the opening;
a support, mounted on an inner side of the base and slidably connected to the base;
a first elastic member, connected between the top wall of the lower housing and the support;
a sensor assembly, fastened to the support and located on a side, away from the top wall of the lower housing, of the support, wherein the sensor assembly comprises a sensor and a puncture needle;
an upper housing, detachably fastened to an end, away from the top wall of the housing, of the side wall of the lower housing, wherein the upper housing covers the opening; and
a stopper, configured to: when the physiological sign monitoring apparatus is in an unused state, prevent the base from moving relative to the lower housing in an axial direction of the physiological sign monitoring apparatus, wherein
when the upper housing is detached from the lower housing, the abutting end abuts against an organism, and the lower housing moves relative to the base in a direction toward the organism, the first elastic member pushes the support toward the organism, the sensor assembly abuts against the organism, and the sensor is implanted into the organism under guidance of the puncture needle.

2. The physiological sign monitoring apparatus according to claim 1, wherein the stopper comprises a first stop structure and a second stop structure, the first stop structure is fastened to an outer side of the base, the second stop structure is fastened to the upper housing, and in the axial direction of the physiological sign monitoring apparatus, the first stop structure and the second stop structure are arranged opposite to each other, and the second stop structure is located on a side, close to the top wall of the lower housing, of the first stop structure.

3. The physiological sign monitoring apparatus according to claim 2, wherein both the first stop structure and the second stop structure are threaded, and a thread pitch of the first stop structure is the same as a thread pitch of the second stop structure.

4. The physiological sign monitoring apparatus according to claim 3, wherein engaging threads are provided on an outer side of the side wall of the lower housing, and the upper housing is detachably fastened to the lower housing through the engaging threads; and
the thread pitch of the first stop structure is the same as a thread pitch of the engaging threads.

5. The physiological sign monitoring apparatus according to claim 1, wherein the lower housing further comprises a first limiting block, the first limiting block is fastened to an inner side of the side wall of the lower housing and is exposed from the opening, the base further comprises a second limiting block, the second limiting block is fastened to an outer side of the abutting end, and in the axial direction of the physiological sign monitoring apparatus, the first limiting block and the second limiting block are arranged opposite to each other;
the stopper is detachably connected to the base, and abuts between the first limiting block and the second limiting block; and
when the physiological sign monitoring apparatus switches from the unused state to an in-use state, the upper housing is detached from the lower housing, the stopper is detached from the base, and the abutting end abuts against the organism.

6. The physiological sign monitoring apparatus according to claim 5, wherein the stopper is of a semi-ring structure, and a central angle of the semi-ring structure is greater than 180°.

7. The physiological sign monitoring apparatus according to claim 1, wherein the abutting end is provided with a limiting groove, and an opening of the limiting groove is located on an outer side surface of the abutting end;
the upper housing comprises a top wall and a side wall, the side wall of the upper housing is connected around a periphery of the top wall of the upper housing, and the stopper is fastened to an inner side of the side wall of the upper housing; and
when the upper housing is mounted to the lower housing, the stopper is partially located in the limiting groove.

8. The physiological sign monitoring apparatus according to claim 7, wherein the stopper is threaded, the limiting groove is a threaded groove, and a thread pitch of the limiting groove is the same as a thread pitch of the stopper.

9. The physiological sign monitoring apparatus according to claim 7 or 8, wherein there are a plurality of stoppers, the plurality of stoppers are spaced apart, and the plurality of stoppers are evenly arranged around the axial direction of the physiological sign monitoring apparatus.

10. The physiological sign monitoring apparatus according to any one of claims 1 to 9, wherein the base further comprises a plurality of seventh resilient arms, the plurality of seventh resilient arms are spaced apart around an axial direction of the base, a tail end of the seventh resilient arm is provided with a hook, and the hook faces the inner side of the base; and
when the support moves in a direction toward the organism and the sensor assembly abuts against the organism, the hook of the seventh resilient arm snaps into the support.

11. The physiological sign monitoring apparatus according to claim 10, wherein the support further comprises a fastening boss; the fastening boss is protrudingly disposed on an outer side surface of the support; when the physiological sign monitoring apparatus is in the unused state, the hook of the seventh resilient arm and the fastening boss are spaced apart; and when the support moves in the direction toward the organism and the sensor assembly abuts against the organism, the hook of the seventh resilient arm snaps into the fastening boss.

12. The physiological sign monitoring apparatus according to claim 11, wherein when the hook of the seventh resilient arm faces away from the abutting end and the sensor assembly abuts against the organism, the hook of the seventh resilient arm snaps into a side, close to the abutting end, of the fastening boss; or
when the hook of the seventh resilient arm faces the abutting end and the sensor assembly abuts against the organism, the hook of the seventh resilient arm snaps into a side, away from the abutting end, of the fastening boss.

13. The physiological sign monitoring apparatus according to any one of claims 1 to 12, wherein the base further comprises a plurality of elastic latches, the elastic latches are spaced apart around the axial direction of the base, an end, away from the top wall of the lower housing, of the elastic latch is spaced away from the abutting end, and the elastic latch protrudes from the inner side of the base; and
when the support moves in the direction toward the organism and the sensor assembly abuts against the organism, the elastic latch abuts against a side, close to the top wall of the lower housing, of the support.

14. The physiological sign monitoring apparatus according to claim 13, wherein the support further comprises a flange, and the flange is protrudingly disposed on the outer side surface of the support; and
when the support moves in the direction toward the organism and the sensor assembly abuts against the organism, the elastic latch abuts against a side, close to the top wall of the lower housing, of the flange.

15. The physiological sign monitoring apparatus according to any one of claims 1 to 14, wherein the sensor comprises a switch magnet, and the switch magnet is configured to control energization/de-energization of the sensor;
the physiological sign monitoring apparatus further comprises a first magnet, the first magnet is fastened to the support and is located between the support and the sensor assembly, and in the axial direction of the physiological sign monitoring apparatus, the first magnet and the switch magnet are arranged opposite to each other; and
when the physiological sign monitoring apparatus is in the unused state, the first magnet is configured to de-energize the sensor.

16. The physiological sign monitoring apparatus according to claim 15, wherein the physiological sign monitoring apparatus further comprises a second magnet, the second magnet is fastened to the upper housing, and in the axial direction of the physiological sign monitoring apparatus, the second magnet and the switch magnet are arranged opposite to each other; and
when the physiological sign monitoring apparatus is in the unused state, the second magnet is configured to de-energize the sensor.

17. The physiological sign monitoring apparatus according to claim 15 or 16, wherein the sensor assembly may further comprise a first electrode plate and a second electrode plate, the first electrode plate and the second electrode plate are electrically connected to a positive electrode and a negative electrode respectively, and in the axial direction of the physiological sign monitoring apparatus, the switch magnet is located between the first electrode plate and the second electrode plate; and
the switch magnet is fastened to the first electrode plate; the first electrode plate is deformable; and when the physiological sign monitoring apparatus is in the unused state, the switch magnet and the second electrode plate are spaced apart, and the sensor is de-energized; or when the physiological sign monitoring apparatus is in the in-use state, the switch magnet is in contact with and electrically connected to the second electrode plate, and the sensor is energized.

18. The physiological sign monitoring apparatus according to claim 17, wherein the first electrode plate comprises an ultra-thin steel sheet.

19. The physiological sign monitoring apparatus according to any one of claims 1 to 18, wherein the sensor assembly further comprises a needle holder, the puncture needle is fastened to the needle holder, a puncture tip of the puncture protrudes from the needle holder, the physiological sign monitoring apparatus further comprises a needle retraction holder, the needle retraction holder snaps into the support, the lower housing, or the base, and the needle holder snaps into the needle retraction holder; and
when the support moves in a direction toward the organism, the support pushes the needle holder toward the organism through the needle retraction holder, and the puncture tip of the puncture needle penetrates into the organism.

20. The physiological sign monitoring apparatus according to claim 19, wherein the physiological sign monitoring apparatus further comprises a second elastic member, and the second elastic member is located between the support and the needle retraction holder, or the second elastic member is located on a side, close to the top wall of the lower housing, of the needle retraction holder; and
after the sensor punctures into and is implanted into the organism, the second elastic member is configured to enable the needle retraction holder to move away from the organism, the needle retraction holder and the puncture needle move toward a side away from the organism, and the puncture needle is detached from the organism.

21. The physiological sign monitoring apparatus according to claim 20, wherein the second elastic member abuts between the support and the needle retraction holder, and the second elastic member is in a compressed state.

22. The physiological sign monitoring apparatus according to claim 20, wherein the second elastic member is located on the side, close to the top wall of the lower housing, of the needle retraction holder, one end of the second elastic member is connected to the needle retraction holder, the other end of the second elastic member is connected to the lower housing, and the second elastic member is in a stretched state.

23. The physiological sign monitoring apparatus according to any one of claims 20 to 22, wherein the second elastic member is located between the support and the needle retraction holder, and the second elastic member is a spring; or
the second elastic member is located on the side, close to the top wall of the lower housing, of the needle retraction holder, and the second elastic member is a needle retraction mainspring or a tension spring.

24. The physiological sign monitoring apparatus according to any one of claims 20 to 23, wherein the support comprises a side plate and a bottom plate, the side plate of the support is in a tubular shape and is fastened to a side, close to the top wall of the lower housing, of the bottom plate of the support, the needle retraction holder is located on an inner side of the side plate of the support, and the sensor assembly is located on a side, away from the top wall of the lower housing, of the bottom plate of the support;
the base comprises a pre-retraction pressure rib and a retraction pressure rib, and both the pre-retraction pressure rib and the retraction pressure rib are protrudingly disposed on an inner side surface of the base;
a snap-fit hole is provided on the side plate of the support, the snap-fit hole passes through the side plate of the support, a wall surface of the snap-fit hole comprises a first stepped surface and a second stepped surface, a distance between the first stepped surface and the base is less than a distance between the second stepped surface and the base, and a distance between the first stepped surface and the bottom plate of the support is less than a distance between the second stepped surface and the bottom plate of the support;
the needle retraction holder comprises an elastic fastener; when the physiological sign monitoring apparatus is in the unused state, one end of the elastic fastener abuts against the first stepped surface, and a middle part of the elastic fastener protrudes from an outer side of the side plate of the support through the snap-fit hole; and in the axial direction of the physiological sign monitoring apparatus, the middle part of the elastic fastener is arranged opposite to the pre-retraction pressure rib and is arranged opposite to the retraction pressure rib; and
in a process in which the first elastic member pushes the support toward the organism until the sensor assembly abuts against the organism, when the support moves to a first position, the middle part of the elastic fastener abuts against the pre-retraction pressure rib, and one end of the elastic fastener abuts against the second stepped surface; when the support moves to a second position, the middle part of the elastic fastener abuts against the retraction pressure rib, and the elastic fastener is fully located on an inner side of the side plate of the support; when the support moves to a third position, the sensor assembly abuts against the organism; and after the sensor punctures into and is implanted into the organism, the second elastic member is configured to enable the needle retraction holder to move away from the organism, the needle retraction holder and the puncture needle move toward a side away from the organism, and the puncture needle is detached from the organism.

25. The physiological sign monitoring apparatus according to any one of claims 19 to 24, wherein the sensor assembly further comprises a sealed cartridge, the sealed cartridge is detachably fastened to the needle holder, the sealed cartridge has a hollow cavity, and a part of the sensor and the puncture tip of the puncture needle are located in the hollow cavity of the sealed cartridge;
the sealed cartridge comprises a plurality of first elastic arms, the first elastic arms are protrudingly disposed on an outer side surface of the sealed cartridge, the upper housing further comprises an unlock ratchet wheel, and the unlock ratchet wheel fits with the plurality of first elastic arms; and
in a process in which the upper housing is tightened relative to the lower housing, the first elastic arm is slidably connected to the unlock ratchet wheel; and in a process in which the upper housing is detached from the lower housing, the first elastic arm snaps into the unlock ratchet wheel, and pushes the sealed cartridge to detach from the needle holder.

26. The physiological sign monitoring apparatus according to claim 25, wherein the sealed cartridge further comprises a plurality of second elastic arms, the second elastic arms are protrudingly disposed on the outer side surface of the sealed cartridge, and the ratchet wheel fits with the plurality of second elastic arms; and
the plurality of first elastic arms form a first elastic arm group, the plurality of second elastic arms form a second elastic arm group, and the first elastic arm group and the second elastic arm group are disposed in a staggered manner in the axial direction of the physiological sign monitoring apparatus.

27. The physiological sign monitoring apparatus according to any one of claims 1 to 6, wherein the sensor assembly further comprises a needle holder, the puncture needle is fastened to the needle holder, and a puncture tip of the puncture protrudes from the needle holder;
the sensor assembly further comprises a sealed cartridge, the sealed cartridge is detachably fastened to the needle holder, the sealed cartridge has a hollow cavity, and a part of the sensor and the puncture tip of the puncture needle are located in the hollow cavity of the sealed cartridge;
the upper housing comprises a bottom wall, a side wall, a clamping arm, and a snap-fit boss; the side wall of the upper housing is connected around a periphery of the bottom wall of the upper housing; an end, away from the bottom wall of the upper housing, of the side wall of the upper housing is detachably fastened to the lower housing; the snap-fit boss is fastened to a side, facing the top wall of the lower housing, of the bottom wall of the upper housing; the snap-fit boss is provided with a snap-fit slot; an opening of the snap-fit slot faces the top wall of the lower housing; and when the physiological sign monitoring apparatus is in the unused state, an end, away from the needle holder, of the sealed cartridge abuts in the snap-fit slot;
a mounting hole is provided on the bottom wall of the upper housing, the mounting hole passes through the bottom wall, one end of the clamping arm is detachably connected to the bottom wall of the upper housing, and the other end of the clamping arm extends, through the mounting hole, into a side, facing the top wall of the lower housing, of the bottom wall of the upper housing, and is detachably connected to the sealed cartridge; and
in a process in which the upper housing is detached from the lower housing, the clamping arm clamps the sealed cartridge, and pushes the sealed cartridge to detach from the needle holder.

28. The physiological sign monitoring apparatus according to claim 27, wherein the sealed cartridge has a limiting groove, and an opening of the limiting groove is located on a side wall of the sealed cartridge; and
an end, away from a top plate of the upper housing, of the clamping arm is provided with a hook, the hook of the clamping arm faces the sealing member, the hook of the clamping arm is at least partially located in the limiting groove, a groove wall of the limiting groove comprises a first limiting surface and a second limiting surface, the first limiting surface and the second limiting surface are spaced apart in a circumferential direction of the sealed cartridge, and the hook of the clamping arm is located between the first limiting surface and the second limiting surface.

29. The physiological sign monitoring apparatus according to claim 28, wherein a groove surface of the limiting groove further comprises a third limiting surface, the third limiting surface is connected to the first limiting surface and the second limiting surface, and in a direction parallel to an axial direction of the sealed cartridge, the third limiting surface is arranged opposite to the hook of the clamping arm.

30. The physiological sign monitoring apparatus according to claim 28 or 29, wherein there are a plurality of limiting grooves, the plurality of limiting grooves are spaced apart in the circumferential direction of the sealed cartridge, there are a plurality of clamping arms, and the plurality of clamping arms and the plurality of limiting grooves are arranged in a one-to-one correspondence.

31. The physiological sign monitoring apparatus according to any one of claims 27 to 30, wherein an outer diameter of the snap-fit boss is greater than an outer diameter of the sealed cartridge.

32. A physiological sign monitoring apparatus, comprising:
a housing, comprising a middle plate, an upper side wall, and a lower side wall, wherein the middle plate comprises a first surface and a second surface that face away from each other, the upper side wall is fastened to the first surface, the lower side wall is fastened to the second surface, both the upper side wall and the lower side wall are connected around a periphery of the middle plate, the upper side wall and the middle plate enclose first space, the lower side wall and the middle plate enclose second space, the middle plate has a first snap-fit hole 9411, and the first snap-fit hole 9411 passes through the middle plate and is communication with the first space and the second space;
a support, wherein the support comprises a body portion, two first snap-fit arms, and at least one second snap-fit arm; the body portion is located on an inner side of the lower side wall and is slidably connected to the lower side wall; both the first snap-fit arm and the second snap-fit arm are fastened to an end, close to the middle plate, of the body portion; the two first snap-fit arms and the second snap-fit arm are connected around a periphery of the body portion at spacings; an end, away from the body portion, of the first snap-fit arm has a first hook; an end, away from the body portion, of the second snap-fit arm has a second hook; both the first snap-fit arm and the second snap-fit arm are partially located in the first snap-fit hole 9411; the first hook is located in the first space and snaps into the middle plate; and the second hook is located in the first space and snaps into the middle plate;
a first elastic member, abutting between the middle plate and the support;
a sensor assembly, fastened to the body portion and located on a side, away from the middle plate of the housing, of the body portion, wherein the sensor assembly is located in the second space, and the sensor assembly comprises a sensor and a puncture needle;
a button module, comprising two buttons, two first push blocks, and at least one second push block, wherein both the first push block and the second push block are located in the first space, the upper side wall has two mounting holes, the mounting hole passes through the upper side wall and is in communication with the first space and the outside, the button is mounted in the mounting hole, one end of the button is exposed to the outside, the other end of the button abuts against the first push block, two ends of the second push block respectively abut against the two first push blocks, the first snap-fit arm and the first push block are arranged opposite to each other in a first direction, the second snap-fit arm and the second push block are arranged opposite to each other in a second direction, and the first direction and the second direction form an included angle; and
a front sealing cover, detachably fastened to an end, away from the middle plate, of the lower side wall, wherein
when the front sealing cover is detached from the lower side wall, the end, away from the middle plate, of the lower side wall abuts against an organism, both of the two buttons are subject to pressure toward a central axis of the physiological sign monitoring apparatus and push the two first push blocks to the central axis of the physiological sign monitoring apparatus, the two first push blocks push the second push block to the central axis of the physiological sign monitoring apparatus, the first hook and the second hook enter the second space from the first space, the first elastic member pushes the support toward the organism, the sensor assembly abuts against the organism, and the sensor is implanted into the organism under guidance of the puncture needle.

33. The physiological sign monitoring apparatus according to claim 32, wherein
the two ends of the second push block are respectively located on sides, close to the central axis of the physiological sign monitoring apparatus, of the two first push blocks;
the first snap-fit arm is partially located on a side, close to the central axis of the physiological sign monitoring apparatus, of the first push block; and
the second snap-fit arm is partially located on a side, close to the central axis of the physiological sign monitoring apparatus, of the second push block.

34. The physiological sign monitoring apparatus according to claim 32 or 33, wherein the housing further comprises a boss, the boss is fastened to a middle part of the middle plate and is located in the first space, and both the two first push blocks and the at least one second push block are located between the boss and the upper side wall; and
the button module further comprises an elastic member, and the elastic member is located between the boss and the second push block.

35. The physiological sign monitoring apparatus according to claim 34, wherein there are two elastic members, the second push block comprises a first abutting end and a second abutting end, the first abutting end abuts between one first push block and one elastic member, and the second abutting end abuts between the other first push block and the other elastic member.

36. The physiological sign monitoring apparatus according to any one of claims 32 to 35, wherein there are two second push blocks, and the two second push blocks are spaced apart in the second direction and are respectively located on two sides of the central axis of the physiological sign monitoring apparatus.

37. The physiological sign monitoring apparatus according to any one of claims 32 to 36, wherein the sensor assembly further comprises a needle holder, the puncture needle is fastened to the needle holder, a puncture tip of the puncture protrudes from the needle holder, the physiological sign monitoring apparatus further comprises a needle retraction holder, the needle retraction holder snaps into the housing, and the needle holder snaps into the needle retraction holder; and
when the support moves in a direction toward the organism, the support pushes the needle holder toward the organism through the needle retraction holder, and the puncture tip of the puncture needle penetrates into the organism.

38. The physiological sign monitoring apparatus according to claim 37, wherein the physiological sign monitoring apparatus further comprises a second elastic member, and the second elastic member is located between the support and the needle retraction holder, or the second elastic member is located on a side, close to the middle plate of the housing, of the needle retraction holder; and
after the sensor punctures into and is implanted into the organism, the second elastic member is configured to enable the needle retraction holder to move away from the organism, the needle retraction holder and the puncture needle move toward a side away from the organism, and the puncture needle is detached from the organism.

39. The physiological sign monitoring apparatus according to claim 37 or 38, wherein the physiological sign monitoring apparatus further comprises a helical fastener, the helical fastener comprises a limiting rib and a rotation portion, the rotation portion is fastened to the support, and the limiting rib is fastened to the rotation portion;
the rotation portion is in a tubular shape, an inner side surface of the rotation portion is provided with inclined teeth, an outer side surface of the needle retraction holder is provided with inclined teeth, the inclined teeth of the rotation portion are engaged with the inclined teeth of the needle retraction holder, and the needle retraction holder is rotatably connected to an inner side of the rotation portion;
the housing further comprises a limiting rib, and when the physiological sign monitoring apparatus is in an unused state, the limiting rib of the helical fastener abuts against the limiting rib of the housing in a circumferential direction of the rotation portion; and
when the support moves in the direction toward the organism, the limiting rib of the helical fastener is detached from the limiting rib of the housing, the second elastic member is configured to enable the needle retraction holder to move away from the organism, the needle retraction holder and the puncture needle move toward a side away from the organism, and the puncture needle is detached from the organism.

40. The physiological sign monitoring apparatus according to claim 37 or 38, wherein the physiological sign monitoring apparatus further comprises a plurality of lever fasteners, the plurality of lever fasteners are arranged around the central axis of the physiological sign monitoring apparatus, the lever fastener comprises a head portion, a middle portion, and a tail portion, the middle portion is connected between the head portion and the tail portion, the head portion abuts against a side, close to the middle plate the housing, of the needle retraction holder, the middle portion is rotatably connected to the support, and the tail portion snaps into the housing; and
when the support moves in the direction toward the organism, the tail portion is detached from the housing, the needle retraction holder and the puncture needle move toward a side away from the organism, the head portion is pushed away from the central axis of the physiological sign monitoring apparatus, and the puncture needle is detached from the organism.

41. The physiological sign monitoring apparatus according to any one of claims 32 to 40, wherein the physiological sign monitoring apparatus further comprises a rear sealing cover that is detachably fastened to an end, away from the middle plate, of the upper side wall, and the rear sealing cover covers the end, away from the middle plate, of the upper side wall and seals the first space.

42. A physiological sign monitoring apparatus, comprising:
a housing, comprising a middle plate, an upper side wall, a lower side wall, and a boss, wherein the middle plate comprises a first surface and a second surface that face away from each other, the upper side wall is fastened to the first surface, the lower side wall is fastened to the second surface, both the upper side wall and the lower side wall are connected around a periphery of the middle plate, the upper side wall and the middle plate enclose first space, the lower side wall and the middle plate enclose second space, the boss is fastened to the middle plate and is located in the first space, the boss and the upper side wall are spaced apart and enclose rotation space, the middle plate has a snap-fit hole, and the snap-fit hole passes through the middle plate and is in communication with the rotation space and the second space;
a support, wherein the support comprises a body portion and a snap-fit arm; the body portion is on an inner side of the lower side wall and is slidably connected to the lower side wall; the snap-fit arm is fastened to an end, close to the middle plate, of the body portion; an end, away from the body portion, of the snap-fit arm has a hook; and the snap-fit arm enters the rotation space through the snap-fit hole;
a first elastic member, abutting between the middle plate and the body portion;
a sensor assembly, fastened to the body portion and located on a side, away from the middle plate of the housing, of the body portion, wherein the sensor assembly is located in the second space, and the sensor assembly comprises a sensor and a puncture needle;
a rear sealing cover, detachably fastened to an end, away from the middle plate, of the upper side wall, wherein the rear sealing cover covers the end, away from the middle plate, of the upper side wall, the rear sealing cover is provided with a mounting hole, and the mounting hole passes through the rear sealing cover and is in communication with the outside and the first space;
a button module, comprising a button, a first reset spring, and a slide block, wherein the button is fastened in the mounting hole, one end of the button is exposed to the first space, the other end of the button is exposed to the outside through the mounting hole, the first reset spring abuts between the button and the middle plate, the slide block is located in the rotation space, the slide block comprises a ring portion and a snap-fit portion, the ring portion is sleeved on the boss, the snap-fit portion is connected to a side, away from the boss, of the ring portion, and the hook of the snap-fit arm snaps into the snap-fit portion of the slide block; and
a front sealing cover, detachably fastened to an end, away from the middle plate, of the lower side wall, wherein the rear sealing cover covers an end, away from the middle plate, of the lower side wall and seals the second space, wherein
when the front sealing cover is detached from the lower side wall, the end, away from the middle plate, of the lower side wall abuts against an organism, the button is subject to pressure toward the first space and pushes the slide block to rotate around a central axis of the physiological sign monitoring apparatus, the hook of the snap-fit arm is detached from the slide block, the first elastic member pushes the support toward the organism, the sensor assembly abuts against the organism, and the sensor is implanted into the organism under guidance of the puncture needle.

43. The physiological sign monitoring apparatus according to claim 42, wherein the button module further comprises a push block, the push block is located between the slide block and the button, the push block comprises a first surface and a second surface that face away from each other, the button abuts against the first surface, and the first reset spring abuts between the second surface and the middle plate;
the second surface of the push block is provided with a first inclined surface, a surface on a side, facing the push block, of the slide block is provided with a second inclined surface, and the first inclined surface fits with the second inclined surface; and
when the button is subject to pressure toward the first space and the button moves relative to the housing in a direction toward the organism, the button enables the push block to move relative to the housing in the direction toward the organism, and the push block pushes, through fitting between the first inclined surface and the second inclined surface of the slide block, the slide block to rotate around the central axis of the physiological sign monitoring apparatus.

44. The physiological sign monitoring apparatus according to claim 43, wherein the push block further comprises a limiting portion, the limiting portion is protrudingly disposed on the second surface, the boss is provided with a limiting groove, an opening of the limiting groove is located on a surface on a side, facing the button, of the boss, and the limiting portion is configured to fit with the limiting groove to prevent the push block from moving relative to the housing around the central axis of the physiological sign monitoring apparatus.

45. The physiological sign monitoring apparatus according to any one of claims 42 to 44, wherein there are a plurality of snap-fit arms, the plurality of snap-fit arms are connected around a periphery of the body portion at spacings, there are a plurality of snap-fit portions, the plurality of snap-fit portions are arranged around the ring portion, and the plurality of snap-fit arms and the plurality of snap-fit portions are arranged in a one-to-one correspondence.

46. The physiological sign monitoring apparatus according to any one of claims 42 to 43, wherein the button module further comprises a second reset spring, the housing further comprises a limiting block, the limiting block and the snap-fit portion are spaced apart around the central axis of the physiological sign monitoring apparatus, and the second reset spring abuts between the limiting block and the snap-fit portion; and
in a process in which the button is subject to pressure toward the first space and pushes the slide block to rotate around the central axis of the physiological sign monitoring apparatus, the snap-fit portion moves close to the limiting block, to compress the second reset spring.

47. The physiological sign monitoring apparatus according to any one of claims 42 to 46, wherein an end face of an end, away from the middle plate, of the button is located in the mounting hole and is recessed toward the first space.

48. The physiological sign monitoring apparatus according to any one of claims 42 to 47, wherein the sensor assembly further comprises a needle holder, the puncture needle is fastened to the needle holder, a puncture tip of the puncture protrudes from the needle holder, the physiological sign monitoring apparatus further comprises a needle retraction holder, the needle retraction holder snaps into the housing, and the needle holder snaps into the needle retraction holder; and
when the support moves in a direction toward the organism, the support pushes the needle holder toward the organism through the needle retraction holder, and the puncture tip of the puncture needle penetrates into the organism.

49. The physiological sign monitoring apparatus according to claim 48, wherein the physiological sign monitoring apparatus further comprises a second elastic member, and the second elastic member is located between the support and the needle retraction holder, or the second elastic member is located on a side, close to the middle plate of the housing, of the needle retraction holder; and
after the sensor punctures into and is implanted into the organism, the second elastic member is configured to enable the needle retraction holder to move away from the organism, the needle retraction holder and the puncture needle move toward a side away from the organism, and the puncture needle is detached from the organism.

50. The physiological sign monitoring apparatus according to claim 48 or 49, wherein the physiological sign monitoring apparatus further comprises a helical fastener, the helical fastener comprises a limiting rib and a rotation portion, the rotation portion is fastened to the support, and the limiting rib is fastened to the rotation portion;
the rotation portion is in a tubular shape, an inner surface of the rotation portion is provided with inclined teeth, an outer side surface of the needle retraction holder is provided with inclined teeth, the inclined teeth of the rotation portion are engaged with the inclined teeth of the needle retraction holder, and the needle retraction holder is rotatably connected to an inner side of the rotation portion;
the housing further comprises a limiting rib, and when the physiological sign monitoring apparatus is in an unused state, the limiting rib of the helical fastener abuts against the limiting rib of the housing in a circumferential direction of the rotation portion; and
when the support moves in the direction toward the organism, the limiting rib of the helical fastener is detached from the limiting rib of the housing, the second elastic member is configured to enable the needle retraction holder to move away from the organism, the needle retraction holder and the puncture needle move toward a side away from the organism, and the puncture needle is detached from the organism.

51. The physiological sign monitoring apparatus according to claim 48 or 49, wherein the physiological sign monitoring apparatus further comprises a plurality of lever fasteners, the plurality of lever fasteners are arranged around the central axis of the physiological sign monitoring apparatus, the lever fastener comprises a head portion, a middle portion, and a tail portion, the middle portion is connected between the head portion and the tail portion, the head portion abuts against a side, close to the middle plate the housing, of the needle retraction holder, the middle portion is rotatably connected to the support, and the tail portion snaps into the housing; and
when the support moves in the direction toward the organism, the tail portion is detached from the housing, the needle retraction holder and the puncture needle move toward a side away from the organism, the head portion is pushed away from the central axis of the physiological sign monitoring apparatus, and the puncture needle is detached from the organism.

52. A physiological sign monitoring apparatus, comprising:
a lower housing, comprising a top wall and a side wall, wherein the side wall of the lower housing is connected around a periphery of the top wall of the lower housing, and an opening is formed on a side, away from the top wall of the lower housing, of the side wall of the lower housing;
a base, mounted on an inner side of the lower housing and slidably connected to the side wall of the lower housing;
a support, mounted on an inner side of the base and slidably connected to the base;
a first elastic member, connected between the top wall of the housing and the support;
a sensor assembly, fastened to the support and located on a side, away from the top wall of the lower housing, of the support, wherein the sensor assembly comprises a sensor and a puncture needle;
an upper housing, detachably fastened to an end, away from the top wall of the housing, of the side wall of the lower housing, wherein the upper housing covers the opening; and
a concealing mechanism, configured to conceal the puncture needle, wherein the concealing mechanism is fastened to a side, away from the top wall of the lower housing, of the base, and a part of the concealing mechanism protrudes from the lower housing through the opening, wherein
when the upper housing is detached from the lower housing, the concealing mechanism abuts against an organism, and the lower housing moves relative to the base in a direction toward the organism, the concealing structure switches from a closed state to an open state, the puncture needle is exposed, the first elastic member pushes the support toward the organism, the sensor assembly abuts against the organism, and the sensor is implanted into the organism under guidance of the puncture needle.

53. The physiological sign monitoring apparatus according to claim 52, wherein
when the physiological sign monitoring apparatus is in an unused state, the upper housing is mounted to the lower housing, and the concealing mechanism is in the open state; and
after the upper housing is detached from the lower housing, the concealing mechanism switches from the open state to the closed state, and the concealing mechanism conceals the puncture needle.

54. The physiological sign monitoring apparatus according to claim 52 or 53, wherein the concealing mechanism comprises a plurality of hinges, and when the concealing mechanism is in the closed state, the plurality of hinges are located on a side, away from the top wall of the lower housing, of the puncture needle, to conceal the puncture needle.

55. The physiological sign monitoring apparatus according to claim 54, wherein when the concealing mechanism is in the open state, an included angle between a plane on which the hinge is located and an axial direction of the physiological sign monitoring apparatus is less than or equal to 10°.

56. The physiological sign monitoring apparatus according to claim 54 or 55, wherein the concealing mechanism further comprises a fastening pillar, a hinge pin, and a torsion spring; a quantity of fastening pillars, a quantity of hinge pins, a quantity of torsion springs, and a quantity of hinges are equal; the fastening pillar is fastened to an end, away from the top wall of the lower housing, of the base; a plurality of fastening pillars are connected around a periphery of the base; the hinge pin is connected to an end, away from the base, of the fastening pillar; the torsion spring is sleeved on the hinge pin; the hinge is rotatably connected to the fastening pillar; and the torsion spring abuts against a side, close to the base, of the hinge.

57. The physiological sign monitoring apparatus according to claim 56, wherein the hinge comprises a rotation portion, a limiting portion, and a concealing portion, the rotation portion is connected between the limiting portion and the concealing portion, the concealing portion is in a plate shape, the rotation portion is rotatably connected to the fastening pillar, the plurality of fastening pillars enclose third space, the concealing portion is located in the third space, and the limiting portion is located outside the third space;
the lower housing has an abutting portion, the abutting portion is protrudingly disposed on an inner side surface of the side wall of the lower housing, and in a direction parallel to the axial direction of the physiological sign monitoring apparatus, the limiting portion is arranged opposite to the abutting portion of the housing, and the limiting portion is located on a side, away from the top wall of the lower housing, of the abutting portion; and
in a process in which the lower housing moves relative to the base in the direction toward the organism, the abutting portion pushes the limiting portion, an end, away from the concealing portion, of the limiting portion moves toward the organism, the concealing portion moves toward a side away from the organism, the concealing structure switches from the closed state to the open state, and the puncture needle is exposed.

58. The physiological sign monitoring apparatus according to claim 57, wherein there are three hinges, and the three hinges are sequentially arranged around a central axis of the physiological sign monitoring apparatus.

59. The physiological sign monitoring apparatus according to claim 57 or 58, wherein the support is provided with a sixth guide structure, and the sixth guide structure is fastened to an end, away from the top wall of the lower housing, of the support and is protrudingly disposed on an outer side of the support;
the physiological sign monitoring apparatus further comprises a guide pillar, the guide pillar is fastened to the end, away from the top wall of the lower housing, of the base, the guide pillar and the fastening pillar are connected around the periphery of the base, and a seventh guide structure is disposed on a side, facing the third space, of the guide pillar; and
in a process in which the first elastic member pushes the support toward the organism, the sixth guide structure is slidably connected to the second guide structure, to implement directional movement of the support.

60. The physiological sign monitoring apparatus according to claim 57 or 58, wherein the concealing mechanism further comprises a first limiting block, the first limiting block is fastened to the fastening pillar and is located on a side, away from the base, of the concealing portion of the hinge, and in a direction parallel to an axial direction of the physiological sign monitoring apparatus, the first limiting block is arranged opposite to the concealing portion of the hinge; and
when the concealing mechanism is in the closed state, the first limiting block abuts against the concealing portion of the hinge.

61. The physiological sign monitoring apparatus according to claim 56, wherein the concealing mechanism further comprises a limiting pillar, a bearing plate, a second limiting block, a first push block reset spring, and a first push block; the bearing plate is fastened to the end, away from the top wall of the lower housing, of the base; both the limiting pillar and the fastening pillar are fastened to a side, away from the base, of the bearing plate; the fastening pillar and the limiting pillar are spaced apart; there are a plurality of limiting pillars; the plurality of limiting pillars are spaced apart around a central axis of the physiological sign monitoring apparatus; and the second limiting block is fastened to the limiting pillar and is spaced away from the bearing plate;
the first push block is movably connected to the limiting pillar, the first push block is partially located between the second limiting block and the bearing plate, the first push block reset spring abuts between the first push block and the bearing plate, the first push block comprises an abutting end, and the abutting end protrudes from the lower housing through the opening;
when the physiological sign monitoring apparatus is in the unused state, the upper housing abuts against the first push block, and the first push block abuts against the hinge, to enable the concealing mechanism to be in the open state;
in a process in which the upper housing is detached from the lower housing, the first push block reset spring pushes the first push block to move toward a side away from the top wall of the lower housing, the first push block and the hinge are spaced apart, the hinge is pushed by the torsion spring to rotate toward the side away from the top wall of the lower housing, and the concealing mechanism switches from the open state to the closed state to conceal the puncture needle; and
when the abutting end abuts against the organism and the lower housing moves relative to the base in a direction toward the organism, the first push block reset spring is compressed, the first push block abuts against the hinge, the concealing mechanism switches from the closed state to the open state, and the puncture needle is exposed.

62. The physiological sign monitoring apparatus according to claim 56, wherein the concealing mechanism further comprises a bearing plate, a drive wheel, a second push block, a second push block reset spring, and a drive wheel reset spring;
the hinge comprises a blade, a first guide pillar, and a second guide pillar, the blade comprises a first surface and a second surface that face away from each other, the first guide pillar is fastened to the first surface, and the second guide pillar is fastened to the second surface;
the bearing plate is fastened to the end, away from the top wall of the lower housing, of the base, the bearing plate is provided with a first guide hole, the drive wheel is movably connected to the base, the drive wheel is provided with a second guide hole, the first guide pillar is slidably connected to the first guide hole, and the second guide pillar is slidably connected to the second guide hole;
the push block reset spring abuts between the push block and the drive wheel, the push block comprises an abutting end, and the abutting end protrudes from the lower housing through the opening;
one end of the drive wheel reset spring is fastened to the base, and the other end of the drive wheel reset spring is fastened to the drive wheel;
when the physiological sign monitoring apparatus is in the unused state, the drive wheel reset spring is in a stretched state;
in a process in which the upper housing is detached from the lower housing, the push block reset spring pushes the push block to move toward a side away from the bottom wall of the lower housing, the drive wheel reset spring resets to pull the drive wheel to rotate reversely, and the plurality of hinges rotate, to enable the concealing mechanism to be in the closed state; and
when the abutting end abuts against the organism and the lower housing moves relative to the base in a direction toward the organism, the push block moves relative to the base in a direction toward the bottom wall of the lower housing to push the directional wheel to rotate, the drive wheel reset spring is stretched, and the plurality of hinges rotate, to enable the concealing mechanism to be in the open state and the puncture needle to be exposed.

63. The physiological sign monitoring apparatus according to claim 52, wherein the concealing mechanism comprises an alloy limiting cap and a plurality of alloy sheets, the alloy is in a curved-surface shape, the alloy is fastened to an inner side of the base, and the alloy limiting cap is fastened to the lower housing;
when the physiological sign monitoring apparatus is in an unused state, an end, away from the base, of the alloy sheet is located between the base and the alloy limiting cap;
after the upper housing is detached from the lower housing, the alloy limiting cap is detached from the base, and ends, away from the base, of the alloy converge to conceal the puncture needle; and
the base comprises an abutting end, the abutting end protrudes from the lower housing through the opening, and when the abutting end abuts against an organism and the lower housing moves relative to the base in a direction toward the organism, the first elastic member pushes the support toward the organism, and the support pushes the alloy sheets open, to enable the concealing mechanism to be in the open state and the puncture needle to be exposed.

64. The physiological sign monitoring apparatus according to claim 52, wherein the concealing mechanism comprises an alloy sheet, the alloy is in a curved-surface shape, and the alloy is at least partially fastened to an inner side of the base;
when the physiological sign monitoring apparatus is in an unused state, an end, away from the base, of the alloy sheet is located between the base and the upper housing;
after the upper housing is detached from the lower housing, ends, away from the base, of the alloy converge to conceal the puncture needle; and
the base comprises an abutting end, the abutting end protrudes from the lower housing through the opening, and when the abutting end abuts against an organism and the lower housing moves relative to the base in a direction toward the organism, the first elastic member pushes the support toward the organism, and the support pushes the alloy sheets open, to enable the concealing mechanism to be in the open state and the puncture needle to be exposed.

65. A physiological sign monitoring apparatus, comprising:
a lower housing, comprising a top wall and a side wall, wherein the side wall of the lower housing is connected around a periphery of the top wall of the lower housing, and an opening is formed on a side, away from the top wall of the lower housing, of the side wall of the lower housing;
a base, mounted on an inner side of the lower housing and slidably connected to the side wall of the lower housing, wherein the base comprises a top plate, a side wall, and a rebound arm, the side wall of the base is connected around a periphery of the top plate, the rebound arm is fastened to a side, away from the side wall of the base, of the top plate, the rebound arm abuts against the top wall of the lower housing, the base comprises an abutting end, and the abutting end protrudes from the lower housing through the opening;
a support, mounted on an inner side of the base and slidably connected to the base;
a first elastic member, abutting between the base and the support;
a sensor assembly, fastened to the support and located on a side, away from the top wall of the lower housing, of the support, wherein the sensor assembly comprises a sensor and a puncture needle;
an upper housing, detachably fastened to an end, away from the top wall of the housing, of the side wall of the lower housing, wherein the upper housing covers the abutting end and closes the opening; and
when the upper housing is detached from the lower housing, the abutting end abuts against an organism, and the lower housing moves relative to the base in a direction toward the organism, the first elastic member pushes the support toward the organism, the sensor assembly abuts against the organism, and the sensor is implanted into the organism under guidance of the puncture needle.

66. The physiological sign monitoring apparatus according to claim 65, wherein the sensor assembly further comprises a needle holder, the puncture needle is fastened to the needle holder, a puncture tip of the puncture protrudes from the needle holder, the physiological sign monitoring apparatus further comprises a needle retraction holder, the needle retraction holder snaps into the support, the lower housing, or the base, and the needle holder snaps into the needle retraction holder;
the physiological sign monitoring apparatus further comprises a second elastic member, and the second elastic member is located between the support and the needle retraction holder, or the second elastic member is located on a side, close to the top wall of the lower housing, of the needle retraction holder; and
when the support moves in a direction toward the organism, the support pushes the needle holder toward the organism through the needle retraction holder, and the puncture tip of the puncture needle penetrates into the organism; after the sensor punctures into and is implanted into the organism, the second elastic member is configured to enable the needle retraction holder to move away from the organism, the needle retraction holder and the puncture needle move toward a side away from the organism, and the puncture needle is detached from the organism; and the first elastic member is sleeved on an outer side of the second elastic member.
